(19) Europäisches Patentamt
European Patent Office
Office européen des brevets

(11) **EP 4 438 107 A2**

(12) **EUROPEAN PATENT APPLICATION**

(43) Date of publication:
**02.10.2024 Bulletin 2024/40**

(21) Application number: **24178442.0**

(22) Date of filing: **12.10.2018**

(51) International Patent Classification (IPC):
***A61N 1/36*** (2006.01)

(52) Cooperative Patent Classification (CPC):
**A61B 5/0245; A61B 5/021; A61B 5/02405;
A61B 5/4869; A61B 5/4884; A61N 1/36002;
A61N 1/40;** A61B 5/352

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.10.2017 US 201762572170 P
21.06.2018 US 201862688282 P
21.06.2018 US 201862688296 P
27.06.2018 US 201862690709 P**

(62) Document number(s) of the earlier application(s) in
accordance with Art. 76 EPC:
**18867286.9 / 3 694 409**

(71) Applicant: **Autem Medical, LLC
Bedford, NH 03110 (US)**

(72) Inventors:
• **COSTA, Frederico Perego
Pittsburgh, 15219-4429 (US)**
• **CORREA DA SILVA, Flavio Soares
05386-3400 São Paulo (BR)**
• **TUSZYNSKI, Jacek Adam
Edmonton, T5N 1Z7 (CA)**
• **IEMMA, Antonio Francisco
13417-430 São Paulo (BR)**

(74) Representative: **HGF
HGF Limited
1 City Walk
Leeds LS11 9DX (GB)**

Remarks:
•This application was filed on 28.05.2024 as a
divisional application to the application mentioned
under INID code 62.
•Claims filed after the date of filing of the application
(Rule 68(4) EPC).

(54) **SYSTEM FOR CHARACTERIZATION, DIAGNOSIS, AND TREATMENT OF A HEALTH CONDITION OF A PATIENT AND MICROTUBULE CONDUCTIVITY, AND METHOD OF USING SAME**

(57) A system and method for treating cancer by integrating a hemodynamic parameter (Hdp) monitoring system and a radiofrequency generator synchronized by a processing system is disclosed. The system is capable of identifying health condition-specific Hdp variation values changes in a patient upon the exposure of low energy amplitude modulated electromagnetic fields frequencies. The exposure of the modulated frequencies influences cellular functions, such as microtubule conductivity, or malfunctions in a patient and can provide both a therapeutic and predictive and prognostic effect for the patient, providing for both treatment of a specific ailment or disease such as hepatocellular carcinoma as well as predictive results of how effective the treatment is likely to be. The construction of a library of frequencies can be used to more efficiently and effectively diagnose and treat a health condition in patients by auto-tuning a treatment regimen specifically to the patient.

FIG. 12

EP 4 438 107 A2

**Description**

**CROSS REFERENCE**

This application claims priority to U.S Provisional Application No. 62/690,709 filed on June 27, 2018; U.S. Provisional Application No. 62/688,296 filed on June 21, 2018; U.S. Provisional Application No. 62/688,282 filed on June 21, 2018; and U.S. Provisional Application No. 62/572,170 filed on October 13, 2017, each of which are incorporated herein by reference.

**SUMMARY**

Health device profile (Hdp) monitors, or any other device capable of registering hemodynamic or cardiac electrical activities, are used to sense and monitor various Hdp values. Various Hdp values can be used to diagnose cardiovascular conditions of a patient. Hdp measurements performed, generally in conjunction with an electrocardiogram (ECG), can include measurements of stroke volume (SV), stroke index (SI) and cardiac output (CO). Such measurements are indicated for the diagnosis and therapy of patients suffering from cardiac conditions, such as heart failure, hypertension, coronary artery disease, and pericardial disease, as well as obstructive lung and pleural disease and renal insufficiency.

Electrocardiography, which involves measuring heart rate metrics, can also be used to measure the bio-potential generated by electrical signals that control the expansion and contraction of heart chambers.

Heart rate variability (HRV) is the physiological phenomenon of variation in the time interval between heartbeats. It is measured by the variation in the beat-to-beat interval. Other terms have also been used to described oscillation in consecutive cardiac cycles, such as heart period variability, R-wave R-wave variability and R-wave R-wave interval (R-R interval or RRI) tachogram. Sample processes for identifying heart rate variability (HRV) variables include RRI values recording, computer digitizing, artifact identification, HRV data editing, HRV interval rejection. HRV data sequence and interpolation and sampling for time domain heart rate variability and frequency domain heart rate variability.

A summary of the above techniques can be found, for example, in Task Force of the European Society of Cardiology and the North American Society of Pacing and Electrophysiology, European Heart Journal 17 (1996) at 354-381.

Some conventional systems that employ one or more of the above techniques can enable a user to diagnose the presence or absence of tumor cell growths or cancers in a patient. In some cases, the identity of the cell growth or tumor can also be identified. However, such systems lack the capability to employ measured Hdp values and HRV to determine either a specific type of cancer or any other form of a health condition of a patient.

In an embodiment, a system for diagnosing a health condition of a patient may include an ECG monitoring system configured to detect, measure and store a plurality of first values for RRI values exhibited by the patient during a basal or non-exposure period and a plurality of second values for RRI values exhibited by the patient during or after an exposure period in which the patient is exposed to low-energy electromagnetic carrier output signals. The system may further include an electrically powered generator adapted to be actuated to generate the low-energy electromagnetic carrier output signals for exposing or applying the low-energy electromagnetic carrier output signals to the patient during the exposure period, and a processing system that mya be configured to synchronize the ECG monitoring system and the electrically powered generator. In some embodiments, the processing system can be internal or external to the ECG monitoring system. In some embodiments, the processing system can be configured to sense and identify one or more of a specific electromagnetic field amplitude modulated frequency. The system may further comprise an interface controller that can be operable communication with the ECG monitoring system and the electrically powered generator. In some embodiments, the ECG monitoring system may measure one or more RRI values, calculate one or more HRV values, record one or more HRV variation values, identify one or more specific electromagnetic field amplitude modulated frequencies, or a combination thereof. In some embodiments, the processing system may integrate the one or more HRV values with an intelligent learning library and the electrically powered generator. In some embodiments, the low-energy electromagnetic carrier output signals of the system may comprise an amplitude modulation frequency in a range from about 0.01 Hz to about 150 kHz.

In some embodiments, an ECG monitoring system may be configured to detect, measure and store a plurality of first values for RRI values exhibited by one or more surrogate patients during a basal or non-exposure period and a plurality of second values for RRI values exhibited by the one or more surrogate patients during or after an exposure period in which the one or more surrogate patients are exposed to low-energy electromagnetic output signals. In some embodiments, the system may comprise an electrically powered generator adapted to be actuated to generate the low-energy electromagnetic carrier output signals for exposing or applying the low-energy electromagnetic carrier output signals to the surrogate patients during said exposure period. The system may further comprise a processing system configured to synchronize the ECG monitoring system and the electrically powered generator wherein the processing system can be internal or external to the ECG monitoring system. In some embodiments, the processing system can be configured to sense and identify one or more of a specific electromagnetic field amplitude modulated frequency. In further embodiments, the system may comprise an interface controller in operable communication with the ECG monitoring system and the electrically powered generator. In some embodiments, the ECG monitoring system can be configured to measure one or more RRI values, calculate one or more HRV values, record one or more HRV variation values, identify one or more specific electromagnetic field amplitude modulated frequencies, or a combination thereof. The processing system may integrate the one or more HRV value with an intelligent learning library and the electrically powered generator. In some embodiments, the low-energy electromagnetic carrier output signals may comprise an amplitude modulation frequency in a range from about 0.01 HZ to about 150 kHz.

In some embodiments, a system for diagnosing a health condition of a patient may comprise a hemodynamic parameter (Hdp) monitoring system that may be configured to detect, measure, and record a plurality of first values for each of a plurality of hemodynamic parameters exhibited by a patient during an exposure period, wherein the exposure period may comprise a time period in which the patient is exposed to a one or more of electromagnetic frequency signals. The system may further comprise an electrically-powered frequency generator adapted to generate the one or more electromagnetic frequency signals during the exposure period, wherein the one or more electromagnetic frequency signals can be configured to influence a cellular function. The system may further include a processing system that can be configured to synchronize the Hdp monitoring system and the frequency generator, auto-tune a carrier signal to adjust forward energy delivered to the patient, and instruct the frequency generator to expose the patient to each of the one or more of electromagnetic frequency signals by modulating an amplitude of the carrier signal to produce an amplitude modulated electromagnetic frequency signal. In some embodiments, the amplitude modulated electromagnetic signal can be selected from within a range of 10 Hz to 1,000 Hz. In further embodiments, the cellular function can be microtubule conductivity. In further embodiments, the plurality of hemodynamic parameters may comprise one or more of RR interval, heart rate, systolic blood pressure, diastolic blood pressure, median blood pressure, pulse pressure, stroke volume, cardiac output, and total peripheral resistance. In some embodiments, the processing system may be configured to actuate the frequency generator to generate one or more highly specific frequency radio frequency (RF) carrier signals based on at least the plurality of first values for each of the plurality of hemodynamic parameters. In some embodiments, the frequency generator may comprise a programmable generator. In further embodiments, the programmable generator may comprise one or more controllable generator circuits, wherein each controllable generator circuit can be configured to generate one or more highly specific frequency RF carrier signals. In some embodiments, each controllable generator circuit may comprise an amplitude modulation (AM) frequency control signal generator that can be configure to control amplitude modulated variations of the one or more highly specific frequency RF carrier signals. In further embodiments, the carrier signal can be a 27.12 MHz signal. In some embodiments, the system may further comprise a computing device that can be operably connected to the processing system and may be configured to store at least one machine learning algorithm configured to output one or more variables for use in auto-tuning the carrier signal.

In some embodiments, a system for treating cancer of a patient may comprise a hemodynamic parameter (Hdp) monitoring system that can be configured to detect, measure, and record a plurality of first values for each of a plurality of hemodynamic parameters exhibited by a patient during an exposure period, wherein the exposure period may comprise a time period in which the patient is exposed to a plurality of electromagnetic frequency signals. In some embodiments, the system may further include an electrically-powered frequency generator adapted to generate the one or more electromagnetic signals during the exposure period, wherein the one or more electromagnetic signals can be configured to influence microtubule conductivity. In further embodiments, a processing system may be included and can be configured to synchronize the Hdp monitoring system and the frequency generator and auto-tune a carrier signal to adjust forward energy delivered to the patient, and instruct the frequency generator to expose the patient to each of the plurality of electromagnetic frequency signals by modulating an amplitude of the carrier signal to produce a desired electromagnetic frequency signal. In some embodiments, the amplitude modulated electromagnetic signal can be selected from within a range of 10 Hz to 1,000 Hz.

In some embodiments, a method of diagnosing a health condition of a patient may include measuring, by a ECG monitoring system, a plurality of first values for RRI values, calculating HRV values exhibited by a patient during exposure of the patient to highly specific frequency radio frequency carrier signals, processing HRV values and exposure of the patient to the highly specific frequency radio frequency carrier signals, recording representative HRV variation values exhibited by one or more surrogate patients during exposure of the one or more surrogate patients to the highly specific frequency radio frequency carrier signals, storing the plurality of first values and a plurality of second values of representative HRV variation values, and transferring the representative HRV variation values from a pre-diagnosis or diagnosed patient to a library for further processing. In some embodiments, the highly specific frequency radio frequency carrier signals may comprise an amplitude modulation frequency in a range from about 0.01 HZ to about 150 kHz.

In some embodiments, a method of diagnosing a health condition of a patient may include determining, by a processing system, a plurality of electromagnetic frequency signals to apply to the patient, auto-tuning, by the processing system, a carrier wave to balance forward energy delivered to the patient, exposing, by a frequency generator operably connected to the processing system, the patient to each of the plurality of electromagnetic frequency signals by modulating an amplitude of the carrier signal to produce a desired electromagnetic frequency signal that can be configured to influence a cellular function of the patient, measuring, by a hemodynamic parameter (Hdp) monitoring system, a plurality of first values for a plurality of hemodynamic parameters exhibited by a patient during exposure of the patient to the plurality of electromagnetic frequency signals, and analyzing the plurality of first values to provide for a diagnosis of a health condition of the patient. In some embodiments, the amplitude modulated signal can be selected from within a range of 10 Hz to 1,000 Hz. In some embodiments, the method may further comprise identifying a specific frequency response to a single frequency exposure of an electromagnetic amplitude modulation signal as non-reactive, reactive, or post-reactive. In further embodiments, the plurality of hemodynamic parameters may comprise one or more of RR interval, heart rate, systolic blood pressure, diastolic blood pressure, median blood pressure, pulse pressure, stroke volume, cardiac output, and total peripheral resistance. In some embodiments, the method may comprise determining highly specific frequency RF carrier signals that cause significant Hdp value changes in the patient. In further embodiments, the carrier signal may comprise a 27.12 MHz signal. In some embodiments, auto-tuning may comprise adjusting the forward energy of the carrier signal based upon an output of a machine learning algorithm. In further embodiments, the cellular function can be microtubule conductivity.

In some embodiments, a method of treating cancer may include administering one or more high frequency carrier signals and at least one amplitude modulation control signal for controlling amplitude modulated variations of the one or more high frequency carrier signals, wherein the amplitude modulation frequency can be selected from within a range of 0.01 Hz to 150 kHz. In some embodiments, the amplitude modulation frequency can be selected from within a range of 10 Hz to 1,000 Hz. In further embodiments, the at least one amplitude modulation control signal may modulate microtubule ion conductivity.

In some embodiments, a programmable generator can be activatable by electrical power and structured to influence cellular functions or malfunctions in a warm-blooded mammalian subject. In some embodiments, the programmable generator may comprise at least one controllable low-energy electromagnetic energy generator circuit for generating one or more high frequency carrier signals, wherein the at least one generator circuit may include at least one amplitude modulation control signal generator for controlling amplitude modulated variations of the one or more high frequency carrier signals, and at least one programmable amplitude modulation frequency control signal generator for controlling frequencies at which amplitude modulations are generated. In some embodiments, each programmable amplitude modulation frequency control signal generator can be adapted to accurately control the frequencies at which the amplitude modulations are generated to within an accuracy of at least 1000 parts per million relative to a reference amplitude modulation frequency selected from within a range of 0.01 Hz to 150 kHz. In some embodiments, the programmable generator may further include at least one data processor constructed and arranged for communication with the at least one generator circuit and for receiving control information from a source of control information, and a connection position configured to connect to an electrically conductive applicator that may be configured to apply one or more amplitude-modulated low energy emissions at a program-controlled frequency to the warm-blooded mammalian subject, wherein the reference amplitude modulation frequencies can be selected dependent on a health condition of the warm-blooded mammalian subject.

## BRIEF DESCRIPTION OF THE DRAWINGS

FIG. 1. depicts a sample use scenario for the portable medical device as described herein in accordance with an embodiment.

FIG. 2 depicts an alternate sample use scenario for the portable medical device in accordance with an embodiment.

FIG. 3 illustrates a sample circuit diagram for the portable medical device in accordance with an embodiment.

FIG. 4 illustrates a sample circuit diagram for a signal modulator in accordance with an embodiment.

FIG. 5 illustrates a sample circuit diagram for an amplifier in accordance with an embodiment.

FIG. 6 illustrates a sample circuit diagram for a power monitor in accordance with an embodiment.

FIG. 7 depicts an illustrative schematic structure for an integrated medical system according to an embodiment.

FIG. 8 depicts an illustrative block diagram of an integrated system according to an embodiment.

FIG. 9 illustrates a flow diagram for a signal modulator in accordance with an embodiment.

FIG. 10 illustrates a flow diagram for a signal modulator in accordance with an embodiment.

FIG. 11 illustrates a flow diagram for a signal modulator in accordance with an embodiment.

FIG. 12 depicts an illustrative patient during experimental setup for continuous monitoring of hemodynamic parameters before and during amplitude modulation (AM) radio frequency (RF) electromagnetic field (EMF) exposure according to an embodiment.

FIG. 13A-E illustrates an amplitude modulation (AM) radio frequency (RF) electromagnetic field (EMF) according to various embodiments.

FIG. 14 illustrates a capacitor as a frequency specific demodulation system.

FIG. 15 illustrates phase-locking synchronization of a demodulated signal electrical discharge.

FIG. 16A illustrates a signal exposure protocol in accordance with an embodiment.

FIG. 16B illustrates an additional signal exposure protocol in accordance with an embodiment.

FIG. 17 illustrates a flow diagram representing a hemodynamic recording performed continuously during non-exposure and exposure periods in a double-blind fashion according to an embodiment.

FIG. 18A illustrates a diagram representing a signal exposure protocol over the course of one or two days in accordance with an embodiment.

FIG. 18B illustrates a diagram representing a signal exposure protocol with reflective energy measurements over the course of one or two days.

FIG. 18C illustrates a diagram representing a signal exposure protocol over the course of one or two days in accordance with an embodiment.

FIG. 18D illustrates a diagram representing a signal exposure protocol over the course of one day in accordance with an embodiment.

FIG. 18E illustrates a diagram representing a signal exposure protocol over the course of one day in accordance with an additional embodiment.

FIG. 19 illustrates a table of eleven illustrative hemodynamic parameters simultaneously measured during each heartbeat according to an embodiment.

FIG. 20 illustrates a table of eleven illustrative hemodynamic parameters simultaneously measured during each heartbeat following exposure to a signal frequency in accordance with an embodiment.

FIG. 21 illustrates a hemodynamic parameter and signal decomposition during a homeostasis and a stress response in accordance with an embodiment.

FIG. 22A illustrates a hemodynamic parameter and signal decomposition during a homeostasis and a stress response in accordance with an additional embodiment.

FIG. 22B illustrates a time domain analysis on R-R interval and heart rate variability in accordance with an embodiment.

FIG. 23A illustrates a Poincare plot from a ten minute electrocardiogram in accordance with an embodiment.

FIG. 23B illustrates a Poincare plot correlation with fourier transformation in accordance with an embodiment.

FIG. 24A illustrates a hemodynamic parameter response to a signal frequency in a subject with hepatocellular carcinoma in accordance with an embodiment.

FIG. 24B illustrates a hemodynamic parameter response to a signal frequency in a healthy subject in accordance with an additional embodiment.

FIG. 24C illustrates a flow diagram of a hemodynamic parameter data analysis following exposing a subject to a signal frequency in accordance with an embodiment.

FIG. 25A illustrates the alterations in a hemodynamic parameter in response to a signal exposure in a subject having hepatocellular carcinoma in accordance with an embodiment.

FIG. 25B illustrates the alterations in a hemodynamic parameter in response to a signal exposure in a healthy subject in accordance with an embodiment.

FIG. 25C illustrates the alterations in a hemodynamic parameter in response to a signal exposure in a subject having hepatocellular carcinoma in accordance with an additional embodiment.

FIG. 25D illustrates the alterations in a hemodynamic parameter in response to a signal exposure in a subject having hepatocellular carcinoma in accordance with a further embodiment.

FIG. 25E illustrates the alterations in heart rate variability in response to a signal exposure in a subject having hepatocellular carcinoma in accordance with an embodiment.

FIG. 25F illustrates the alterations in a hemodynamic parameter in response to a signal exposure in a subject in accordance with an embodiment.

FIG. 26 illustrates a hemodynamic parameter in response to a signal exposure in a subject over two days in accordance with an embodiment.

FIG. 27 illustrates a hemodynamic parameter in response to a signal exposure over two days in multiple healthy subjects or subjects having hepatocellular carcinoma in accordance with an embodiment.

FIG. 28A illustrates an intrapatient hemodynamic parameter in response to a signal exposure over two days in a subject having hepatocellular carcinoma in accordance with an embodiment.

FIG. 28B illustrates an intrapatient hemodynamic parameter in response to a signal exposure over two days in a healthy subject in accordance with an embodiment.

FIG. 29A-G illustrates mean hemodynamic parameter values in a subject having different periods and diagnosis in accordance with various embodiments.

FIG. 30 A-B illustrates intrapatient variation of hemodynamic parameter values in a subject in accordance with various embodiments.

FIG. 31 A-C illustrates hemodynamic parameter values in multiple healthy or hepatocellular carcinoma subjects in accordance with various embodiments.

FIG. 32 illustrates support vector machine analysis of a hemodynamic parameter in a subject in accordance with various embodiments.

FIG. 33 illustrates diagnostic results by analyzing a hemodynamic parameter in a subject following exposure to a signal in accordance with an embodiment.

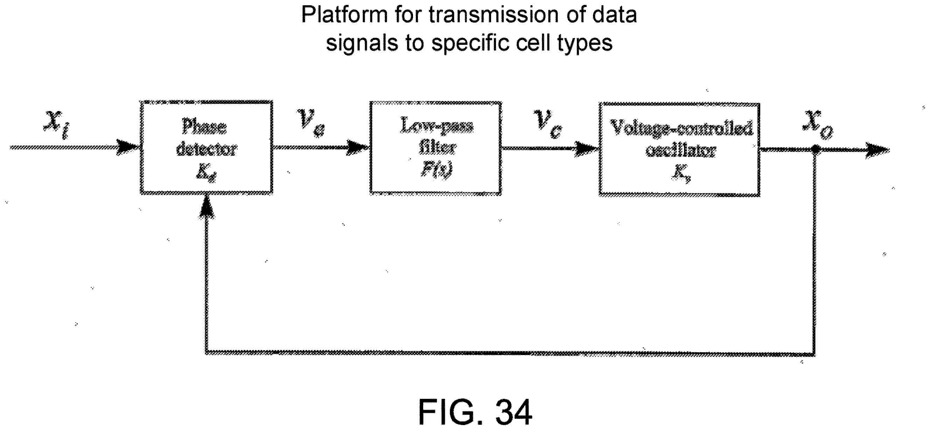

FIG. 34 illustrates a phase-locked loop system having synchronization solution and cell based specificity as a platform for transmission of data signals to specific cell types.

FIGS. 35A-F illustrate microtubules in metastatic and non-metastatic cells in accordance with an embodiment.

FIGS. 36A-E illustrate a microtubule electrical conductibility system in accordance with an embodiment.

FIGS. 37A-C illustrate a microtubule electrical conductibility system in accordance with an additional embodiment.

FIGS. 38A-B illustrate microtubules as an electrical wire having nanopores in accordance with an embodiment.

FIG. 39 illustrates pulse transmission as a soliton-like wave in accordance with an embodiment.

FIG. 40A illustrates electrodes fabricated on a glass surface to observe responses to AC voltage in accordance with an embodiment.

FIG. 40B illustrates a microtubule response to AC signals in accordance with an embodiment.

FIG. 41 illustrates geometries of microtubules near electrodes at various field frequencies in accordance with some embodiments; **A,** 100kHz; **B,** 250 kHz; **C,** 500 kHz; **D,** 1 MHz; E, 2.5 MHz.

FIG. 42A illustrates molecular dynamics of C-termini in a microtubule ring in accordance with an embodiment.

FIG. 42B illustrates the effects of an acidic environment on C-termini in a microtubule ring in accordance with an embodiment.

FIG. 42C illustrates the effects of neutral environment on C-termini in a microtubule ring in accordance with an embodiment.

FIG. 43 illustrates effects of C-termini oscillations on ion movement through nanopores into the microtubule lumen in accordance with an embodiment.

FIG. 44 illustrates a cross-linked conformation of C-termini stabilizes a straight orientation of a tubulin dimer in accordance with an embodiment.

FIG. 45 illustrates an ordered water structure between charged surfaces in accordance with an embodiment.

FIG. 46 illustrates an exponential drop of an electric potential in an ionic solution that is near a charged surface in accordance with an embodiment.

FIG. 47A illustrates a counter-ion charge distribution surrounding a microtubule in accordance with an embodiment.

FIG. 47B illustrates a counter-ion charge distribution surrounding a C-terminal tail in accordance with an embodiment.

FIG. 48 illustrates a calculation of a microtubule capacitance in accordance with an embodiment.

FIG. 49A illustrates an extended terminal tail in accordance with an embodiment.

FIG. 49B illustrates a tilting terminal tail in accordance with an embodiment.

FIG. 50 illustrates an electrical cable of an ionic surrounding of a microtubule having 13 protofilaments in accordance with an embodiment. Notation used: Pr-j : protofilament 'j', j=1..13; $R_1$ : resistance along a tubulin; $R_2$: resistance perpendicular to the tubulin; $R_3$: resistance between protofilament, in series with the partial inductance $L_1$; $C_1$: capacitance of a dimer; $L_1$: fraction of the inductance of a full turn.

FIG. 51 illustrates an effective circuit diagram for the n-th unit with characteristic elements for Kirchhoff's laws applied to a microtubule as an ionic cable in accordance with an embodiment.

FIG. 52 illustrates ions and ordered water within a cell and an extracellular matrix in accordance with an embodiment.

FIG. 53 illustrates a condensation and a decondensation due to cationic charge movement in a cytoplasm.

FIG. 54A illustrates alpha-fetoprotein (AFP) tumor marker measurements in a patient over time in accordance with an embodiment.

FIG. 54B illustrates a liver CT scan in a patient in accordance with an embodiment.

FIG. 55A illustrates a coronal view of a CT liver image having a tumor mass in accordance with an embodiment.

FIG. 55B illustrates a sagittal view with tumor infiltrating the inferior vena cava (arrow) in accordance with an embodiment.

FIG. 55C illustrates a large tumor mass in accordance with an embodiment.

FIG. 55D illustrates a tumor invading a right branch of a portal vein in accordance with an embodiment.

FIG. 55E illustrates multiple lung metastasis (arrows) in accordance with an embodiment.

FIG. 56 illustrates a subphrenic tumor mass (left), a hepatic tumor mass in Seg II/III (center), and a tumor mass in the peritoneal cavity (right) in accordance with an embodiment.

FIG. 57A illustrates cavitation of pulmonary metastasis as depicted by a liver CT scan in a patient in accordance with an embodiment.

FIG. 57B illustrates disappearance of a hepatic tumor mass and residual peritoneal implant as depicted by a liver CT scan in a patient in accordance with an embodiment.

FIG. 58 illustrates a liver MRI scan in a patient in accordance with an embodiment.

FIG. 59 illustrates a further liver MRI scan in a patient in accordance with an embodiment.

FIG. 60A illustrates a liver CT scan in a patient after two weeks of receiving an exposure protocol in accordance with an embodiment.

FIG. 60B illustrates a liver CT scan in a patient after four weeks following receiving an exposure protocol in accordance with an embodiment.

FIG. 61 illustrates a liver CT scan in a patient after twelve days following receiving an exposure protocol in accordance with an embodiment.

FIG. 62A illustrates electromagnetic field absorption, transmission, or reflection in accordance with an embodiment.

FIG. 62B illustrates electromagnetic field reflection coefficients in accordance with an embodiment.

FIG. 63A-D illustrates reflection energy differences in accordance with some embodiments.

FIG. 64 illustrates a sample process flow for treating a patient according to an embodiment.

FIG. 65 illustrates a sample flow for training a machine learning algorithm according to an embodiment.

FIG. 66 illustrates a set of patient response graphs according to an embodiment.

FIG. 67A illustrates a diagram representing a signal exposure protocol over the course of one day in accordance with an embodiment.

FIG. 67B illustrates the determination of new active frequencies in comparison with a first frequency exposure protocol in accordance with an embodiment.

FIG. 68 illustrates the distribution of active frequencies in accordance with an embodiment.

FIG. 69 illustrates the distribution of active frequencies in accordance with a further embodiment.

FIG. 70 illustrates a normal probability plot construction in accordance with an embodiment.

FIG. 71 illustrates a bar coding system in accordance with an embodiment.

FIG. 72 illustrates a bar coding system in accordance with a further embodiment.

FIG. 73 illustrates a bar coding system for diagnosing healthy patients and hepatocellular carcinoma patients in accordance with an embodiment.

FIG. 74 illustrates a bar coding system for diagnosing healthy patients and hepatocellular carcinoma patients in accordance with a further embodiment.

FIG. 75A illustrates Hdp values recorded during 23 consecutive heart-beats in accordance with an embodiment.

FIG. 75B illustrates new attribute parameters values recorded during 23 consecutive heart-beats in accordance with an embodiment.

FIG. 76 illustrates rates of correct classification using centroids from representative Hdp variation values in accordance with an embodiment.

FIG. 77 illustrates rates of correlation of representative Hdp variation values and a bio-feedback procedure in accordance with an embodiment.

FIG. 78 illustrates differences of representative Hdp variation values determined during the exposure to different SFq in accordance with an embodiment.

FIG. 79A illustrates the distribution of 1,054 cancer-specific frequencies from four cancers types in accordance with an embodiment.

FIG. 79B illustrates the distribution of disease-specific Sfq and healthy-specific Sfq during exposure to three different groups of cancer-specific frequencies in 21 patients.

FIGS. 80A-B illustrates dynamic instant variability calculation by heart-beat in accordance with an embodiment.

FIG. 81 illustrates a dynamic construction of a cancer treatment program by a range of modulated frequencies in accordance with an embodiment.

FIG. 82 illustrates cellular injury following exposure to electromagnetic frequencies in accordance with an embodiment.

## DETAILED DESCRIPTION

This disclosure is not limited to the particular systems, devices and methods described, as these may vary. The terminology used in the description is for the purpose of describing the particular versions or embodiments only, and is not intended to limit the scope.

As used in this document, the singular forms "a," "an," and "the" include plural references unless the context clearly dictates otherwise. Unless defined otherwise, all technical and scientific terms used herein have the same meanings as commonly understood by one of ordinary skill in the art. Nothing in this disclosure is to be construed as an admission that the embodiments described in this disclosure are not entitled to antedate such disclosure by virtue of prior invention. As used in this document, the term "comprising" means "including, but not limited to."

The embodiments of the present teachings described below are not intended to be exhaustive or to limit the teachings to the precise forms disclosed in the following detailed description. Rather, the embodiments are chosen and described so that others skilled in the art may appreciate and understand the principles and practices of the present teachings.

This disclosure relates to an electronic portable medical device for characterization, diagnosis, treatment, and frequency discovery in a warm-blooded mammalian subject. This disclosure more particularly involves an integrated system capable of measuring, monitoring and recording electrical activity of the heart by RR interval (RRI) values and computing those values in order to identify specific heart rate variability (HRV) values resulting from exposure of the warm-blooded mammalian subject to specific frequencies of amplitude modulated radio frequency electromagnetic fields (AM RF EMF) thereof in order to diagnose and treat health conditions. The portable medical device is capable of measuring and recording RRI values during exposure to one or more such frequencies of AM RF EMF to diagnose, treat, and discovery frequencies in a warm-blooded mammalian subject in terms of the invention.

In some embodiments, the health condition may include, without limitation, hepatocellular carcinoma, colorectal cancer, breast cancer, ovarian cancer, pancreatic cancer, head and neck cancer, bladder cancer, liver cancer, renal cancer, melanoma, gastrointestinal cancer, prostate cancer, small cell lung cancer, non-small cell lung cancer, sarcoma, glioblastoma, T- and B-cell lymphoma, endometrial cancer, or cervical cancer.

Through the course of numerous clinical trials in which Hdp values have been recorded for numerous patients, it has been determined that patients' HRV values differ based on the type of health condition that a patient faces. In particular, different HRV values have been associated with different types of cancer. Such determinations have provided a basis for proposing a diagnostic procedure to diagnose a particular form of cancer harbored by a patient based on HRV values. These determinations further suggest that many health conditions suffered by a patient, including viral, parasitical or other pathogenic invasions, organ dysfunctions leading to undesirable components such as toxins being present in the blood of a patient, drug abuse, poisons, high low-density lipoprotein (LDL) cholesterol levels, venom from a snake-bite and the like, could be diagnosed on the basis of certain identified measured HRV values in a patient.

A frequency synthesizer can be used to generate a specific precise frequency or a series of such frequencies. A user can use a keyboard or other input device to select such frequencies, which in turn can cause a circuit to turn the generated signal ON or OFF within well-defined time intervals.

In an embodiment, a processing system can calculate HRV values from measured and recorded RRI values using an ECG monitoring system connected to a warm-blooded mammalian subject when exposing the subject to one or more high specific frequency radio frequency carrier signals emitted by a programmable generator. The ECG monitoring system measures and records RRI values for further processing. A processing system that is either internal to the portable medical device or external can execute a series of computing processes intended to calculate HRV values. HRV values are organized, synchronized, aggregated, recorded and stored as representative HRV variation values for further mathematical and Artificial Intelligence computing procedures to be performed in the intelligent library of frequencies (ILf). The processing system provides the sensing capability of the portable medical device by using various measured and recorded HRV values of the patient and identifies specific electromagnetic field amplitude modulated frequencies (SFq), characterized by recognizable patterns of HRV value changes (herein referred to as representative HRV variation values). The SFq is a subset of highly specific frequency radio frequency carrier signals that influence cellular functions or malfunctions in a warm-blooded mammalian subject (i.e., the patient). The portable medical device exposes the patient to highly specific frequency radio frequency carrier signals to the patient using a probe (more broadly described as an electrically conductive applicator) for applying the emissions to the patient via a topical contact at one or more determined parts of the body as part of the a programmable generator. The portable medical device measures and records RRI values from the patient using electrodes placed in topical contact in one or more determined parts of the body as part of the ECG monitoring system.

In an embodiment, the portable medical device can calculate and identify representative HRV variation values that can be stored in an internal temporary storage device for asynchronous transmission to the ILf. In an alternate embodiment, the portable medical device can simultaneously transmit the representative HRV variation values to the ILf by a wireless connection to an encrypted network. In an alternate embodiment, the portable medical device can transmit the RRI values and the information related to the patient's exposure to highly specific frequency radio frequency carrier signals to an external device with a processing system in order to calculate and identify the representative HRV variation values before transmission to the ILf.

In an embodiment, the portable medical device and/or an external device with a processing system can be connected to an encrypted network. The representative HRV variation values can be transmitted to the ILf via a synchronous or asynchronous connection to an encrypted web platform for further mathematical and Artificial Intelligence computing procedures.

In an embodiment, the computing processing system can include a device synchronizer, a data aggregator, a storage device and/or a storage interface, and an interface controller. The interface controller can be configured to match the calculated HRV values and the exposure to one or more highly specific frequency radio frequency carrier signals (synchronization). Additionally or alternatively, the interface controller can further be configured to consolidate the records (data aggregation) to be stored (storage) for further processing and transmission such that HRV values are linked to an exposure to one or more highly specific frequency radio frequency carrier signals from which such HRV values were measured. The ECG monitoring system and the programmable generator can be connected via the interface controller. The modules corresponding to synchronization and data aggregation and the storage interface can be arranged in a portable medical device integrated hardware solution or in an external device with external computing capabilities as an integrated hardware solution.

In another embodiment, a patient can be treated using the present portable medical device. The treatment procedure can include exposing the patient to one or more highly specific frequency radio frequency carrier signals. The programmable generators can be loaded with instructions configured to produce a health condition-specific group of SFq for warm-blooded mammalian subjects with a specific health condition. In an embodiment, the group of SFq can be accurately controlled. In an embodiment, the group of SFq can have a resolution of about 0.5 Hz from the intended determined or predetermined modulation frequency. In another embodiment, the group of SFq can have a resolution of about 0.1 Hz from the intended determined or predetermined modulation frequency. In yet another embodiment, the group of SFq can have a resolution of about 0.01 Hz from the intended determined or predetermined modulation frequency. In still another embodiment, the group of SFq can have a resolution of about 0.001 Hz from the intended determined or predetermined modulation frequency.

In an embodiment, the patient may be exposed to SFq emissions at a relatively low and safe energy level that results in low levels of absorption by the patient. It is believed that physiological exchanges or flow of electrical impulses within warm-blooded animals (which are to be affected by application of the emissions of the disclosed system) are similar at low energy levels. Regardless, in certain implementations, in the region of exposure (at or near to the point of contact or by induction of the electrically conductive applicator with a subject receiving treatment), the specific absorption rate (SAR) should be and is most preferably substantially less than 1.6 mW/g weight of living tissue.

Furthermore, emissions can be stably maintained during emission, such that the stability should preferably be of the order of $10^{-5}$, more preferably $10^{-6}$, and most preferably $10^{-7}$. The stability of the emissions can be determined as the relative deviation of frequency divided by the desired frequency, e.g., 0.01 Hz (deviation)/1,000 Hz (desired freq.)=$10^{-5}$.

In certain implementations, the programmable generator can include a microprocessor (or other similar integrated circuit) configured to operate according to control information that is, for example, loaded from the processing system. In some examples, the programmable generator can combine other medical devices, such as an ECG monitoring system and other computing servers, all of them operating together and synchronized by the processing system as one single new medical device. As a result, the new and improved programmable generator can be loaded with an updated series of specific SFq identified in a single warm-blooded mammalian subject or from a group of warm-blooded mammalian subjects with same health conditions. In addition, the new and improved programmable generator as described herein can support different applications besides treatment of a patient, such as diagnosis, searching for SFq, and treatment follow-up. The microprocessor (or integrated circuit) can control the function of the programmable generator to produce the desired therapeutic emissions. In some examples, the programmable generator can include an impedance transformer connected to an emitter of low energy electromagnetic emissions and a probe (e.g., an electrically conductive applicator) that applies the emissions to a patient. The impedance transformer can be configured to substantially match the impedance of the patient sensed by the emitter circuit with the impedance of the output of the emitter circuit.

The ECG monitoring system can be configured to measure RRI values, calculate HRV values, and record representative HRV variation values used for SFq identification. The identified SFq can be used for diagnosis and treatment of a health condition of a patient. The system as described herein can thus include an ECG monitoring system that measures RRI values and records various identified representative HRV variation values of the patient. Accordingly, the system can generally measure various identified RRI values of the patient using electrodes placed in topical contact with various determined parts of the body. The ECG monitoring system can further include a subsystem configured to record the various identified RRI values of the patient. The calculated and recorded HRV values can be stored in one or more storage devices.

The HRV values can be calculated and recorded following established procedures. Initial measurements of RRI values during non-exposure highly specific frequency radio frequency carrier signals periods in an individual or patient or RRI values thereof are herein identified as basal measurements or basal RRI values. In terms of procedure, initial measurements of the above parameters are performed on warm-blooded mammalian subjects after a period of relaxation, such as about 15 minutes, while the patient is lying in a supine position (face and preferably also palms of the hands facing upwardly) or in another comfortable and relaxed position.

After obtaining the above initial measurements, a selected series of one or more highly specific frequency radio frequency carrier output signals can be applied to the subject, thereby providing for exposure measurements or exposure RRI values.

The above-mentioned one or more highly specific frequency radio frequency carrier signals can be AM RF EMF output signals produced according to a control program loaded into the programmable generator, which generates the AM RF EMF output signals at certain predetermined amplitude modulation (AM) frequencies. In some implementations, patients are preferably exposed to AM RF EMF output signals, or such signals are applied to patients, during a determined period of time, most preferably over a period of at least ten heart-beats of the patient or a period of at least 10 seconds. This procedure can generally take place while the patient is connected to both a synchronized ECG monitoring system and the programmable generator so that RRI values can be measured and HRV values can be calculated and recorded during the period of exposure or application. The HRV values can, however, also or alternatively be the initial data source to identify SFq and/or representative HRV variation values after software processing as is described above.

The above-identified RRI values that are measured during or after the above-identified exposures or applications to subjects or patients are herein referred to as exposure RRI values and post-exposure RRI values, respectively.

The procedures above, as generally applied to multiple patients diagnosed with a health condition can provide multiple basal RRI values, multiple exposure RRI values and multiple post-exposure RRI values as related to the diagnosed health condition. These multiple RRI values can in general be somewhat scattered values and, after calculation, can produce HRV values that are similarly scattered. Accordingly, for purposes of defining representative HRV variation values, such scattered values can be regularly submitted to the processing system so that the processing system can perform mathematical and Artificial Intelligence calculations that identify SFq using the representative HRV variation values.

Continuing the above example, the ECG monitoring system can be part of the portable medical device as described herein and provide a means for software processing RRI values for use in the identification of SFq and the diagnosis of health conditions of a patient. The identification of SFq and representative HRV variation values are termed representative surrogate markers that are determined through the processing system from the ECG monitoring system that measures and records RRI values performed during non-exposure and exposure periods on patients pre-diagnosed and diagnosed to be either healthy or suffering from a known form of poor health condition.

Representative surrogate markers employed for the purpose of diagnosis, SFq identification, treatment and treatment follow-up, can be derived from computative combinations of information from representative basal RRI measured values, representative exposure RRI measured values, and/or representative post-exposure RRI values. Those computative combinations can be performed in the ILf.

The time periods of exposure or application of the AM RF EMF frequency output signals by means of a variable frequency programmable generator device may be within a broad range of frequencies; for example, AM RF EMF frequencies within a range between about 0.01 to about 150 MHz can require a short period of time for RRI values to be varied at any particular frequency value. Thus, consecutive exposures or applications of sections of the range of AM RF EMF frequencies may be required in order to identify AM RF EMF frequency values at which basal, exposure and post-exposure HRV values actually occur during the heart-beat times at which RRI values are measured by the ECG monitoring system and processed by the computing processing system to calculate the HRV values and to determine the representative HRV variation values.

In addition to being integrated within or coupled to the recording means as described above, the processing system can be located at a central server and connected to the portable device by an encrypted web-based platform, which can perform the analysis based on recorded representative HRV variation values, received or communicated to the central server.

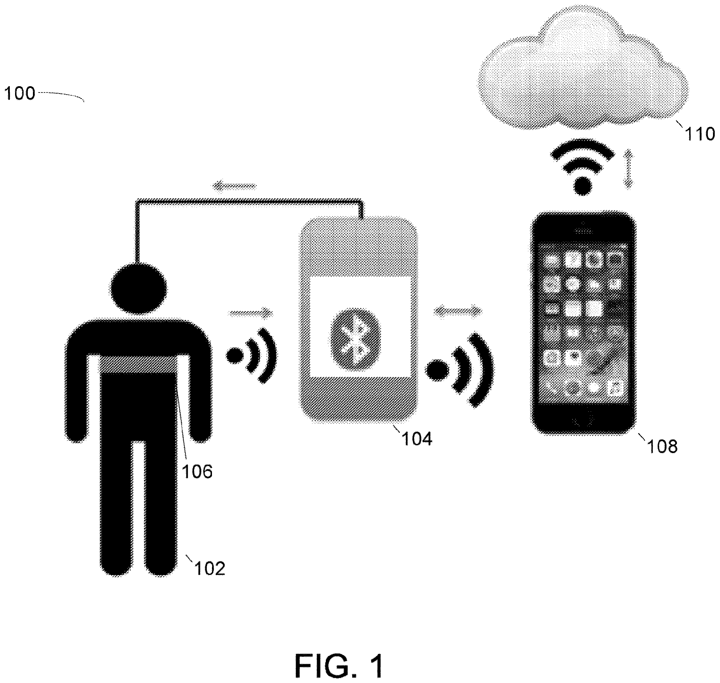

The following discussions of FIGS. 1-6 illustrate various examples of the portable device as described above, and particular environments in which the device can be utilized.

FIG. 1 illustrates a sample system 100 used to monitor a patient 102. In certain implementations, a portable medical device 104 (similar to the portable medical devices as described above) can be operably connected to a set of electrical sensors (e.g., connected to the patient 102 using a belt such as electrode belt 106). The medical device 104 can be configured to produce a set of electrical signals (e.g., via the programmable generator device described above) and transmit the signals to the patient 102 via the sensors. The sensors can be configured to measure an electrical response produced by the patient 102. The sensors can return a value to the medical device 104 indicative of the measured electrical response. In certain implementations, the medical device 104 can transmit this information to an external computing device, such as smartphone 108, for further processing. Additionally, in some examples, the smartphone 108 can be operably connected to an encrypted network 110 for transmission of any processed information for remote storage and/or additional calculations by a remote computing device such as a remote server.

As noted above, the portable medical device 104 can work in concert with an external computing device, such as the example shown in FIG. 1. In such an arrangement, the internal processing components of the portable medical device 104 can be reduced, thereby reducing the overall size of the portable medical device. However, as also noted above, the portable medical device 104 can be designed such that it can perform analysis of the received electrical response from the patient 102.

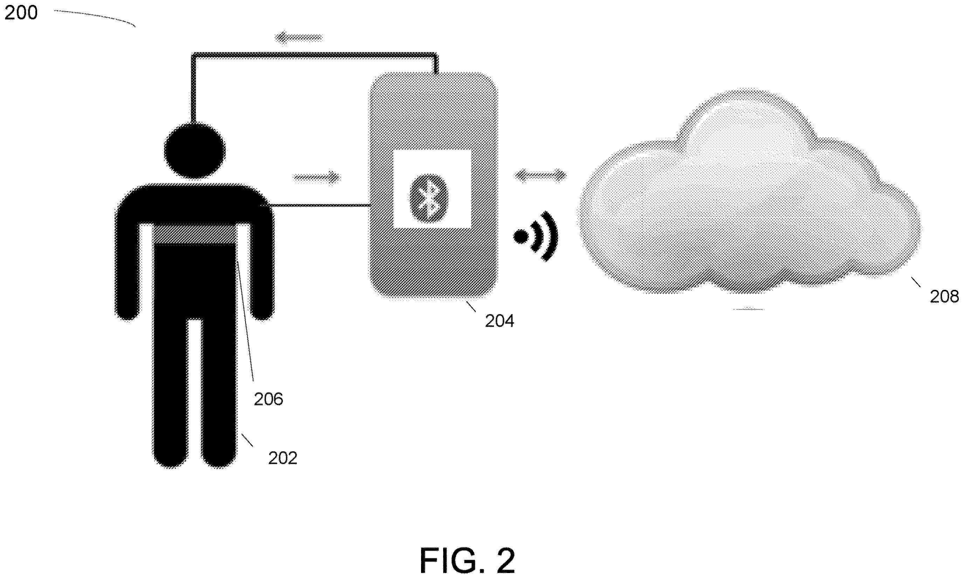

FIG. 2 illustrates a sample system 200 used to monitor a patient 202. In certain implementations, a portable medical device 204 (similar to the portable medical devices described above) can be operably connected to a set of electrical sensors (e.g., connected to the patient 202 using a belt such as electrode belt 206). The medical device 204 can be configured to produce a set of electrical signals (e.g., via the programmable generator device as described above) and transmit the signals to the patient 202 via the sensors. The sensors can be configured to measure an electrical response produced by the patient 202. The sensors can return a value to the medical device 204 indicative of the measured electrical response. However, unlike in FIG. 1, the medical device 204 can be configured to perform additional analysis on the returned value. Additionally, in some examples, the medical device 204 can be operably connected to an encrypted network 208 for transmission of any processed information for remote storage and/or additional calculations by a remote computing device such as a remote server.

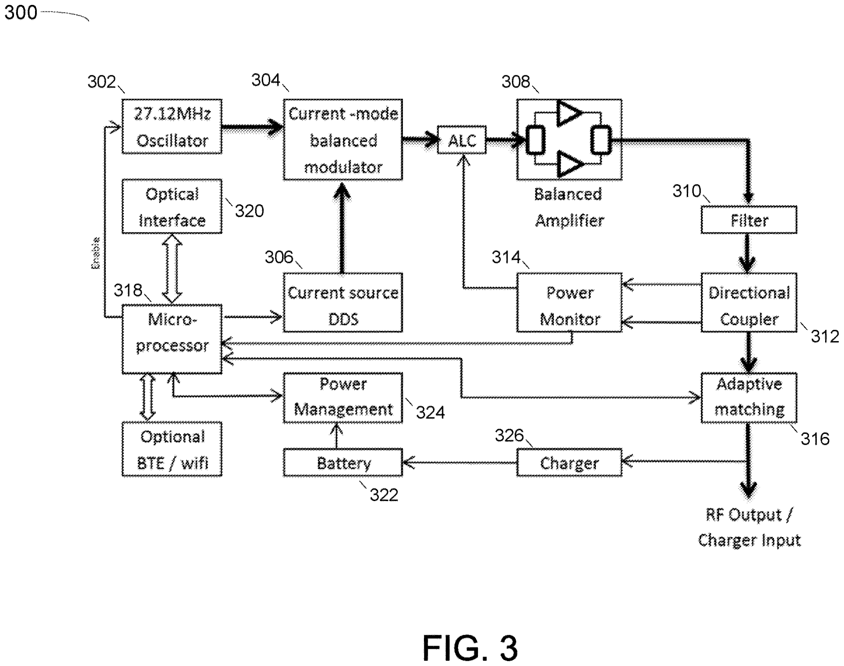

FIG. 3 illustrates a sample circuit diagram of a portable medical device 300 such as those described herein. The medical device 300 can include a fixed carrier frequency oscillator 302 and a current mode balanced modulator 304. In certain implementations, the frequency oscillator 302 can be configured to produce a 27.12 MHz signal. However, it should be noted that this signal frequency is provided by way of example only and can vary based upon the intended functionality of the portable medical device 700. In certain implementations, the frequency oscillator 702 can be configured to produce a signal having a frequency between 1.0 mHz and 1.0 Hz, 1.0 Hz and 1.0 kHz, 1.0 kHz and 1.0 MHz, 1.0MHz and 1.0 GHz, and frequencies above 1.0 GHz.

In some examples, the modulator 304 can be modulated by a sinusoid current generated by a direct digital synthesiser 306, thereby producing a modulated carrier signal. In certain implementations, the sinusoid current can have a 10 MHz frequency resolution and a frequency accuracy of 10ppm.

The modulated carrier signal can be amplified by an amplifier 308. In certain implementations, the amplifier 308 can be a class AB balanced RF amplifier. The output of the amplifier 308 can be filtered by a filter 310 to reduced harmonic content. The filtered signal may be processed by a directional coupler 312 and passed to a power monitor 314 and an adaptive matching component 316. The power monitor 314 can be configured to monitor the current power level of the filtered signal and, if necessary, provide an adjustment signal to a microprocessor 318. The microprocessor 318 can alter the sinusoid current generated by the synthesizer 306, thereby altering the output power. In some implementations, the microprocessor 318 can be operably connected to an optical interface 320 of the medical device 300 that is configured to display information received from the microprocessor. In some examples, the optical interface 320 can include an input device such as a touchscreen to receive input information from a patient or a user of the medical device 300.

The medical device 300 can also include a battery 322 and a power management component 324 configured to manage and distribute power generated by the battery. In some examples, the battery 322 can be a rechargeable battery coupled to a charging component 326 configured to provide a charging signal to the battery when the medical device 300 is connected to an exterior power source.

As noted above, the output of the directional coupler 312 can be passed to an adaptive matching component 316. The adaptive matching component 316 can also be connected to and controlled by the microprocessor 318. Upon receiving instructions from the microprocessor 318, the adaptive matching component 316 can be configured to provide an RF output signal to one or more sensors positioned on the patient's body.

In certain implementations, the filtered power can be maintained at a desired level by means of an automatic level control loop including, as described above, the power monitor 314 and the microprocessor 318, ensuring that the change in voltage as the battery 322 discharges does not significantly affect the treatment. The reflected power is minimized by adjusting the adaptive matching component 316 to ensure efficient transfer of therapeutic signals to the patient. In some examples, the medical device 300 can be controlled directly from a computer over an optical link using a frequency and duration command and return to a low power state after a defined time period.

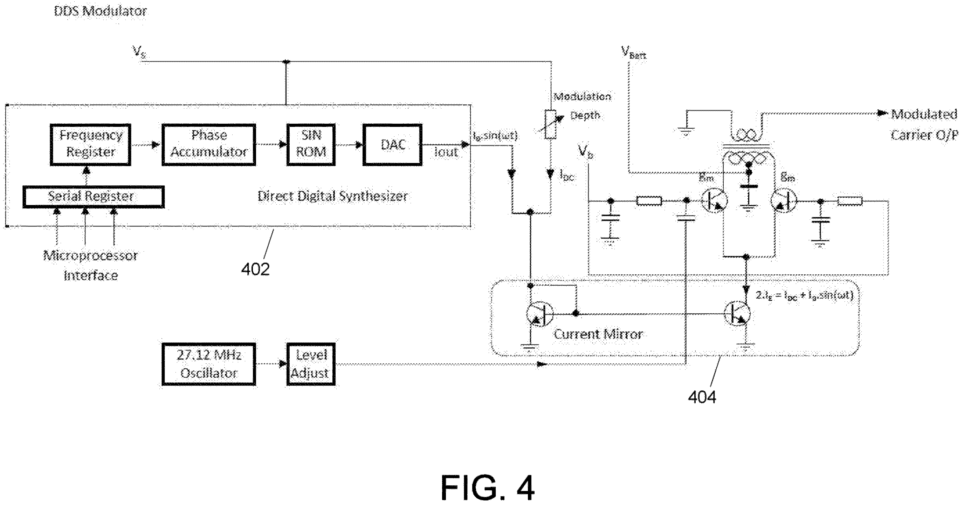

FIG. 4 illustrates a sample circuit diagram for producing a modulated carrier signal as described above in the description of FIG. 3. As noted above, the modulation sinusoidal signal can be generated by a direct digital synthesizer (DDS) 402, the DDS includes an integrated current output digital to analog converter (DAC). In certain implementations, the modulation sinusoidal signal can be in the frequency range of about 10 Hz to about 50 kHz.

A current mirror 404 can be used to drive the current mode balanced amplitude modulator. The input to the current mirror 404 can include two components, the sinusoid from the DDS 402 and a DC current which is adjustable to set the modulation depth to a desired range, for example, 85% to 90% of the original signal. The output from the current mirror 404 can form the tail of a transistor pair operating as a high impedance current source with current $2I_E = I_{DC} + I_0 \sin(\omega t)$, the current setting the gain, $g_m$, of the transistor amplifiers in the differential pair. The bases of the transistors can be biased at the voltage $V_b$, one transistor can be fed with the carrier signal at 27.12 MHz, and the second transistor can be held at a small signal ground. The carrier signal can be amplified differentially with a gain varying in sympathy with the modulation signal, and the two outputs can be combined using, for example, a center-tapped balun transformer. The combined modulated signal can attain signal levels twice as high as would be available from a single-ended configuration for the same battery voltage. The output of the modulator is thus the desired AM modulated signal, e.g., a 27.12 MHz carrier signal.

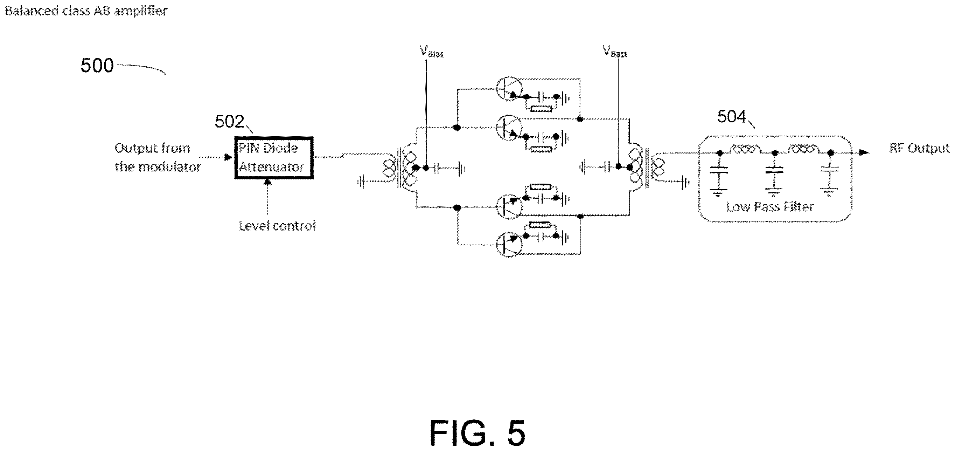

FIG. 5 illustrates a sample circuit diagram for an amplifier 500 (e.g., amplifier 308 as described above in the description of FIG. 3). The signal from the current mode modulator can be amplified to achieve a required signal level using a balanced amplifier 500.

The gain of the amplifier 500, and hence the output level, is somewhat dependant on the battery voltage, therefore the amplifier can be preceded by a variable attenuator 502 that is adjustable to maintain a constant output power as the battery discharges. The attenuator 502 can use a shunt PIN diode in a T attenuator configuration in which a higher current through the diode results in higher attenuation. As noted above, in certain implementations, the amplifier 500 can be a class AB balanced design with parallel RF devices to increase the current capacity.

The input transformer can convert the input from a single-ended signal to a balanced signal and can match the input impedance of the devices. Each pair of devices can amplify one half cycle of the signal. As such, the output signal can approach twice the battery voltage in peak amplitude before clipping. The output transformer can combine the two signals to produce the final output signal. The class AB bias ensures that any cross-over distortion is minimized. The impedance transformation ratio of the output balun transformer can be chosen such that the desired output power can be generated given the limited supply (battery) voltage. The balanced amplifier design suppresses even-order distortion products; however, odd-order harmonics can be suppressed using a low pass filter 502, for example, a 5th order low pass filter.

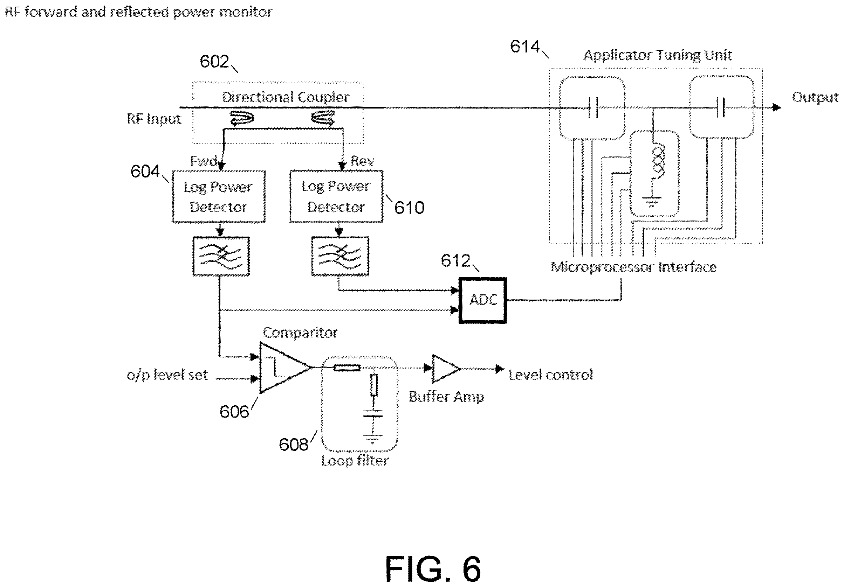

FIG. 6 illustrates a sample circuit diagram for monitoring the RF forward and reflected power produced by the amplifier. The output from the amplifier (e.g., amplifier 500) can be sampled using a directional coupler 602, and the signal detected using a log power detector 604 with, for example, >30 dB dynamic range. The output signal can be low-pass filtered to obtain the average envelope power from the amplitude modulated signals. In certain implementations, the average envelope power can be measured by an analog-to-digital converter (ADC) 612 to allow the microprocessor to report the value to the control computer for use in a control loop that maintains a constant output power as the battery voltage changes.

In some examples, the detected voltage equating to the average output power can be compared to the desired value using a comparator 606. The digital output can be filtered using a passive lead-lag loop filter 608 with appropriate time constants to maintain stability of the control loop. The output of the loop filter 608 can be buffered and used to drive a PIN diode attenuator to adjust the overall gain to obtain the desired output power.

The directional coupler 602 can also sample the power reflected from the load, in this case the applicator, using a log power detector 610. The average level can be determined using a low-pass filter and can be sampled using an ADC 612. The control computer can compare the forward and reflected powers and adjust an applicator tuning unit 614 to find the settings with the lowest reflected power or optimum power transfer to the patient. Various algorithms can be used. For example, the control computer can adjust each of the three elements in turn, up or down in value, and determine whether the reflection reduces or not, repeating this process iteratively until a minimum is found.

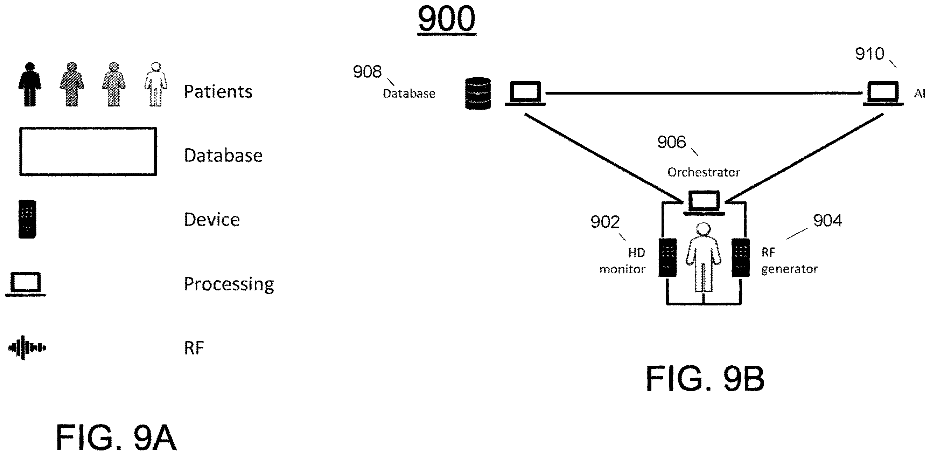

As described herein, patient diagnosis may be performed with the aid of measured Hdp values and recorded Hdp values. The recorded Hdp values may be measured in a number of patients that either are pre-diagnosed to be suffering from an identified poor health condition or are in a healthy condition. The Hdp values may be stored at determined times and for determined periods of time, as described in greater detail below (FIGS. 9-11).

In an embodiment, a system includes an Hdp monitoring system used to measure and record Hdp values and a frequency generator configured to provide one or more highly specific frequencies to a patient via radio frequency (RF) carrier signals.

In an embodiment, the system may identify SFq, which are a subgroup of the highly specific frequency RF carrier signals. The SFq may be used to influence cellular functions or malfunctions in a warm-blooded mammalian subject. The exposure of a warm-blooded mammalian subject to the SFq may cause representative Hdp variation values to change in a manner that indicates whether or not one or more highly specific frequency RF carrier signals have a potential biological effect in the warm-blooded mammalian subject. The specificity of changes in representative Hdp variation values may be a surrogate marker for the diagnosis and treatment of the warm-blooded mammalian subject.

In an embodiment, the system may store one or more groups of identified SFq in a server connected by a protected Internet-based platform to form an intelligent library of SFq (ILf). The stored data may be combined, organized, compared and characterized for use in the diagnosis of patients or individuals and in the treatment of health conditions in patients having similar diagnoses.

The integrated frequency generator used to emit or expose a warm-blooded mammalian subject to one or more high specific frequency RF carrier signals may be a programmable generator and may be an electronic component that is activatable by electrical power as part of an integrated system. The programmable generator may be employed to influence cellular functions or malfunctions in a warm-blooded mammalian subject. The programmable generator may include one or more controllable low energy electromagnetic energy generator circuits configured to generate one or more highly specific frequency RF carrier signals. One or more microprocessors or integrated circuits that include or communicate with the one or more generator circuits are provided. In an embodiment, the one or more microprocessors may also be used to control the transmission and reception of control information from a processing system. In an embodiment, the one or more generator circuits may include one or more amplitude modulation (AM) frequency control signal generators configured to control amplitude modulated variations of the one or more highly specific frequency RF carrier signals. The one or more generator circuits may further include one or more programmable AM frequency control signal generators configured to control the frequency at which the amplitude modulations are generated.

The system may further include a processing system configured to integrate and synchronize the Hdp monitoring system and the one or more programmable generators. Various Hdp values measured and recorded by the Hdp monitoring system while exposing a warm-blooded mammalian subject to one or more highly specific frequency RF carrier signals emitted by the one or more programmable generators may be processed by the processing system. The information resulting from such processing may be stored, for example, in the ILf. The processing system may further control, synchronize and load a control program into the one or more programmable generators with a specific series of SFq. As such, the processing system may integrate and synchronize the Hdp monitor, the ILf and the one or more programmable generators to support an integrated solution.

In an embodiment, the processing system and the ILf may be part of a server connected to the remainder of the system by a protected web platform. The ILf may include an artificial intelligence capability used for storing, combining, organizing, comparing, characterizing and processing SFq and recorded representative Hdp variation values. The ILf may store and organize a series of SFq and representative Hdp variation values identified in a warm-blooded mammalian subject or patient. One or more series of SFq may then be loaded into the one or more programmable generators. The one or more programmable generators may accurately control the emission of the frequency of the amplitude modulations with an accuracy of at least 1000 parts per million (ppm) relative to one or more determined or predetermined reference AM frequencies. In an embodiment, the AM frequencies may be within a range of 0.01 Hz to 150 kHz. The processing system may further include a connection or a coupling position. The connection or coupling position may be used to connect or couple the processing system to an electrically conductive applicator that applies the one or more amplitude-modulated low energy emissions at the accurately controlled modulation frequencies to the warm-blooded mammalian subject.

Through the course of performing numerous clinical trials in which multiple measurements of various Hdp values in patients have been recorded, it has been determined that such Hdp values differ based on the type of health condition that a patient faces. In particular, different Hdp values have been identified with respect to different types of cancer. Such determinations have provided a basis for proposing a diagnostic procedure based on measured Hdp values that is used to diagnose a particular form of cancer in a patient. These determinations further suggested that many health conditions suffered by a patient, including viral, parasitic or other pathogenic invasions, organ dysfunctions, which could lead to toxins being present in the blood of a patient, drug abuse, poisons, high low-density lipoprotein (LDL) cholesterol levels, venom from a snake-bite and the like, may be diagnosed in a patient on the basis of certain identified measured Hdp values.

A frequency synthesizer may be used to generate a particular frequency or a series of frequencies with precision. For example, a user may use a keyboard or other input device to select one or more frequencies, which in turn may cause a circuit to turn a generated signal ON or OFF within well-defined time intervals.

In an embodiment, a processing system processes Hdp values measured and recorded by an Hdp monitoring system connected to warm-blooded mammalian subject during the exposure to one or more highly specific frequency RF carrier signals emitted by a programmable generator. The Hdp monitoring system may measure and record Hdp values for further processing. The processing system may incorporate one or more algorithms that analyze the recorded Hdp values obtained by the Hdp monitoring system. The processing system uses various measured and recorded Hdp values of the subject and identifies SFq, characterized by recognizable patterns of Hdp variation value changes, herein referred to as representative Hdp variation values. The SFq is a subset of the highly specific frequency RF carrier signals that influence cellular functions or malfunctions in a warm-blooded mammalian subject (i.e., a patient). The processing system generally identifies the various measured and recorded Hdp values of the patient using electrodes placed in topical contact with various determined parts of the body as part of the Hdp monitoring system. The Hdp monitoring system further comprises a recording component that records the various identified measured Hdp values of the patient. In an embodiment, the recording component may store the measured Hdp values in a storage device of the Hdp monitoring system. In an alternate embodiment, the recording component may store the measured Hdp values in any storage device on which the various identified measured Hdp values of the patient can be recorded for immediate and/or future processing. Hdp values may include the values of, for example, one or more of the following hemodynamic parameters:

- RR interval (interval from an R peak to the next R peak as shown, for example, on an electrocardiogram (ECG)) (RRI);
- heart rate (HR);
- systolic blood pressure (sBP);
- diastolic blood pressure (dBP);
- median blood pressure (mBP);
- pulse pressure (PP);
- stroke volume (SV);
- cardiac output (CO); and

- total peripheral resistance (TPR).

In an embodiment, the processing system may include a device synchronizer, a data aggregator, a storage device and/or a storage interface, and an interface controller. The interface controller may be responsible for matching the Hdp values and the exposure to one or more high specific frequency radio frequency carrier signals (synchronization). Additionally, or alternatively, the interface controller may be responsible for consolidating the records (data aggregation) to be stored (storage) for further processing (interface controller) in such a way that Hdp values are linked to an exposure to one or more high specific frequency radio frequency carrier signals from which such Hdp values were measured. The Hdp monitoring system and the programmable generator may be connected via the interface controller. The modules corresponding to synchronization and data aggregation, and the storage interface may be packed as a portable integrated hardware solution.

In another embodiment, the processing system may include, *inter alia,* two components: a statistical mining component and a machine learning/evolutionary game theory component. It should be noted that, as used herein, machine learning refers to various types of machine learning including, for example, deep machine learning and layered machine learning. The statistical mining component may include a series of mathematical procedures based on discriminant analysis and support vector machine (SVM). Hdp values may be constant selected metric variables and their dependent new attributes that are analyzed based on different well-established statistical methods. Using multivariate discriminant analysis and other coordinate transformation based on relevant component analysis, Hdp values may be represented as centroids of representative Hdp variation values with well-defined threshold values in order to optimize common metrics. The machine learning/evolutionary game theory component may include permanently refined, cluster analysis and updated mathematical algorithms that or by new discriminating attributes, perform cutoff refining for (1) identify patterns of responses for health condition-specific frequencies, herein named representative Hdp variation values, and (2) store representative Hdp variation values and the corresponding health condition-specific frequencies. These components may be implemented on a central and secure server-side system connected to the integrated hardware solution via encrypted communication over a network, such as the Internet.

In yet another embodiment, an ILf may be located in the central and secure server system. In an embodiment, the library may be connected to all instances of the integrated hardware solution via encrypted communication over a network, such as the Internet. In such an embodiment, the network solution may provide a real-time, integrated and evolutionary system combining all working devices. Permanent updated de-identified patient's demographic and clinical information data gathered from physician and patient-reported outcomes combined with records of representative Hdp variation values and the correspondent health condition-specific frequencies (SFq) and the data may be stored in the ILf. Threshold values for representative Hdp variation values may be refined based on newly added values. Such data may be structured and processed to refine the procedures for diagnosis, treatment and follow-up for a health condition of a patient. ILf may have computing capabilities to support statistical data mining and machine learning for pattern recognition and evolutionary game theory for identification of points of equilibrium, which characterize the best possible matching for each series of SFq and/or representative Hdp variation values and corresponding to the diagnosis and treatment outcome information. Refinement procedures are implemented as artificial intelligence based meta-programs, which take into account patient segmentation. The programmable generator is connected by the interface controller in the processing system in order to transfer data between the processing system and the programmable generator. Refined procedures are then downloaded back to the processing system module of the integrated hardware solution, in order to re-program the programmable generator.

The interface controller may connect the programmable frequency generator to the processing system in order to allow for the transfer of data. Refined procedures may be downloaded to the processing system in order to update the programmable frequency generator prior to or during a treatment session.

In an embodiment, a diagnosis of a health condition of a patient may be determined based on one or a group of SFq and/or representative Hdp variation values identified by the processing system. In an embodiment, a plurality of measured and recorded Hdp values may be submitted to the processing system during the exposure of the patient to one or more high specific frequency radio frequency carrier signals. The processing system may identify SFq and/or representative Hdp variation values in a patient diagnosed with a health condition. In an embodiment, the identified SFq and representative Hdp variation values may be stored in the ILf. The warm-blooded mammalian subject, during exposure to a selected group of SFq (i.e., a subgroup of high specific frequency radio frequency carrier signals emitted by a programmable generator) may have various Hdp values that are measured and recorded by the Hdp monitoring system processed to identify the characteristic hemodynamic response pattern to SFq exposure. The processing system identifies representative Hdp variation values related to the selected group of SFq. The processed information may be stored in the ILf for instant and/or future database comparisons. The diagnosis identification may be the result of searching for patterns of response that are consistent with a specific health condition of a patient. The processing system may diagnose a health condition of a patient by incorporating a series of mathematical algorithms that analyze the recorded Hdp data obtained by the Hdp monitoring system.

In another embodiment, a user may be enabled to search for SFq. The searching procedure may be conducted during the exposure of a patient to one or more high specific frequency radio frequency carrier signals. For example, searching for SFq may include a process that involves the Hdp monitoring system reviewing measured and recorded Hdp stored in the processing system during exposure of the patient to one or more high specific frequency radio frequency carrier signals. The searching procedure for SFq may involve the application of mathematical algorithms to determine a series of specific frequencies to be provided by the programmable generators. In an embodiment, the searching process may include processing of measured and recorded Hdp values by the Hdp monitoring system during the exposure to a series of specific frequencies, such as a subgroup of high specific frequency radio frequency carrier signals, produced by a programmable generator in a warm-blooded mammalian subject with an unknown health condition or a patient with a known health condition. With respect to the exposure of a subject or patient to a predetermined sequence of one or more high specific frequency radio frequency carrier signals, the term "accurately controlled" means that modulated low energy electromagnetic emissions are modulated to within a resolution of at most about 1 Hz of higher frequencies (greater than about 1000 Hz). For example, if a determined or predetermined modulation frequency to be applied to the warm-blooded mammalian subject is about 2000 Hz, accurate control of such modulated low energy emission requires the generated frequency to be between about 1999 Hz and about 2001 Hz. The processing system identifies SFq and representative Hdp variation values during the searching procedure.

In an embodiment, new SFq may be discovered. The discovery procedure may be conducted during exposure of an individual or patient to one or more high specific frequency radio frequency carrier signals. Discovery of new SFq may include having the processing system received measured and recorded Hdp values from the Hdp monitoring system during exposure to one or more high specific frequency radio frequency carrier signals. The discovery of new SFq may further involve the application of mathematical algorithms to determine a series of specific frequencies by the programmable generators. In an embodiment, the searching process may process measured and recorded Hdp values from the Hdp monitoring system during exposure to a series of specific frequencies that are a subgroup of high specific frequency radio frequency carrier signals produced by a programmable generator in a warm-blooded mammalian subject with a known health condition. In an embodiment, the processing system identifies SFq and representative Hdp variation values during the process of discovering new SFq.

In an embodiment, a diagnosis of a health condition of a patient may be determined based on one or a group of SFq and/or representative Hdp variation values identified by the processing system. In an embodiment, a plurality of measured and recorded Hdp values may be submitted to the processing system during the exposure of the patient to one or more high specific frequency radio frequency carrier signals. The processing system may identify SFq and/or representative Hdp variation values in a patient diagnosed with a health condition. In an embodiment, the identified SFq and representative Hdp variation values may be stored in the ILf. The warm-blooded mammalian subject, during exposure to a selected group of SFq (i.e., a subgroup of high specific frequency radio frequency carrier signals emitted by a programmable generator) may have various Hdp values that are measured and recorded by the Hdp monitoring system processed to identify the characteristic hemodynamic response pattern to SFq exposure. The processing system identifies representative Hdp variation values related to the selected group of SFq. The processed information may be stored in the ILf for instant and/or future database comparisons. The diagnosis identification may be the result of searching for patterns of response that are consistent with a specific health condition of a patient. The processing system may diagnose a health condition of a patient by incorporating a series of mathematical algorithms that analyze the recorded Hdp data obtained by the Hdp monitoring system.

In another embodiment, a user may be enabled to search for SFq. The searching procedure may be conducted during the exposure of a patient to one or more high specific frequency radio frequency carrier signals. For example, searching for SFq may include a process that involves the Hdp monitoring system reviewing measured and recorded Hdp stored in the processing system during exposure of the patient to one or more high specific frequency radio frequency carrier signals. The searching procedure for SFq may involve the application of mathematical algorithms to determine a series of specific frequencies to be provided by the programmable generators. In an embodiment, the searching process may include processing of measured and recorded Hdp values by the Hdp monitoring system during the exposure to a series of specific frequencies, such as a subgroup of high specific frequency radio frequency carrier signals, produced by a programmable generator in a warm-blooded mammalian subject with an unknown health condition or a patient with a known health condition. With respect to the exposure of a subject or patient to a predetermined sequence of one or more high specific frequency radio frequency carrier signals, the term "accurately controlled" means that modulated low energy electromagnetic emissions are modulated to within a resolution of at most about 1 Hz of higher frequencies (greater than about 1000 Hz). For example, if a determined or predetermined modulation frequency to be applied to the warm-blooded mammalian subject is about 2000 Hz, accurate control of such modulated low energy emission requires the generated frequency to be between about 1999 Hz and about 2001 Hz. The processing system identifies SFq and representative Hdp variation values during the searching procedure.

In an embodiment, new SFq may be discovered. The discovery procedure may be conducted during exposure of an individual or patient to one or more high specific frequency radio frequency carrier signals. Discovery of new SFq may include having the processing system received measured and recorded Hdp values from the Hdp monitoring system during exposure to one or more high specific frequency radio frequency carrier signals. The discovery of new SFq may further involve the application of mathematical algorithms to determine a series of specific frequencies by the programmable generators. In an embodiment, the searching process may process measured and recorded Hdp values from the Hdp monitoring system during exposure to a series of specific frequencies that are a subgroup of high specific frequency radio frequency carrier signals produced by a programmable generator in a warm-blooded mammalian subject with a known health condition. In an embodiment, the processing system identifies SFq and representative Hdp variation values during the process of discovering new SFq.

In yet another embodiment, the system may be used to construct and update the ILf. The process used to construct and update the ILf library with frequencies may use the processing system to identify SFq and/or representative Hdp variation values in warm-blooded mammalian subjects. The processing system may store identified SFq and representative Hdp variation values in a central server connected by a protected Internet platform. The ILf may store newly identified SFq from warm-blooded mammalian subjects with a known health condition. The stored SFq that are originated from warm-blooded mammalian subjects with known health conditions may undergo artificial intelligence processing to allow future diagnosis, identification, and treatment program generation for the treatment of patients diagnosed with the same health conditions. For example, one or more SFq identified in a patient diagnosed with a specific health condition may be used with other warm-blooded mammalian subjects for diagnosis and treatment proposes.

In another embodiment, a patient may be treated using the present system. The treatment procedure may include exposing patients to one or more high specific frequency radio frequency carrier signals. The programmable generators may be loaded with a program control in order to produce a selected and health condition-specific group of SFq that are to be provided to the warm-blooded mammalian subjects with the specific health condition. The ILf may store and update a plurality of selected groups of SFq identified in warm-blooded mammalian subjects with the same health conditions. The processing system may load the programmable generators with program controls to expose warm-blooded mammalian subjects with selected health condition to the specific group of SFq. In an embodiment, the group of SFq may be accurately controlled. In an embodiment, the group of SFq may have a resolution of about 0.5 Hz from the intended determined or predetermined modulation frequency. In another embodiment, the group of SFq may have a resolution of about 0.1 Hz from the intended determined or predetermined modulation frequency. In yet another embodiment, the group of SFq may have a resolution of about 0.01 Hz from the intended determined or predetermined modulation frequency. In still another embodiment, the group of SFq may have a resolution of about 0.001 Hz from the intended determined or predetermined modulation frequency.

In an embodiment, the system may be used to provide follow-up treatment to a patient. The follow-up procedure may include testing procedures in patients under treatment for a heath condition during a determined period of time or may real-time testing in patients during the exposure to one or more high specific frequency radio frequency carrier signals while under a treatment cycle. The follow-up testing procedure may be conducted during the exposure to one or more high specific frequency radio frequency carrier signals in an individual or patient. The follow-up testing of a patient with a known health condition may involve the application of mathematical algorithms to determine a series of specific frequencies to be loaded into the one or more programmable generators. The follow-up testing may include providing the processing system with measured and recorded Hdp from the Hdp monitoring system during exposure to one or more high specific frequency radio frequency carrier signals for follow-up comparison. The processing system may identify SFq and representative Hdp variation values during the follow-up testing procedure that may or may not modify as result of the treatment to a patient. The follow-up testing procedure result may be able to identify patterns of response consistent with a non-invasive prediction of treatment response of a health condition. The processing system may incorporate a series of mathematical algorithms that analyze the recorded Hdp data obtained by the Hdp monitoring system.

Of importance is the exposure of SFq emissions to be at a very low and safe energy level and result in low levels of absorption, the reason believed to be that physiological exchanges or flow of electrical impulses within warm-blooded animals (which are to be affected by application of the emissions of the present invention) are similarly at very low energy levels. In any event, in the region (at or near to the position of contact or close-by induction of the electrically conductive applicator with a subject receiving treatment), the specific absorption rate (SAR) should be and is most preferably substantially less than 1.6 mW/g weight of living tissue.

Furthermore of importance to achieve the intended biological therapeutic effect is that the stability of the emissions be maintained during emission, and that such stability should preferably be of the order of $10^{-5}$, more preferably $10^{-6}$, and most preferably $10^{-7}$, stability being determined as the relative deviation of frequency divided by the desired frequency, e.g., 0.01 Hz (deviation)/1,000 Hz (desired freq.)=$10^{-5}$.

The exposure of one or more high specific frequency radio frequency carrier signals by the programmable generators integrated in the invention. The programmable generator is an electronic component with significant improvements from a patented medical device that includes a microprocessor (which may more recently be replaced by an integrated circuit). The programmable generator in the invention uses control information that is loaded from the processing system. The other improvements consist in the programmable generator in the invention is the integrated part of the invention, that combines other medical devices such as a Hdp monitoring system and other computing servers, all of them operating together and synchronized by the processing system as one single new medical device. As a result, the new and improved programmable generator can be instantly loaded with updated series of specific SFq identified in a single warm-blooded mammalian subject or identified in a group of warm-blooded mammalian subjects with same health conditions. In addition, the new and improved programmable generator in the invention supports different applications besides treatment of a patient, such as diagnosis, searching for SFq and follow-up of a treatment. The microprocessor (or now alternatively integrated circuit) then controls the function of the programmable generator to produce the desired therapeutic emissions. Also described is the provision in the programmable generator of an impedance transformer connected intermediate the emitter of low energy electromagnetic emissions and a probe (here more broadly described as an electrically conductive applicator) for applying the emissions to the patient. The impedance transformer substantially matches the impedance of the patient seen from the emitter circuit with the impedance of the output of the emitter circuit. The Hdp monitoring system is a currently available and patented medical device with different brands and used in different applications that is integrated in the present invention. The Hdp monitoring system is necessary for measurement and record of Hdp values used by the processing system for SFq identification. The identified SFq is used for diagnosis and treatment of a health condition of a patient. The system integrates the Hdp monitoring system that measures and records various identified Hdp values of the patient. The system generally measures various identified Hdp values of the patient utilizing electrodes placed in topical contact with various determined parts of the body. The Hdp monitoring system further comprises recording means that records the various identified Hdp values of the patient. The recording means can utilize any storage device on which the various identified measured Hdp values of the patient can be recorded. The storage of measured and recorded Hdp in accordance with the invention, that a variety of Hdp values need to be measured and recorded, which include the values of at least the following nine Hdp's:

- RR interval (interval from the R peak to the next as shown on an electrocardiogram (ECG) (RRI);
- heart rate (HR);
- systolic blood pressure (sBP);
- diastolic blood pressure (dBP);
- median blood pressure (mBP);
- pulse pressure (PP);
- stroke volume (SV);
- cardiac output (CO); and
- total peripheral resistance (TPR).

The Hdp values are measured and recorded following established procedures. Initial measurements during non-exposure high specific frequency radio frequency carrier signals periods in an individual or patient or Hdp values thereof are herein named basal measurements or basal Hdp values. In terms of procedure, initial measurements of above parameters are performed on warm-blooded mammalian subjects after a period of relaxation, for example about 15 minutes, while the patient is lying in a supine position (face and preferably also palms of the hands facing upwardly) or in other comfortable and relaxed position.

Following on having performed the above initial measurements, the diagnosed or pre-diagnosed warm-blooded mammalian subjects are exposed to or application of the above described procedures, i.e. diagnosis, searching for SFq, discovering new SFq, treatment's follow-up involving exposure to or application of selected series of one or more high specific frequency radio frequency carrier output signals, thereof are herein named exposure measurements or exposure Hdp values.

The above-mentioned one or more high specific frequency radio frequency carrier signals are electromagnetic field frequency (EMF) output signals may be produced by a loaded control program into the programmable generator capable to generate EMF output signals at certain predetermined amplitude modulation (AM) frequencies. The subjects or patients are most preferably exposed to or the EMF output signals are applied to patients during heart-beat times over a determined period of time, most preferably over the time of at least ten heart-beat times of the patient or a period of at least 10 seconds. In some embodiments, the subjects or patients are exposed to or the EMF output signals are applied to patients over the time of at least three heart-beat times of the patient or a period of at least 3 seconds. This procedure is part of the integrated solution of the invention and it would in general take place while the patient remains connected to or is reconnected to both synchronized Hdp monitoring system and programmable generator of the system of the invention so that Hdp values may be measured and recorded during the period of exposure or application. The Hdp values may, however, also or alternatively be the data source to identify SFq and/or representative Hdp variation values after software processing described above.

The above Hdp values measured during or after above exposures or applications to subjects or patients are herein referred to as exposure or exposure Hdp values and post-exposure or post-exposure Hdp values, respectively.

The procedures above, in general as applied to multiple patients pre-diagnosed or diagnosed patients with known health conditions to be suffering from an identified form of poor health condition, provide multiple basal Hdp values, multiple exposure Hdp values and multiple post-exposure Hdp values as related to the identified pre-diagnosed or diagnosed form of poor health condition. These multiple Hdp values, for example for most if not all of the nine Hdp parameters listed above, may in general be somewhat scattered values. Accordingly, for purposes of defining representative Hdp values, such scattered values would regularly be submitted to the processing system that integrates multiple mathematical calculations for purposes of identifying SFq and representative Hdp variation values.

In line with above, the Hdp monitoring system is only part of the present invention, provides means for software processing Hdp values for use in the identification of SFq and the diagnosis of health conditions of a patient. The identification of SFq and representative Hdp variation values are termed representative surrogate markers that are determined through the processing system from the Hdp monitoring system that measures and records Hdp values performed during non-exposure and exposure periods on patients pre-diagnosed and diagnosed to either healthy or suffering from a known form of poor health condition.

Representative surrogate markers employed for diagnosis, searching for SFq, treatment and treatment's follow-up purposes, in terms of the present invention, are derived from computative combinations of information from both representative basal Hdp measured values, representative exposure Hdp measured values and representative post-exposure Hdp values. Since the exposure EMF frequencies employed for influencing Hdp values are different for each health condition and post-exposure Hdp values are similarly different, the computative combinations for deriving representative surrogate markers for a specified health condition requires different computations uses discriminant analysis and well-established statistical methods for ideal threshold values determination.

The reliability of representative surrogate markers values is of course dependent on the number of pre-diagnosed and diagnosed surrogates included for each type of poor health condition examined. Thus, the incidence of poor health conditions among populations, more particularly high incidence poor health conditions that are difficult to diagnose, such as Hepatocellular Carcinoma (HCC) or related liver diseases, has received particular attention. Similarly, the relatively high incidence of breast cancer has thus far also received particular attention, as reported below.

Post-exposure Hdp measured values, insofar as may be reflected following on exposure to or application of low energy EMF carrier signals, in terms of the invention, may be compared with Hdp values which occur following on exposure or application to a patient of predetermined EMF frequency values pre-determined to alleviate a cause of a specified poor health condition of a patient. Matching basal, exposure and post-exposure Hdp values, on their own, or after using the processing system may support the efficacy of treatment by application of said predetermined EMF carries signals and provide a preliminary indication of diagnosis of the health condition of the patient. Reference to the further scientific details related, for example, specifically to two different forms of cancer diagnoses are described below. Here, mention is made to patient's diagnosis following on the basal non-exposure period and correlations of patient's diagnosis with hemodynamic patterns in male HCC and female breast cancer in comparison with healthy controls. Similarly, mention is made to tumor-specific hemodynamic response pattern during exposure periods.

The time periods of exposure or application of EMF frequency output signals by means of a variable frequency programmable generator device within a broad range of frequencies; for example, EMF frequencies within a range between from about 0.01 to about 150 MHz, may require a short period of time for Hdp values to be varied at any particular frequency value. Thus, consecutive exposures or applications of sections of the range of EMF frequencies may be required in order to identify EMF frequency values at which basal, exposure and post-exposure Hdp value variations actually occur during the heart-beat times at which Hdp values are, measured and recorded by the Hdp monitoring system and processed by the processing system.

The system of the invention includes, besides programmable generator of EMF frequency output signal, the processing system and the ILf central server, the output signal frequency measurement and recording means for measuring and recording such frequency values at which frequencies Hdp variances of at least certain of the Hdp values are exhibited, herein threshold values. Similarly, Hdp value recording means for recording each of the measured values for each of the identified Hdp values, preferably separately of one another, measured and recorded before, during or after the period of time of exposure to or application of output signals to the patient.

A further component of the integrated invention, additional to those described above, is the processing system component that may be integrated with or coupled to the recording means for recording Hdp values before, during or after performing or exposing the patient to a cellular excitation procedure. The processing system component may include program-controlled calculation means for performing a series of mathematical analysis of various of the recorded Hdp values to obtain representative surrogate values, such as the identification of SFq and representative Hdp variation values, for each of the different recorded Hdp values, optionally making a determination of ratios between different representative Hdp values, and comparing either or both of such representative values or ratios between different values, with predetermined representative values or ratios (threshold values) characteristics of a SFq and/or representative Hdp variation value changes while exposing the patient to a cellular excitation procedure, predetermined in patients known to be healthy or known to be suffering from or likely to develop an identified poor health condition. The comparison of calculated representative surrogate values, such as recorded Hdp values or ratios and identified SFq and/or representative Hdp variation values in patients diagnosed with same health condition, which match with predetermined representative Hdp values or ratios and/or identified SFq and/or representative Hdp variation values, leads to providing an indication of a diagnosis of a health condition of a patient.

The processing system component may, alternative or additional to being integrated or coupled to the recording means as described above, be located at a central server connected to the invention by a protected web platform, which may perform the analysis based on recorded Hdp information, received or communicated to the center.

The exposure of identified SFq has demonstrated biological activity and supports its use as a novel treatment modality.

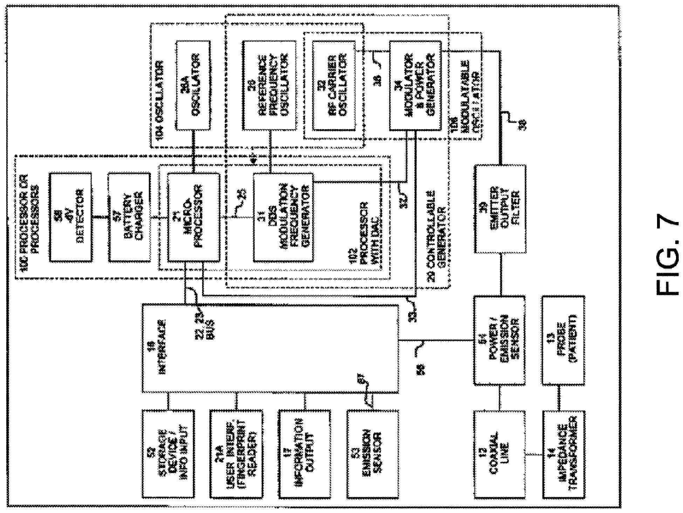

FIG. 7 depicts an illustrative schematic structure for an integrated medical system according to an embodiment. As shown in FIG. 7, a system for diagnosing a health condition of a patient integrates an Hdp monitoring system and a frequency generator. In an embodiment, the system may further include a processing system.

The Hdp monitoring system determines a cardiovascular performance reserve for each individual patient. In an embodiment, the Hdp monitoring system may receive input physiological data from a patient. The input physiological data may be used to obtain a parameter Z which is or approximates a product of the patient's Stroke Volume (SV) and the patient's Systemic Vascular Resistance (SVR). The Hdp monitoring system may further provide a value representing the Respiratory Rate (RR) of the patient. The RR value may be determined by one or more of a measurement using a dedicated device, a calculation performed using the input physiological data or manually by using best estimate, such as making an estimate based on the heart rate of the patient

A modulated low energy electromagnetic emission application system generator may be used to emit low energy radio frequency (RF) electromagnetic waves to a warm-blooded mammalian subject. The low energy RF electromagnetic waves may be used to treat a warm-blooded mammalian subject suffering some limited number of described health conditions. The system described herein integrates and synchronizes the Hdp monitoring system and the generator via the processing system, which may further be connected to a central server by a protected web based platform.

The system described herein may be an integrated solution having a patient side component and a server side component. The patient side component may include the Hdp monitoring system connected to the programmable generator. Both the Hdp monitoring system and the programmable generator may be connected to the processing system to enable synchronization of the devices and allow for compatible data aggregation. The central server side component may be connected with the patient side component via the protected web based platform and may provide artificial intelligence based computation and data storage.

In certain implementations, the radiofrequency generator can be configured to measure and store reflection power values during exposure that are synchronized with Hdp monitoring values for further artificial intelligence-based computation and data storage as described in additional detail below.

The two components of the system may enable bidirectional data transfer in real-time as described above. For example, once the programmable generator is loaded with one or more control programs of selected series of SFq, the programmable generator may be disconnected from the integrated solution to enable outpatient use. The programmable generator may be reconnected in the integrated solution to permit batch upload of update data and to allow automatic treatment profiling to be transferred back to the processing system.

Referring to FIG. 7, the new medical device related to the invention is the integration of other medical devices. The Hdp monitor provides a method for determining a cardiovascular performance reserve for each individual patient, comprising the steps of: a) receiving input physiological data from the patient for obtaining a parameter Z which is or approximates the product of the Stroke Volume (SV) by the Systemic Vascular Resistance (SVR); b) providing a value representing the Respiratory Rate (RR) of said patient, wherein the Respiratory Rate (RR) value is provided by measurements using dedicated device(s), calculations from the input physiological data or manually by using best estimate, as described in U.S. Patent Application Publication No. 2015/0005647 c) Electrocardiography (ECG) and photoplethysmography (PPG), where ECG measures the bio-potential generated by electrical activity of the heart and PPG senses the rate of blood flow, d) Heart rate variability (HRV) that is oscillation in consecutive cardiac cycles.

Electromagnetic emission application system 11 generator, relates to the practice of emissions of low energy radio frequency (RF) electromagnetic waves to a warm-blooded mammalian subject for treating a warm-blooded mammalian subject suffering some limited number of described health conditions as described in prior U.S. Patent Nos. 4,649,935 and 4,765,322. The new device related to the invention integrates and synchronizes both medical devices by the processing system that connects the new device to a central server by protected web based platform.

Referring to FIG. 7, the new medical device may be an integrated solution with two components: the patient side component and the central server side component. The patient side component may include the Hdp monitoring system connected to the programmable generator, both connected to the processing system in the integrated hardware to make synchronization and compatible data aggregation. The central server side component is connected with the patient side component by protected web based platform and provides artificial intelligence based computation and data storage.

Referring to FIG. 7, the two components of the new medical device related to the invention are real-time connected to bidirectional data transfer as described above. The programmable generator once loaded with control programs of selected series of SFq can be disconnected from the integrated solution in the invention for outpatient use. The programmable generator can be reconnected in the integrated solution in the invention for batch upload of update data and automatic treatment profiling transfer back to the processing system.

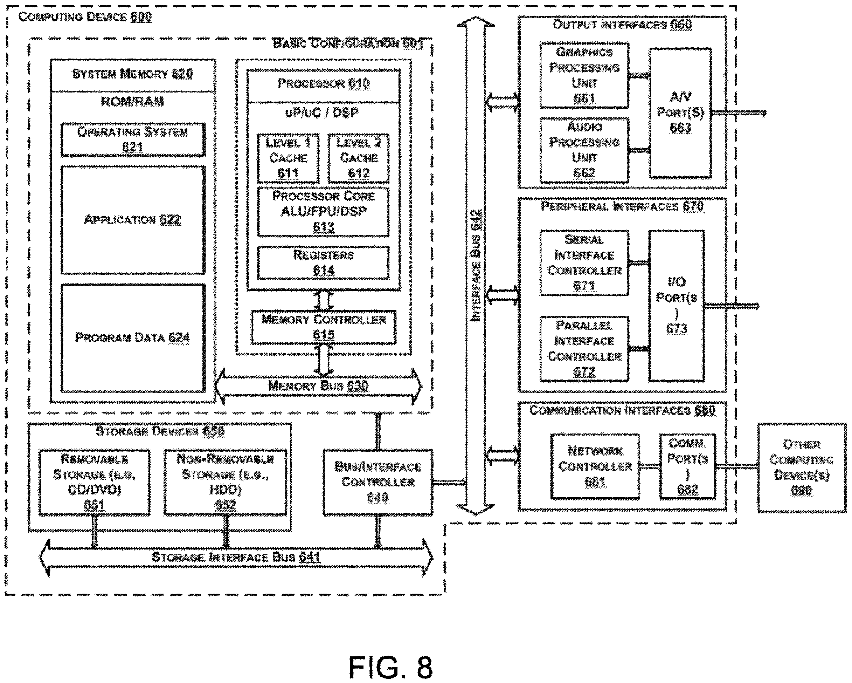

The system 11 includes an electrically conductive applicator 12, 13 for applying one or more electromagnetic emissions to the warm-blooded mammalian subject. There are a number of different forms of applicators that may consist of an electrically conductive probe 13 which has a close contact with a subject undergoing treatment. Probe 13 is connected to an electromagnetic energy emitter (see also FIG. 8), through coaxial cable 12 and impedance matching transformer 14.

Electronic system 11 also includes a connector or coupler for connection to a programmable device such as a computer or an interface or receiver 16 which is adapted to receive an application storage device 52 such as, for example, magnetic media, semiconductor media, optical media or mechanically encoded media, or programmed emissions programmed with control information employed to control the operation of system 11 so that the desired type of low energy emission therapy is applied to the patient.

Application storage device 52 can be provided with a microprocessor which, when applied to interface 16, operates to control the function of system 11 to apply the desired low energy emission therapy. The application storage device 52 is provided with a microprocessor which is used in combination with microprocessor 21 within system 11. In such case, the microprocessor within device 52 assists in the interfacing of storage device 52 with system 11 and other central servers.

System 11 may also include a display 17 which can display various indications of the operation of system 11. In addition, system 11 may include on and off power buttons 18 and 19, optionally replaced by user interface 21A (refer to FIG. 8).

FIG. 8 depicts an illustrative block diagram of an Hdp monitoring system according to an embodiment. The system includes a computing device 600. The computing device may include various additional components such as basic configuration 601, a bus/interface controller 640, storage devices 650, output devices 660, peripheral devices 670, communication interfaces 680, and/or other computing devices 690. One or more electrical busses may be configured to operably connect the above-identified components. For example, storage interface bus 641 may be configured to operably connect the storage devices 650 and the bus/interface controller 640. Additionally, an interface bus 642 may be configured to operably connect the bus/interface controller 640 with the output interfaces 660, the peripheral interfaces 670, and the communication interfaces 680.

The basic configuration 601 may include a processor 610, a system memory 620, and a memory bus 630 configured to operably connect the processor and the system memory. In some examples, the processor 610 may include a level 1 cache 611, a level 2 cache 612, a processor core 613, one or more registers 614, and a memory controller 615. In some implementations, the system memory 620 may include various software or operating modules such as an operating system 621, one or more applications 622, and program data 624.

In some examples, the storage devices 650 may include a removable storage device 651 including, for example, a USB storage device or other similar removable media. The storage devices 650 may also include a non-removable storage device 652 such as a hard disk drive. In some implementations, the output interfaces 660 may include a graphics processing unit 661, an audio processing unit 662, an one or more A/V ports 663 operably connected to the graphics processing unit and the audio processing unit. In some examples, an external output device such as a monitor or other similar display and/or a speaker or other similar audio output device can be operably connected to the A/V ports 663.

In some examples, the peripheral interfaces 670 may include a serial interface controller 671, a parallel interface controller 672, and one or more I/O ports 673 operably connected to the serial interface controller 671 and the parallel interface controller 672. In some examples, an external device such as a printing device may be operably connected to the computing device 600 via the one or more I/O ports 673. In some implementations, the communication interfaces 680 may include a network controller 681 configured to facilitate communication with the other communication devices 690. In some examples, the network controller 681 may be operably connected to one or more communication ports 682 for establishing communications with the other communication devices 690. For example, the established communications can be via a wired or wireless data communication link.

In some implementations, the system as illustrated in FIG. 8 may be configured to measure Hdp values before, during and/or after application of electromagnetic output signals to a patient. In an embodiment, circuitry may be provided with a connector configured to connect with the Hdp monitoring system. Alternatively, the circuitry may be integrated into the Hdp monitoring system. Descriptions of each of the blocks of the block diagram or functions thereof are included to facilitate an understanding thereof.

The block diagram of electronic circuitry of the Hdp monitoring system applies AM RF output signals to a patient at predetermined selected AM frequencies. The predetermined selected frequencies are controlled by AM frequency values stored in the storage device 52 and/or other servers. Various predetermined selected AM frequencies applied to a patient are indicated for treatment of patients suffering from a poor health condition for which the patient has been diagnosed.

In an embodiment, an integrated or combined device may enable sensing of Hdp values and reflection power energy of a patient prior to, during or after application of AM RF electromagnetic signals or other such signals. Of particular interest in this regard is that the measured and recorded Hdp and reflection power energy values may differ dependent upon the patient condition. For example, measured and recorded Hdp and reflection power energy values may differ among patients suffering from different forms of cancer. In addition, the measured and recorded Hdp and reflection power energy values may differ from patients suffering from a form of cancer and healthy patients. However, such Hdp and reflection power energy values may be substantially similar for patients suffering from the same or a closely related poor health condition. The measured and recorded representative Hdp and reflection power energy variation values and the identification of SFq accordingly offer diagnosis and treatment opportunities for various forms of poor health conditions. In addition, such Hdp variation values may permit the diagnosis of healthy patient conditions.

Referring back to FIG. 7, here describing exclusively the programmed generator portion of the invention, the microprocessor 21 operates as the controller for the application system and is connected to control the various components of the system through address bus 22, data bus 23 and input/output (I/O) lines 25. Microprocessor 21 preferably includes internal storage for the operation code, control program, and temporary data. In addition, microprocessor 21 includes input/output (I/0) ports and internal timers. Microprocessor 21 may be, for example, an 8-bit single-chip microcontroller, 8048 or 8051 available from Intel Corporation of Santa Clara, California. The timing for microprocessor 21 is provided by system clock 24 which includes a clock crystal 26 along with capacitors 27 and 28. System clock 24 may run at any clock frequency suitable for the particular type of microprocessor used. In accordance with one embodiment, system clock 24 operates at a clock frequency of 8.0 MHz.

In general, microprocessor 21 functions to control controllable electromagnetic energy generator circuit 29 to produce a desired form of modulated low energy electromagnetic emission for application to a patient through probe 13. Controllable generator circuit 29 includes modulation frequency generator circuit 31 and carrier signal oscillator 32. Microprocessor 21 operates to activate or de-activate controllable generator circuit 29 through oscillator disable line 33. Controllable generator circuit 29 also includes an AM modulator and power generator 34 which operates to amplitude modulate a carrier signal produced by carrier oscillator 32 on carrier signal line 36, with a modulation signal produced by modulation signal generator circuit 31 on modulation signal line 37. Modulator 34 produces an amplitude modulated carrier signal on modulated carrier signal line 38, which is then applied to the filter circuit 39. The filter circuit 39 is connected to probe 13 via coaxial cable 12 and impedance transformer 14.

Microprocessor 21 controls modulation signal generator circuit 31 of controllable generator circuit 29 through address bus 22, data bus 23 and I/O lines 25. In particular, microprocessor 21 selects the desired waveform stored in modulation waveform storage device 43 via I/O lines 25. Microprocessor 21 also controls waveform address generator 41 to produce on waveform address bus 42 a sequence of addresses which are applied to modulation signal storage device 43 in order to retrieve the selected modulation signal. The desired modulation signal is retrieved from wave form look-up table 43 and applied to modulation signal bus 44 in digital form. Modulation signal bus 44 is applied to digital to analog converter (DAC) 46 which converts the digital modulation signal into analog form. This analog modulation signal is then applied to selective filter 47 which, under control of microprocessor 21, filters the analog modulation signal by use of a variable filter network including resistor 48 and capacitors 49 and 51 in order to smooth the wave form produced by DAC 46 on modulation signal line 20.

In the present embodiment, the various modulation signal wave forms are stored in look-up table 43. In an embodiment, a look-up table 43 may contain up to 8 different modulation signal wave forms, although more or fewer may be stored in the lookup table. Wave forms which have been successfully employed include square wave forms or sinusoidal wave forms. Other possible modulation signal wave forms include rectified sinusoidal, triangular, and combinations of all of the above.

In an embodiment, each modulation signal wave form uses 256 bytes of memory and is retrieved from look-up table 43 by running through the 256 consecutive addresses. It is noted that each wave form may use more or fewer bytes of memory within the scope of this disclosure as will be apparent to one of ordinary skill in the related art. The frequency of the modulation signal is controlled by how fast the wave form is retrieved from look-up table 43. In an embodiment, this is accomplished by downloading a control code from microprocessor 21 into programmable counters contained within wave form address generator 41. The output of the programmable counters then drives a ripple counter that generates the sequence of addresses on the wave form address bus 42.

Waveform address generator 41 may, for example, be a programmable timer/counter uPD65042C, available from NEC. Modulation signal storage device or look-up table 43 may, for example, be a type 28C16 Electrical Erasable Programmable Read Only Memory (EEPROM) programmed with the desired wave form table. Digital to analog converter 46 may, for example, be a DAC port, such as AD557JN available from Analog Devices, and selective filter 47 may be a type 4052 multiplexer available from National Semiconductor or Harris Semiconductor. Additional or alternate components may be used within the scope of this disclosure.

The particular modulation control information used by microprocessor 21 to control the operation of controllable generator circuit 29, in accordance with the present invention, is stored in application storage device 52 or, in terms of the present invention may be a variable AM frequency tuning device adapted to load the interface 16 with AM frequencies between and high and low frequency levels. Application storage device 52 may be any storage device capable of storing information for later retrieval. Application storage device 52 is connected to the processing system by interface 16 to complete the integrated solution in the invention.

It should be emphasized that although the Figures illustrate microprocessor 21 separate from application storage device 52, microprocessor 21 and application storage device 52 where loaded control programs from the processing system are stored into the programmable generator. The control programs once loaded into the system control the operation of the system as described herein. In this case, interface 16 would exist between the combination of microprocessor 21 and application storage device 52 and the rest of the system.

Interface 16 is configured as appropriate for the particular application storage device 52 in use. Interface 16 translates the control information stored in application storage device 52 into a usable form for storage within the memory of microprocessor 21 to enable microprocessor 21 to control controllable generator circuit 29 to produce the desired modulated low energy emission. Interface 16 may directly read the information stored on application storage device 52, or it may read the information through communication link with the processing system. When application storage device 52 and microprocessor 21 are incorporated in the same device, interface 16 is configured to connect microprocessor 21 to the rest of system.

The control information stored in application storage device 52 specifies various controllable parameters of the modulated low energy RF electromagnetic emission which is applied to a patient through probe 13. Such controllable parameters include, for example, the frequency and amplitude of the carrier, the amplitudes and frequencies of the modulation of the carrier, the duration of the emission, the power level of the emission, the duty cycle of the emission (i.e., the ratio of on time to off time of pulsed emissions applied during an application), the sequence of application of different modulation frequencies for a particular application, and the total number of treatments and duration of each treatment prescribed for a particular patient.

For example, the carrier signal and modulation signal may be selected to drive the probe 13 with an amplitude modulated signal in which the carrier signal includes spectral frequency components below 1 GHz, and preferably between 1 MHz and 900 MHz, and in which the modulation signal comprises spectral frequency components between about 0.1 Hz and about 10 MHz, between about 1 Hz and about 150 KHz, between about 0.01 Hz and about 1,000 Hz, or between about 0.01 Hz and about 2,000 Hz. In an embodiment, one or more modulation frequencies may be sequenced to form the modulation signal.

As an additional feature, an electromagnetic emission sensor 53 may be provided to detect the presence of electromagnetic emissions at the frequency of the carrier oscillator 32. Emission sensor 53 provides to microprocessor 21 an indication of whether or not electromagnetic emissions at the desired frequency are present. Microprocessor 21 then takes appropriate action, for example, displaying an error message on information output display 17, disabling controllable generator circuit 29, or the like.

The system may further include a power sensor 54, which detects the amount of power applied to the patient through probe 13 compared to the amount of power returned or reflected from the patient. This ratio is indicative of the proper use of the system during a therapeutic session. Power sensor 54 applies to microprocessor 21 through power sense line 56 an indication of the amount of power applied to patient through probe 13 relative to the amount of power reflected from the patient.

The indication provided on power sense line 56 may be digitized and used by microprocessor 21, for example, to detect and control a level of applied power, and to record on application storage device 52, information related to the actual treatments applied. Data transfer information to the processing system may include, for example: the number of treatments applied for a given time period; the actual time and date of each treatment; the number of attempted treatments; the treatment compliance (i.e., whether the probe was in place or not in place during the treatment session); and the cumulative dose of a particular modulation frequency.

The level of power applied is preferably controlled to cause the specific absorption rate (SAR) of energy absorbed by the patient to be from 1 microwatt per kilogram of tissue to 50 Watts per kilogram of tissue. Preferably, the power level is controlled to cause an SAR of from 100 microwatts per kilogram of tissue to 10 Watts per kilogram of tissue. Most preferably, the power level is controlled to deliver whole body mean SAR in the range of only 0.2 to 1mW/kg, with a 1g peak spatial SAR between 150 and 350mW/kg. These SARs may be in any tissue of the patient. The system also includes powering circuitry including battery and charger circuit 57 and battery voltage change detector 58.

In the integrated solution, combination or use of two medical devices of the nature described above but executing different and synchronized tasks that derivate from their initial conception and application such as measuring and recording Hdp values, at least nine parameter values mentioned, before, during or after exposure or application of EMF frequency output signals and identifying representative Hdp variation values and SFq have provided a scientific and reproducible method of diagnosing and treating health conditions of a patient, further scientific details related, for example, specifically to one different form of cancer diagnoses are provided below:

The identification of changes in pulse amplitude in patients with a diagnosis of cancer when exposed to low and safe levels of 27.12 MHz radiofrequency electromagnetic fields amplitude-modulated at specific frequencies have previously been reported. (Barbault, A. et al., Amplitude-modulated electromagnetic fields for the treatment of cancer: discovery of tumor-specific frequencies and assessment of a novel therapeutic approach, J. Exp. Clin. Cancer Res. 28, 51, doi:10.1186/1756-9966-28-51 (2009)). The observation that changes in pulse amplitude occur at exactly the same frequencies in patients with the same type of cancer led to a hypothesis that each type of cancer possesses a specific frequency signature. (*Id.*) In vitro experiments have shown that tumor-specific frequencies have anti-proliferative effects on cancer cells, modulate the expression of genes involved in cell migration and invasion, and are capable of disrupting the mitotic spindle. (Zimmerman, J. W. et al., Cancer cell proliferation is inhibited by specific modulation frequencies, British Journal of Cancer 106, 307-313 (2012)). The clinical activity of these tumor-specific frequencies was assessed in two separate studies in which patients were treated with intrabuccally administered AM RF EMF, which were modulated at tumor-specific frequencies. Antitumor activity was observed in patients with metastatic breast cancer (Barbault, A. et al. (2009)) and advanced hepatocellular carcinoma (Costa, F. P. et al., Treatment of advanced hepatocellular carcinoma with very low levels of amplitude-modulated electromagnetic fields, British Journal of Cancer 105, 640-648 (2011)) and stable disease was observed in patients with other tumor types.

This study was designed to test the hypothesis that analysis of changes in Hdp upon exposure to tumor-specific frequencies is a novel, non-invasive diagnostic approach in warm-blooded mammalian subjects. The method is also capable of identifying SFq to be used in the treatment of health condition in patients.

The experimental procedures described below were reviewed and approved by the Hospital Sírio Libanês Institutional Review Board (IRB), Rua Dona Adma Jafet,50 Conj.41/43, São Paulo SP 01.308-050 Brazil. All patients and healthy individuals enrolled in this study signed an informed consent, which was approved by the IRB. The protocol was registered prior to enrolment of the 1st patient: clinicaltrial.gov identified no. NCT 01686412. 87 individuals were screened, and 82 individuals were prospectively enrolled. The patient's diagnosis and the nature of AM RF EMF exposure (HCC-specific, breast cancer specific, and randomly chosen frequencies) were disclosed before computational analysis in order to constitute a knowledge base. The validation group included patients with biopsy-proven cancer (advanced HCC and advanced breast cancer), and healthy controls. The last group included patients with potentially resectable HCC.

The AM RF EMF device used for this study has been described in detail previously. (Costa, F. P. et al., British journal of cancer 105, 640-648 (2011)). While patients receiving treatment with AM RF EMF are exposed to one, two, or three daily treatments of about 35 minutes to about 1 hour, the diagnostic feasibility of AM RF EMF administration was tested during a single exposure of about 10 minutes or about 35 minutes in order to expose all individuals once to each of the 194 tumor-specific frequencies (HCC specific and breast cancer specific), which are each emitted for about three or about ten seconds. (Barbault, A. et al., J. Exp. Clin. Cancer Res. 28, 51, doi:10.1186/1756-9966-28-51 (2009); Zimmerman, J. W. et al., British Journal of Cancer 106, 307-313 (2012); Costa, F. P. et al., British Journal of Cancer 105, 640-648 (2011)). Similarly, 194 of the previously reported 236 randomly chosen frequencies were selected (Zimmerman, J. W. et al., British Journal of Cancer 106, 307-313 (2012)) to match the number and exposure duration of tumor-specific frequencies. Hence, each individual was exposed to all frequencies included in each of the treatment programs (HCC specific, breast cancer specific, and randomly chosen frequencies). Each modulation frequency was emitted for about three seconds from the lowest to the highest frequency as previously described. ((Barbault, A. et al., J. Exp. Clin. Cancer Res. 28, 51, doi:10.1186/1756-9966-28-51

(2009); Zimmerman, J. W. et al., British journal of cancer 106, 307-313 (2012); Costa, F. P. et al., British journal of cancer 105, 640-648 (2011)).

Various examples of using the above system for recording and analyzing Hdp values are described in U.S. Patent Application No. 15/844,214 filed December 15, 2017 and entitled "System for Characterization, Diagnosis, and Treatment of a Health Condition of a Patient and Methods of Using Same,: the content of which is incorporated herein by reference in its entirety.

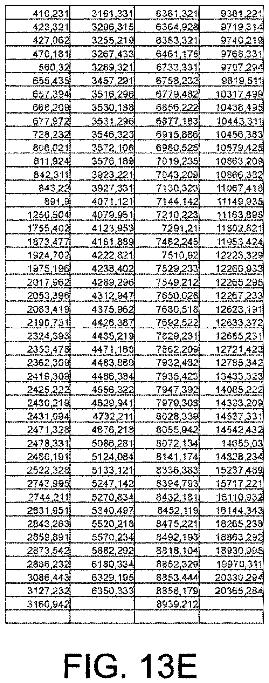

In certain applications of the systems and techniques as described herein, application of a specific frequency signal or set of frequencies can be beneficial in both providing a prognostic and predictive benefit for a patient as well as providing a treatment to the patient. For example, when treating particular types of cancer such as HCC, application of a series of specific electromagnetic frequencies (e.g., a set of 194 specifically selected frequencies as shown in FIG. 13E), or a series of electromagnetic frequencies that occur every 3 Hz or 10 Hz in a range from about 0.01 Hz to about 20 KHz, or in a range from about 0.01 Hz to about 2 KHz, can be therapeutic in nature as well as provide prognostic and predictive information (by, for example, measuring patient HRV response to application of the frequencies). Additionally information can be measured as well such as amount of energy absorbed by the patient, amount of energy reflected by the patient, and other similar measurable quantities that can be recorded and analyzed to provide further information as described below.

As noted above, in order to administer the selected frequencies to a patient, a carrier signal can be used. For example, oscillator 302 can be configured to produce a carrier signal having a specific frequency such as 27.12 MHz.

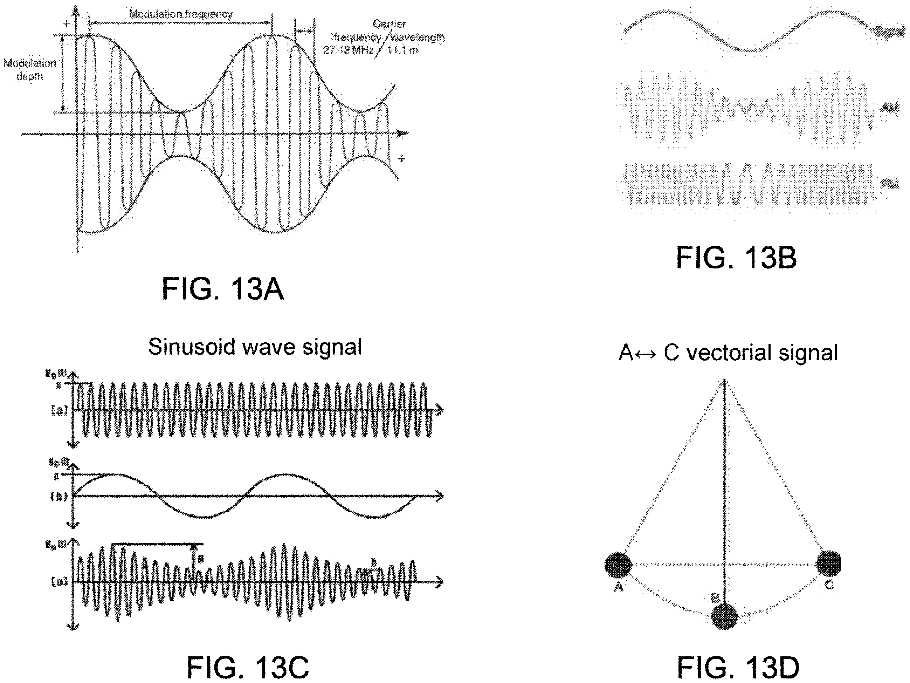

However, both modulating the amplitude of the carrier wave, a modulated signal can be produced at, for example, each of the 194 frequencies, as shown in FIG. 13E, or a series of electromagnetic frequencies that occur every 3 Hz or 10 Hz in a range from about 0.01 Hz to about 20 KHz, in a range from about 10 Hz to about 1,000 Hz, or in a range from about 10 Hz to about 2,000 Hz. In some embodiments, a modulated signal can be produced at a series of electromagnetic frequencies that occur every 3 Hz in a range from about 0.01 Hz to about 20 KHz. In some embodiments, a modulated signal can be produced at a series of electromagnetic frequencies that occur every 3 Hz in a range from about 10 Hz to about 1,000 Hz, or in a range from about 10 Hz to about 2,000 Hz. In some embodiments, a modulated signal can be produced at a series of electromagnetic frequencies that occur every 10 Hz in a range from about 0.01 Hz to about 20 KHz. In some embodiments, a modulated signal can be produced at a series of electromagnetic frequencies that occur every 10 Hz in a range from about 10 Hz to about 1,000 Hz, or in a range from about 10 Hz to about 2,000 Hz. In some embodiments, a modulated signal can be produced at a series of electromagnetic frequencies as illustrated in table 1 below. In yet other embodiments, a modulated signal can be produced at a series of electromagnetic frequencies that occur about every 3 Hz in a range from about 0.01 Hz to about 20 KHz. In some embodiments, a modulated signal can be produced at a series of electromagnetic frequencies that occur about every 3 Hz in a range from about 10 Hz to about 1,000 Hz, or in a range from about 10 Hz to about 2,000 Hz. In some embodiments, a modulated signal can be produced at a series of electromagnetic frequencies that occur about every 10 Hz in a range from about 0.01 Hz to about 20 KHz. In some embodiments, a modulated signal can be produced at a series of electromagnetic frequencies that occur about every 10 Hz in a range from about 10 Hz to about 1,000 Hz, or in a range from about 10 Hz to about 2,000 Hz. In some embodiments, a modulated signal can be produced at a series of electromagnetic frequencies as illustrated in table 1 below.

Table 1 (in Hz)

| 10 | 76 | 142 | 208 | 274 | 340 | 406 | 472 | 538 | 604 | 670 | 736 | 802 | 868 | 934 | 1000 |
|---|---|---|---|---|---|---|---|---|---|---|---|---|---|---|---|
| 13 | 79 | 145 | 211 | 277 | 343 | 409 | 475 | 541 | 607 | 673 | 739 | 805 | 871 | 937 | |
| 16 | 82 | 148 | 214 | 280 | 346 | 412 | 478 | 544 | 610 | 676 | 742 | 808 | 874 | 940 | |
| 19 | 85 | 151 | 217 | 283 | 349 | 415 | 481 | 547 | 613 | 679 | 745 | 811 | 877 | 943 | |
| 22 | 88 | 154 | 220 | 286 | 352 | 418 | 484 | 550 | 616 | 682 | 748 | 814 | 880 | 946 | |
| 25 | 91 | 157 | 223 | 289 | 355 | 421 | 487 | 553 | 619 | 685 | 751 | 817 | 883 | 949 | |
| 28 | 94 | 160 | 226 | 292 | 358 | 424 | 490 | 556 | 622 | 688 | 754 | 820 | 886 | 952 | |
| 31 | 97 | 163 | 229 | 295 | 361 | 427 | 493 | 559 | 625 | 691 | 757 | 823 | 889 | 955 | |
| 34 | 100 | 166 | 232 | 298 | 364 | 430 | 496 | 562 | 628 | 694 | 760 | 826 | 892 | 958 | |
| 37 | 103 | 169 | 235 | 301 | 367 | 433 | 499 | 565 | 631 | 697 | 763 | 829 | 895 | 961 | |
| 40 | 106 | 172 | 238 | 304 | 370 | 436 | 502 | 568 | 634 | 700 | 766 | 832 | 898 | 964 | |
| 43 | 109 | 175 | 241 | 307 | 373 | 439 | 505 | 571 | 637 | 703 | 769 | 835 | 901 | 967 | |
| 46 | 112 | 178 | 244 | 310 | 376 | 442 | 508 | 574 | 640 | 706 | 772 | 838 | 904 | 970 | |
| 49 | 115 | 181 | 247 | 313 | 379 | 445 | 511 | 577 | 643 | 709 | 775 | 841 | 907 | 973 | |
| 52 | 118 | 184 | 250 | 316 | 382 | 448 | 514 | 580 | 646 | 712 | 778 | 844 | 910 | 976 | |
| 55 | 121 | 187 | 253 | 319 | 385 | 451 | 517 | 583 | 649 | 715 | 781 | 847 | 913 | 979 | |
| 58 | 124 | 190 | 256 | 322 | 388 | 454 | 520 | 586 | 652 | 718 | 784 | 850 | 916 | 982 | |
| 61 | 127 | 193 | 259 | 325 | 391 | 457 | 523 | 589 | 655 | 721 | 787 | 853 | 919 | 985 | |
| 64 | 130 | 196 | 262 | 328 | 394 | 460 | 526 | 592 | 658 | 724 | 790 | 856 | 922 | 988 | |
| 67 | 133 | 199 | 265 | 331 | 397 | 463 | 529 | 595 | 661 | 727 | 793 | 859 | 925 | 991 | |
| 70 | 136 | 202 | 268 | 334 | 400 | 466 | 532 | 598 | 664 | 730 | 796 | 862 | 928 | 994 | |
| 73 | 139 | 205 | 271 | 337 | 403 | 469 | 535 | 601 | 667 | 733 | 799 | 865 | 931 | 997 | |

In certain implementations, the signal as shown in FIG. 13A may be representative of an AM RF EMF signal produced by modulating the amplitude of the carrier signal. As shown in FIG. 13A, by modulating the amplitude of the signal, varying modulation depths can be achieved, thereby producing a modulated signal having a specific modulation frequency. However, as further shown in FIG. 13A, during the amplitude modulation, the frequency of the carrier signal remains unchanged, e.g., 27.12 MHz in this example.

For the precise measurement of HRV, in order to identify the biological surrogate in humans, a highly precise and integrated system capable to identify signals in time-domain non-linear systems in very short period intervals. For example, a Real-time Orchestrated Low-energy Electromagnetic Exposure (R.O.L.E.X.) system such as system 900 as shown in FIG. 9B can synchronize a monitoring device 902, a radiofrequency generator 904, a database 908 configured to store the dynamic data library for hemodynamic recording and data processing in a precise, reliable and reproducible way, and a computing device configured to store and execute the Artificial Intelligence computing algorithms and processing techniques.

In certain implementations, and considering that all hemodynamic parameters are recorded for every heartbeat (beat-to-beat) and the frequency modulation exposure time is measured in a few seconds, a program orchestrator 906 can be configured to controlled the monitor 902 and the frequency generator 904 as well as to send intervention signals to the monitor for every new frequency modulation exposure for posterior data synchronization. Data filtering, data synchronization, data processing for calculation of time-domain parameters and A.I. data transformation were all performed automatically following strict computing processes and using the techniques and processes as described below.

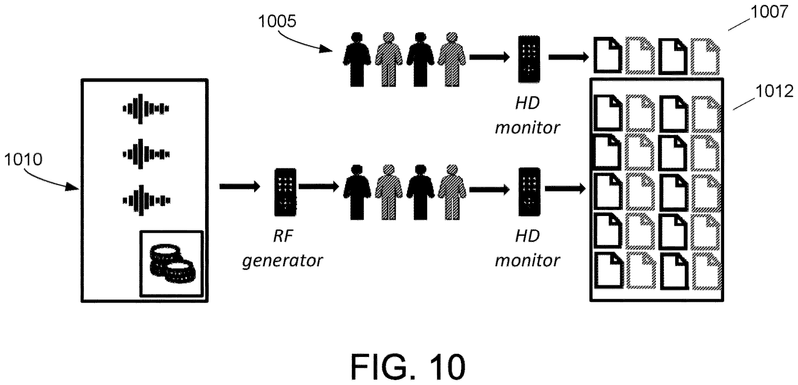

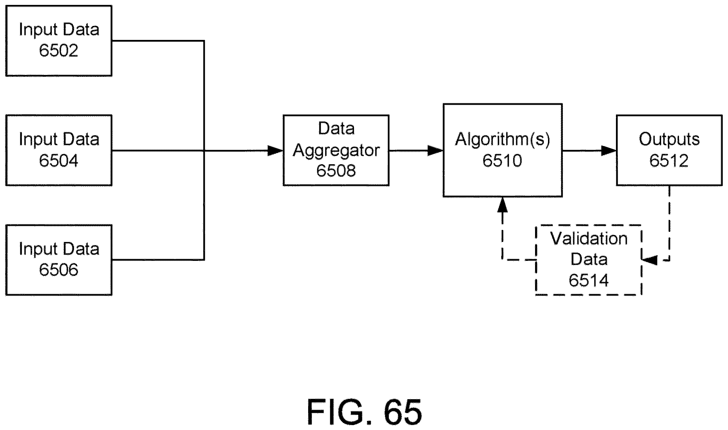

In order to collect data to train and improve the A.I. as described herein, initial patient data can be collected to train the initial machine learning algorithms (explained in greater detail below in the discussion of FIG. 65). For example, FIG. 10 illustrates two data flows that can represent data collection for a set of patients. An initial flow 1005 includes collecting conventional treatment information for a set of patients. These patients may be diagnosed as healthy or having one or more ailments or diseases such as HCC. The patients' hemodynamic parameters can be monitored and collected into an initial data set 1007.

Similar, a second data flow 1010 can include collecting data for patients using electromagnetic exposure as well. For example, a set of specific frequencies can be determined (or a standard set of frequencies can be used) such as the set of frequencies shown in FIG. 13E. An RF generator can produce those frequencies and expose a set of patients to the frequencies. An HD monitor can measure the patients' hemodynamic response to the exposure and collect and record that data as data set 1012.

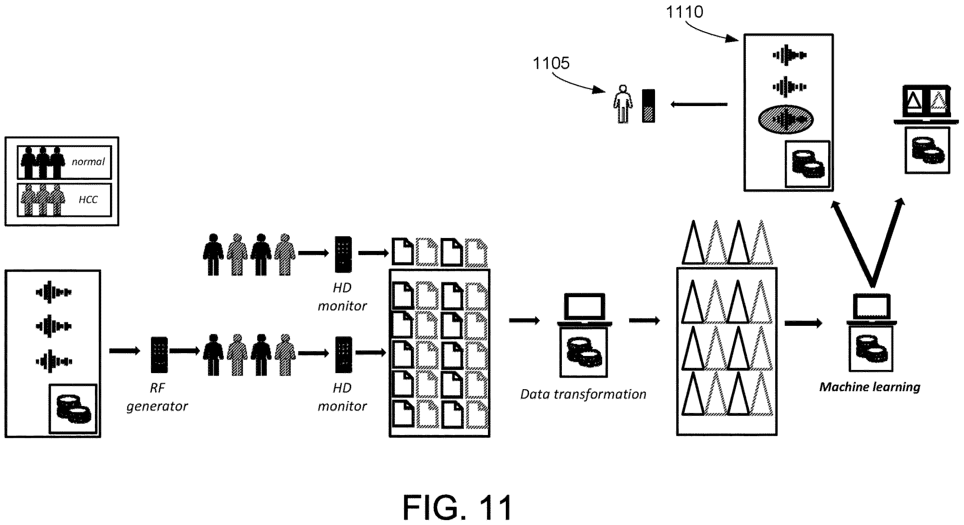

In certain implementations, the processes and techniques as described in FIG. 10 can be used to tune a treatment to a specific patient. For example, as shown in FIG. 11, a specialized or tuned treatment for patient 1105 can be determined or calculated automatically based upon the records collected for other patients, initial test results for the patient 1105, and machine learning/A.I. techniques. For example, as shown in FIG. 11, patient records can be collected as described above in the discussion of FIG. 10. The patient records can then be analyzed and further processed to transform the records into a data format configured to be input into one or more machine learning algorithms. The output of the machine learning algorithms can then be further analyzed and compared to a specific patient's information (e.g., demographic information, prior test results, initial energy exposure information) to determine a specific treatment regimen for that patient. In certain implementations, the personalized treatment regimen can include an auto-tuned set 1110 of frequencies or energy levels for use during exposure of the patient 1105 to the electromagnetic energy as described herein.

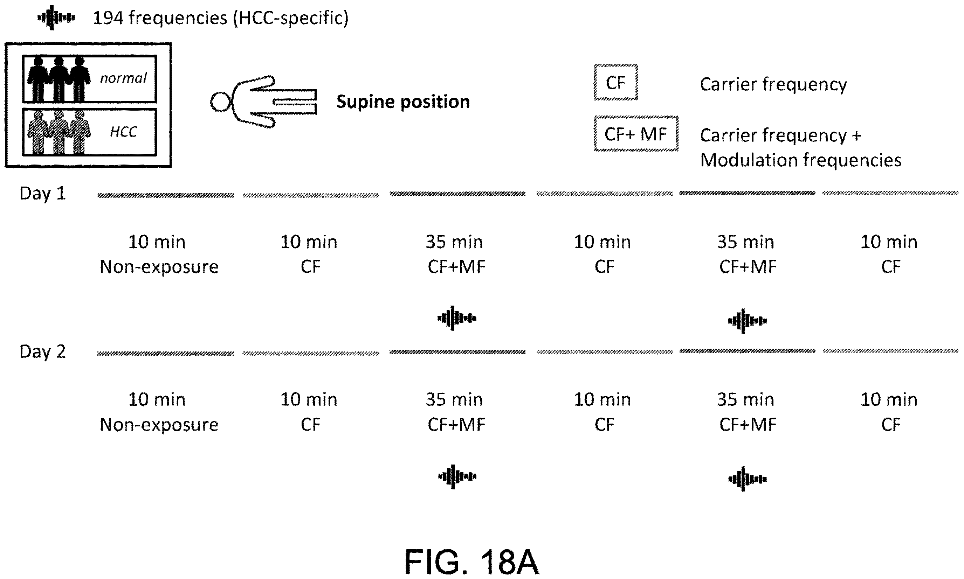

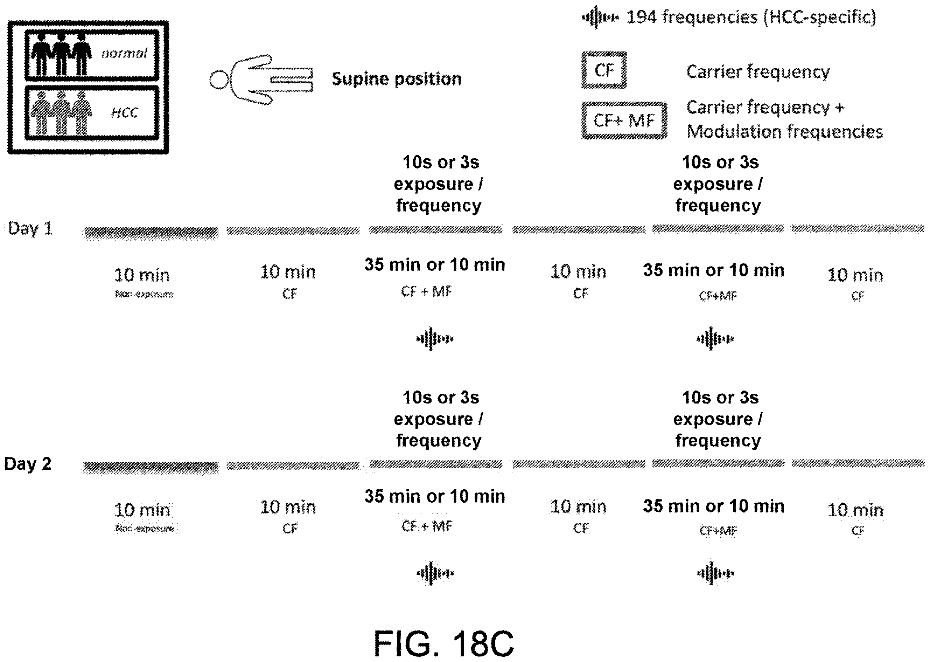

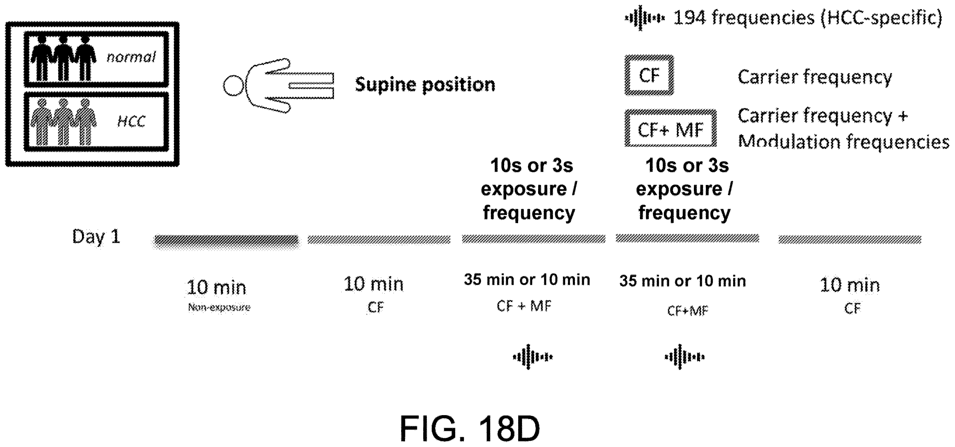

As shown in FIGS. 18A, 18C, and 18D a specific treatment timeline can be implemented for exposure of a patient to a set of modulated frequency signals. As shown in FIGS. 18A, 18C, and 18D the patient can be relaxed in a supine position. An initial period of about ten minutes of non-exposure can be included to allow for the patient to relax and establish baselines for various hemodynamic parameters and HRV. After the initial non-exposure period, the patient may be exposed to the carrier signal (i.e., at a constant amplitude without amplitude modulation) for an about ten minute period. After the initial exposure period, the patient may be exposed to a modulated frequency exposure period for about 120 minutes, about 60 minutes, about 35 minutes, or about 10 minutes. During this time, each of the set of frequencies (e.g., 194 frequencies or a series of electromagnetic frequencies that occur every 3 Hz or 10 Hz in a range from about 0.01 Hz to about 20 KHz, in a range from about 10 Hz to about 1,000 Hz, or in a range from about 10 Hz to about 2,000 Hz as described herein) can be applied to the patient for a particular period of time. In some embodiments, the series of electromagnetic frequencies in a range from about 0.01 Hz to about 20 KHz, from about 10 Hz to about 1,000 Hz, or from about 10 Hz to about 2,000 Hz, can occur about every 3 Hz. In some embodiments, the series of electromagnetic frequencies in a range from about 0.01 Hz to about 20 KHz, from about 10 Hz to about 1,000 Hz, or from about 10 Hz to about 2,000 Hz can occur about every 4 Hz. In some embodiments, the series of electromagnetic frequencies in a range from about 0.01 Hz to about 20 KHz, from about 10 Hz to about 1,000 Hz, or from about 10 Hz to about 2,000 Hz can occur about every 5 Hz. In some embodiments, the series of electromagnetic frequencies in a range from about 0.01 Hz to about 20 KHz, from about 10 Hz to about 1,000 Hz, or from about 10 Hz to about 2,000 Hz can occur about every 6 Hz. In some embodiments, the series of electromagnetic frequencies in a range from about 0.01 Hz to about 20 KHz, from about 10 Hz to about 1,000 Hz, or from about 10 Hz to about 2,000 Hz can occur about every 7 Hz. In some embodiments, the series of electromagnetic frequencies in a range from about 0.01 Hz to about 20 KHz, from about 10 Hz to about 1,000 Hz, or from about 10 Hz to about 2,000 Hz can occur about every 8 Hz. In some embodiments, the series of electromagnetic frequencies in a range from about 0.01 Hz to about 20 KHz, from about 10 Hz to about 1,000 Hz, or from about 10 Hz to about 2,000 Hz can occur about every 9 Hz. In some embodiments, the series of electromagnetic frequencies in a range from about 0.01 Hz to about 20 KHz, from about 10 Hz to about 1,000 Hz, or from about 10 Hz to about 2,000 Hz can occur about every 10 Hz. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 3 Hz to about every 10 HZ. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 4 Hz to about every 10 HZ. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 5 Hz to about every 10 HZ. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 6 Hz to about every 10 HZ. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 7 Hz to about every 10 HZ. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 8 Hz to about every 10 HZ. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 9 Hz to about every 10 HZ. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 3 Hz to about every 9 HZ. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 3 Hz to about every 8 HZ. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 3 Hz to about every 7 HZ. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 3 Hz to about every 6 HZ. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 3 Hz to about every 5 HZ. In some embodiments, the series of electromagnetic frequencies in a range from about 0.01 Hz to about 20 KHz, from about 10 Hz to about 1,000 Hz, or from about 10 Hz to about 2,000 Hz can occur in a range from about every 3 Hz to about every 4 HZ. In some embodiments, the particular period of time is about 2 or about 3 seconds per frequency. In further embodiments, the particular period of time is about 10 seconds per frequency. In some embodiments, the particular period of time is about 2 seconds per frequency. In some embodiments, the particular period of time is about 3 seconds per frequency. In some embodiments, the particular period of time is about 4 seconds per frequency. In some embodiments, the particular period of time is about 5 seconds per frequency. In some embodiments, the particular period of time is about 6 seconds per frequency. In some embodiments, the particular period of time is about 7 seconds per frequency. In some embodiments, the particular period of time is about 8 seconds per frequency. In some embodiments, the particular period of time is about 9 seconds per frequency. In some embodiments, the particular period of time is about 10 seconds per frequency.

In some embodiments, the modulated frequencies applied to the patient can be a series of electromagnetic frequencies that occur every 3 Hz in a range from about 0.01 Hz to about 20 KHz. In some embodiments, the modulated frequencies applied to the patient can be a series of electromagnetic frequencies that occur every 3 Hz in a range from about 10 Hz to about 1,000 Hz. In some embodiments, the modulated frequencies applied to the patient can be a series of electromagnetic frequencies that occur every 3 Hz in a range from about 10 Hz to about 2,000 Hz. In some embodiments, the modulated frequencies applied to the patient can be a series of electromagnetic frequencies that occur every 10 Hz in a range from about 0.01 Hz to about 20 KHz. In some embodiments, the modulated frequencies applied to the patient can be a series of electromagnetic frequencies that occur every 10 Hz in a range from about 10 Hz to about 1,000 Hz. In some embodiments, the modulated frequencies applied to the patient can be a series of electromagnetic frequencies that occur every 10 Hz in a range from about 10 Hz to about 2,000 Hz. The system can cycle repeatedly through each of the frequencies for the exposure period. In some embodiments, the exposure period is about 35 minutes (FIGS. 18A, 18C, 18D). In further embodiments, the exposure period is about 10 minutes (FIGS. 18C and 18D). In some embodiments, the exposure period is about 120 minutes or about 60 minutes. After the initial exposure period, the system may expose the patient to another carrier signal period for about 10 minutes during which the patient's body can recover from the initial exposure period of about 120 minutes, about 60 minutes, about 35 minutes, or about 10 minutes (FIGS. 18A and 18C). At the conclusion of the second carrier signal period of about ten minutes, the patient may again be exposed to a second modulated frequency signal period of about 120 minutes, about 60 minutes, about 35 minutes, or about 10 minutes (FIGS. 18A and 18C). Like before, during this time, each of the set of frequencies (e.g., 194 frequencies or a series of electromagnetic frequencies that occur every 3 Hz or 10 Hz in a range from about 0.01 Hz to about 20 KHz, from about 10 Hz to about 1,000 Hz, or from about 10 Hz to about 2,000 Hz as described herein) can be applied to the patient for a particular period of time (e.g., from about 2 seconds to about 3 seconds per frequency, or about 10 seconds per frequency). The system can cycle repeatedly through each of the frequencies for the exposure period, wherein the exposure period can be about 120 minutes, about 60 minutes, about 35 minutes, or be about 10 minutes. In some embodiments, the modulated frequencies applied to the patient can be a series of electromagnetic frequencies that occur every 3 Hz in a range from about 0.01 Hz to about 20 KHz. In some embodiments, the modulated frequencies applied to the patient can be a series of electromagnetic frequencies that occur every 3 Hz in a range from about 10 Hz to about 1,000 Hz. In some embodiments, the modulated frequencies applied to the patient can be a series of electromagnetic frequencies that occur every 3 Hz in a range from about 10 Hz to about 2,000 Hz. In some embodiments, the modulated frequencies applied to the patient can be a series of electromagnetic frequencies that occur every 10 Hz in a range from about 0.01 Hz to about 20 KHz. In some embodiments, the modulated frequencies applied to the patient can be a series of electromagnetic frequencies that occur every 10 Hz in a range from about 10 Hz to about 1,000 Hz. In some embodiments, the modulated frequencies applied to the patient can be a series of electromagnetic frequencies that occur every 10 Hz in a range from about 10 Hz to about 2,000 Hz. Following this second exposure period, the patient may again be exposed to the carrier frequency for about ten minutes, after which the treatment session is concluded.

The treatment timelines described above may comprise a treatment session. As shown in FIG. 18A and 18C, the treatment session can be repeated for a patient on a second day. The treatment session can occur for one or more consecutive or non-consecutive days. In some embodiments, the treatment session occurs for one day. In some embodiments, the treatment session occurs for two consecutive days. In some embodiments, the treatment session occurs for two non-consecutive days. In some embodiments, the treatment session occurs once per week, twice per week, three times per week, or combinations thereof. In some embodiments, the treatment session occurs 1 time per month, 2 times per month, 3 times per month, 4 times per month, 5 times per month, 6 times per month, 7 times per month, 8 times per month, 9 times per month, 10 times per month, 11 times per month, 12 times per month, 13 times per month, 14 times per month, 15 times per month, 16 times per month, 17 times per month, 18 times per month, 19 times per month, 20 times per month, 21 times per month, 22 times per month, 23 times per month, 24 times per month, 25 times per month, 26 times per month, 27 times per month, 28 times per month, 29 times per month, 30 times per month, or combinations thereof.

In some embodiments, the treatment session occurs for any number of consecutive or non-consecutive days described above, or any number of monthly treatment sessions described above for a total of about 1 year, about 2 years, about 3 years, about 4 years, about 5 years, about 6 years, about 7 years, about 8 years, about 9 years, about 10 years, about 11 years, about 12 years, about 13 years, about 14 years, about 15 years, about 16 years, about 17 years, about 18 years, about 19 years, about 20 years, about 21 years, about 22 years, about 23 years, about 24 years, about 25 years, about 26 years, about 27 years, about 28 years, about 29 years, about 30 years, about 31 years, about 32 years, about 33 years, about 34 years, about 35 years, about 36 years, about 37 years, about 38 years, about 39 years, about 40 years, about 41 years, about 42 years, about 43 years, about 44 years, about 45 years, about 46 years, about 47 years, about 48 years, about 49 years, about 50 years, or combinations thereof. In some embodiments, the treatment session occurs for any number of consecutive or non-consecutive days described above, or any number of monthly treatment sessions described above for a total of about 1 year. In some embodiments, the treatment session occurs for any number of consecutive or non-consecutive days described above, or any number of monthly treatment sessions described above for a total of about 2 years. In some embodiments, the treatment session occurs for any number of consecutive or non-consecutive days described above, or any number of monthly treatment sessions described above for a total of about 3 years. In some embodiments, the treatment session occurs for any number of consecutive or non-consecutive days described above, or any number of monthly treatment sessions described above for a total of about 4 years. In some embodiments, the treatment session occurs for any number of consecutive or non-consecutive days described above, or any number of monthly treatment sessions described above for a total of about 5 years. In some embodiments, the treatment session occurs for any number of consecutive or non-consecutive days described above, or any number of monthly treatment sessions described above for a total of about 6 years. In some embodiments, the treatment session occurs for any number of consecutive or non-consecutive days described above, or any number of monthly treatment sessions described above for a total of about 7 years. In some embodiments, the treatment session occurs for any number of consecutive or non-consecutive days described above, or any number of monthly treatment sessions described above for a total of about 8 years. In some embodiments, the treatment session occurs for any number of consecutive or non-consecutive days described above, or any number of monthly treatment sessions described above for a total of about 9 years. In some embodiments, the treatment session occurs for any number of consecutive or non-consecutive days described above, or any number of monthly treatment sessions described above for a total of about 10 years.

In some embodiments, the treatment session occurs for any number of consecutive or non-consecutive days described above or any number of monthly treatment sessions described above for any number of years described above until tumor progression is reduced. In some embodiments, tumor progression is reduced by about 1% to about 10%, about 1% to about 20%, about 1% to about 30%, about 1% to about 40%, about 1% to about 50%, about 1% to about 60%, about 1% to about 70%, about 1% to about 80%, about 1% to about 90%, about 1% to about 99%. In some embodiments, tumor progression is reduced by about 20% to about 30%, about 20% to about 40%, about 20% to about 50%, about 20% to about 60%, about 20% to about 70%, about 20% to about 80%, about 20% to about 90%, about 20% to about 99%. In some embodiments, tumor progression is reduced by about 40% to about 50%, about 40% to about 60%, about 40% to about 70%, about 40% to about 80%, about 40% to about 90%, about 40% to about 99%. In some embodiments, tumor progression is reduced by about 60% to about 70%, about 60% to about 80%, about 60% to about 90%, about 60% to about 99%.

In some embodiments, the treatment session occurs for any number of consecutive or non-consecutive days described above or any number of monthly treatment sessions described above for any number of years described above until tumor regression is observed. In some embodiments, tumor regression is determined by measuring the percent reduction in tumor size. In some embodiments, tumor regression is about 1% to about 10%, about 1% to about 20%, about 1% to about 30%, about 1% to about 40%, about 1% to about 50%, about 1% to about 60%, about 1% to about 70%, about 1% to about 80%, about 1% to about 90%, about 1% to about 99%. In some embodiments, tumor regression is about 20% to about 30%, about 20% to about 40%, about 20% to about 50%, about 20% to about 60%, about 20% to about 70%, about 20% to about 80%, about 20% to about 90%, about 20% to about 99%. In some embodiments, tumor regression is about 40% to about 50%, about 40% to about 60%, about 40% to about 70%, about 40% to about 80%, about 40% to about 90%, about 40% to about 99%. In some embodiments, tumor regression is about 60% to about 70%, about 60% to about 80%, about 60% to about 90%, about 60% to about 99%.

In some embodiments, the treatment session occurs for any number of consecutive or non-consecutive days described above or any number of monthly treatment sessions described above for any number of years described above until the tumor is no longer measurable.

It should be noted that the treatment schedule as shown in FIG. 18A is provided by way of example only. In certain implementations or patient-specific treatment timelines, various specifics as shown in FIG. 18A can be changed or otherwise altered. For example, the number of modulated frequency exposure periods can be changed to less than or more than two. Further, the number of carrier frequency exposure periods can be changed to less than or more than two. Similarly, the length of the carrier frequency and/or modulated frequency exposure periods can be changed. Further, the modulated frequencies can cycle repeatedly through each of the frequencies for one or more exposure periods.

In some embodiments, the patient can be relaxed in a supine position. An initial period of about ten minutes of non-exposure time can be included to allow for the patient to relax and establish baselines for various hemodynamic parameters and HRV. After the initial non-exposure period, the patient may be exposed to a carrier signal (i.e., at a constant amplitude without amplitude modulation) period for about ten minutes. After the initial exposure period, the patient may be exposed to a modulated frequency exposure period for about 35 minutes. During this time, each of the set of frequencies (e.g., 194 frequencies or a series of electromagnetic frequencies that occur every 3 Hz or 10 Hz in a range from about 0.01 Hz to about 20 KHz, from about 10 Hz to about 1,000 Hz, or from about 10 Hz to about 2,000 Hz as described herein) can be applied to the patient for a particular period of time (e.g., 10 seconds per frequency or 3 seconds per frequency). In some embodiments, the modulated frequencies applied to the patient can be a series of electromagnetic frequencies that occur every 3 Hz in a range from about 0.01 Hz to about 20 KHz. In some embodiments, the modulated frequencies applied to the patient can be a series of electromagnetic frequencies that occur every 3 Hz in a range from about 10 Hz to about 1,000 Hz . In some embodiments, the modulated frequencies applied to the patient can be a series of electromagnetic frequencies that occur every 3 Hz in a range from about 10 Hz to about 2,000 Hz.. In some embodiments, the modulated frequencies applied to the patient can be a series of electromagnetic frequencies that occur every 10 Hz in a range from about 0.01 Hz to about 20 KHz. In some embodiments, the modulated frequencies applied to the patient can be a series of electromagnetic frequencies that occur every 10 Hz in a range from about 10 Hz to about 1,000 Hz. In some embodiments, the modulated frequencies applied to the patient can be a series of electromagnetic frequencies that occur every 10 Hz in a range from about 10 Hz to about 2,000 Hz. The system can cycle repeatedly through each of the frequencies for the exposure period. After the initial exposure period of about 35 minutes, the system may expose the patient to another carrier signal period of about 10 minutes during which the patient's body can recover from the initial exposure period of about 35 minutes. At the conclusion of the second carrier signal period of about ten minutes, the patient may be exposed to a modulated frequency signal period of about 10 minutes. During this time, each of the set of frequencies (e.g., 194 frequencies or a series of electromagnetic frequencies that occur every 3 Hz or 10 Hz in a range from about 0.01 Hz to about 20 KHz, from about 10 Hz to about 1,000 Hz, or from about 10 Hz to about 2,000 Hz as described herein) can be applied to the patient for a particular period of time (e.g., 2 or 3 seconds per frequency or 10 seconds per frequency). In some embodiments, the modulated frequencies applied to the patient can be a series of electromagnetic frequencies that occur every 3 Hz in a range from about 0.01 Hz to about 20 KHz. In some embodiments, the modulated frequencies applied to the patient can be a series of electromagnetic frequencies that occur every 3 Hz in a range from about 10 Hz to about 1,000 Hz. In some embodiments, the modulated frequencies applied to the patient can be a series of electromagnetic frequencies that occur every 3 Hz in a range from about 10 Hz to about 2,000 Hz. In some embodiments, the modulated frequencies applied to the patient can be a series of electromagnetic frequencies that occur every 10 Hz in a range from about 0.01 Hz to about 20 KHz. In some embodiments, the modulated frequencies applied to the patient can be a series of electromagnetic frequencies that occur every 10 Hz in a range from about 10 Hz to about 1,000 Hz. In some embodiments, the modulated frequencies applied to the patient can be a series of electromagnetic frequencies that occur every 10 Hz in a range from about 10 Hz to about 2,000 Hz. The system can cycle repeatedly through each of the frequencies for the exposure period. Following this second modulated frequency exposure period, the patient may again be exposed to the carrier frequency for about ten minutes, after which the treatment session is concluded. The same treatment session can be repeated for a patient on a second day. The treatment session can occur for one or more consecutive or non-consecutive days. In some embodiments, the treatment session occurs for one day. In some embodiments, the treatment session occurs for two consecutive days. In some embodiments, the treatment session occurs for two non-consecutive days.

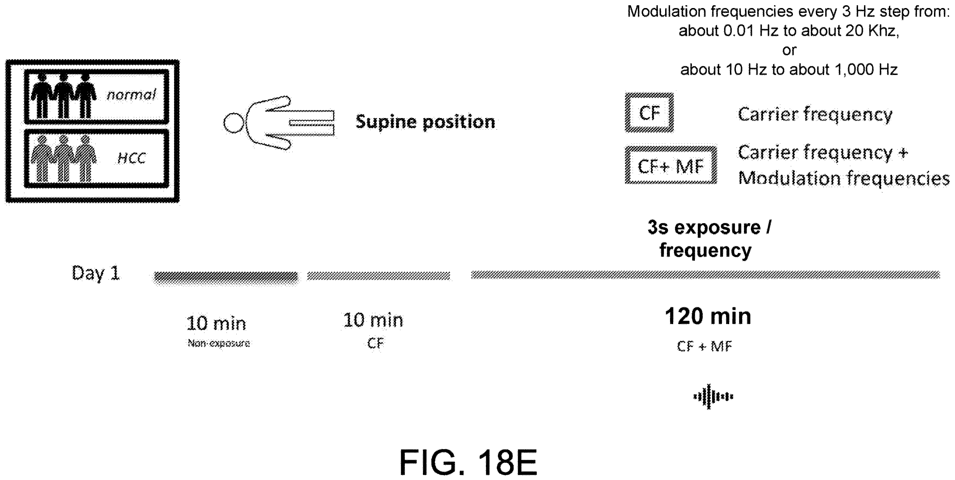

In some embodiments, the patient can be relaxed in a supine position. An initial non-exposure period of about ten minutes can be included to allow for the patient to relax and establish baselines for various hemodynamic parameters and HRV. After the initial non-exposure period, the patient may be exposed to a carrier signal (i.e., at a constant amplitude without amplitude modulation) period for about ten minutes. After the initial exposure period, the patient may be exposed to a modulated frequency exposure period of about 50 minutes, about 60 minutes, or about 120 minutes. During this time, the series of electromagnetic frequencies that occur every 3 Hz in a range from about 0.01 Hz to about 20 KHz, from about 10 Hz to about 1,000 Hz, from about 100 Hz to about 1,000 Hz, or from about 10 Hz to about 2,000 Hz can be applied to the patient for about 3 seconds per frequency (FIG. 18E). The system can cycle repeatedly through each of the frequencies for the exposure period. After this initial exposure period, the system may expose the patient to another carrier signal period for about 10 minutes during which the patient's body can recover from the initial exposure period, after which the treatment session is concluded. The same treatment session can be repeated for a patient on a second day. The treatment session can occur for one or more consecutive or non-consecutive days. In some embodiments, the treatment session occurs for one day. In some embodiments, the treatment session occurs for two consecutive days. In some embodiments, the treatment session occurs for two non-consecutive days.

In some embodiments, the patient can be relaxed in a supine position. An initial non-exposure period of about ten minutes can be included to allow for the patient to relax and establish baselines for various hemodynamic parameters and HRV. After the initial non-exposure period, the patient may be exposed to a carrier signal (i.e., at a constant amplitude without amplitude modulation) period for about ten minutes. After the initial exposure period, the patient may be exposed to a modulated frequency exposure period for about 50 minutes, about 60 minutes, or about 120 minutes. During this time, the series of electromagnetic frequencies that occur every 3 Hz in a range from about 0.01 Hz to about 20 KHz, from about 10 Hz to about 1,000 Hz, from about 100 Hz to about 1,000 Hz, from about 10 Hz to about 2,000 Hz can be applied to the patient for about 3 seconds per frequency (FIG. 18E). The system can cycle repeatedly through each of the frequencies for the exposure period. After this initial exposure period, the system may expose the patient to another carrier signal period for about 10 minutes during which the patient's body can recover from the initial exposure period. In some embodiments, the system can determine if the patient is to be exposed for an additional period to the modulated frequency signal. If the patient is to be exposed for another period, the system may expose the patient to another modulated frequency exposure period. In some embodiments, the additional modulated frequency exposure comprises the modulated frequencies that altered a hemodynamic parameter, for example heart rate variability, in the initial modulated frequency exposure period. In some embodiments, the additional modulated frequency exposure comprises the series of electromagnetic frequencies that occur every 3 Hz or 10 Hz in a range from about 0.01 Hz to about 20 KHz, from about 10 Hz to about 1,000 Hz, or from about 10 Hz to about 2,000 Hz. Following the initial or additional modulated frequency exposure, the system can expose the patient to another carrier wave exposure period for about ten minutes. If the patient is not to be exposed to another modulated frequency period, the process can end. The same treatment session can be repeated for a patient on a second day. The treatment session can occur for one or more consecutive or non-consecutive days. In some embodiments, the treatment session occurs for one day. In some embodiments, the treatment session occurs for two consecutive days. In some embodiments, the treatment session occurs for two non-consecutive days.

As illustrated in FIG. 18D, in some embodiments the patient can be relaxed in a supine position. An initial non-exposure period of about ten minutes can be included to allow for the patient to relax and establish baselines for various hemodynamic parameters and HRV. After the initial non-exposure period, the patient may be exposed to a carrier signal (i.e., at a constant amplitude without amplitude modulation) period for about ten minutes. After the initial exposure period, the patient may be exposed to a modulated frequency exposure period for about 35 minutes or about 10 minutes. During this time, each of the set of frequencies (e.g., 194 frequencies or a series of electromagnetic frequencies that occur every 3 Hz or 10 Hz in a range from about 0.01 Hz to about 20 KHz, from about 10 Hz to about 1,000 Hz, or from about 10 Hz to about 2,000 Hz as described herein) can be applied to the patient for a particular period of time (e.g., about 10 seconds per frequency or about 3 seconds per frequency). In some embodiments, the modulated frequencies applied to the patient can be a series of electromagnetic frequencies that occur every 3 Hz in a range from about 0.01 Hz to about 20 KHz. In some embodiments, the modulated frequencies applied to the patient can be a series of electromagnetic frequencies that occur every 3 Hz in a range from about 10 Hz to about 1,000 Hz. In some embodiments, the modulated frequencies applied to the patient can be a series of electromagnetic frequencies that occur every 3 Hz in a range from about 10 Hz to about 2,000 Hz. In some embodiments, the modulated frequencies applied to the patient can be a series of electromagnetic frequencies that occur every 10 Hz in a range from about 0.01 Hz to about 20 KHz. In some embodiments, the modulated frequencies applied to the patient can be a series of electromagnetic frequencies that occur every 10 Hz in a range from about 10 Hz to about 1,000 Hz. In some embodiments, the modulated frequencies applied to the patient can be a series of electromagnetic frequencies that occur every 10 Hz in a range from about 10 Hz to about 2,000 Hz. The system can cycle repeatedly through each of the frequencies for the exposure period. After the initial exposure period of about 35 minutes or about 10 minutes, the system may expose the patient to another exposure period that comprises the initial modulated frequencies that altered a hemodynamic parameter (e.g. heart rate variability). In some embodiments, after the initial exposure period of about 35 minutes or about 10 minutes, the system may expose the patient to another exposure period that comprises the initial modulated frequencies that did not alter a hemodynamic parameter (e.g. heart rate variability). In some embodiments, after the initial exposure period of about 35 minutes or about 10 minutes, the system may expose the patient to another exposure period that comprises all of the 194 frequencies or a series of electromagnetic frequencies that occur every 3 Hz or 10 Hz in a range from about 0.01 Hz to about 20 KHz, from about 10 Hz to about 1,000 Hz, or from about 10 Hz to about 2,000 Hz as described herein. In some embodiments, the modulated frequencies applied to the patient can be a series of electromagnetic frequencies that occur every 3 Hz in a range from about 0.01 Hz to about 20 KHz. In some embodiments, the modulated frequencies applied to the patient can be a series of electromagnetic frequencies that occur every 3 Hz in a range from about 10 Hz to about 1,000 Hz. In some embodiments, the modulated frequencies applied to the patient can be a series of electromagnetic frequencies that occur every 3 Hz in a range from about 10 Hz to about 2,000 Hz. In some embodiments, the modulated frequencies applied to the patient can be a series of electromagnetic frequencies that occur every 10 Hz in a range from about 0.01 Hz to about 20 KHz. In some embodiments, the modulated frequencies applied to the patient can be a series of electromagnetic frequencies that occur every 10 Hz in a range from about 10 Hz to about 1,000 Hz. In some embodiments, the modulated frequencies applied to the patient can be a series of electromagnetic frequencies that occur every 10 Hz in a range from about 10 Hz to about 2,000 Hz. Following this second modulated frequency exposure period, the patient may again be exposed to the carrier frequency for about ten minutes, after which the treatment session is concluded. The same treatment session can be repeated for a patient on a second day. The treatment session can occur for one or more consecutive or non-consecutive days. In some embodiments, the treatment session occurs for one day. In some embodiments, the treatment session occurs for two consecutive days. In some embodiments, the treatment session occurs for two non-consecutive days.

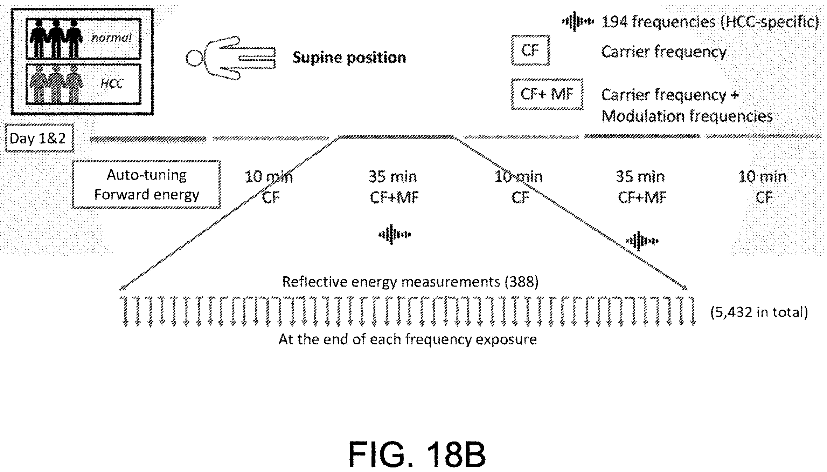

As noted above, for a specific patient the treatment can be customized or auto-tuned such that the forward energy (e.g., energy used in the carrier and modulate frequency signals) is chosen specifically for a particular patient. This auto-tuning can be determined for a particular patient based upon various factors such as patient information (e.g., health condition, size and weight, prior treatment information) such that the forward energy applied to the patient during treatment, and the energy absorbed by the patient's cells during treatment, is within a specific range or threshold of the normal or average energy values for similar other patients. As shown in FIG. 18B, this auto-tuning can be performed prior to the initial about ten minute carrier frequency period.

Additionally, as shown in FIG. 18B, during the modulated frequency exposure period, reflective energy can be measured as well. Reflective energy provides an indication of how much of the forward energy is not being absorbed or transmitted through the patient. As transmission energy is generally constant for a patient, the transmission energy can be considered negligible for a patient during treatment. Thus, by measuring the reflective energy, the system can approximate the amount of energy being absorbed by the patient during the exposure periods.

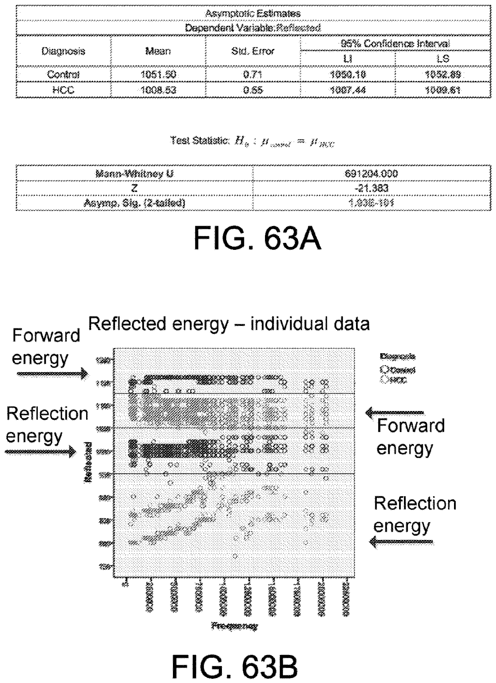

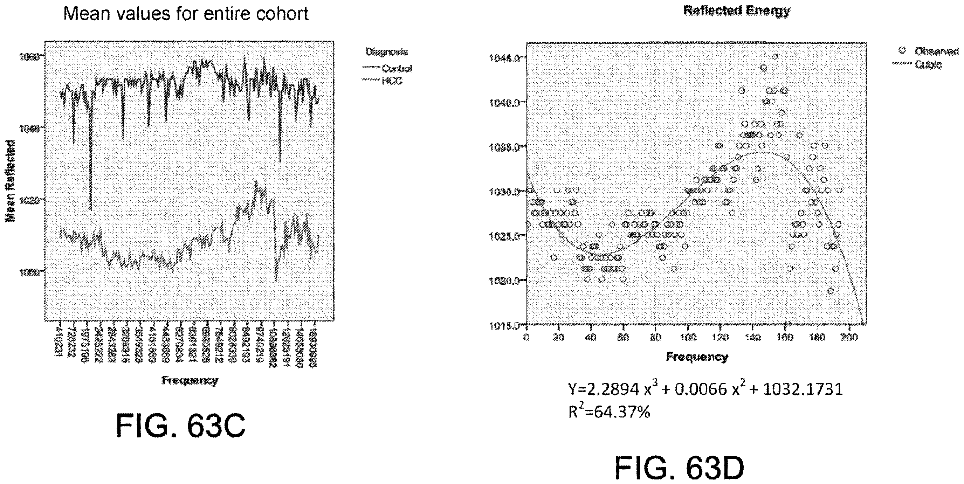

Additionally, by measuring reflective energy, the system can also determine an alternative data point for use in evaluation and diagnosis of a particular ailment or disease. For example, in related studies done during the development of the processes and techniques as described herein, the data in FIG. 63B was observed. As shown in FIG. 63B, by measuring and plotting forward energy versus reflective energy for a patient population, various bands of energy levels can be seen. Additionally, for HCC patients, the reflective energy falls into a band generally below the band of reflective energy for healthy patients. Such an occurrence can be used as another factor when determining whether a patient is to be (or likely to be) diagnosed with HCC. Additionally, as the reflective energy levels are lower for HCC patients, this would potentially indicate that HCC patients are absorbing more energy on a cellular level than healthy patients, thus possibly providing at least a portion of an explanation for why modulated frequency exposure as described herein causes cellular malfunction in cancer cells.

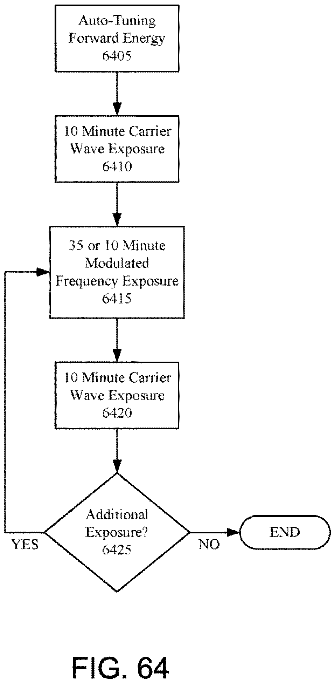

As noted above, FIGS. 18A - 18D illustrate various treatment timelines for patient treatment. These timelines are also summarized in the process flow as shown in FIG. 64. As shown in FIG. 64, the system (e.g., the orchestrator 906 as shown in FIG. 9B) can initially auto-tune 6405 the forward energy for a specific patient. Following auto-tuning, the system can expose 6410 the patient to the initial about ten minute carrier wave signal. Following the initial about ten minute exposure, the system can expose 6415 the patient to the initial modulated frequency signal for about 120 minutes, about 60 minutes, about 35 minutes, or about 10 minutes as described herein. Following the initial modulated frequency exposure, the system can expose 6420 the patient to another carrier wave exposure period for about ten minutes. The system can then determine 6425 if the patient is to be exposed for an additional period to the modulated frequency signal. If the patient is to be exposed for another period, the process can return to step 6415 where the system exposes the patient to another modulated frequency exposure period for about 120 minutes, about 60 minutes, about 35 minutes, or about 10 minutes. If the patient is not to be exposed to another modulated frequency period, the process as shown in FIG. 64 can end.

In some embodiments, the system can initially auto-tune 6405 the forward energy for a specific patient. Following auto-tuning, the system can expose 6410 the patient to the initial carrier wave signal for about ten minutes. Following the initial about ten minute exposure, the system can expose 6415 the patient to the initial modulated frequency signal for about 120 minutes, about 60 minutes, about 35 minutes, or about 10 minutes as described herein. The system can then determine 6425 if the patient is to be exposed for an additional period to the modulated frequency signal. If the patient is to be exposed for another period, the process can return to step 6415 where the system exposes the patient to another modulated frequency exposure period for about 120 minutes, about 60 minutes, about 35 minutes, or about 10 minutes. In some embodiments, following the initial modulated frequency exposure, the system can expose 6420 the patient to another modulated frequency exposure. In some embodiments, the additional modulated frequency exposure comprises the modulated frequencies that altered a hemodynamic parameter, for example heart rate variability. In some embodiments, the additional modulated frequency exposure comprises the modulated frequencies that did not alter a hemodynamic parameter, for example heart rate variability. In some embodiments, the additional modulated frequency exposure comprises the 194 amplitude modulated frequencies or a series of electromagnetic frequencies that occur every 3 Hz or 10 Hz in a range from about 0.01 Hz to about 20 KHz, from about 10 Hz to about 1,000 Hz, or from about 10 Hz to about 2,000 Hz. In some embodiments, the modulated frequencies applied to the patient can be a series of electromagnetic frequencies that occur every 3 Hz in a range from about 0.01 Hz to about 20 KHz. In some embodiments, the modulated frequencies applied to the patient can be a series of electromagnetic frequencies that occur every 3 Hz in a range from about 10 Hz to about 1,000 Hz. In some embodiments, the modulated frequencies applied to the patient can be a series of electromagnetic frequencies that occur every 3 Hz in a range from about 10 Hz to about 2,000 Hz. In some embodiments, the modulated frequencies applied to the patient can be a series of electromagnetic frequencies that occur every 10 Hz in a range from about 0.01 Hz to about 20 KHz. In some embodiments, the modulated frequencies applied to the patient can be a series of electromagnetic frequencies that occur every 10 Hz in a range from about 10 Hz to about 1,000 Hz.. In some embodiments, the modulated frequencies applied to the patient can be a series of electromagnetic frequencies that occur every 10 Hz in a range from about 10 Hz to about 2,000 Hz. Following the initial or additional modulated frequency exposure, the system can expose 6420 the patient to another carrier wave exposure period for about ten minutes. If the patient is not to be exposed to another modulated frequency period, the process as shown in FIG. 64 can end.

As noted above, the timing and order of exposure periods as shown in FIG. 64 is provided by way of example only. Additionally, in certain implementations, the determination of whether to perform another exposure period can be determined by another decision maker such as a patient's physician.

The A.I. and related algorithms as described herein can include machine learning or other similar statistical-based modeling techniques. For example, the algorithm used may depend on an expected outcome of the algorithm. For example, a processing device can be configured to use a first process or algorithm to calculate refinements to a derived weight as described above based upon a first set of outcomes data while using a second or different process/algorithm to calculate refinements to a derived weight as described above based upon a second set of outcomes data. Different methods and algorithms may be used to calculate the refined weights in concert or substantially simultaneously. The output of each of the different methods and algorithms can then be compared/further analyzed to determine which output is highest rated, or the output of each method and algorithm can be combined into a combinational metric.

In some implementations, a machine learning model as described in further detail below can be trained on a large population, for example, a population that can range from several thousand to tens of thousands of patient records comprising electrophysiology, demographic and medical history information. The machine learning tool can include but is not limited to penalized regression/classification techniques such as random forest and gradient boosting, (e.g., implemented using R or any other statistical/mathematical programming language), Bayesian belief networks, collaborative filters, support vector machines, and other similar machine learning and classification techniques. Any other classification based machine learning tool can be used, including neural networks (as described in more detail below) and support vector machines. Because the machine learning tool may be computationally intensive, some or all of the processing for the machine learning tool may be performed on a server that is separate from the medical device.

An overview of how a random forest tool may be applied to a given dataset can illustrate how a classification tool may work in interpreting given input data. A random forest is a collection of decision trees. A decision tree is a flow chart-like structure in which each node represents a test on a metric and each branch represents the outcome of the test. The tree culminates in a classification label, e.g., a decision taken at the end after computing each of the metrics. Each tree in a random forest tool gets a "vote" in classifying a given set of metrics. There are two components of randomness involved in the building of a random forest. First, at the creation of each tree, a random subsample of the total data set is selected to grow the tree. Second, at each node of the tree, a "splitter variable" is selected and the underlying patients are separated into two classes. For example, patients in one class (e.g., positive response to a specific drug) can be separated from those in another class (e.g., negative response to a specific drug). The tree is grown with additional splitter variables until all terminal nodes (leaves) of the tree are purely one class or the other. The tree is "tested" against patient records that have been previously set aside. Each patient testing record traverses the tree, going down one branch or another depending on the metrics included in the record for each splitter variable. The patient testing record is assigned a predicted outcome based on where the record lands in the tree (a vote). The entire process may be repeated with new random divisions of the underlying dataset to produce additional trees and ultimately a "forest". In each case, a different subset of patients can be used to build the tree and test its performance.

In developing the results described in the below example implementation, a predetermined number of model variations are trained. For example, each model variation is labeled sequentially, (e.g., for 100 runs, labeled from 1-100). In each run of the model, the software randomly samples a predetermined portion (e.g. an 80% portion) of the population as the training set and sets aside the remainder (e.g., 20%) as the validation set.

As noted above, the machine learning tool can train the model on a first portion of the underlying dataset and validate the model on a second portion of the dataset or on another separate dataset. When evaluating the performance of each model, the performance of the underlying decisions within the decision trees in the random forest can be evaluated based on specificity and sensitivity parameters. For example, the sensitivity parameter can be based on a measure of the model's ability to correctly predict whether a patient is at risk of reacting negatively to a drug treatment. For example, the sensitivity parameter may be based on a proportion of patients who are treated that the model correctly predicts will react negatively to the treatment. The specificity parameter can be based on the proportion of patients who to be treated with a specific drug, and who are predicted by the relevant model as reacting positive to the drug treatment. It may be advantageous to optimally balance individual performance variables such as sensitivity and specificity at a high level. For example, by setting the specificity at a relatively high value, e.g., 95%, the underlying thresholds within the classifier model may be adjusted to minimize false positives. After the specificity is defined, the measure of sensitivity can be treated as a type of performance measure, e.g., generally in the range of 15-35% for a given model, however, smaller or larger values of sensitivity are also possible.

A validation protocol, for example, as described below, can be employed to validate the predictive performance of trained models. In an implementation, the validation phase can be used to ascertain appropriate threshold scores for classifying future patients (where an outcome is currently unknown and a prediction of the outcome is desired) and to determine the predictive performance of each classifier model generated by the machine learning tool. For validating the various models and associated threshold scores, a second group of individuals, e.g., a validation population (or cohort), can be used. For example, the validation population used can be a new validation population. The outcome for the patients in the validation cohort is eventually learned as these patients progress through treatment. In an embodiment, the patients in the validation population can be different from the group of training and test patients described above for training the model. For example, a validation population of patients and their associated metrics (validation metrics) can be independent from a training population of patients and associated metrics (training metrics). In some implementations, there may be an overlap between the validation metrics and the training metrics.

In some implementations, the validation population can be updated by at least one of 1) adjusting one or more of the metrics in the validation metrics, and 2) expanding the validation metrics based on appending additional one or more subjects to population of subjects that make up the validation population. The thresholds for classifying future patients can be refined based on the updated validation metrics. For example, metrics of a patient that is currently being treated or monitored or has otherwise not progressed through the treatment can be used to adjust the one or more metrics in the validation metrics or the patient's metrics can be added to the validation population as metrics from a new subject. The validation metrics can be adjusted as new metrics for the patient are determined during the monitoring or treatment of the patient. In some examples, as a monitored patient progresses through treatment, the patient's metrics can be added to the validation population and/or used to adjust the metrics in the validation metrics after the patient has progressed through the treatment.

In some implementations, the training population can be updated by at least one of 1) adjusting one or more of the metrics in the training metrics, and 2) expanding the training metrics based on appending additional one or more subjects to the first plurality of subjects. The machine learning classifier models can be retrained based on the updated training metrics. For example, as additional patient metrics are determined from current patients and/or metrics from new patients are determined, the machine learning model can be retrained, e.g., on the increased number of metrics or on new, different metrics, to provide updated classifier models. The training population can be updated as new metrics for current patients and/or metrics for new patients are determined or after patients have progressed through treatment.

FIG. 65 illustrates a sample flow for training and validating one or more classifier models for a machine learning algorithm as described above. A set or population of known input data 6502, 6504, 6506 can be provided as the data set used to train and validate the classifier models. For example, the known patient records data set may include 1000 patients that have been diagnosed with a specific ailment such as HCC, their drug treatment regimens, and the associated outcomes for each patient. A percentage of the known patient data records can be used as the input data 6502, 6504, 6506. For example, 80%, or 800, of the patient records can be used as the input 6502, 6504, 6506.

The input data 6502, 6504, 6506 can be fed into a data aggregator 6508. The data aggregator 6508 can be configured to match patient data into a single training input for the machine learning algorithm and configure the training input into a format readable by the machine learning algorithm. The data aggregator 6508 can feed the training data into algorithm 6510. The algorithm 6510 can include one or more untrained data structures such as a series of data trees (e.g., organized using a random forest tool as described above). Using the training input variables and known outcomes from the input data 6502, 6504, 6506, the algorithm 6510 can iteratively process each data point in the training set, thereby training the data structures to more accurately produce the expected (and known) outcomes.

Once the algorithm 6510 has exhausted the input data 6502, 6504, 6506, the algorithm can generate one or more outputs 6512. The outputs 6510 can be compared against the expected output (as know from the initial population) to determine the specificity and sensitivity of the now-trained algorithm 6510. In certain implementations, validation data 6514 can be used to further refine the trained algorithm 6510 using additional patient records. For example, the validation data 6514 can be input into a validation module for validation of the one or more trained algorithms 6510. To continue the above example, the validation data 6514 can include 200 patient records. Typically, there is no overlap between a training data set and a validation data set as there is no advantage to running the same data set twice.

As the validated classifier models as used to classify new patients (e.g., to produce new outputs for a set of patient metrics as described herein), the produced outcomes can be used to better validate the process using a closed loop feedback system. For example, as a patient is classified and treated, the result of that treatment can be included in the patient record and verified by, for example, the patient's physician. The patient's record, now updated to include a known outcome, can then be provided as feedback to the validation module. The validation module can process the feedback, comparing a generated output against the known outcome for the patient. Based upon this comparison, the validation module can further refine the validated algorithms, thereby providing a closed loop system where the models are updated and upgraded regularly.

Once trained, the algorithms 6510 can be implemented into the Artificial Intelligence as described herein. For example, the algorithms 6510 can be implemented into Artificial Intelligence 6510 as discussed above. Then, using the trained algorithms, a system such as system 900 can monitor a patient's response to applied electromagnetic energy (e.g., by measuring the patient's HRV during the exposure) to both diagnose whether the patient has, for example, HCC and what the prognosis is for treatment of the patient. As noted above, an added benefit of the exposure to the electromagnetic energy can be simultaneous treatment of the cancer during diagnosis and long-term prognostication.

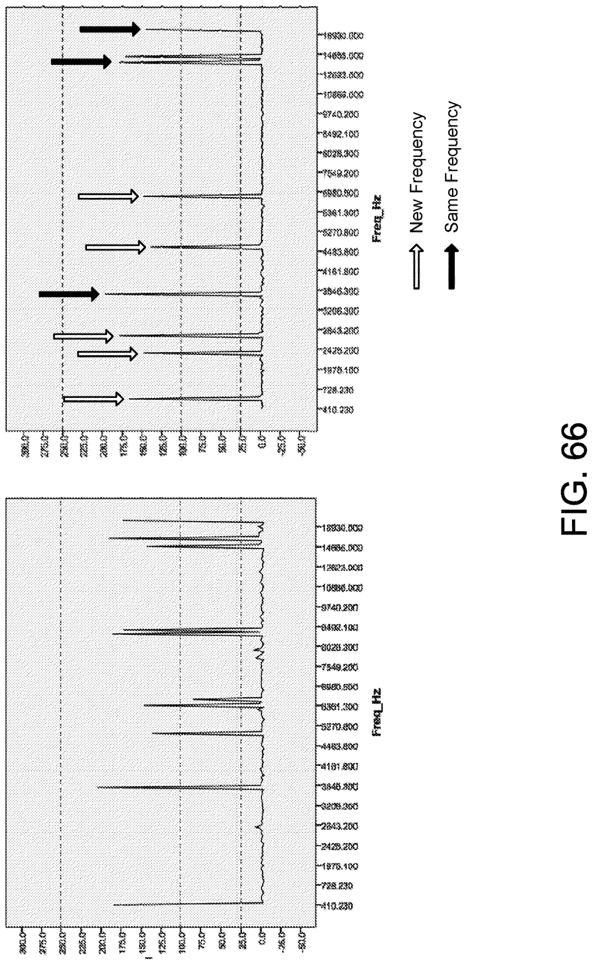

In certain examples, immediate changes to a patient's response to the applied energy can be observed. For example, as shown in FIG. 66, the left graph indicates a patient's response to various applied frequencies during a first about 35 minute therapy session (e.g., as described above in relation to FIGS. 18A, 18C, or 18D). The right graph of FIG. 66 illustrates a patient's response to the same set of frequencies after an about ten minute rest period. In some embodiments, a patient's response to the same set of frequencies occurs without an about ten minute rest period as depicted in FIG. 18D. As shown in FIG. 66, while several frequencies cause a similar reaction in the patient, there are now new frequencies that cause a reaction in the patient (e.g., noticeable changes in the patient's HRV) and several previously identified frequencies that the patient is not responding to after the rest period. Such a result can be indicative of an indication of immediate cellular changes in the patient causes by the initial about 35 minute exposure period.

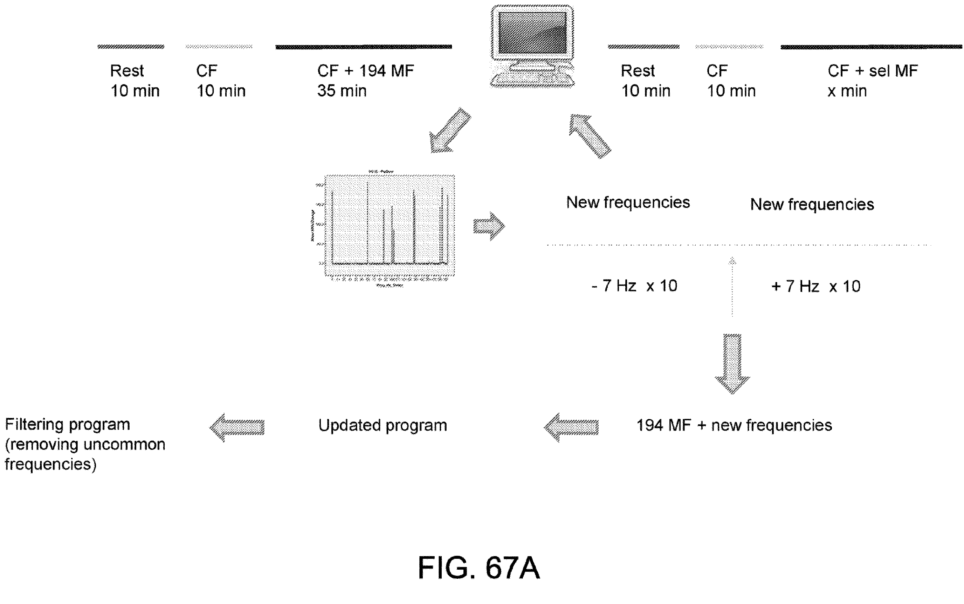

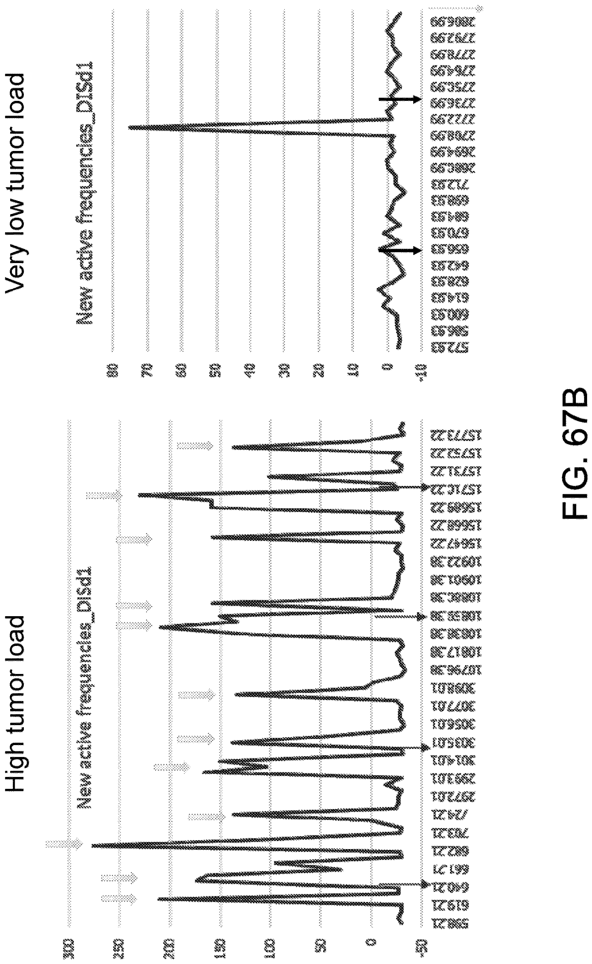

As illustrated in FIG. 67A, in some embodiments the patient can be relaxed in a supine position. An initial non-exposure period (i.e. rest) of about ten minutes can be included to allow for the patient to relax and establish baselines for various hemodynamic parameters and heart rate variability (HRV). After the initial non-exposure period, the patient may be exposed to a carrier frequency (i.e., at a constant amplitude without amplitude modulation) for about ten minutes. After the initial exposure period, the patient may be exposed a minute modulated frequency exposure period for about 120 minutes, about 60 minutes, about 35 minutes, or about 10 minutes. During this time, each of the set of frequencies (e.g., 194 frequencies or a series of electromagnetic frequencies that occur every 3 Hz or 10 Hz in a range from about 0.01 Hz to about 20 KHz, from about 10 Hz to about 1,000 Hz, or from about 10 Hz to about 2,000 Hz as described herein) can be applied to the patient for a particular period of time (e.g., 10 seconds per frequency or 3 seconds per frequency). In some embodiments, the modulated frequencies applied to the patient can be a series of electromagnetic frequencies that occur every 3 Hz in a range from about 0.01 Hz to about 20 KHz. In some embodiments, the modulated frequencies applied to the patient can be a series of electromagnetic frequencies that occur every 3 Hz in a range from about 10 Hz to about 1,000 Hz. In some embodiments, the modulated frequencies applied to the patient can be a series of electromagnetic frequencies that occur every 3 Hz in a range from about 10 Hz to about 2,000 Hz. In some embodiments, the modulated frequencies applied to the patient can be a series of electromagnetic frequencies that occur every 10 Hz in a range from about 0.01 Hz to about 20 KHz. In some embodiments, the modulated frequencies applied to the patient can be a series of electromagnetic frequencies that occur every 10 Hz in a range from about 10 Hz to about 1,000 Hz. In some embodiments, the modulated frequencies applied to the patient can be a series of electromagnetic frequencies that occur every 10 Hz in a range from about 10 Hz to about 2,000 Hz. The system can cycle repeatedly through each of the frequencies for the exposure period. After the initial exposure period of 120 minutes, about 60 minutes, about 35 minutes, or about 10 minutes, the system may determine the set of frequencies (i.e. "active frequencies") that altered a hemodynamic parameter (e.g. heart rate variability). In some embodiments, the system may determine a set of new frequencies that altered or did not alter a hemodynamic parameter during the first exposure period. In some embodiments, the set of new frequencies is within a range from about 0 Hz to about 10 Hz of the first set of frequencies that altered or did not alter a hemodynamic parameter (e.g. heart rate variability), preferably from about 0 Hz to about 7 Hz as depicted in FIG. 67B in patients with high tumor load and with low tumor load. In some embodiments, the set of new frequencies can be combined with the original 194 frequencies or a series of electromagnetic frequencies that occur every 3 Hz or 10 Hz in a range from about 0.01 Hz to about 20 KHz, in a range from about 10 Hz to about 1,000 Hz, or in a range from about 10 Hz to about 2,000 Hz as described herein to provide a patient-specific treatment protocol. In some embodiments, the modulated frequencies applied to the patient can be a series of electromagnetic frequencies that occur every 3 Hz in a range from about 0.01 Hz to about 20 KHz. In some embodiments, the modulated frequencies applied to the patient can be a series of electromagnetic frequencies that occur every 3 Hz in a range from about 10 Hz to about 1,000 Hz. In some embodiments, the modulated frequencies applied to the patient can be a series of electromagnetic frequencies that occur every 3 Hz in a range from about 10 Hz to about 2,000 Hz. In some embodiments, the modulated frequencies applied to the patient can be a series of electromagnetic frequencies that occur every 10 Hz in a range from about 0.01 Hz to about 20 KHz. In some embodiments, the modulated frequencies applied to the patient can be a series of electromagnetic frequencies that occur every 10 Hz in a range from about 10 Hz to about 1,000 Hz. In some embodiments, the modulated frequencies applied to the patient can be a series of electromagnetic frequencies that occur every 10 Hz in a range from about 10 Hz to about 2,000 Hz. In some embodiments, the patient-specific treatment protocol is administered to the patient. In some embodiments, the patient-specific treatment protocol is administered prior to or after a rest period of about ten minutes. In some embodiments, the patient-specific treatment protocol removes the frequencies that did not produce an alteration in the hemodynamic parameter (e.g. heart rate variability).

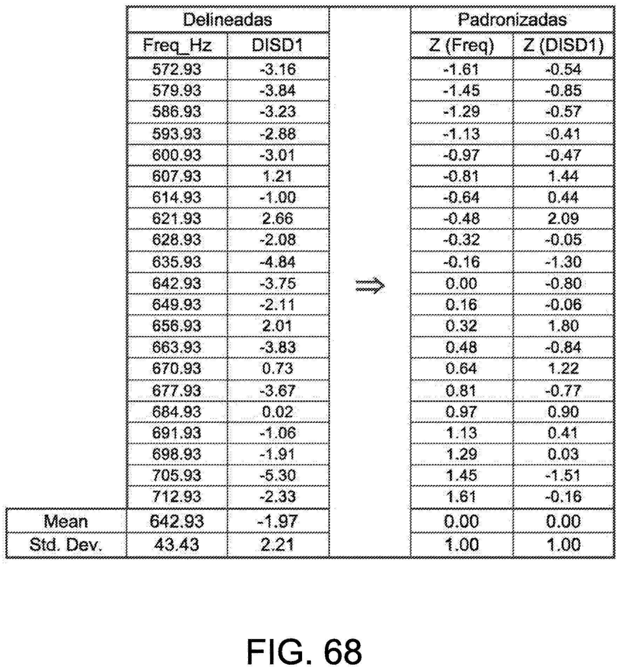

In some embodiments, new active frequencies are determined by the distribution of active frequencies, as illustrated in FIG. 68. In some embodiments, the distribution of active frequencies is transformed by the equation:

$X \cap N (\mu ; \sigma^2)$, wherein X is a random variable with normal distribution of mean $\mu$ and variance $\sigma^2$. So $\sigma = +\sqrt{\sigma^2}$ is the standard deviation. The Gaussian transformation that takes a random variable $X \cap N (\mu ; \sigma^2)$ to the standard normal distribution $Z \cap N (0 ; 1)$ can be: $Z_i (X) = x_i - \mu(x) / \sigma(x)$. $(\pi.1)$ (Standardized variable). In some embodiments, with this transformation of variables, a random variable $X$ with mean $\mu(x)$ and variance $\sigma^2(x)$ may be transformed into a random variable with mean $\mu(z) = 0$ and variance $\sigma^2(z) = 1$, whichever $\mu(x)$ is and $\sigma^2(x)$.

In some embodiments, the central limit theorem may provide a sample distribution of means wherein $Z(\overline{x_i}) = \overline{x_i} - \mu(x) / \sigma(x)/\sqrt{n} \cdot (\pi.2)$. In some embodiments, the sample distribution of means tends to a normal distribution as it grows: $Z(\overline{x_i}) \cap N (0 ; 1)$. In some embodiments, special groups of frequencies may be referred to as outlined frequencies of the form $\theta_i, i = 1, 2, ..., 2 \mu + 1$, where $\theta_i =$

$$\begin{cases} - (u - j) \delta, & se\ f < \tilde{f} \\ 0, & se\ f = \tilde{f} \\ + (u - j)\delta, & se\ f > \tilde{f} \end{cases} ;$$

$j = 0, 1, ..., (u$-$1)$
$f - (u$-$0)\delta, f - (u$-$1)\delta, f - (u$-$2)\delta, ..., f$-$\delta, f = \overline{f}, f$+$\delta, ..., f$+$(u$-$2)\delta, f$+$(u$-$1)\delta, f$+$(u$-$0)\delta (\pi.3)$ At where:

    $f$ may be an active frequency selected in the previous step. By construction $f = 103\overline{f}$ ;
    $\mu$ may be the number of frequencies that can be added below and above $f$ ; $(\pi.4)$
    $\delta$ may be the constant difference between these frequencies.

Then, the number of frequencies under study can be: $n = 2u + 1$.

By construction, the delineated frequency distribution ($\pi.3$) can have a mean $\overline{\theta} = \overline{f}$, where it can be naturally an active frequency $\overline{f} = f$ obtained in the previous exposures. Further, it may prove that its sample standard deviation can be:

$$. S(\theta) = \left[\frac{\delta^2}{6}(u+1)(2u+1)\right]^{1/2}(\pi.5)$$

The set of frequencies delineated may be normally distributed:

$$. \theta \cap N\{\overline{f}; \frac{\delta^2}{6}(u+1)(u+2)\}$$

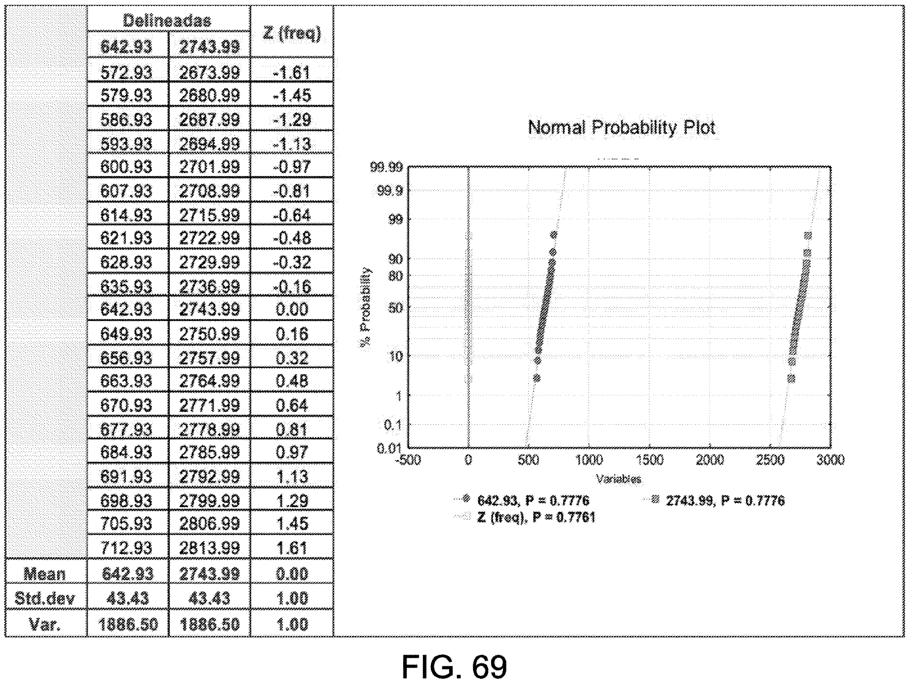

In some embodiments, normality may not occur with the baseline distal variables (DISD1, heart rate variability). In some embodiments, there may be occurrences of outliers. As illustrated in FIG. 69, two types of atypical values may be considered: the outliers and the influential points or leverage points.

In some embodiments, identification of outliers and influential points may be performed. In some embodiments, identification of outliers and influential points may be performed by the graphical criterion of Normal Probability Plot to detect potentially outliers and / or influential points, the studentized residue $(RS)_i$ and Cook's distance $(D_i)$ to "confirm or not" the influential points.

In some embodiments, a linear regression model may be considered:

$$y = X\beta + e \; ; \; \beta = \begin{bmatrix} \beta_0 \\ \beta_1 \end{bmatrix};$$

characterized by:

$$Z(DIDS1)_i = \beta_0 + \beta_1 (freq_{Hz})_i + e_i$$

(a) The observations (DISD1) submitted may be considered outliers. $\|RS\| > 2.0$
(b) Cook's distance to analyze the influence of the $i$ - order of the answer may be defined by:

$$D_i = \frac{r_i^2 \, h_{ii}}{k \, (l - h_{ii})^2}$$

Where, in a linear regression model of Z(DISD1) as a function of Z (freq):

$r_i$ can be the studies residue of the response;
$k$ can be the number of parameters of the model;
$h_{ii}$ the - nth diagonal value of the orthogonal projector of the vector of the responses on the space generated by the columns of the matrix $X$ of the design: $H = X(X'X)^{-1} X'$ also known as Hat matrix $y$.

In some embodiments, $(DISD1)_i$ can be influential if $D_i > \frac{2(k+1)}{2u+1}$

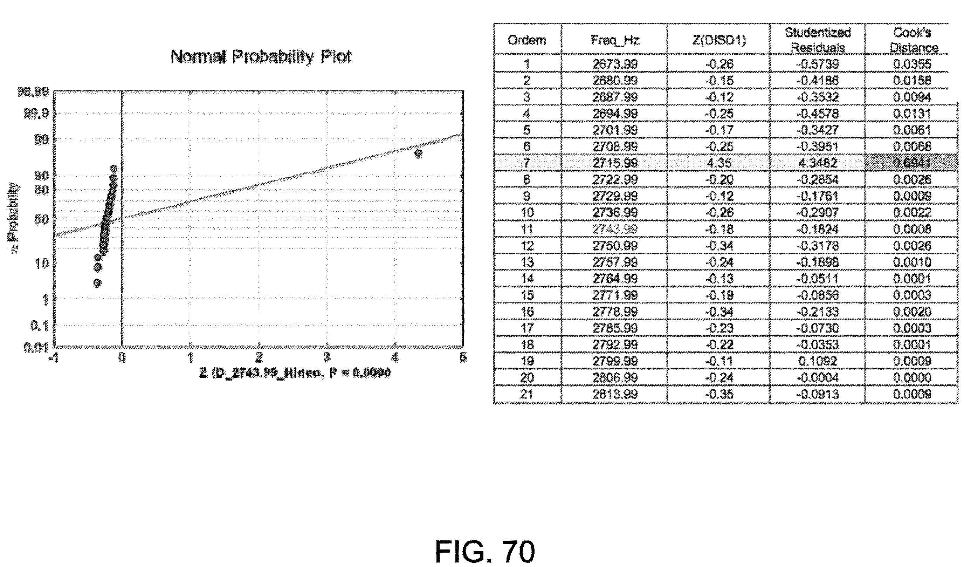

As depicted in FIG. 70, an influential point example of a normal probability plot construction, in accordance with embodiments disclosed above, may have a Delta for frequency response of about 3 Hz. In some embodiments, the delta for frequency response of about 3 Hz may be independent of frequency value.

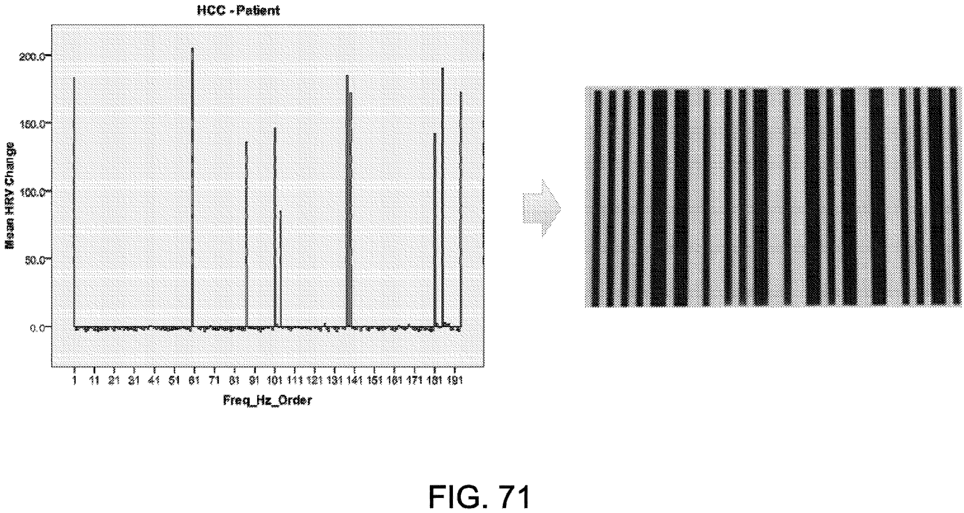

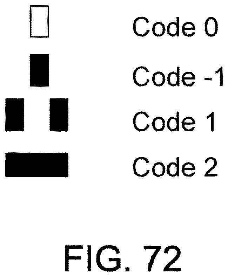

In some embodiments the patient can be relaxed in a supine position. An initial non-exposure period (i.e. rest) of about ten minutes can be included to allow for the patient to relax and establish baselines for various hemodynamic parameters and heart rate variability (HRV). After the initial non-exposure period, the patient may be exposed to a carrier frequency (i.e., at a constant amplitude without amplitude modulation) for about ten minutes. After the initial exposure period, the patient may be exposed a modulated frequency exposure period for about 120 minutes, about 60 minutes, about 35 minutes, or about 10 minutes. During this time, each of the set of frequencies (e.g., 194 frequencies or a series of electromagnetic frequencies that occur every 3 Hz or 10 Hz in a range from about 0.01 Hz to about 20 KHz, from about 10 Hz to about 1,000 Hz, or from about 10 Hz to about 2,000 Hz as described herein) can be applied to the patient for a particular period of time (e.g., 10 seconds per frequency or 3 seconds per frequency). In some embodiments, the modulated frequencies applied to the patient can be a series of electromagnetic frequencies that occur every 3 Hz in a range from about 0.01 Hz to about 20 KHz. In some embodiments, the modulated frequencies applied to the patient can be a series of electromagnetic frequencies that occur every 3 Hz in a range from about 10 Hz to about 1,000 Hz. In some embodiments, the modulated frequencies applied to the patient can be a series of electromagnetic frequencies that occur every 3 Hz in a range from about 10 Hz to about 2,000 Hz. In some embodiments, the modulated frequencies applied to the patient can be a series of electromagnetic frequencies that occur every 10 Hz in a range from about 0.01 Hz to about 20 KHz. In some embodiments, the modulated frequencies applied to the patient can be a series of electromagnetic frequencies that occur every 10 Hz in a range from about 10 Hz to about 1,000 Hz. In some embodiments, the modulated frequencies applied to the patient can be a series of electromagnetic frequencies that occur every 10 Hz in a range from about 10 Hz to about 2,000 Hz. The system can cycle repeatedly through each of the frequencies for the exposure period. After the initial exposure period of about 120 minutes, about 60 minutes, about 35 minutes, or about 10 minutes, the system may determine the set of frequencies (i.e. "active frequencies") that altered a hemodynamic parameter (e.g. heart rate variability). In some embodiments, the system may determine a set of new frequencies that altered or did not alter a hemodynamic parameter during the first exposure period. In further embodiments, the system may determine a frequency attribute to the set of frequencies that altered or did not alter a hemodynamic parameter. In some embodiments, the frequency attribute can be, as a non-limiting example, a bar coding system as depicted in FIG. 71. In some embodiments, the frequency attribute can be ascribed a code wherein the code can be code 0, code -1, code 1, code 2, or a combination thereof. As illustrated in FIG. 72, in some embodiments, the frequency attribute can be code 0 wherein code 0 is illustrated as 0 bar in the bar coding system, the frequency attribute can be code -1 wherein code -1 is illustrated as 1 bar in the bar coding system, the frequency attribute can be code 1 wherein code 1 is illustrated as 2 bars in the bar coding system, the frequency attribute can be code 2 wherein code 2 is illustrated as 3 bars, 3 sequential bars with no void in between the 3 sequential bars, in the bar coding system, or a combination thereof. In further embodiments, the code 0 may indicate a frequency did not produce a change in a hemodynamic parameter (e.g. heart rate variability), the code -1 may indicate a frequency produced a decrease in a hemodynamic parameter (e.g. heart rate variability), the code 1 may indicate a frequency produced an increase in a hemodynamic parameter (e.g. heart rate variability), and the code 2 may indicate a frequency produced a very high increase or decrease in a hemodynamic parameter (e.g. heart rate variability).

In some embodiments, the bar coding system has a frequency interval. In some embodiments, the bar coding system has a frequency interval of about 1 Hz, about 3 Hz, about 5 Hz, about 10 Hz, about 20 Hz, about 30 Hz, about 40 Hz, about 50 Hz, about 60 Hz, about 70 Hz, about 80 Hz, about 90 Hz, about 100 Hz, about 125 Hz, about 150 Hz, about 175 Hz, about 200 Hz, or any range in between inclusive. In some embodiments, the bar coding system has a frequency interval of about 100 Hz.

The Real-time Orchestrated Low energy Electromagnetic Exposure System (R.O.L.E.X. system), as described herein (e.g., system 900 as shown in FIG. 9B) can provide for storage of complete raw data from the monitoring device, radiofrequency generator command logs, merged and transformed data files for every patient exposure, and other related data. The data can be encrypted and stored in accordance with an Intelligent Dynamic Library format to facilitate data capture, new data processing and data retrieval to support a number of data analysis for future research activities. For quality control, all data can be organized to support independent data audit and data validation.

Additionally, the biological surrogate can be a biomarker that can be traceable in humans using a non-invasive technology. Like any other biomarker, this biological surrogate can provide proof (or reflect a physiological response to an intervention) that amplitude modulated electromagnetic fields have significant interference in an organ function or other aspects of health. Like any other biomarker, this biological surrogate may correlate with the risk or progression of a disease, or with the susceptibility of the disease to a given treatment.

The biological surrogate, by means of significant HRV changes during the exposure to specific frequency modulation identified by the R.O.L.E.X. system, can allow the construction of individual list of active frequencies modulation (able to cause change in HRV) to be used in precise and non-invasive systemic treatment modality. This technical solution supports the evaluation of an infinite number of patients and, consequently, can generate a large volume of data for data mining and machine learning.

Besides the instant construction of cancer specific frequencies in different tumor types as described herein, the biological surrogate can be studied as a potential prognostic and predictive cancer marker with an immediate potential clinical application.

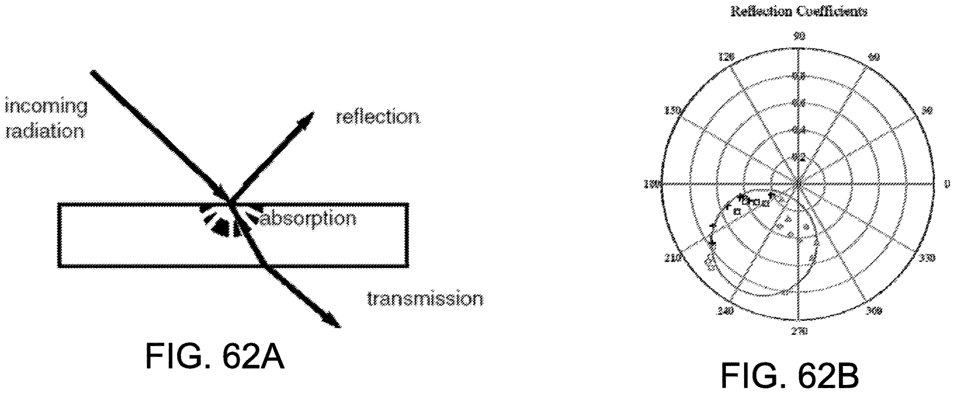

In some embodiments, the AM RF EMF frequencies can be absorbed, transmitted or reflected in a subject (FIG. 62A). In some embodiments, the reflected frequencies may have a reflection coefficient (FIG. 62B). In some embodiments, the reflected frequencies can be measured. In further embodiments, the reflected frequencies can be used to determine the diagnosis of a patient. In some embodiments, the reflected frequencies can be used to auto tune the AM RF EMF frequencies in which a patient is exposed to. In some embodiments, a subject with a diagnosis of a disease may have a change in their reflection frequency compared to healthy controls. In some embodiments, a subject with a diagnosis of hepatocellular carcinoma may have a reduction in their reflection frequency compared to healthy controls (FIGS. 63A-D).

In some embodiments, cancer cells exposed to 27.12 MHz radiofrequency electromagnetic fields may replicate *in vitro* or *in vivo* conditions. In some embodiments, the cancer cells are hepatocellular carcinoma (HCC) cells. In further embodiments, cancer cells can be exposed to tumor-specific modulation frequencies that may have been previously identified by biofeedback methods in patients with a diagnosis of cancer. In some embodiments cancer that are exposed to tumor-specific modulation frequencies may undergo cell death, modify their proliferative capacity, or a combination thereof. Control modulation frequencies may consist of randomly chosen modulation frequencies within a range of about 100 Hz to about 21 kHz, about 0.01 Hz to about 20 KHz, about 10 Hz to about 1,000 Hz, or about 10 Hz to about 2,000 Hz. In some embodiments, the modulated frequencies applied to the patient can be a series of electromagnetic frequencies that occur every 3 Hz in a range from about 0.01 Hz to about 20 KHz. In some embodiments, the modulated frequencies applied to the patient can be a series of electromagnetic frequencies that occur every 3 Hz in a range from about 10 Hz to about 1,000 Hz. In some embodiments, the modulated frequencies applied to the patient can be a series of electromagnetic frequencies that occur every 3 Hz in a range from about 10 Hz to about 2,000 Hz. In some embodiments, the modulated frequencies applied to the patient can be a series of electromagnetic frequencies that occur every 10 Hz in a range from about 0.01 Hz to about 20 KHz. In some embodiments, the modulated frequencies applied to the patient can be a series of electromagnetic frequencies that occur every 10 Hz in a range from about 10 Hz to about 1,000 Hz.. In some embodiments, the modulated frequencies applied to the patient can be a series of electromagnetic frequencies that occur every 10 Hz in a range from about 10 Hz to about 2,000 Hz. The alternating fields may interfere with polymerizing and depolymerizing electrostatics of microtubules due to the force of the alternating fields acting on the tubulin dimers that make up the microtubules. The tubulin dimers' dipole moment could be affected by these external fields, especially in mitosis due to decreased shielding. Theoretical calculations may explain how electrostatic forces generated by microtubules are at work during mitosis and influence chromosomal motion, or suggest other effects that could be involved when alternating fields are applied to cells in mitosis.

In some embodiments, a cancer cell may have a rate of energy metabolism. In further embodiments, a cancer cells rate of energy metabolism may be altered when compared to a non-cancer cell. In further embodiments, a cancer cells altered rate of energy metabolism may indicate a phase transition. In further embodiments, a phase transition may include a cell transitioning from a non-excitable cell to an excitable cell. In some embodiments, a phase transition may include a cell transitioning from an excitable cell to a non-excitable cell. In further embodiments, a cancer cell may be an excitable cell that absorbs an electromagnetic field. In additional embodiment, a cancer cell may be an excitable cell that absorbs more of an electromagnetic field when compared to a non-cancerous cell, a non-excitable cell, or a combination thereof. In some embodiments, a carrier frequency of an electromagnetic field may provide a microtubule an increase in sensitivity to an amplitude modulated frequency.

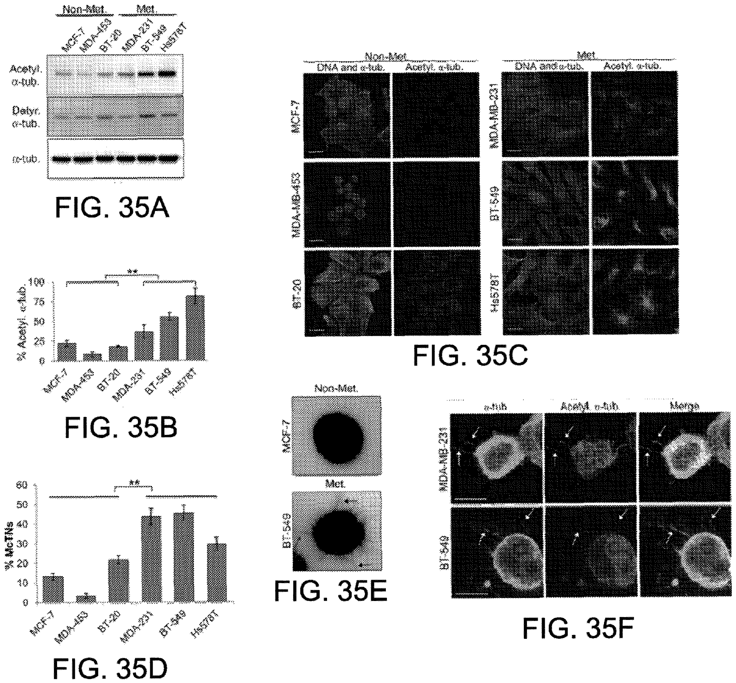

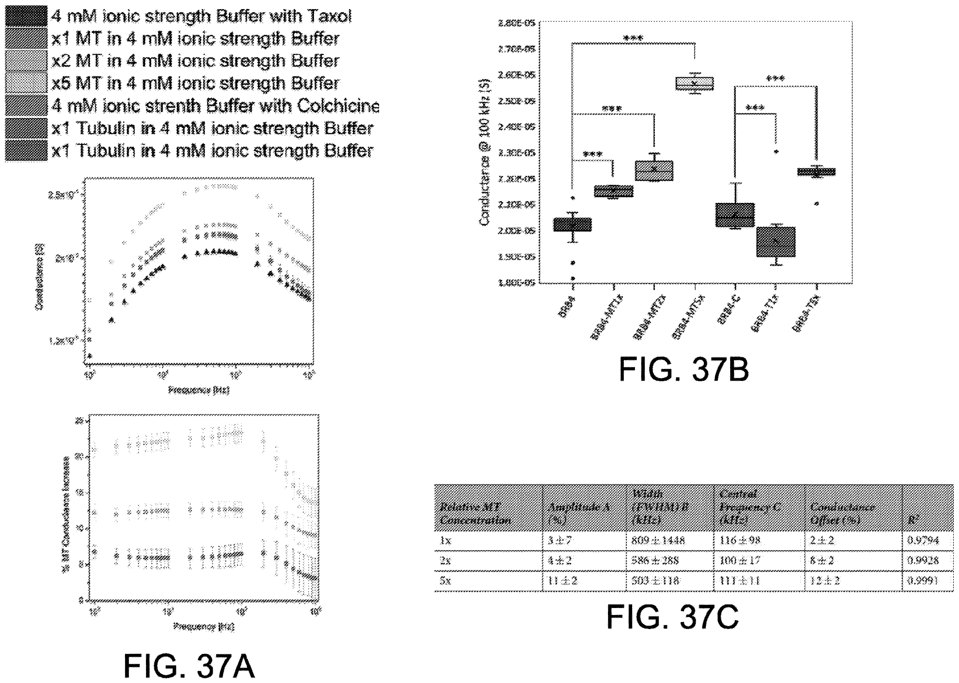

In some embodiments, biological polymers may be made up of various proteins, such as actin or tubulin, or nucleic acids such as DNA or RNA (FIG. 35-36). These structures may have uncompensated electrical charges when immersed in water but ionic solutions such as the cytoplasm may provide a bath of counter-ions that at least partially may neutralize the net electric charge. This may result in dipolar and higher-moment electric field distributions (FIG. 37). Biological water may create structures with ordered dipole moments and complex dynamics at multiple scales. Further, free ions may endow the cell with conducting properties along polymeric pathways as well as in a diffusive way. Membranes may support electric fields, which, due to screening, may exponentially decay but do not completely disappear when measured in the cell interior.

In further embodiments, proteins in organisms may have semiconducting properties. Protein conductivity can be dependent on the hydration state of proteins.

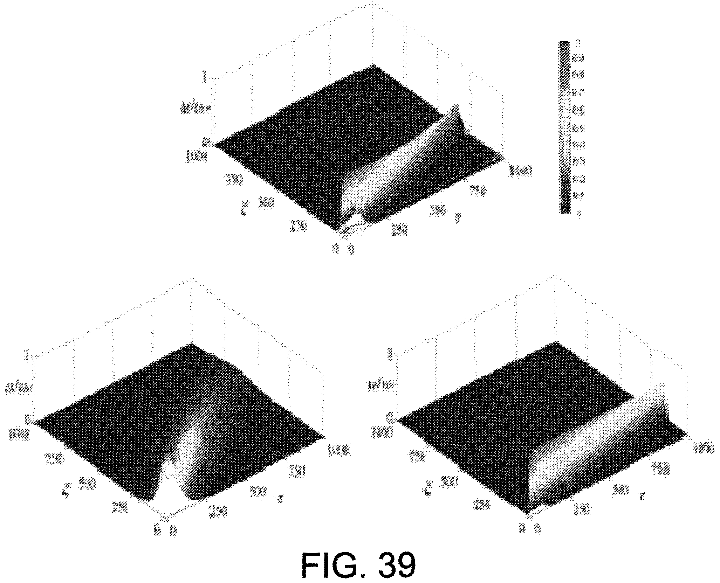

In some embodiments, organisms may conduct electricity for the role of membrane potentials, ionic currents, and endogenous electromagnetic fields in proliferation, morphogenesis, and regeneration. Water may also transmit electrical pulses due to the structure imparted by hydrophilic surfaces. Charge carriers related to protein semiconduction can be electrons, protons or ions surrounding proteins in physiological solution. This protonic conduction in proteins may be found for collagen, keratin, cytochrome c and hemoglobin. In some embodiment, actin filaments (AFs) and microtubules (MTs) may be implicated in numerous forms of electrical processes involving ionic and electronic conduction. In further embodiments, AFs and MTs may support dipolar and/or ionic kink-like soliton waves, which may travel at speeds in the range of about 2-100 m/s (FIG. 39).

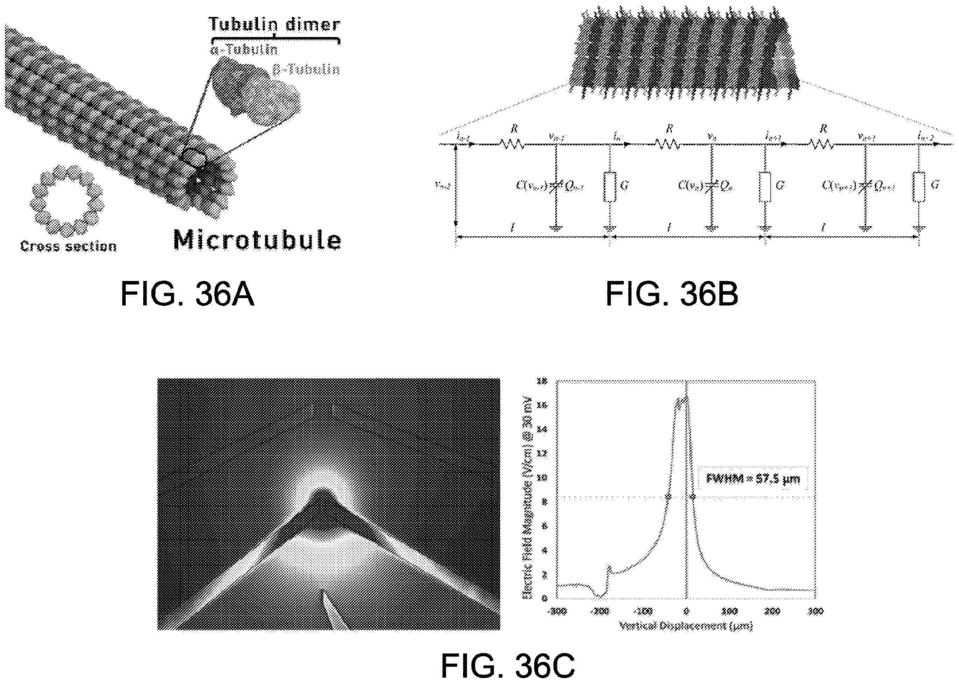

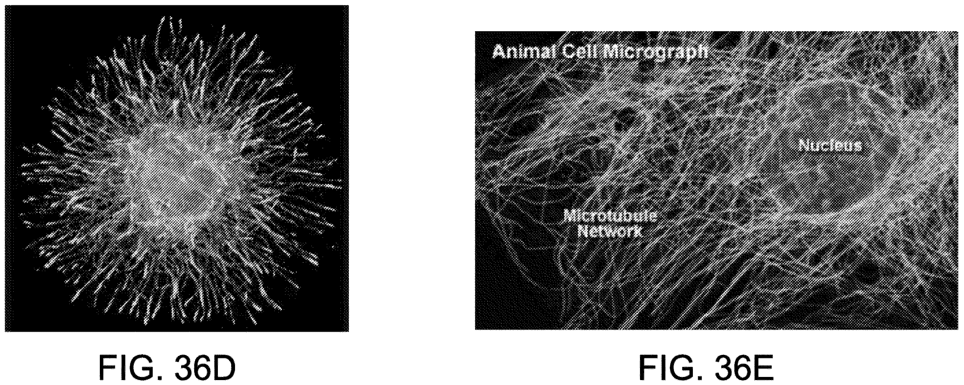

In some embodiments, MTs can be made from polymerized tubulin (FIG. 36). MTs can maintain the structure of the cell and may provide tracks for intracellular transport, and in creating the mitotic spindle in cell division. The building block of a MT can be a tubulin dimer having about 900 amino acid residues and may comprise about 14,000 atoms with a combined mass of about 110 kDa. The tubulin dimer can be made up of two distinct monomers called $\alpha$ and $\beta$ tubulin. Further, each of the tubulin monomers can be expressed by a different gene leading to a set of tubulin isotypes expressed differently by different cells.

In further embodiments, each dimer in the MT may have a length of about 8 nm along a MT cylinder axis, a width of about 6.5 nm and the radial dimension of about 4.6 nm. The inner core of the cylinder, known as the lumen, is about 15 nm in diameter. The wall of MTs is mostly characterized by a so-called B-lattice structure, which is approximately hexagonal with at least one protofilament forming a seam, thus having an offset between two neighboring dimers meeting at the seam.

In some embodiments, tubulin's electric charges may depend on the isotypes used and range from about -5 to about -35 elementary charges. The dipole moments can vary from about 500 to about 4000 debye.

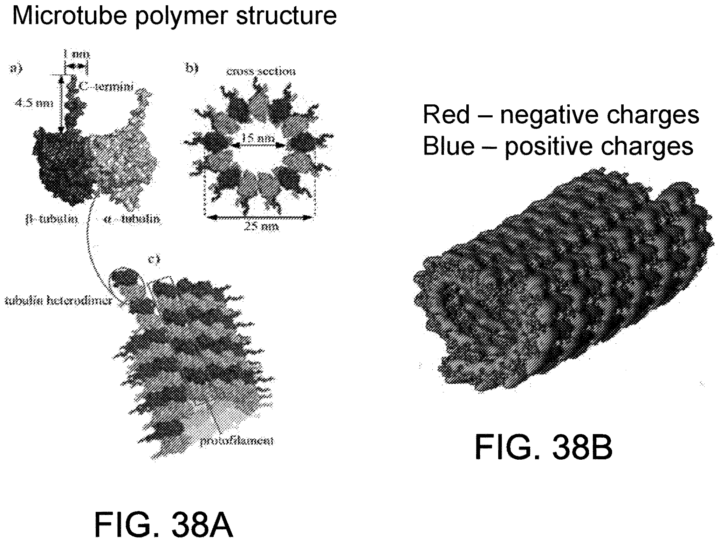

In some embodiments, microtubules may exhibit intrinsic conductivity as well as ionic conductivity along their length. Intrinsic conductivity may include electronic conductivity through the macromolecule itself, with electrons traversing through a semiconducting property of tubulin. In further embodiments, due to the large electric charges on tubulin, microtubules may have an electro-negatively charged outer surface as well as C-terminal tails (TTs), resulting in a cloud of counter-ions surrounding them (FIG. 38). Ionic waves may be amplified along MTs. In further embodiments, MTs may form a cylinder with a hollow inner volume (lumen) and may have conducting properties involving the lumen.

Table 2 summarizes data that measures the various conductivities of microtubules. In some embodiments, conductivities along MTs are may not be length dependent which may indicate a non-Ohmic resistance.

Table 2. Conductivity data for microtubules

Table 2

| Method used | Conductivity type | Conductivity $\sigma$ (S/m) |
|---|---|---|
| Electro-orientation | Ionic | 0.15 |
| AC electro-kinetics | Ionic | 0.25 |
| TMR-coated, 4-point probe | Ionic | ~40 |
| Cytoplasm | | 2.5 |
| dry | Intrinsic | Less than 3 |
| Axoplasm | | 3.4 |
| DC | Intrinsic | 0.6 to 136 |
| Gold electrodes, channel | Ionic/Intrinsic | Less than 80 |
| Patch-pipette | Ionic | 367 |
| Air | | 200-2000 |
| AC. Water inside. | Intrinsic | $10^3 - 10^5$ |
| RF reflectance spectroscopy | Ionic/Intrinsic | $1\text{-}5 \times 10^6$ |
| Kiethley semiconductor characterization device | Ionic/protonic | 20 S/m |

In some embodiments, MTs may increase their ionic conductivity compared to the buffer solution free of tubulin. In further embodiments, MTs conductivity may increase to about 15-fold compared to that of the surrounding solution. In some embodiments, MTs may amplify ionic charge conductivity having a current transmission increase by about 69% along the MTs in a buffer similar to that of the intracellular ionic concentration, using about 135 mM KCl. In further embodiments, ionic current amplification along MTs may be explained by the highly negative surface charge density along the outsides of microtubules that may create a counter-ionic cloud, which may allow amplification of axially transferred signals. In some embodiments, the MTs have a conductivity of about 367 S/m.

As depicted in FIG. 37, in some embodiments, microtubule electrical conductibility is increased in an ionic buffer when the concentration of microtubules is increased.

In some embodiments, microtubule conductivity is measured in solution and an increased ionic conductivity due to a counter-ion cloud formed around the highly electronegative surface of MTs is measured. In further embodiments, dry protein conductivity is measured. In some embodiments, the conductivity may be due to the microelectrodes and not the protein filament. In further embodiments, MTs are adsorbed to a glass substrate and yield an intrinsic conductivity of about less than about 3 S/m. In some embodiments, MTs intrinsic conductivity is measured in an ultra-pure water solution that bridge gold electrodes. In some embodiments, MT ionic conductivity is measured along the periphery of MTs. In further embodiments, MTs are adsorbed onto glass and HOPG substrates and a four-probe measurement is performed of DC and AC conductivities. In some embodiments, the DC intrinsic conductivity of MTs, from a gap of about 200 nm, can be in the range of about $10^{-1}$ to about $10^2$ S/m. In some embodiments, MTs in several AC frequency ranges become about 1000 times more conductive and having values for the MT conductivities in the range of about $10^3$ to about $10^5$ S/m. In further embodiments, resonance peaks for solubilized tubulin dimers can be about 37 MHz, about 46 MHz, about 91 MHz, about 137 MHz, about 176 MHz, about 281 MHz, about 430 MHz, about 9 GHz, about 19 GHz, about 78 GHz, about 160 GHz, about 224 GHz, about 28 THz, about 88 THz, about 127 THz, or about 340 THz. In some embodiments, MTs may have resonance peaks of about 120 kHz, about 240 kHz, about 320 kHz, about 12 MHz, about 20 MHz, about 22 MHz, about 30 MHz, about 101 MHz, about 113 MHz, about 185 MHz, about 204 MHz about 3 GHz, about 7 GHz, about 13 GHz, about 18 GHz. In some embodiments, MTs having an ionic conductance may overlap with the 100 KHz range which may indicate a sensitivity of MTs to this frequency range. In some embodiments, the water channel inside the MT may have high conductivity of the MT at specific AC frequencies. In further embodiments, MTs have a ballistic conductivity in the range of about 12 to about 30 MHz. In some embodiments, thin films of water can be adsorbed onto a solid surface and may have high conductivity measured by using a scanning tunneling microscope (STM). In some embodiments, MTs may have an AC conductivity of about 1 kHz to about 1 MHz range and found it to be about 20 S/m.

In some embodiments, MTs conductivity and dielectric constant may be measured using an electro-orientation method. In further embodiments, in the absence of electric fields, MTs exhibit random 'Brownian' movements. In some embodiments, in fields with AC frequencies below about 10 kHz MTs may exhibit a flow motion as a result of ionic convection. In further embodiments, the convection effect can be avoided by applying electric fields with a frequency greater than about 10 kHz. In some embodiments, a field strength above about 500 V/cm and frequencies in the range of about 10 kHz to about 5 MHz MTs may be oriented in solution by an electric field. In some embodiments, a field of about 90 kV/m at about 1 MHz, MTs may aligned within several seconds. In further embodiments, MT ionic conductivity can be about 150 mS/m. In some embodiments, tubulin has a dielectric constant of about $\varepsilon = 8.41$.

In some embodiments, resistance values can be measured in microtubules, both intrinsically and as ionic conduction cables. In some embodiments, electrical contacts can be made to single microtubules following dry-etching of a substrate containing gold microelectrodes. In further embodiments, the intrinsic resistance of a microtubule of about 12 $\mu$m in length is in the range of about 500 M$\Omega$ giving a value of resistivity of about 40 M$\Omega/\mu$m in a dry state. In further embodiments, measurements can be performed on gold-coated microtubules which may be covered with a layer of gold following sputtering of about 30-nm. In further embodiments, the resistance of a configured metallized MT can be below about 50 $\Omega$.

In some embodiments, conductivity of MTs can be measured by radio frequency reflectance spectroscopy. In further embodiments, the conductivity of MTs can be about $10^6$ S/m. In some embodiments, conductivity of MTs can be measured by RF reflectance spectroscopy of MT samples containing a buffer solution, free tubulin in buffer, microtubules in buffer, and, microtubules with MAP's in buffer. The concentration of tubulin can be about 5 mg/ml and in the concentration of MAP2 and tau protein can be about 0.3 mg/ml. The average DC resistance can be about 0.999 k$\Omega$ (buffer), about 0.424 k$\Omega$ (tubulin), about 0.883 k$\Omega$ (microtubules) and about 0.836 k$\Omega$ (MT's + MAP's). In some embodiments, the resistance of a microtubule of about 10 $\mu$m in length, having a basic electrical element in such a circuit, can be about 8 M$\Omega$ resistance. In some embodiments, a microtubule of about 1-$\mu$m in length may be in the range of about 200 k$\Omega$, and a microtubule of about 10-$\mu$m may have an intrinsic resistance of about 2 M.

Table 3 illustrates various conductivities through microtubules.

Table 3

| Type | Result |
| --- | --- |
| Intrinsic | Conductivity $10^5$-$10^9$ S/m using different assumptions |
| Ionic | Ionic pulse velocity: 0.26 m/s |
| Ionic, C-termini | Importance of C-termini and MAP2 in transmitting ionic signals |

In some embodiments, MT conductance may occur along the outer rim of the MT, intrinsic conductivity through the MT itself, and possible proton jump conduction and conductivity through the inner MT lumen.

In some embodiments, an electrical field can be measured around a MT. In some embodiments, MTs can be ferroelectric. In some embodiments, electric fields can be applied in the range of about 300 V/m to suspended microtubules, which move at pH of about 6.8 from the negative electrode to the positive one indicating a negative net charge. In further embodiments, an electrophoretic mobility of about 2.6x10-4 cm2/Vs may be determined. In some embodiments, MTs may align in parallel arrays due to the application of electric fields. The electric field intensities may be in a range of about 20 V/m to about 50 V/m and can be of pulsed shape.

In some embodiments, MTs in solution can be taxol-stabilized and exposed to an AC field. In some embodiments, MTs display an electro-osmotic or a electro-thermal flow, as well as a MT dielectrophoresis effect. In further embodiments, the conductivity of MTs can be about 0.25 S/m at about 5 MHz.

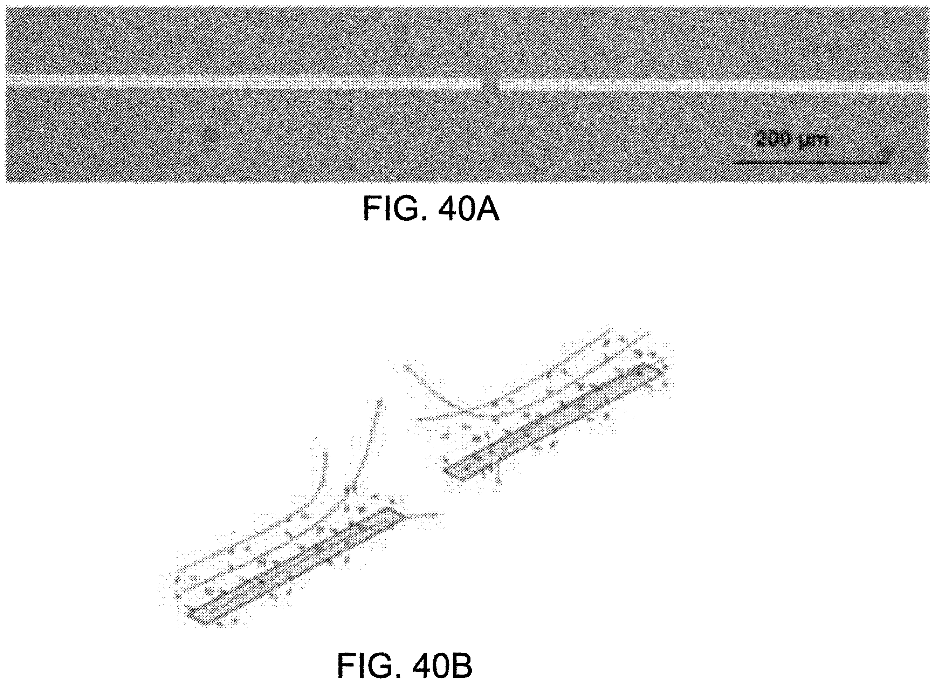

FIG. 40A depicts pair of electrodes fabricated on a glass surface to observe response to AC voltage. Electrode dimensions are about: 15 $\mu$m $\times$ 12mm, the gap: 20$\mu$m, and a 40 V voltage bias is applied.

FIG. 40B depicts a schematic representation of a microtubule response to AC signals.

In some embodiments, below about 500 kHz microtubules may flow toward the centerline of electrodes. The *electro-osmotic force* may cause the movement of the fluid in a vortex-like manner. This represents the Coulomb-force experienced by the ionic fluid due to the applied voltage.

In some embodiments, the fluid flow velocity may be proportional to the electric field amplitude E, surface charge density $\sigma$, inversely proportional to viscosity $\eta$ and the inverse Debye length $\kappa$ such that: $v=E\,\sigma/\kappa\eta$. In some embodiments, at lower frequencies, flow velocity is larger. In further embodiments, the *electro-thermal force* may cause motion of microtubules along the length of the electrodes due to strong heating effects of the AC field.

In some embodiments, above about 500 kHz, microtubules may flow toward the gap between the electrodes due to *dielectrophoresis.* This is the force experienced by microtubules in a non-uniform electric field and given by the formula:

$$\langle F_{DEP} \rangle = \frac{1}{4} v\varepsilon_{\mathrm{m}} \left[ \frac{\omega^2 \varepsilon_{\mathrm{m}} \left( \varepsilon_{\mathrm{p}} - \varepsilon_{\mathrm{m}} \right) + \sigma_{\mathrm{m}} \left( \sigma_{\mathrm{p}} - \sigma_{\mathrm{m}} \right)}{\omega^2 \varepsilon_{\mathrm{m}}^2 + \sigma_{\mathrm{m}}^2} \right] \nabla \left| E \right|^2$$

where the symbols with subscript "m" refer to the medium and "p" to the particle. As such, this process may be driven by a difference between the conductivities and permittivities of the microtubules and the medium, ($\sigma_{\mathrm{p}} - \sigma_{\mathrm{m}}$) and ($\varepsilon_{\mathrm{p}} - \varepsilon_{\mathrm{m}}$), respectively. Lowering the pH of the solution to the isoelectric point of MTs of about pH 5 may reduce this effect and additionally lowering the frequency may reduce it further due to the dependence of the first term on the square of the frequency. The difference between the dielectric constant will remain (about 7.8 for water and about 8.4 for tubulin).

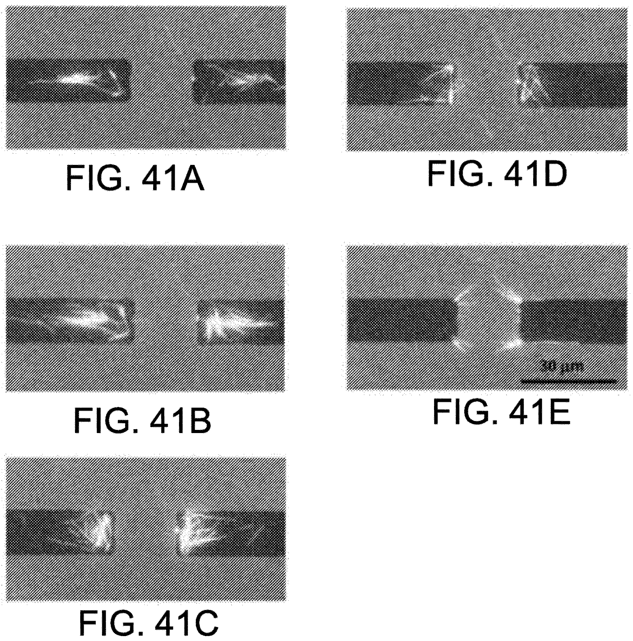

FIGS. 41A-E depicts the geometries of the MT bundles near the electrodes at different field frequencies: A=100kHz, B=250 kHz, C=500 kHz, D=1 MHz, and E=2.5 MHz. In some embodiments, at about 5 MHz, the electro-osmotic and electro-thermal flow may balance each other out, the flow of microtubules may now be due to dielectrophoresis. In some embodiments, the geometry of the MTs in the vicinity of the electrodes may have a curvature that is opposite to that seen in mitotic spindles which may indicate that such high frequency fields could disrupt mitotic spindle formation. Further, FIGS. 41A-E provide an independent measurement of the MT conductivity as $\sigma_{\mathrm{m}}$ about 250 mS m$^{-1}$ as depicted in Table 2 above.

It is important to compare the dielectrophoretic force to Brownian motion in order to be able to evaluate whether or not electric fields are sufficiently strong to overcome random motion. The easiest way to make this comparison is to determine if the dielectric potential exceeds the thermal energy, i.e.:

$$\pi\, r^3\, \varepsilon_{\mathrm{m}}\, [(\varepsilon_{\mathrm{p}}-\varepsilon_{\mathrm{m}})/(\varepsilon_{\mathrm{p}} + 2\varepsilon_{\mathrm{m}})]\, E^2 > kT$$

where $\varepsilon_{\mathrm{m}}$ is the dielectric constant of the medium and $\varepsilon_{\mathrm{p}}$ is the dielectric constant of the particle. E is the electric field strength and r the radius of the particle. Taking as an example a tubulin dimer in solution and the corresponding values of the dielectric constants, E may exceed about 25 V/m for the field to be effective in orienting polarizable tubulin dimers. Similarly, for a microtubule of about 10-$\mu$m in length, replacing the factor $\pi\, r^3$ with $\pi\, r^2\, L$, where r is the radius of a MT (12.5 nm) and L its length, a condition that E > 1 V/m may be obtained. The electric field values of about 100 V/m may be sufficient to exert electrophoretic effects on tubulin and MTs. In some embodiments, the longer the microtubule, the more pronounced the dielectrophoretic effect could be predicted to occur.

In some embodiments, MT electric properties can be composed of both intrinsic protein effects and ionic effects due to condensed counter-ion clouds, which could arise due to the electrostatic attraction of positive ions by the negative surface charge of MTs. In some embodiments, aligned MTs can assemble *in vitro* in the presence of electric fields with strengths in a range of about 10 V/cm to about 300 V/m (FIG. 36). In some embodiments, MTs can be ferroelectric. In further embodiments, electric fields can be applied to suspended MTs and to MTs interacting with a kinesin-coated glass surface. In suspension, MTs without MT-associated proteins (MAPs) may move at pH of about 6.8 from a negative electrode to a positive electrode which may suggest a negative net charge and may suggest an electrophoretic mobility of about $2.6\times10^{-4}$ cm$^2$/Vs was determined.

In some embodiments, MTs can be labelled with DNA to adjust the amount of net charge and observe the mobility of these MT structures. This was compared to control where MTs where only labelled fluorescently with two different tags. In some embodiments, control MTs may have an electrophoretic mobility of about $2\times10^{-8}$ m$^2$ V$^{-1}$ s$^{-1}$. For

filed strengths of about 100 V/m, one can estimate the average velocity of MT translocations as about 2 $\mu$m/s with $\lambda_D$ = 0.74 nm as the Debye length, $\eta$ = 8.90 $\times$ 10$^4$ kg m$^{-1}$ s$^{-1}$ and $\varepsilon$ = 6.93 $\times$ 10$^{-10}$ C V$^{-1}$ m$^{-1}$ as the viscosity and dielectric constant of the buffer. The estimated effective charges of the TAMRA- and AlexaFluor 488-tagged tubulin dimer can be about 10 e$^-$ and about 9.7 e$^-$.

In some embodiments, ionic waves may trigger MTs C-termini to change from upright to downward conformations. This model considers MAP2 to function as an ionic wave-guide that may transfer a conformational change in a C-terminus state to an adjacent MT. Perturbations applied to condensed counter-ions at the end of MAP2 may force them from equilibrium initiating a travelling wave. This wave may travel as a "kink" solitary wave with a phase velocity of $v_{ph}$ = 2 nm/ps. A time scale for C-termini motion could be about 100 ps, which could be is too fast for a 100 kHz frequency range.

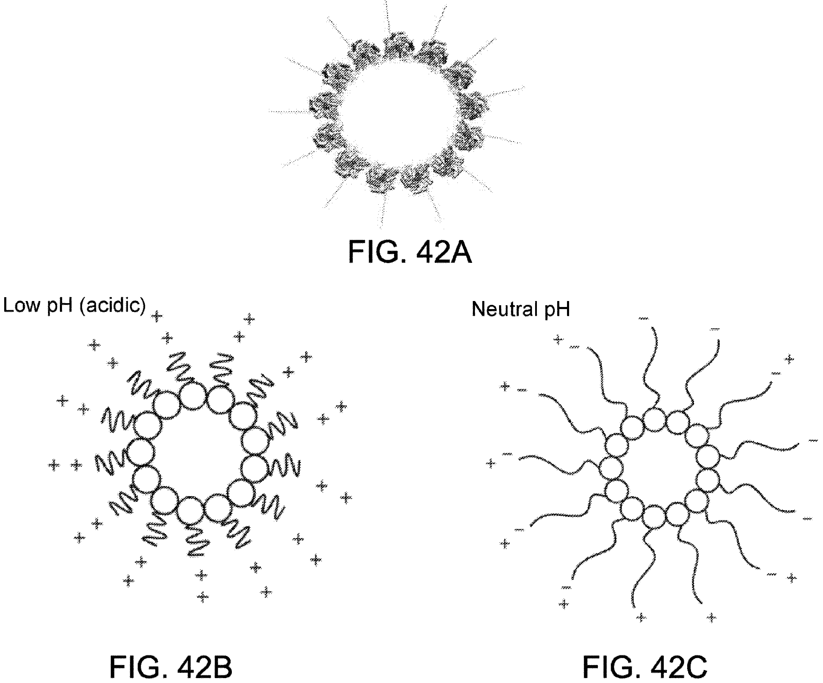

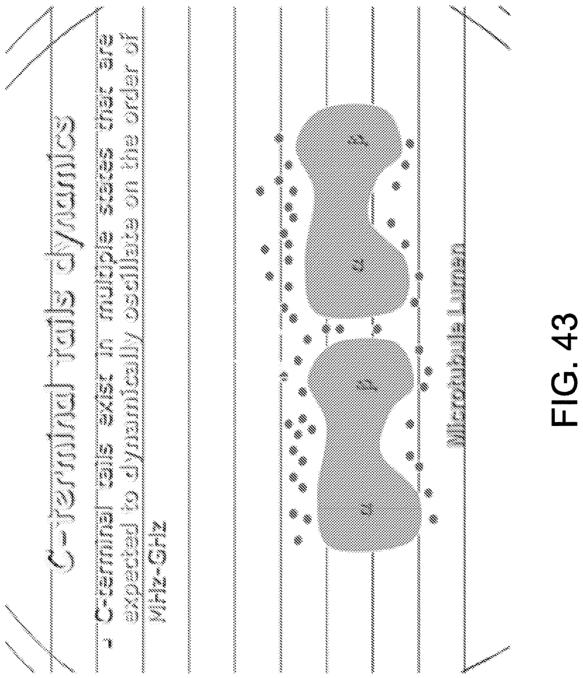

In some embodiments, a MT C-termini being flexible and charged, with about 40% of the tubulin's charge located at the C-termini, could dynamically respond to electric fields as depicted in FIGS. 42A-C where local changes of pH may be correlated with positive and negative electric field's polarities, respectively. In further embodiments, this effect may cause MT instability as well as interference with motor protein transport. In some embodiments, the C-termini may oscillate dynamically in a range between MHz and GHz (FIG. 43).

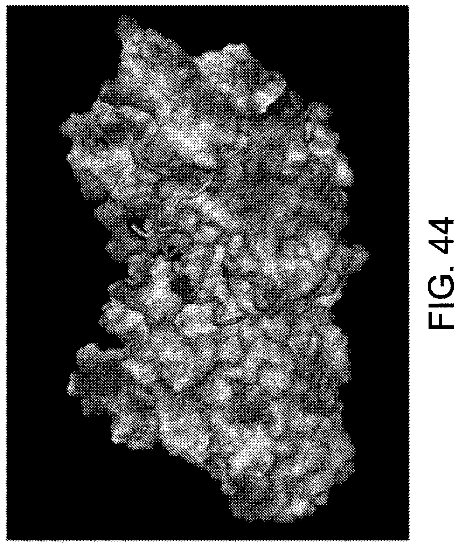

In some embodiments, a stable dimer conformation may have C-termini cross-linked between the monomers (FIG. 44). In further embodiments, a cross-linked conformation of the C-termini stabilizes a straight orientation of a tubulin dimer.

In some embodiments, H-bond strength in MTs can range from about 11.9 k/mol to about 42.2 kJ/mol. In further embodiments, H-bond strength in MTs can have a total of about 462 kJ/mol for the $\alpha$-tubulin/$\beta$-tubulin interactions and about 472 kJ/mol for the $\beta$-tubulin $\beta$-tubulin interactions, which translates based on the Planck relationship between frequency and energy into a range of frequency values between about $0.3 \times 10^{14}$ Hz and about $1.3 \times 10^{15}$ Hz. Therefore, hydrogen bond breaking as a result of MHz range carrier waves is not expected to occur.

In some embodiments, electromagnetic resonances may occur in biological molecules including proteins, DNA and RNA in the THz, GHz, MHz and kHz ranges. In some embodiments, a resonant recognition model (RRM) can be based on the distribution of energy of delocalized proteins in a biological system and charge transfer under resonance with a velocity of about $7.87 \times 10^5$ m/s and may covering distances of about 3.8A between amino acids, giving a frequency in a range of about $10^{13}$ to about $10^{15}$ Hz. In some embodiments, a charge transfer velocity may have various resonant frequencies. In some embodiments, a charge transfer velocity of about v=0.0005 m/s may produce an EMF in the range of about 108 to about 325 kHz for telomerase reverse transcriptase (TERT), TERT mRNA, or a Telomere. This velocity may correspond to the propagation of solitons on $\alpha$-helices. In some embodiments, a ranges of resonant frequencies of about 97 to about 101 THz, about 340 to about 350 THz, or about 445 to about 470 THz can be predicted by the RRM approach for tubulin and microtubules. In some embodiments, electromagnetic dipole-dipole interactions having a high frequency may lead to an attraction between oscillating dipoles over distances that is comparable to the size of the cell.

In some embodiments, polyelectrolytes may have condensed ions in their surroundings. In further embodiments, counter-ions may "condense" along a stretch of polymer if a high linear charge density is present on the polymer's surface. In some embodiments, a linear polymer may be surrounded by counter-ions from a saline solution such that counter-ions are more closely surrounding the polymer's surface, and co-ions of a salt solution can be repelled such that a depletion area is created. In further embodiments, the sum of surface charges and associated counter-ions may drop to a value given by a formula that depends on the valence of the counter-ions and the Bjerrum length. The Bjerrum length is a phenomenological property of the ion's ability to compensate for the surface charges, depending on the solvent and temperature of the solution. The Bjerrum length, $\lambda_B$, describes the distance beyond which thermal fluctuations are stronger than the electrostatic attractions or repulsions between charges in solution whose dielectric constant is $e$ for a given temperature $T$ in Kelvin. Here $e$ is the electronic charge, $\varepsilon_0$ the permittivity of the vacuum and $k_B$ is Boltzmann's constant. Counter-ion condensation may occur when the mean distance between charges, $b$, is such that $\lambda_B/b=S>1$. Assuming for simplicity a linear charge distribution about the actin filament, the linear charge density, $\xi$, is much greater than $1/z$ where $z$ is the valence of the counter-ions in solution. The parameter, $\xi$, is given by:

$$\xi = \frac{e^2}{4\pi [epsilon]_0 [epsilon]_r k_B T b}$$

where $e$ is the electronic charge, $[epsilon]_r$ the dielectric constant of water, and $b$ the average axial spacing of charges on the poly electrolyte. In some embodiments, the cylindrical volume of depleted ions outside the cloud of ions surrounding the polymer may serve as an electrical shield. The "cable-like" behavior of such a structure may be supported by the polymer itself, and the "adsorbed" counter-ions could be bound to the polymer. In some embodiments, the strength of this interaction could be such that even under infinite dilution conditions (i.e. ionic strength >0), the counter-ions could be attracted to the polymer and may not diffuse out from the polymer's vicinity.

In some embodiments, F-actin or microfilaments can be about 7nm in diameter having a helix repeating about every 37nm. F-actin or microfilaments may have a high electrostatic charge density and may be able to conduct ionic currents through a counter-ionic cloud. Actin may account for about 1-5% of cellular protein, and up to about 10% of cellular protein in muscle cells.

In some embodiments, actin filaments can be one-dimensional polymers with an uneven distribution of electric charges along the polymer's length giving rise to a spatially dependent electric field arranged in peaks and. This may provide large changes in the density of small ions around the polymer with a large dielectric discontinuity in the ionic distribution. This uneven ionic distribution along a short stretch of the polymer (the average pitch about 35 to about 40 nm) can be an electrical circuit with non-linear components which may include (a) a non-linear capacitor associated with the spatial charge distribution between the ions located in the outer and inner regions of the polymer (b) an inductance and (c) a resistor. F-actin may act as a polyelectrolyte which may contain a fraction of its surrounding counter-ions in the form of a condensed cloud about its surface. Such a cloud may be highly insensitive to large changes in the ionic strength of the surrounding saline solution. Due to the presence of the sheath of counter-ions around the actin filament, these polymers may act as biological "electrical wires", and can be modeled as non-linear inhomogeneous transmission lines propagating non-linear dispersive solitary waves.

In further embodiments, long-range ionic wave propagation along actin filaments (and also microtubule networks) may lead to various synchronization functions including subcellular control of ionic channel activity. Cytoskeletal biopolymers, including actin filaments (AFs) and microtubules (MTs), may constitute the backbone for wave propagation, and in turn may interact with membrane components to modulate synaptic connections and membrane channels. In further embodiments, a direct interaction between AFs and ion channels may exist along with a regulatory functional role associated with actin suggesting the cytoskeleton may have a direct connection to membrane components, in particular channels and synapses. In further embodiments, the condensed cloud of counter-ions may separate the filament core from the rest of the ions in the bulk solution and may act as a dielectric medium between the two. It may have both resistive and capacitive components associated with each monomer that makes up the AF. Ion flow is may occur at a radial distance from the surface of the filament approximately equal to the Bjerrum length. An inductive component could emerge due to the actin's double stranded helical structure that induces the ionic flow in a solenoidal manner. Due to the presence of the sheath of counter-ions around the AF, these polymers may act as biological "electrical wires", and could be modeled as non-linear inhomogeneous transmission lines propagating non-linear dispersive solitary waves. In some embodiments, an input voltage pulse with amplitude of about 200 *mV and* duration of about 800 ms to an AF may be applied and measured electrical signals at the opposite end of the AF could indicate that AFs support ionic waves in the form of axial non-linear currents. In further embodiments, the wave patterns observed in electrically-stimulated single AFs could be similar to recorded solitary waveforms for electrically-stimulated non-linear transmission lines. Considering the AF's highly non-linear complex physical structure and thermal fluctuations of the counter-ionic cloud, the observation of soliton-like ionic waves could be consistent with the idea of AFs functioning as biological transmission lines.

Actin filaments can be modelled as electrical transmission lines for ion flows and it may be estimated these flows have a velocity of propagation between about 1 and about 100 m/s, which may provide a realistic model of actin that can support soliton-like ionic traveling waves. In some embodiments, calcium ionic condensation along actin in particular may have a role in signal transduction within the cell. In further embodiments, and similar to MTs described above, actin filaments can be manipulated by external electric fields.

In some embodiment, water inside an organism may be gel-like. Proteins with hydrophilic surfaces may create structured interfacial water. This interfacial layer may extend beyond the 1 to 2 layers previously expected. The cytosol of cells can be a packed volume of proteins, and thus the water in a cell is may be structured to a large degree. This structured water, absorbed to collagen for example, could then support proton jump conduction. Interfacial water along membranes may also support rapid migration of protons. In some embodiments, a substantial portion of water may not be bulk-like. Water may also be necessary for protein electronic conductivity, as hydration may result in albumin conductivity increasing by eight orders of magnitude. In further embodiments, water may support short and long range hopping motions of protons. Proton mobility in water can be about five times that of $Na^+$. Its mobility may be enhanced by the Grotthuss mechanism, which is the proton hopping that occurs along water chains without the need for hydrodynamic diffusion.

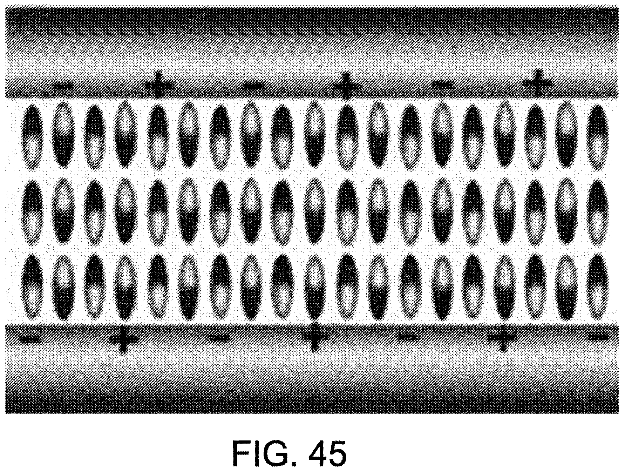

FIG. 45 illustrates an ordered water structure between charged surfaces.

In some embodiments, NMR evidence may indicate that cell water possesses more structure than liquid water, and that much of the Na and K in the cell is not free in aqueous solution, but is associated with charged sites on macromolecules. In further embodiments, complexed Na and K cations have been compared to valence electrons in solid conductors and free cations to conduction band electrons. With activation energy barriers and solid-liquid interfaces present in the cell, the liquid-state-free cation model of the cell may not feasible while a model based on structured water and associated cations is compatible with thermodynamic evidence. A substantial part of water molecules in the cell is in the form of hydration water bound to various macromolecules.

In some embodiments, the biological cell may create a "living matrix" of semiconducting macromolecules that are able to transmit, store, and process information involved in the regulation of physiological processes.

In some embodiments, activation of a Na+ pumping mode with an oscillating electric field of the strength of about 20 V/cm may occur at a frequency of about 1.0 MHz. In further embodiments, neither Rb+ efflux nor Na+ influx may be stimulated by the applied field in the frequency range from about 1 Hz to about 10 MHz. These results may indicate that only those transport modes that require ATP splitting under the physiological condition were affected by the applied electric fields, although the field-stimulated Rb+ influx and Na+ efflux did not depend on the cellular ATP concentration in the range of about 5 to about 800 $\mu$M. Computer simulation of a four-state enzyme electro-conformationally coupled to an alternating electric field may reproduce the main features of the above results.

In some embodiments, channel densities may vary among different neuronal phenotypes reflecting different stabilities of resting potentials and signal reliabilities. In model cell types such as in mammalian medial entorhinal cortex cells (MECs), modeled and experimental results may match for an average of about $5.10^5$ fast conductance $Na^+$ and delayed rectifier $K^+$ channels per neuron. In unmyelinated squid axons counts can reach up to about $10^8$ channels per cell. In model channels such as the bacterial KcsA channel one $K^+$ ion crosses the channel per 10-20 ns under physiological conductance of roughly about 80to about 100 pS (Roux & Schulten, 2004), which may be consistent with the frequencies of external electric stimulation mentioned above. This may allow for a maximum conduction rate of about $10^8$ ions/s. Estimating the distances between the center of the channel pore and the membrane surface to scale along $5.10^{-9}$ m (5nm) and assuming the most simple watery-hole and continuum electro-diffusion model of channels, this may provide an average speed of about $5.10^{-1}$ m/s per ion (0.5 m/s). Ion transition may occur through a sequence of stable multi-ion configurations through the filter region of the channels, which may allow rapid and ion-selective conduction. The motion of ions within the filter can be studied by applying molecular dynamics (MD) methods and density functional studies. MD methods used in the simulations may solve Newtons's equations of motion for the trajectory of ions.

In some embodiments, ionic signals can be related to morphogenesis and cancer. Currents of injury and associated electrical signals can be both necessary and sufficient for regeneration. Patterning information during embryogenesis and regenerative repair can be influenced by bioelectric ionic signals. Ionic signaling, and endogenous voltage gradients affected by ionic flow, may be implicated in proliferation and cell cycle progression, apoptosis, migration and orientation, and differentiation and de-differentiation. Ions may allow for message passing taking place on time scales, which are orders of magnitude faster than molecules.

In some embodiments, number of effects can be observed, which would implicate electric fields and/or currents in the cytoskeletal or cytoplasmic self-organization processes. Electrotherapies and wound healing may occur which may involve ionic current flows for a number of phenomena at a cell level, e.g. the cytochrome oxidase enzyme particle, in cell division, coherent polarization waves have been implicated as playing the key role chromosome alignment as well as their subsequent separation. Numerous examples of plant and animal cells may have steady electric fields in the range of about 1 to about 40 V/m induced significant changes (in both directions, depending on the case) in regeneration growth rates. In some embodiments, EM fields may influence mitosis and meiosis. In some embodiments, a reduction of about 55% in the mitotic index in exposed to about 60-Hz electric fields at about 430 V/m intensity and after about 4-hours of exposure may occur. In further embodiments, the effect of an electric field of about 50-Hz at an intensity of about 50 kV/m on the mitotic index of cultured human embryo fibroblastoid cells may occur and a reduction of up to about 30% after about 48 hours of exposure can be measured. These frequencies may be close to those of the envelope wave indicating that this can provide an additional effect at a cell and tissue level.

The impact of external electric fields on a cell may depend on the cell's shape. In some embodiments, calculations on the electric field strength in a spherical cell may indicate that, assuming the conductivities of the extracellular and intracellular fluids of the cell are the same, due to the small conductivity of the membrane versus these fluids, the electric field strength inside a typical cell is may be about 5 orders of magnitude less than that outside the cell. In some embodiment, given an applied electric field in air of about 300 V/m, a cell with a diameter of about 10 ^m would theoretically experience a field of $5.4 \times 10^{-10}$ V/m, thereby making the intracellular space shielded to a large degree from extracellular electric fields. In further embodiments, calculations for a spherical cell may indicate that the electric field in the protoplasm of the cell is negligibly small compared with the saline tissue around the cell, again about 5 orders of magnitude smaller. In further embodiments, when the cell is not spherical, as becomes the case increasingly when cells enter mitosis. In further embodiments, shielding occurs in elongated cells such as those found in muscle and long nerve cells. In sufficiently elongated cells, no shielding may occur. In bundles of elongated cells such as those in muscle, still no shielding may occur.

In some embodiments, endogenous current flows may be detected in animal cells. In the phase between fertilization and the first cleavage, a steady current may enter the animal pole and leaves the vegetal pole (equator). (b) In the silkmoth oocyte-nurse complex, the oocyte cytoplasm could be slightly more positive (by about 10 mV) than the nurse cell cytoplasm despite their connection through a broad cytoplasmic bridge whose resistance was estimated to be on the order of about 10 k$\Omega$ and it allows for the passing of a small electric current. This potential difference may be required for macromolecular transport across the bridge with a current on the order of about $5\ 10^{-8}$ A, (c) A steady current may enter the prospective cleavage furrow in both frog and sea urchin eggs during the about 10 min period prior to cleavage initiation and about 8 min after initiation, this current reverses its direction and leaves the furrow region.

In some embodiments, the magnitudes of the measured current densities, j, in cells could range from about 0.2 to about 60 $\mu$A/cm$^2$, which translates into a range from about 0.002 to about 0.6 A/m$^2$. In further embodiments, endogenous potentials may exist across metazoan cells in epithelia where potential differences on the order of about 30 to about 100 mV or more have been measured. Bioelectric fields may act to orient various growth-guiding filaments. The electric field magnitudes measured or estimated to exist in the cell appear to be too weak to be of use in reorienting such molecules as collagen (about 4 10$^5$ V/m needed) or long DNA strands (about 5 10$^4$ V/m needed) with a possible exception of actin filaments where fields as low as about 5 10$^3$ V/m suffice.

In some embodiments, metabolic oscillations in cells may occur with a period of about 10 seconds to about 12 seconds, which may be two or three orders of magnitude slower than any envelope electromagnetic field effects described above. In further embodiments, the envelope or the carrier waves may not interfere with the metabolic cycles at either a cell or tissue level.

Two aspects relevant to electrostatic effects occurring during mitosis may relate to external electric fields and possible endogenous electric fields. Electromagnetic (EM) fields are becoming increasingly relevant to understanding life. Electromagnetic (EM) fields may directly modulate the regulation of cellular growth and differentiation, including the growth of tumors. Both static magnetic and electric fields can alter the mitotic index and cell cycle progression of a number of cell types in various species.

In some embodiments, there may exist similarities between the pattern of the mitotic spindle apparatus and the field geometry of electric dipole moments. In further embodiments, the G$_2$->M transformation may be associated with a ferroelectric phase transition which establishes an axis of oscillation for the cellular polarization wave. The mitotic spindle apparatus may delineate the polarization field with microtubules lined up along the electric field lines. The poles could represent regions of the highest field intensity and the equatorial plane would provide a nodal manifold. The chromosome condensation during the G$_2$->M transformation could be induced by the static dielectric polarization of the chromatin complex resulting from the cellular ferroelectric phase transition. The field is highly non-homogeneous giving rise to a force acting on the dipole moment d of a molecule according to: F = d $\times$ grad E. It has been further assumed that throughout the S->G$_2$->M and mitosis processes, the cellular polarization could increase in amplitude. The electric field during metaphase should be very high inducing rapid motion of chromosomes. Coulomb repulsive forces may act to separate the chromatids and electrical fields strengthen the tactile forces of the spindles.

Regulation of cellular growth and differentiation (including the growth of tumors) may be directly modulated by EM fields. Static electric fields may alter the mitotic index and cell cycle progression of a number of cell types in various species. EM low-frequency fields in the range of about 50 Hz to about 75 Hz may cause perturbations in the mitotic activity of plant and animal cells and a significant inhibiting effect on mitotic activity occurs early during exposure. The onset of mitosis may be associated with a ferroelectric phase transition, which establishes an axis of oscillation for the cellular polarization wave. The mitotic spindle apparatus could delineate the polarization field with microtubules lined up along the electric field lines. The poles may represent regions of the highest field intensity and the equatorial plane would provide a nodal manifold. The chromosome condensation during this transformation could be induced by the static dielectric polarization of the chromatin complex resulting from the cellular ferroelectric phase transitions.

The impact of external electric fields on a cell theoretically may depend on the cell's shape. Calculations of the electric field strength in a spherical cell may indicate that, assuming the conductivities of the extracellular and intracellular fluids of the cell are the same, due to the small conductivity of the membrane versus these fluids, the electric field strength inside a typical cell is about 5 orders of magnitude less than that outside the cell. Given an applied electric field in air of about 300 V/m, a cell with diameter of about 10 $\mu$m could experience a field of about 5.4 $\times$ 10$^{-10}$ V/m, thereby making the intracellular space shielded to a large degree from extracellular electric fields. The electric field in the protoplasm of the cell could be negligibly small compared with the saline tissue around the cell, again about 5 orders of magnitude smaller. In some embodiments, the cell is not spherical, as becomes the case increasingly when cells enter mitosis. The shielding occurring in elongated cells, such as those found in muscle and long nerve cells by calculating the theoretical electric field inside a cell modelled as a cylinder, could be minimal to no shielding. In bundles of elongated cells such as those in muscle, still no shielding could occur. The DNA contents in the nucleus might still be protected from external fields due to being enclosed in the spherical nuclear membrane. During mitosis, when cell elongation typically occurs, external electric field effects may be much more relevant to cellular processes. A more interesting picture emerges when the cell is not spherical, as becomes the case increasingly when cells enter mitosis.

During mitosis, when cell elongation typically occurs, external electric field effects may be much more relevant to cellular processes. Low-intensity, intermediate frequency (e.g. about 100 to about 300 kHz), alternating electric fields may have a profoundly inhibitory effect on the growth rate of various human and rodent tumor cell lines. The effects may include both arrest of cell proliferation due to interference of proper formation of the mitotic spindle and destruction of cells undergoing division. This could be effective in increasing survival rates as an adjuvant treatment to chemotherapy in glioblastoma multiforme (GBM), and has subsequently been FDA approved. During mitosis, instead of the electric field in the cell being uniform, the field may become focused to the cleavage furrow. This may have the effect of attracting any dipoles toward the furrow, and causing cell membrane rupture and post-mitotic apoptotic cell death. Dividing cells of the hematopoietic system may not be affected by these alternating fields due to muscles surrounding the bone marrow serving as a shield to the external electric field effects. In the presence of an external electric field, the field may align cell division, making a high proportion of cells having their cleavage plain orthogonal to the electric field. During mitosis, electric field effects may be relevant toward cell functioning, especially in the creation of the mitotic spindle.

Cells may generate electric fields through microtubules and mitochondria and possibly other systems. The electromagnetic activity of yeast cells may peak during mitosis, suggesting yeast cells in M phase may emit the strongest electric field. Electric field strengths have typically only been able to be measured inside membranes with voltage dye and patch-clamp techniques, leaving the strength of electric fields within the cytoplasm hitherto unknown. Initial attempts using nanosensors embedded in the cytoplasm of cells to measure the endogenous electric field in cells have shown large field strengths (about $10^7$ V/m) which were dissipated upon the addition of a mitochondrial uncoupler. This may create 3D electric field profiles of the cell, which may allow the study of the change of electric field profiles that occur specifically during mitosis.

The morphogenetic field of the cell, being defined as the sum of patterning signals that carry information about the existing and future pattern of the organism, may be important to embryonic development, regeneration after injury, and proper aging. Bioelectric cues in this field may be altered in cancer cells. Cancer cells may have membranes that are depolarized, and this is associated with cell proliferation. Artificial depolarization may make somatic cells have neoplastic-like properties. Biophoton emissions from cancer cells may differ from normal cells. Cancer may be impacted by a loss in the endogenous electrodynamic fields of cells, which may affect structured water in cells and may lead to damping of microtubules. A cancer detection device, which emits waves at about 465 MHz, which is in use to detect prostate cancer, may detect these damped vibrations of microtubules in cancer cells. Nuclear magnetic resonance, used as a primary method in the detection of cancer, may detect the increased motional freedom of cancer water molecules. Forced biophysical changes may also suppress carcinogenesis. Artificial hyperpolarization of somatic cells may induce differentiation and suppress proliferation.

In some embodiments, a quantitative model may explain these effects. An energetic constraint may exist, both from below and above. For an effect to be of significance at a molecular level, its interaction energy must exceed kT per degree of freedom (i.e. about $4 \times 10^{-21}$ J). Otherwise, thermal fluctuations may disrupt the action of electric fields. It must not produce so much thermal energy as to seriously increase the temperature of the cell. In terms of practical comparisons, a cell may generate about $10^{-12}$ W of power (about $10^{-12}$ J/s), much of which is used to maintain a constant temperature. In terms of subcellular forces at work, a minimal amount of useful force at a nanometer scale is about 1 pN. Motor proteins generate forces on the order of several pN. A force of about 1 pN applied to a tip of a microtubule may be used to bend it by as much as about 1 $\mu$m.

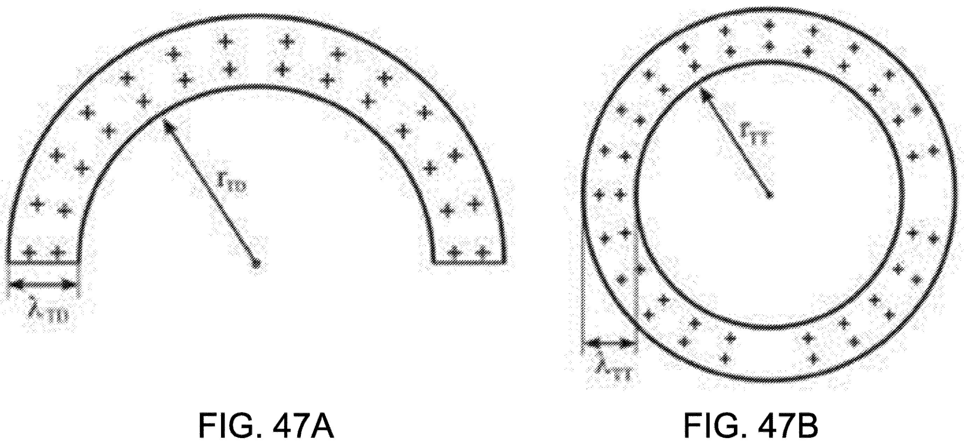

In some embodiments, the net charge on a tubulin dimer may depend on pH and changes from about +5 at pH of about 4.5 to about 0 at pH of about 5 and drops to about -30 at pH of about 8. These estimates may be charges in vacuum. In ionic solutions such as the cytoplasm, a vast majority of electrostatic charges are screened over the distances greater than the Debye length (see FIG. 47). The Debye length varies between about 0.6 and about 1.5 nm depending on the ionic strength.

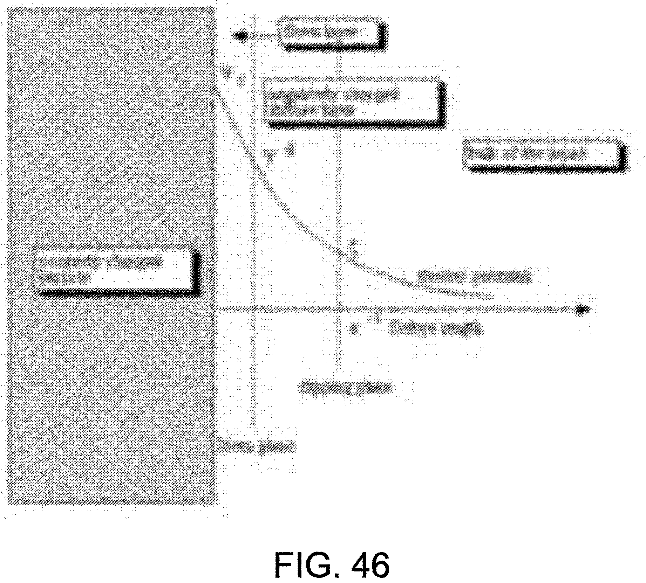

FIG. 46 depicts of the exponential drop of the electric potential in an ionic solution in the vicinity of a charged surface.

In some embodiments, calculating the force due to an electric field of about 100 V/m on a microtubule of about 10 $\mu$m in length, using F=q E, with q = $10^{-13}$ C for an unscreened charges, that F=10 pN assuming the field is also undiminished when penetrating a cell. However, with the Debye screening of electrostatic charges, only less than about 5% of the charge may remain exposed to the field resulting in a force of at most about 0.5 pN, most likely insufficient to exert a major influence on the cytoskeleton, especially since the field is oscillating and the net force would cancel out over the period of oscillations, i.e. on a time scale of tens of microseconds.

In some embodiments, microtubule electrostatics may have tubulin and microtubules that may affect the dipole moment. The next charge may vary between about -5e and about -30e per monomer depending on the isotype of tubulin. The dipole moment of tubulin (excluding the very flexible and dynamic C-termini) can be about 566 debye for the $\alpha$-monomer to about 1714 for the $\beta$-monomer. However, this is also strongly isotype dependent, so these numbers vary a lot between various tubulin isotypes from about 500 to about 4000 debye.

Taking the dipole moment of a free tubulin dimer as p=3000 debye as a representative number, the interaction energy with an electric field of E=100 V/m, we may obtain U=-p E, where p is the dipole moment, and hence U=$10^{-24}$ J. This may be too small (about 4000 times smaller than thermal energy kT) to affect the dynamics of an individual tubulin dimer. A microtubule may contain about 1625 dimers per about 1 $\mu$m of its length, so it could eventually accumulate enough net dipole to interact sufficiently strongly with the field.

Individual dipole moments may cancel out in the radial arrangement. There may be a small non-cancellation effect along the axis that may amount to less than about 10% of the next dipole moment. An entire microtubule may not be aligned in fields lower than about 1000 V/m. The torque between a dipole moment and an electric field may be proportional to the vector product of the electric field and the electric field strength ($\mathbf{F}= \mathbf{p} \times \mathbf{E}$). For the force to have a meaningful effect on a microtubule, it should exceed about 1 pN for lever arm on the order of about 1 $\mu$m giving a torque of about $10^{-18}$ Nm. With fields on the order of about 100 V/m, and a dipole moment of about 2,000 debye per tubulin dimer, even if they were perfectly aligned, it may result in a microtubule of about 1 $\mu$m only experiencing a torque of about $10^{-21}$ Nm, which could be about 1000 times too low.

A force between a charge and an electric field may be given by F = q E(x) where E(x) is screened exponentially by the Debye length. A test charge of about +5e, a distance of about 5 nm from the MT surface for a microtubule of about 5 $\mu$m, experiences a force of about 6 pN in water and about 0.5 pN in ionic solution. A dipolar molecule with a moment of about 200 debye, which is typical for protein charges, located about 5 nm away from a microtubule may have an interaction energy of about 0.03 meV, below thermal energy of about 25 meV. A tubulin dimer of about 3000 debye in the vicinity of a microtubule may experience an energy of about 3 meV. MT-MT interactions due to net charges with Debye screening accounted for lead to a net force of about 9 pN when separated by about 40 nm may result in net repulsion between them. At longer distance, attractive forces may prevail and the corresponding dipole-dipole attraction at about 90 nm is only about 0.08 pN. The maximum electrostatic force in the mitotic plate may be F= 6 $n^2$ pN/MT where n is the number of elementary charges on each protofilament. Since F is estimated to be about 1-74 pN/MT, the estimate is about 0.4-3.5 uncompensated elementary charges per protofilament. The range of values of the forces involved is could be within the realm of possible force requirements for chromosome segregation (about 700 pN for chromosome).

A detailed electric charge distribution on the tubulin surface may be calculated, and the landscape of an electrostatic potential around tubulin within an extension to an MT can be observed. These results exhibit non-uniformity of the potential along the MT radius with periodically repeating peaks and troughs along the MT axis for each of the 13 protofilaments. Based on these observations, MTs can be viewed as "conducting cables" composed of 13 parallel currents of ionic flux.

Since MTs have a predominantly negatively charged outer surface, including C-termini, they can be viewed as typical poly electrolyte polymers. Thus, Manning's theory of polyelectrolytes could be safely applied in this case. Accordingly, every MT should attract an ionic cloud (IC) of positive counter-ions close to its surface and along C-terminal tails (TTs), while negative ions of the cytosol are repelled away from the MT surface so that a roughly cylindrical ionic depletion area around MTs emerges. The thickness of such a depleted area corresponds to the Bjerrum length. This is the distance from the edge of an IC at which the Coulomb electrostatic energy of the screened condensed ion charges balances out the thermal molecular energy such that

$$\frac{e^2}{4\pi\varepsilon_0\varepsilon l_B} = k_B T; \quad \varepsilon_0 = 8.85 \times 10^{-12}\,\mathrm{F/m}.$$

At a physiological temperature ($T$ = 310K), taking the elementary charge as $e$ = 1.6$\times10^{-19}$C, Boltzmann's constant as $k_B$ =1.38$\times$10$^{-23}$J/K and the relative dielectric permittivity of the cytosol as $\varepsilon$ = 80, one may obtain the corresponding value of the Bjerrum length as $l_B$ = 0.67 $\times$ 10$^{-9}$m = 0.67nm. An estimate of the respective condensate thickness $\lambda$ of the counter-ion sheath for the tubulin dimer ($\lambda_{TD}$) and TT ($\lambda_{TT}$) has been found as follows $\lambda_{TD}$ = 2.5nm; $\lambda_{TT}$ =1.1nm.

These values will be used in the calculations of the corresponding capacity and resistivity of an ionic layer around MTs.

FIGS. 47A-47B depict a schematic representation of the counter-ion charge distributions surrounding a microtubule (left panel) and a C-terminal tail (right panel).

A detailed Poisson-Boltzmann approach can be used to evaluate the capacitance of an elementary ring of an MT, which consists of 13 dimers as

$$C_0 = \frac{2\pi\varepsilon_0\varepsilon l}{\ln\left(1 + \dfrac{l_B}{R_{IC}}\right)}.$$

where $l$ stands for the length of a polymer unit and $R_{IC}$ = $r_{TD}$ + $\lambda_{TT}$ for the outer radius of an IC.

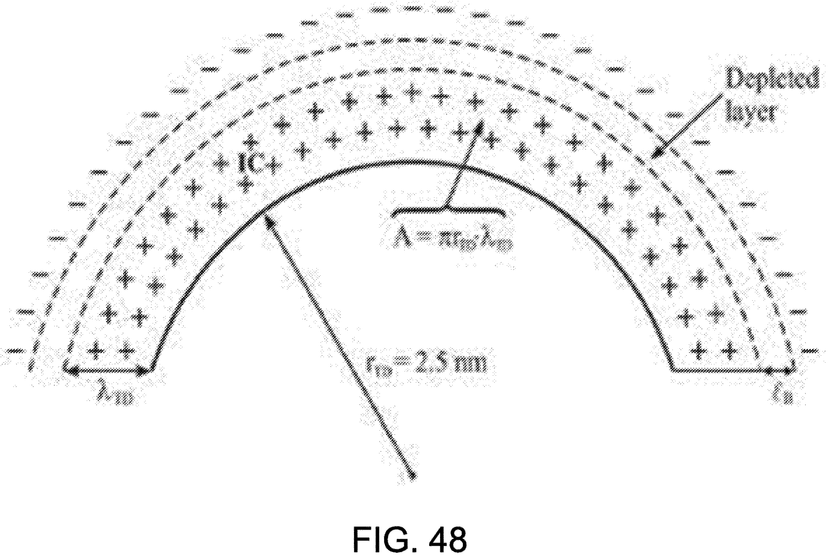

FIG. 48 depicts a schematic illustration for the calculation of the MT capacitance. With $l_{TD}$ = 8nm and $R_{IC}$ = $r_{TD}$ + $\lambda_{TT}$ = 2.5nm + 2.5nm = 5nm, we find for tubulin dimer

$$C_{TD} = \frac{3.14 \times 80 \times 8.85 \times 10^{-12} \times 8 \times 10^{-9}}{\ln\left(1 + \frac{0.67}{5}\right)} F = 1.4 \times 10^{-16} F.$$

Analogously, an extended TT as a smaller cylinder with the radius $r_{TT} = 0.5$nm and the thickness of its IC equal to $\lambda_{TT} = 1$nm. Its extended effective length should be $l_{TT}^{eff} = 4.5$nm - 2.5nm = 2nm, meaning that its part close to the tubulin surface is already embedded in the IC accounted for before. Thus, the corresponding capacitance is:

$$C_{TT} = \frac{2 \times 3.14 \times 80 \times 8.85 \times 10^{-12} \times 2 \times 10^{-9}}{\ln\left(1 + \frac{0.67}{1.5}\right)} F = 0.26 \times 10^{-16} F.$$

Accounting for the fact that two TTs are present in each tubulin dimer, we finally obtain

$$2xC_{TT} = 0.52x1^{-16}F.$$

The two capacitance values above may be considered to be in parallel with each other so that the total maximal capacitance of a tubulin dimer seen as an elementary unit in an ionic conduction cable is readily estimated as

$$C_0 = C_{TD} + 2xC_{TT} = 1.92x10^{-16}F.$$

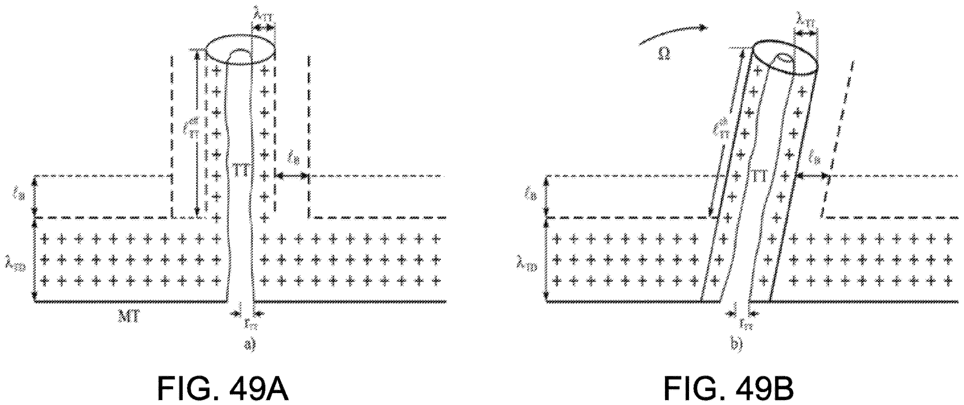

TTs capacitance must change with an increasing concentration of condensed cations due to the shrinking of flexible TTs. These changes are slightly different due to the different structures of $\alpha$ and $\beta$ type TTs. Additionally, this accounts for the tilting movements of TTs under the combined action of thermal fluctuations and a changing voltage due to an incoming ionic wave. The contribution to the capacitance due to TTs should also change by TTs tilt as shown in FIGS. 49A-B.

The change of the effective length $l_{TT}^{eff}$ of a TT is an additional factor affecting the capacitance $\Delta C_{TT}$.

FIGS. 49A-B depicts a comparison between an extended TT (A) and a tilting TT due to oscillations (B).

Regarding the Ohmic resistance, if we ignore ionic current leaks through the depleted layer, the dominant current flows in parallel with the MT axis charging the capacitors and partly leaking through nanopores in the MT surface (FIG. 38). The resistance attributed to this kind of ionic flow can be estimated theoretically or obtained on the basis of experimental evidence provided by the electro-orientation method performed on MTs *in vitro*. Taking a value of MT conductivity as $\sigma = (0.15 \pm 0.01)$Sm$^{-1}$, and adopting a simplifying assumption that the resistivity within an IC patch is homogeneous, the resistance of an ionic cable with a unit length of $l = 8$nm and the cross-sectional area A = $\pi r_{DT}\lambda_{DT}$ = 3.14 $\times$ 2.5nm $\times$ 2.5nm = 19.625nm$^2$, is estimated as $R_0 = \frac{l}{\sigma A} = 2.7 \times 10^6 \Omega$. Theoretically estimating the outer sheath-outer sheath resistance for complete may give R$_{13}$ = 4.75 $\times$ 10$^6$ $\Omega$. The value of resistance for a ring surrounding a microtubule segment the length of a dimer could be 13 times greater, i.e. $R_0 = 6.2 \times 10^7$ $\Omega$.

Finally, we include the conductance of both nanopores to account for the leakage of IC cations into the lumen area. Thus, we have

$$G_0 = \sigma_1 + \sigma_2 = (2.93 + 7.8)\text{nS} = 10.7\text{nS}; \quad \frac{1}{G_0} = 9.3 \times 10^7.$$

Below we develop the underlying equations governing the ionic flows along MTs.

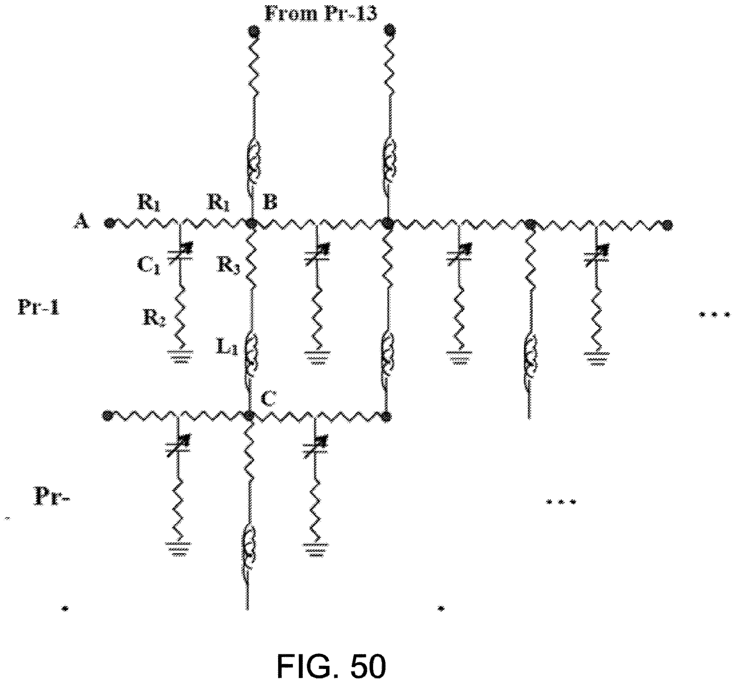

FIG. 50 depicts an electrical cable representation of the ionic surrounding of a microtubule composed of 13 protofilaments. Notation used: Pr-j : protofilament 'j', j=1..13; $R_1$ : resistance along a tubulin; $R_2$: resistance perpendicular to the tubulin; $R_3$: resistance between protofilament, in series with the partial inductance $L_1$; $C_1$: capacitance of a dimer; $L_1$: fraction of the inductance of a full turn.

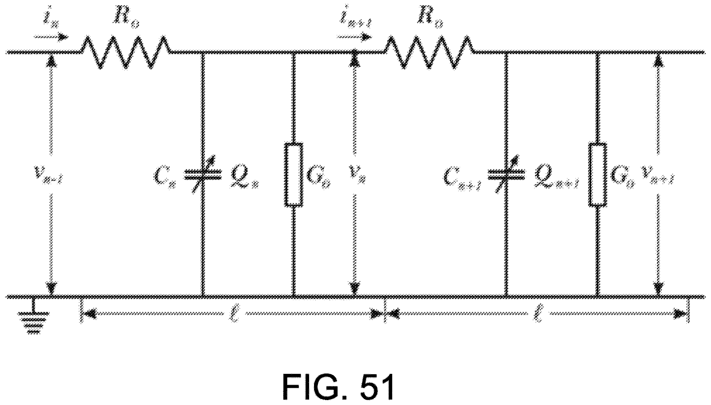

A periodic electric circuit simulating one protofilament of a microtubule may be constructed. It may consist of a long ladder network composed of lumped sections equal to identical EUPs as represented in FIG. 51.

The longitudinal ionic current may be represented by the series of Ohmic resistance $R_0$ for one ionic conduction unit. The nonlinear capacity with the charge $Q_n$ for the n-th site of the ladder may be in parallel with the total conductance $G_0$ of the two TTs. This may be true if the bulk cytosol and MT lumen are considered as grounded. This permits the application of Kirchhoff s law as

$$i_n - i_{n+1} = \frac{\partial Q_n}{\partial t} + G_0 v_n,$$

$$v_{n-1} - v_n = R_0 i_n.$$

These sets of equations can be transformed into

$$\frac{\partial Q}{\partial t} = C_0 \frac{\partial v_n}{\partial t} - C_0 \Gamma_0 \Omega v_n - C_0 \Gamma_0 \Omega (t - t_0) \frac{\partial v_n}{\partial t} - 2 b_0 C_0 v_n \frac{\partial v_n}{\partial t}.$$

The next step is to establish the auxiliary function $u(x,t)$ unifying the voltage and its accompanying IC current as follows

$$u_n = Z^{1/2} i_n = Z^{-1/2} v_n,$$

where the characteristic impedance of EUP is defined in the usual way as

$$Z = \frac{1}{\omega C_0}.$$

Using a Taylor series with respect to a small spatial parameter $l$ we obtain

$$-2 \frac{\partial u}{\partial x} - \frac{l^2}{3} \frac{\partial^3 u}{\partial x^3} - \frac{Z C_0}{l} \frac{\partial u}{\partial t} + \frac{Z C_0 \Gamma_0 \Omega}{l} (t - t_0) \frac{\partial u}{\partial t} + 2 \frac{Z^{3/2} b_0 C_0}{l} u \frac{\partial u}{\partial t} - \frac{1}{l} \left( Z G_0 + Z^{-1} R_0 - Z C_0 \Gamma_0 \Omega \right) u = 0.$$

In order to introduce dimensionless variables, we first estimate the characteristic time and length scales. It is convenient that the charging (discharging) time of an EUP capacitor $C_0$ through the resistance $R_0$ is given by $T_0 = R_0 C_0$. which can be found as $T_0 = 6.2 \times 10^7 \Omega \times 1.92 \times 10^{-16} F = 1.2 \times 10^{-8} s$.

Its inverse, namely the frequency can be found as about 83 MHz, which may be within the range of the carrier wave frequency in the instant application. Moreover, it strongly depends on the length of the MT and hence can be tuned either down or up the frequency scale depending on the average length of MTs involved.

The characteristic propagation velocity of the ionic wave is $v = \frac{l}{T0}$ which yields

$$v_0 = \frac{8 \times 10^{-9} \, \text{m}}{1.2 \times 10^{-8} \, \text{s}} = 0.67 \frac{\text{m}}{\text{s}}.$$

We then estimate the characteristic impedance of each ionic conduction unit as

$$Z > \frac{2R_0}{s}$$

or $Z > 1.24 \times 10^8 \Omega$ for $s = 1$.

For $s = 1$ one obtains the cut off frequency

$$\omega_{max} = 4.3 \times 10^7 \, s^{-1} \quad or \quad \nu_{max} = 6.8 \times 10^6 \, Hz.$$

This indicates that the characteristic frequency matches the order of magnitude of frequency $\Omega$, which describes the TTs oscillations. We now establish the compact form of the ionic current equation as:

$$\frac{\partial u}{\partial \tau} + \beta \frac{\partial^3 u}{\partial \xi^3} + \alpha u \frac{\partial u}{\partial \xi} + \gamma(\xi) \frac{\partial u}{\partial \xi} + \delta u = 0,$$

where the abbreviations were introduced as follows

We can further perform the following transformation of space-time variables

$$(\xi, \tau) \to (\rho, \theta); \quad \rho(\xi, \tau) = (\xi - \xi_0) \exp(-\gamma_0 \tau),$$

$$\theta(\tau) = -\frac{1}{3\gamma_0} \exp(-3\gamma_0 \tau),$$

$$u(\xi, \tau) = W(\rho, \theta) \exp(-2\gamma_0 \tau),$$

so that

$$\frac{\partial W}{\partial \theta} + \alpha W \frac{\partial W}{\partial \rho} + \beta \frac{\partial^3 W}{\partial \rho^3} + \frac{\Lambda}{\theta} W = 0,$$

we then obtain

$$Z = 1.87 \times 10^8 \, \Omega \quad and \quad \omega = 2.7 \times 10^7 \, s^{-1}.$$

Subsequently equating $\omega = \Omega = 2.7 \times 10^7 s^{-1}$ we estimate the parameter $\Gamma_0$ as 0.18. Again, the frequency value is similar to that of the carrier wave of the instant invention and it represents the oscillations of C-terminal tails.

The spatial non-homogeneity parameter can be found to be $\Gamma_0 \Omega = 4.8 \times 10^6 \, s^{-1}$, which is one order of magnitude smaller than the inverse characteristic time $T_0^{-1} = 1.83 \times 10^7 s^{-1}$, In general, by introducing a new transformation so that the function $u(\xi, \tau)$ has the form

$$u(\xi, \tau) = \frac{u_0 \exp(-2\gamma_0 \tau)}{\cosh^2 \left\{ \left[ \frac{\alpha u_0}{4\beta} \exp(-2\gamma_0 \tau) \right]^{1/2} \left[ \xi - \xi_0 (1 - \exp(\gamma_0 \tau)) + \frac{\alpha u_0}{3\beta} (1 - \exp(3\gamma_0 \tau)) \exp(-2\gamma_0 \tau) \right] \right\}},$$

The soliton solution preserves its width but its amplitude decays rather rapidly so that over the length of about 500l it becomes negligible. The velocity of the soliton solution decreases very slightly. We estimate its average velocity

as follows:

$$\Delta x \approx 400\ \ell = 400 \times 8\,\mathrm{nm} = 3.2\,\mu\mathrm{m},$$

$$\Delta t = 1000\, T_0 = 10^3 \times 1.2 \times 10^{-8}\,\mathrm{s} = 1.2 \times 10^{-5}\,\mathrm{s},$$

$$v = \frac{\Delta x}{\Delta t} = 0.26\,\frac{\mathrm{m}}{\mathrm{s}}.$$

The range of this soliton is about $3.2\mu$m, which is of the order of the cell's diameter. The ionic pulse with such parameter values could be efficiently transferred within the cell at high velocities of propagation.

Ionic conduction along and away from charged protein filaments such as microtubules may involve cable equations resulting from RLC circuits surrounding each protein unit in the network. Conduction along the filaments may experience resistances due to viscosity in the ionic fluid. Capacitance may be caused by charge separation between the surface and ions according to the Bjerrum length. Inductance may be caused by helical nature of the microtubule surface and consequently, solenoidal flows of the ionic fluid along and around the microtubule.

Below we summarize the key numerical estimates of the RLC circuit components. For a single dimer, we find that $C = 6.6\ 10^{-16}$ F, $R1 = 6\ 10^{6}\ \Omega$ (along the MT), $R_2 = 1.2\ 10^{6}\ \Omega$ (perpendicular to the MT) and $L = 2\ 10^{-12}$ H. These numbers can be used to estimate characteristic time scales for the oscillations (LC) and exponential decay (RC) taking place in this equivalent circuit. We obtain for decay times ($\tau = RC$) the following values: (a) $\tau_1 = 10^{-8}$ s along the MT length and (b) $x_2 = 2 \times 10^{-9}$ s away from the MT surface. However, due a low value of inductance L, the corresponding time for electromagnetic oscillations is found using $\tau_0 = (LC)^{1/2}$ as $\tau_0 = 0.2\ 10^{-12}$ s = 0.2 ps.

Repeating these calculations for a microtubule, we note that R1 scales with length of a microtubule, while R2 is length independent. The corresponding capacitance in both cases scales with length, therefore $\tau_1$ scales with length squared ($l^2$) while $\tau2$ scales with length. To obtain actual values, we need to multiple the values for a single ring by the number of rings in a microtubule. We use the values found for a single ring, i.e. $\tau_1 = 10^{-8}$ s and $\tau_1 = 2 \times 10^{-9}$ s and scale them accordingly to estimate the length of microtubules that could experience resonant effects in terms of ionic currents along and away from their surface we find the scaling factor that leads to the characteristic times on the order of about 40 ns. Therefore, for longitudinal effects, on the order of about 2 rings (i.e. microtubule nucleation), or microtubules about 20 nm long would respond to about 27 MHz stimulation. On the other hand, for ionic flows pulsating radially around a microtubule with about 20 rings or, a microtubule about 160 nm in length would be required. We should stress, however, that these results are very sensitive regarding the choice of parameter values, especially the resistivity where diverse estimates can be found in the literature. In general, there is strong overlap between the time scales of ionic wave propagation and electric field stimulation. It is conceivable that both effects play a role depending on the orientation of the field *vis a vis* the geometry of mitotic spindles and the MTs forming them. It appears that short MTs would be more sensitive to the longitudinal wave generation by E-fields while long MTs to perpendicular wave generation.

For actin in solution, a key feature is that the positively charged end assembles more quickly than the negatively charged end. This results in an asymmetry in the charges at the ends of the filaments and F-actin's electric polarization. Actin monomers may arrange themselves head to head to form actin dimers resulting in an alternating distribution of electric dipole moments along the length of the filament. In some embodiments, there is a helical distribution of ions winding around the filament at approximately one Bjerrum length. This may correspond to a solenoid in which a fluctuating current flows as a result of voltage gradient between the two ends. For a filament with n monomers, we get an effective resistance, inductance, and capacitance as follows:

$$R_{eff} = \left( \sum_{i=1}^{n} \frac{1}{R_{2,i}} \right)^{-1} + \sum_{i=1}^{n} R_{1,i}$$

$$L_{eff} = \sum_{i=1}^{n} L_i$$

$$C_{eff} = \sum_{i=1}^{n} C_{0i}$$

EP 4 438 107 A2

where $R_{1,i}$ =6.11 $\times 10^6$ Ω, and $R_{2,i}$ =0.9$\times 10^6$ Ω, such that $R_{1,i}$ = 7 $R_{2,i}$. Hence, for a 1 μm length of actin filament we find *that* $R_{eff}$ = 1.2 $\times$ 10$^9$ Ω, $L_{eff}$ = 340 $\times$ 10$^{-12}$ $H$ , $C_{eff}$ = 0.02 $\times$ 10$^{-12}$ $F$. The electrical model of the actin filament is an application of Kirchhoff s laws to one section of the effective electrical circuit that is coupled to neighboring monomers. Taking the

$$LC_0 \frac{\partial^2 V}{\partial t^2} = a^2 \left( \partial_{xx} V \right) + R_2 C_0 \frac{\partial}{\partial t} \left( a^2 \left( \partial_{xx} V \right) \right) - R_1 C_0 \frac{\partial V}{\partial t} + R_1 C_0 2bV \frac{\partial V}{\partial t}$$

continuum limit, for a large number of monomers along an actin filament we derive the following equation, which describes the spatio-temporal behavior of the potential along the actin filament:

In some embodiments, an input voltage pulse may be applied with amplitude of about 200 *mV* and duration of about 800 μs to an actin filament, and electrical signals at the opposite end of the actin filament can be measured. Such types of measurements may indicate actin filaments support ionic waves in the form of axial non-linear currents. These ionic waves may be described by the solutions of the above nonlinear partial differential equation.

In a similar manner to the analysis of the time scales for microtubules as ionic conduction cables with RLC components, we can now estimate similar time scales for actin and actin filaments. In some embodiments, for a single actin monomer, the time scale for LC oscillations can be very fast, namely $\tau_0$=(LC)$^{1/2}$ and $\tau_0$=6$\times$ 10$^{-14}$ s. The decay time for longitudinal ionic waves is $\tau_i$=R$_i$C=6$\times$10$^{-10}$s while the corresponding time for radial waves is $\tau_2$=R$_2$C=0.9$\times$10$^{-10}$s. All of the above time scales are not compatible with interactions involving electric fields in the range of about 27 MHz. However, the situation changes drastically for actin filaments where there is a similar scaling with the length of the filament as described for microtubules. Taking an example of an actin filament of about 10 μm, we find $\tau_0$ = 10$^{-10}$ s, which is still too short but for a short filament with about 100 layers, i.e. about 400 nm long, $\tau_i$=R$_i$C=2.4 $\times$10$^{-8}$ s which is in the correct range for interactions with interactions with the carrier electromagnetic fields in the range of about 27 MHz. We conclude that although less likely than MTs, actin filaments may also be affected by the carrier electromagnetic waves.

Current densities are disclosed herein in relation to those mentioned above for dividing cells where the values were measured in the range of about 0.002<j<0.6 A/m$^2$. Since j=σ E where E = 100 V/m and σ had a large range of values reported (see Table 2) between about 0.1 and about 100, taking the lower limit of about 0.1 may result in ionic currents along microtubules that would overwhelm the intrinsic ion flows in a dividing cell. It is possible that these externally stimulated currents cause a major disruption of the process of mitosis.

In some embodiments, power dissipated due to a current flowing along a microtubule may occur. Taking as an example a microtubule of about 10 μm in length, we estimate the average power drain as

$$<P> = (1/2) V_0^2 [R/(R^2 + X_c^2)]$$

where $X_c$ = 1/ωC is the capacitive resistance. Substituting the relevant numbers, we obtain the power dissipated to be about10$^{-11}$ W which is comparable to the power generated by the cell in metabolic processes (about 100 W of power generation in the body / about 3$\times$ 10 $^{13}$ cells in the body). Consequently, additional heat generated by these processes may be disruptive to living cells.

Time scales for the processes in ion channels can be estimated by the time for translocations (t$_{tr}$) between two filter sites separated by about 0.3 nm, i.e. about 5x 10$^{-10}$ s and about 5$\times$ 10$^{-11}$ s. Transition rates (from potential mean force (PMF)) maps and Kramer transition rate model may be consistent with these numbers. Changes between a non-conductive and a conductive state in the KcsA may occur at about 7.1 $\times$ 10$^3$ s$^{-1}$, giving a life-time of the non-conducting state of about 0.14 ms (about 10$^{-4}$s). As the duration of the non-conducting state scales along about 10 $^{-3}$ to about 10 $^{-4}$ s and the within filter translocation time is about 10$^{-11}$s, we can expect about 10 $^7$ filter state changes during a non-conducting state and about 10$^{10}$ switches per second (about 10 GHz). Consequently, these time scales may not be compatible with those resulting from the effects of MHz range electric fields.

In some embodiments, the DNA contents in the nucleus may be protected from external fields due to being enclosed in the spherical nuclear membrane. In addition to the screening effects of being shielded both by the cell membrane and the nuclear wall, the irregular geometry of the DNA strands and their short persistence length may indicate that while highly charged, DNA is unlikely to participate in ionic conduction effects shown either in actin filaments or microtubules.

Kinesin (along MT) may propagate at a maximum speed of about 10$^{-6}$ m/s. This value may depend on the concentration of ATP and the ionic concentrations in the medium. In the case of MTs kinesin transports various crucial cargo and for actin filaments, dynein does the same at similar speeds. Each step of a motor protein may take place over the period of a millisecond or less which is much longer than the period of AC field oscillations. Kinesin binds to MTs through C-termini which may be sensitive to electric field fluctuations and it is possible that kinesin transport would be very strongly disrupted by these rapid oscillations of C-termini. Therefore, disruption of motor traffic in a living cell exposed to MHz range electromagnetic fields could be an important downstream effect.

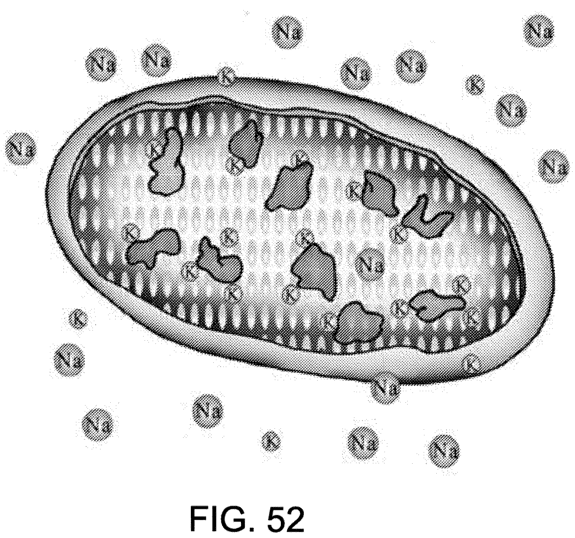

FIG. 52 depicts a schematic illustration of ions and ordered water within a living cell and in the extracullar matrix.

The cytoplasm may provide a medium in which fundamental biophysical processes, e.g. cellular respiration, take place. Most cells maintain a neutral pH (about 7.25 to about 7.35) and their dry matter is composed of at least 50% of protein, cancer cells have a pH of about 6.85 (acidic). The remaining dry material consists of nucleic acids, trace ions, lipids, and carbohydrates. Most of the trace ions are positively charged. A few metallic ions are found which are required for incorporation into metallo-proteins, e.g. $Fe^{2+}$, typically in nanomolar concentrations. Below is a summary of the composition of the cytoplasm regarding the most abundant and important components (Table 4).

Table. 4 Composition of the cytoplasm

| Ions | Concentration mM | Non-ionic constituents | Concentration mg/mL |
|---|---|---|---|
| $K^+$ | 140 | protein | 200 - 300 |
| $Na^+$ | 10 | actin | 2 - 8 |
| $Cl^-$ | 10 | tubulin (electrolyte) | 4 |
| $Ca^{2+}$ | $10^{-4}$ | pH | ~ 7.2 |
| $Mg^{+2}$ | 0.5 | Specific tissues may differ | Specific tissues may differ |
| Negative protein charge: 1.6 mol/kg<br>Positive protein charge: 1.01 mol/kg<br>Net protein charge: 0.6 mol/kg (-)<br>Potassium ion: 0.5 mol/kg (+)<br>Chloride ion: 0.2 mol/kg (-)<br>Net ion charge: 0.3 mol/kg (+)<br>NET CYTOPLASM CHARGE: 0.3 mol/kg (-) | | | |

The viscosity of plasma is about $\eta$= 0.001 Pa.s (Howard, 2001), hence the friction coefficient for an ion in solution can be estimated as about $\gamma=6\pi\eta r$ where r is the ionic radius (hydration shells radius) and find $\gamma=2\ 10^{-12}$ Pa.s.m. In an oscillating electric field of amplitude of about 100 V/m and a frequency of about f=20 MHz, an ion's position may follow periodic motion given by: x(t) = 0.1 A sin ($2\pi$ft), i.e. will execute harmonic motion out of phase with the field, with the same frequency and an amplitude about 10% of the radius.

The net force on the total charge in the cytoplasm can be estimated as F=qE, q=4 $\times$ $10^{11}$ e and E=100 V/m, so the total force is about 6 $\mu$N, which may be sufficient to cause major perturbations in the cell interior. This may depend on the ability of the electric field to penetrate into the cell's interior which is easier in the case of non-spherical cells. The net outcome of these ionic oscillations away and towards attractively interacting protein surfaces inside the cytoplasm can be a concomitant series of oscillations of the structures affected by the ionic clouds as schematically shown below.

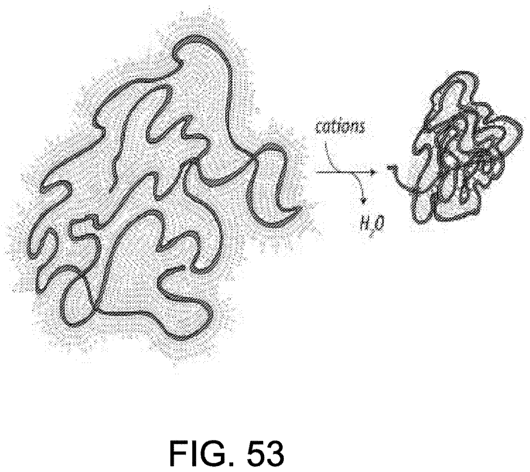

FIG. 53 depicts an illustration of the condensation/decondensation effect due to cationic charge movement in the cytoplasm.

The cytoskeleton microtubules may participate in numerous interactions with electromagnetic forces due to the complex charge distribution in and around these protein filaments surrounded by poly-ionic solutions. There are large net charges on tubulin, which are largely but not completely screened by counter-ions. Some of the charges are localized on C-termini, which are very flexible leading to oscillating charge configurations. There are ions surrounding the protein that can be partially condensed and susceptible to collective oscillations. There are large dipole moments on tubulin and microtubules whose geometric organization affects their response to external fields. Finally, there can be induced dipole moments especially in the presence of electric field gradients.

Based on the extensive analysis of the various possible effects AC electric fields can have on living cells, we conclude the following. Electric field gradients, especially in dividing cells, cause substantial dielectrophoretic forces on tubulin dimers and microtubules. The longer the microtubule, the more pronounced the effect. Additionally, another likely scenario is that ionic current flows along and perpendicular to MT surfaces (as well as actin filaments, but less likely) take place which can be generated by electromagnetic oscillations in the range of about 27 MHz. The specific frequency selection depends critically on the length of each filament.

Depending on the orientation of the electromagnetic fields with the cell axis and in particular with the microtubule axis (however, they fan out from centrosomes in mitotic cells, so there will be at different angles to any field), there could in general be three types of ionic waves generated:

a) longitudinal waves propagating along the microtubule surface. In the case each protofilament of a microtubule acts like a cable with its inherent resistance r, so the resistance of an entire microtubule would be R=r/13 since all these cables are in parallel to each other,

b) helical waves propagating around and along each microtubule, there could be 3 or 5 such waves propagating simultaneously mimicking the 3-start or 5-start geometry of a microtubule. The effective resistance of such cables would be the individual resistance divided by the number of cables in parallel

c) radial waves propagating perpendicularly to the microtubule surface.

If a field is oriented at an angle to the microtubule axis, it is expected that all these wave types may be generated simultaneously. Elongation of dividing cells facilitates penetration of these fields into cells while spherical cells would largely shield the fields and prevent them from entering into their interiors. Once electromagentic fields generate oscillating ionic flows, these can in turn cause the following downstream effects:

a) interfere with ion flows in the cleavage area of dividing cells

b) interfere with motor protein motion and MAP-MT interactions

c) may to a lesser degree affect ion channel dynamics

d) may in general affect the net charge of the cytoplasm.

There are also expected to be some measurable heating effects in the cytoplasm.

These fields are not expected to affect permanent dipoles of proteins such as tubulin and actin.

In some embodiments, exposure of amplitude modulated frequencies to a patient may cause cell injury. In some embodiments, the cell injury can be hydropic degeneration, apoptosis, or a combination thereof. In some embodiments, the cell injury is hydropic degeneration. Hydropic degeneration can be observed when the cell membrane or cellular integrity is disrupted thus causing impairment of the mechanisms to maintain cellular homeostasis. In some embodiments, hydropic degeneration can result in the cytoplasm membrane being more permeable leading to cellular swelling, cellular rupture, or cellular death.

In some embodiments the subject has hepatocellular carcinoma. In some embodiments, the subject has a cancer. In some embodiments, the cancer is selected from the group consisting of colon carcinoma, breast cancer, pancreatic cancer, ovarian cancer, prostate cancer, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, merkel cell carcinoma, craniopharyngioma, ependymoma, pinealoma, hemangioblastoma, acoustic neuroma, oligodendroglioma, meningioma, melanoma, neuroblastoma, retinoblastoma; leukemias, e.g., acute lymphocytic leukemia and acute myelocytic leukemia, chronic leukemia; polycythemia vera, lymphoma, multiple myeloma, Waldenstrom's macroglobulinemia, heavy chain disease, or a combination thereof.

In some embodiments, the subject receives carrier frequency modulation therapy alone or in combination with an anti-cancer therapy. In some embodiments, the subject receives carrier frequency modulation therapy alone or in combination with an anti-cancer therapy prior to or in preparation for tumor resection surgery. In some embodiments, the additional anti-cancer therapy can be Sorafenib (Nexavar), Nivolumab (Opdivo), Regorafenib (Stivarga), or a combination thereof. In some embodiments, the additional anti-cancer therapy can be abemaciclib, abiraterone acetate, abitrexate (methotrexate), abraxane (paclitaxel albumin-stabilized nanoparticle formulation), ABVD, ABVE, ABVE-PC, AC, acalabrutinib, AC-T, actemra (tocilizumab), adcetris (brentuximab vedotin), ADE, ado-trastuzumab emtansine, adriamycin (doxorubicin hydrochloride), afatinib dimaleate, afinitor (everolimus), akynzeo (netupitant and palonosetron hydrochloride), aldara (imiquimod), aldesleukin, alecensa (alectinib), alectinib, alemtuzumab, alimta (pemetrexed disodium), aliqopa (aopanlisib hydrochloride), alkeran for injection (melphalan hydrochloride), alkeran (melphalan), aloxi (palonosetron hydrochloride), alunbrig (brigatinib), ambochlorin (chlorambucil), amboclorin (chlorambucil), ameluz (aminolevulinic acid), amifostine, aminolevulinic acid, anastrozole, apalutamide, aprepitant, aranesp (darbepoetin alfa), aredia (pamidronate disodium), arimidex (anastrozole), aromasin (exemestane), arranon (nelarabine), arsenic trioxide, arzerra (ofatumumab), asparaginase erwinia chrysanthemi, atezolizumab, avastin (bevacizumab), avelumab, axicabtagene ciloleucel, Axitinib, Azacitidine, Bavencio (Avelumab), BEACOPP, Becenum (Carmustine), Beleodaq (Belinostat), Belinostat, Bendamustine Hydrochloride, Bendeka (Bendamustine Hydrochloride), BEP, Besponsa (Inotuzumab Ozogamicin), Bevacizumab, Bexarotene, Bicalutamide, BiCNU (Carmustine), Bleomycin, Blinatumomab, Blincyto (Blinatumomab), Bortezomib, Bosulif (Bosutinib), Bosutinib, Brentuximab Vedotin, Brigatinib, BuMel, Busulfan, Busulfex (Busulfan), Cabazitaxel, Cabometyx (Cabozantinib-S-Malate), Cabozantinib-S-Malate, CAF, Calquence (Acalabrutinib), Campath (Alemtuzumab), Camptosar (Irinotecan Hydrochloride), Capecitabine, CAPOX, Carac (Fluorouracil--Topical), Carboplatin, CARBOPLATIN-TAXOL, Carfilzomib, Carmubris (Carmustine), Carmustine, Carmustine Implant, Casodex (Bicalutamide), CEM, Ceritinib, Cerubidine (Daunorubicin Hydrochloride), Cervarix (Recombinant HPV Bivalent Vaccine), Cetuximab, CEV, Chlorambucil, CHLORAMBUCIL-PREDNISONE, CHOP, Cisplatin, Cladribine, Clafen (Cyclophosphamide), Clofarabine, Clofarex (Clofarabine), Clolar (Clofarabine), CMF, Cobimetinib, Cometriq (Cabozantinib-S-Malate), Copanlisib Hydrochloride, COPDAC, COPP, COPP-ABV, Cosmegen (Dactinomycin), Cotellic (Cobimetinib), Crizotinib, CVP, Cyclophosphamide, Cyfos (Ifosfamide), Cyramza (Ramucirumab), Cytarabine, Cytarabine Liposome, Cytosar-U (Cytarabine), Cytoxan (Cyclophosphamide), Dabrafenib, Dacarbazine, Dacogen (Decitabine), Dactinomycin, Daratumumab, Darbepoetin Alfa, Darzalex (Daratumumab), Dasatinib, Daunorubicin Hydrochloride, Daunorubicin Hydrochloride and Cytarabine Liposome, Decitabine, Defibrotide Sodium, Defitelio (Defibrotide Sodium), Degarelix, Denileukin Diftitox, Denosumab, DepoCyt (Cytarabine Liposome), Dexamethasone, Dexrazoxane Hydrochloride, Dinutuximab, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), DTIC-Dome (Dacarbazine), Durvalumab, Efudex (Fluorouracil--Topical), Eligard (Leuprolide Acetate), Elitek (Rasburicase), Ellence (Epirubicin Hydrochloride), Elotuzumab, Eloxatin (Oxaliplatin), Eltrombopag Olamine, Emend (Aprepitant), Empliciti (Elotuzumab), Enasidenib Mesylate, Enzalutamide, Epirubicin Hydrochloride, EPOCH, Epoetin Alfa, Epogen (Epoetin Alfa), Erbitux (Cetuximab), Eribulin Mesylate, Erivedge (Vismodegib), Erleada (Apalutamide), Erlotinib Hydrochloride, Erwinaze (Asparaginase Erwinia chrysanthemi), Ethyol (Amifostine), Etopophos (Etoposide Phosphate), Etoposide, Etoposide Phosphate, Evacet (Doxorubicin Hydrochloride Liposome), Everolimus, Evista (Raloxifene Hydrochloride), Evomela (Melphalan Hydrochloride), Exemestane, 5-FU (Fluorouracil Injection), 5-FU (Fluorouracil--Topical), Fareston (Toremifene), Farydak (Panobinostat), Faslodex (Fulvestrant), FEC, Femara (Letrozole), Filgrastim, Firmagon (Degarelix), Fludara (Fludarabine Phosphate), Fludarabine Phosphate, Fluoroplex (Fluorouracil-Topical), Fluorouracil Injection, Fluorouracil-Topical, Flutamide, Folex (Methotrexate), Folex PFS (Methotrexate), FOLFIRI, FOLFIRI-BEVACIZUMAB, FOLFIRI-CETUXIMAB, FOLFIRINOX, FOLFOX, Folotyn (Pralatrexate), FU-LV, Fulvestrant, Fusilev (Leucovorin Calcium), Gardasil (Recombinant HPV Quadrivalent Vaccine), Gardasil 9 (Recombinant HPV Nonavalent Vaccine), Gazyva (Obinutuzumab), Gefitinib, Gemcitabine Hydrochloride, GEMCITABINE-CISPLATIN, GEMCITABINE-OXALIPLATIN, Gemtuzumab Ozogamicin, Gemzar (Gemcitabine Hydrochloride), Gilotrif (Afatinib Dimaleate), Gleevec (Imatinib Mesylate), Gliadel (Carmustine Implant), Gliadel wafer (Carmustine Implant), Glucarpidase, Goserelin Acetate, Halaven (Eribulin Mesylate), Hemangeol (Propranolol Hydrochloride), Herceptin (Trastuzumab), HPV Bivalent Vaccine, HPV Nonavalent Vaccine, HPV Quadrivalent Vaccine, Hycamtin (Topotecan Hydrochloride), Hydrea (Hydroxyurea), Hydroxyurea, Hyper-CVAD, Ibrance (Palbociclib), Ibritumomab Tiuxetan, Ibrutinib, ICE, Iclusig (Ponatinib Hydrochloride), Idamycin (Idarubicin Hydrochloride), Idarubicin Hydrochloride, Idelalisib, Idhifa (Enasidenib Mesylate), Ifex (Ifosfamide), Ifosfamide, Ifosfamidum (Ifosfamide), IL-2 (Aldesleukin), Imatinib Mesylate, Imbruvica (Ibrutinib), Imfinzi (Durvalumab), Imiquimod, Imlygic (Talimogene Laherparepvec), Inlyta (Axitinib), Inotuzumab Ozogamicin, Interferon Alfa-2b, Interleukin-2 (Aldesleukin), Intron A (Recombinant Interferon Alfa-2b), Ipilimumab, Iressa (Gefitinib), Irinotecan Hydrochloride, Irinotecan Hydrochloride Liposome, Istodax (Romidepsin), Ixabepilone, Ixazomib Citrate, Ixempra (Ixabepilone), Jakafi (Ruxolitinib Phosphate), JEB, Jevtana (Cabazitaxel), Kadcyla (Ado-Trastuzumab Emtansine), Keoxifene (Raloxifene Hydrochloride), Kepivance (Palifermin), Keytruda (Pembrolizumab), Kisqali (Ribociclib), Kymriah (Tisagenlecleucel), Kyprolis (Carfilzomib), Lanreotide Acetate, Lapatinib Ditosylate, Lartruvo (Olaratumab), Lenalidomide, Lenvatinib Mesylate, Lenvima (Lenvatinib Mesylate), Letrozole, Leucovorin Calcium, Leukeran (Chlorambucil), Leuprolide Acetate, Leustatin (Cladribine), Levulan (Aminolevulinic Acid), Linfolizin (Chlorambucil), LipoDox (Doxorubicin Hydrochloride Liposome), Lomustine, Lonsurf (Trifluridine and Tipiracil Hydrochloride), Lupron (Leuprolide Acetate), Lupron Depot (Leuprolide Acetate), Lupron Depot-Ped (Leuprolide Acetate), Lutathera (Lutetium Lu 177-Dotatate), Lutetium (Lu 177-Dotatate), Lynparza (Olaparib), Marqibo (Vincristine Sulfate Liposome), Matulane (Procarbazine Hydrochloride), Mechlorethamine Hydrochloride, Megestrol Acetate, Mekinist (Trametinib), Melphalan, Melphalan Hydrochloride, Mercaptopurine, Mesna, Mesnex (Mesna), Methazolastone (Temozolomide), Methotrexate, Methotrexate LPF (Methotrexate), Methylnaltrexone Bromide, Mexate (Methotrexate), Mexate-AQ (Methotrexate), Midostaurin, Mitomycin C, Mitoxantrone Hydrochloride, Mitozytrex (Mitomycin C), MOPP, Mozobil (Plerixafor), Mustargen (Mechlorethamine Hydrochloride), Mutamycin (Mitomycin C), Myleran (Busulfan), Mylosar (Azacitidine), Mylotarg (Gemtuzumab Ozogamicin), Nanoparticle Paclitaxel (Paclitaxel Albumin-stabilized Nanoparticle Formulation), Navelbine (Vinorelbine Tartrate), Necitumumab, Nelarabine, Neosar (Cyclophosphamide), Neratinib Maleate, Nerlynx (Neratinib Maleate), Netupitant and Palonosetron Hydrochloride, Neulasta (Pegfilgrastim), Neupogen (Filgrastim), Nexavar (Sorafenib Tosylate), Nilandron (Nilutamide), Nilotinib, Nilutamide, Ninlaro (Ixazomib Citrate), Niraparib Tosylate Monohydrate, Nivolumab, Nolvadex (Tamoxifen Citrate), Nplate (Romiplostim), Obinutuzumab, Odomzo (Sonidegib), OEPA, Ofatumumab, OFF, Olaparib, Olaratumab, Omacetaxine Mepesuccinate, Oncaspar (Pegaspargase), Ondansetron Hydrochloride, Onivyde (Irinotecan Hydrochloride Liposome), Ontak (Denileukin Diftitox), Opdivo (Nivolumab), OPPA, Osimertinib, Oxaliplatin, Paclitaxel, Paclitaxel Albumin-stabilized Nanoparticle Formulation, PAD, Palbociclib, Palifermin, Palonosetron Hydrochloride, Palonosetron Hydrochloride and Netupitant, Pamidronate Disodium, Panitumumab, Panobinostat, Paraplat (Carboplatin), Paraplatin (Carboplatin), Pazopanib Hydrochloride, PCV, PEB, Pegaspargase, Pegfilgrastim, Peginterferon Alfa-2b, PEG-Intron (Peginterferon Alfa-2b), Pembrolizumab, Pemetrexed Disodium, Perjeta (Pertuzumab), Pertuzumab, Platinol (Cisplatin), Platinol-AQ (Cisplatin), Plerixafor, Pomalidomide, Pomalyst (Pomalidomide), Ponatinib Hydrochloride, Portrazza (Necitumumab), Pralatrexate, Prednisone, Procarbazine Hydrochloride, Procrit (Epoetin Alfa), Proleukin (Aldesleukin), Prolia (Denosumab), Promacta (Eltrombopag Olamine), Propranolol Hydrochloride, Provenge (Sipuleucel-T), Purinethol (Mercaptopurine), Purixan (Mercaptopurine), Radium 223 Dichloride, Raloxifene Hydrochloride, Ramucirumab, Rasburicase, R-CHOP, R-CVP, Recombinant Human Papillomavirus (HPV) Bivalent Vaccine, Recombinant Human Papillomavirus (HPV) Nonavalent Vaccine, Recombinant Human Papillomavirus (HPV) Quadrivalent Vaccine, Recombinant Interferon Alfa-2b, Regorafenib, Relistor (Methylnaltrexone Bromide), R-EPOCH, Retacrit (Epoetin Alfa), Revlimid (Lenalidomide), Rheumatrex (Methotrexate), Ribociclib, RICE, Rituxan (Rituximab), Rituxan Hycela (Rituximab and Hyaluronidase Human), Rituximab, Rituximab and Hyaluronidase Human, Rolapitant Hydrochloride, Romidepsin, Romiplostim, Rubidomycin (Daunorubicin Hydrochloride), Rubraca (Rucaparib Camsylate), Rucaparib Camsylate, Ruxolitinib Phosphate, Rydapt (Midostaurin), Sclerosol Intrapleural Aerosol (Talc), Siltuximab, Sipuleucel-T, Somatuline Depot (Lanreotide Acetate), Sonidegib, Sorafenib Tosylate, Sprycel (Dasatinib), STANFORD V, Sterile Talc Powder (Talc), Steritalc (Talc), Stivarga (Regorafenib), Sunitinib Malate, Sutent (Sunitinib Malate), Sylatron (Peginterferon Alfa-2b), Sylvant (Siltuximab), Synribo (Omacetaxine Mepesuccinate), Tabloid (Thioguanine), TAC, Tafinlar (Dabrafenib), Tagrisso (Osimertinib), Talc, Talimogene Laherparepvec, Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Tecentriq (Atezolizumab), Temodar (Temozolomide), Temozolomide, Temsirolimus, Thalidomide, Thalomid (Thalidomide), Thioguanine, Thiotepa, Tisagenlecleucel, Tocilizumab, Tolak (Fluorouracil--Topical), Topotecan Hydrochloride, Toremifene, Torisel (Temsirolimus), Totect (Dexrazoxane Hydrochloride), TPF, Trabectedin, Trametinib, Trastuzumab, Treanda (Bendamustine Hydrochloride), Trexall (Methotrexate), Trifluridine and Tipiracil Hydrochloride, Trisenox (Arsenic Trioxide), Tykerb (Lapatinib Ditosylate), Unituxin (Dinutuximab), Uridine Triacetate, VAC, Valrubicin, Valstar (Valrubicin), Vandetanib, VAMP, Varubi (Rolapitant Hydrochloride), Vectibix (Panitumumab), VeIP, Velban (Vinblastine Sulfate), Velcade (Bortezomib), Velsar (Vinblastine Sulfate), Vemurafenib, Venclexta (Venetoclax), Venetoclax, Verzenio (Abemaciclib), Viadur (Leuprolide Acetate), Vidaza (Azacitidine), Vinblastine Sulfate, Vincasar PFS (Vincristine Sulfate), Vincristine Sulfate, Vincristine Sulfate Liposome, Vinorelbine Tartrate, VIP, Vismodegib, Vistogard (Uridine Triacetate), Voraxaze (Glucarpidase), Vorinostat, Votrient (Pazopanib Hydrochloride), Vyxeos (Daunorubicin Hydrochloride and Cytarabine Liposome), Wellcovorin (Leucovorin Calcium), Xalkori (Crizotinib), Xeloda (Capecitabine), XELIRI, XELOX, Xgeva (Denosumab), Xofigo (Radium 223 Dichloride), Xtandi (Enzalutamide), Yervoy (Ipilimumab), Yescarta (Axicabtagene Ciloleucel), Yondelis (Trabectedin), Zaltrap (Ziv-Aflibercept), Zarxio (Filgrastim), Zejula (Niraparib Tosylate Monohydrate), Zelboraf (Vemurafenib), Zevalin (Ibritumomab Tiuxetan), Zinecard (Dexrazoxane Hydrochloride), Ziv-Aflibercept, Zofran (Ondansetron Hydrochloride), Zoladex (Goserelin Acetate), Zoledronic Acid, Zolinza (Vorinostat), Zometa (Zoledronic Acid), Zydelig (Idelalisib), Zykadia (Ceritinib), Zytiga (Abiraterone Acetate), or a combination thereof.

In some embodiments, the subject receives carrier frequency modulation therapy alone or in combination with an anti-cancer therapy. In some embodiments, the anti-cancer therapy can be at a dose in a range from about 10 mg/day to about 1000 mg/day. In some embodiments, the anti-cancer therapy can be at a dose in a range from about 20 mg/day to about 1000 mg/day, from about 30 mg/day to about 1000 mg/day, from about 40 mg/day to about 1000 mg/day, from about 50 mg/day to about 1000 mg/day, from about 60 mg/day to about 1000 mg/day, from about 70 mg/day to about 1000 mg/day, from about 80 mg/day to about 1000 mg/day, from about 90 mg/day to about 1000 mg/day, from about 100 mg/day to about 1000 mg/day, from about 200 mg/day to about 1000 mg/day, from about 300 mg/day to about 1000 mg/day, from about 400 mg/day to about 1000 mg/day, from about 500 mg/day to about 1000 mg/day, from about 600 mg/day to about 1000 mg/day, from about 700 mg/day to about 1000 mg/day, from about 800 mg/day to about 1000 mg/day, from about 900 mg/day to about 1000 mg/day, from about 100 mg/day to about 200 mg/day, from about 200 mg/day to about 800 mg/day, from about 200 mg/day to about 600 mg/day, or from about 200 mg/day to about 500 mg/day.

The following Examples are provided solely for illustrative and exemplary purposes, and are not intended to limit the invention in any way.

## EXAMPLE 1

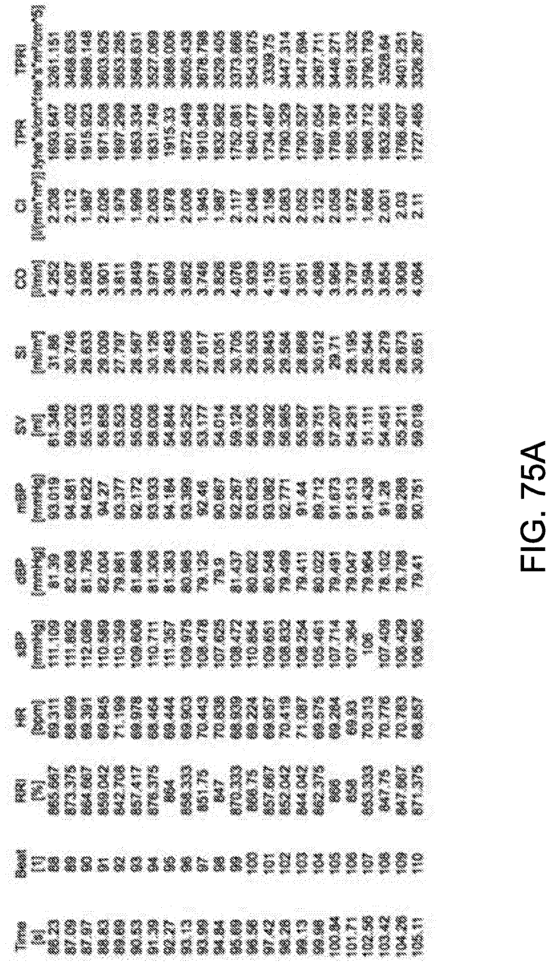

Exemplary of the Hdp values recorded during 23 consecutive heart-beats are set forth in FIG. 75A. The measured and recorded Hdp values for each of the nine hemodynamic parameters are exemplary of such values exhibited by a single patient.

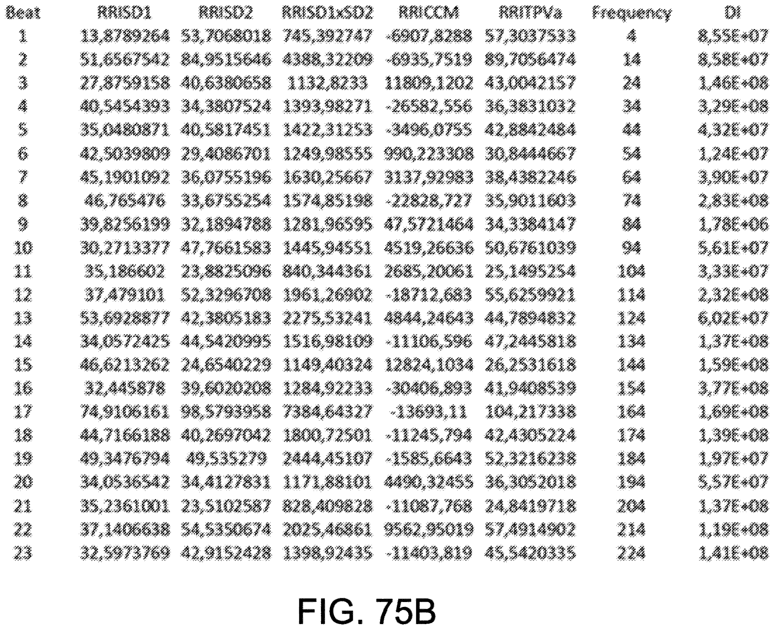

Exemplary of the dependent new attributes parameters values recorded during 23 consecutive heart-beats are set forth in FIG. 75B. The dependent new attributes parameters values may be used as representative Hbp variation values exhibited by a single patient.

### EXAMPLE 2

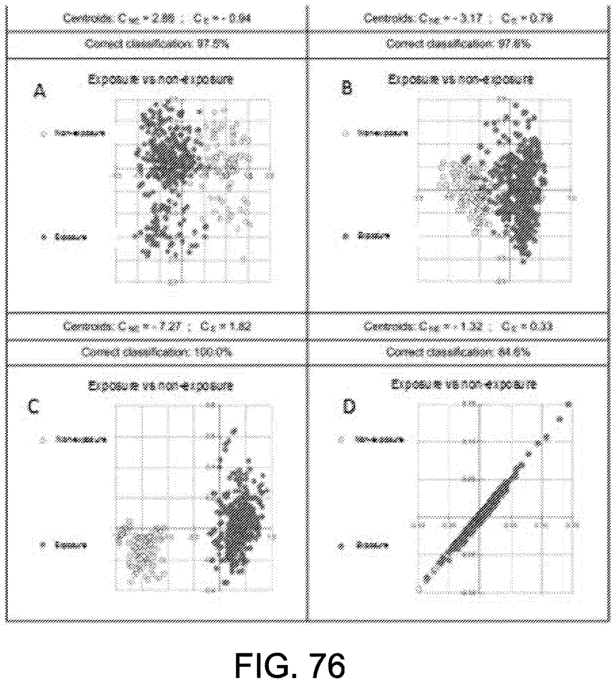

FIG. 76 demonstrates high rates of correct classification using centroids from representative Hdp variation values during basal Hdp values (white) and exposure Hdp values (dark) in four patients with diagnosis of hepatocellular carcinoma during systemic treatment. Patients A, B and C were not responding to the cancer treatment and Patient D was experiencing good response to cancer treatment. The centroids from representative Hdp variation values patterns were significant different between exposure versus non-exposure periods and responding versus non-responding to treatment using four different well-established statistical methods (p < 0.0001).

### EXAMPLE 3

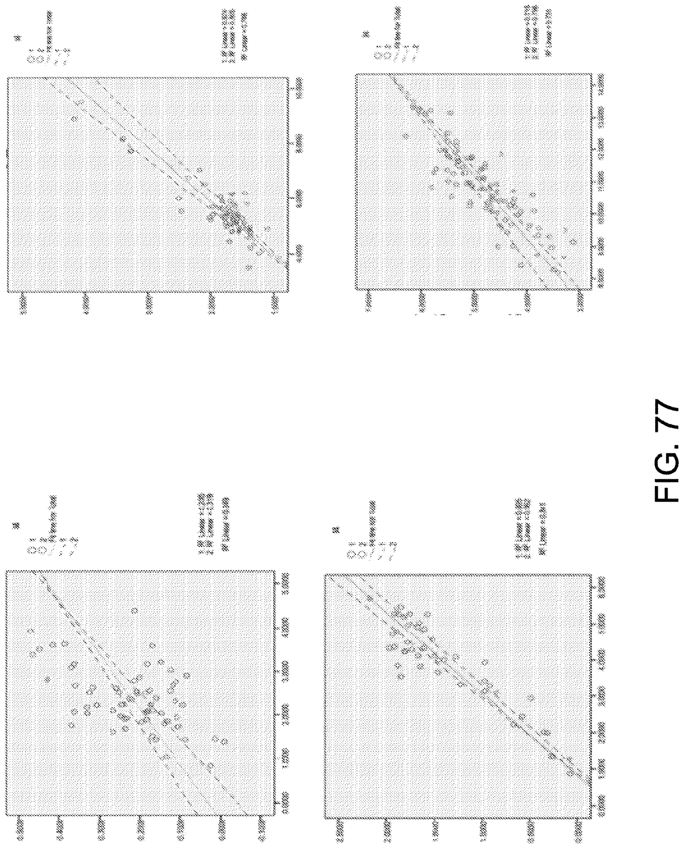

FIG. 77 demonstrates high rates of correlation of representative Hdp variation values and a bio-feedback procedure involving very substantial observations and measurements of physiological responses (at certain well defined AM frequencies) for reactive pulse (e.g., shown as blue in a related plot) and non-reactive pulse (e.g., shown as red in a related plot) in four patients with diagnosis of hepatocellular carcinoma. The from representative Hdp variation values patterns were significant different between reactive versus non-reactive pulse alterations using different well-established statistical methods (p < 0.0001).

### EXAMPLE 4

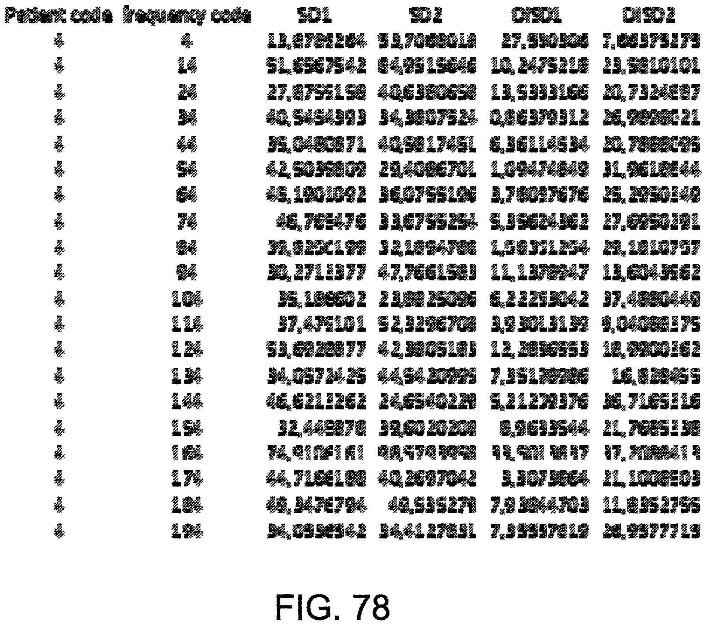

FIG. 78 illustrates differences of representative Hdp variation values determined during the exposure to different SFq. The application of mathematical algorithms and artificial intelligence processing identify EMF frequencies that may cause Hdp variation value changes consistent with a healthy condition of a patient. EMF frequencies causing demonstrated in this example is named SFq.

### EXAMPLE 5

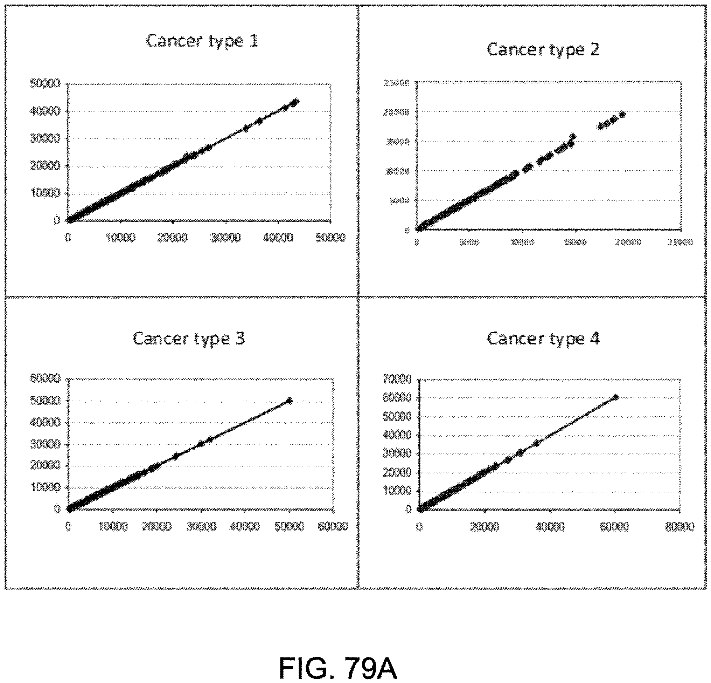

FIG. 79A illustrates a health condition-specific SFq from amplitude modulated from 100 Hz to 40,000 Hz are distributed in an apparent chaotic manner in different health conditions. By reorganizing different health condition-specific SFq using mathematical calculations described in the invention, we obtain linear equation defined by $Hz = a + \beta x$ with $R^2$=99.9%. The artificial intelligence algorithms allow the construction of series of SFq for the diagnosis and treatment of patients. The example showed the distribution of 1,054 cancer-specific frequencies from four cancers types.

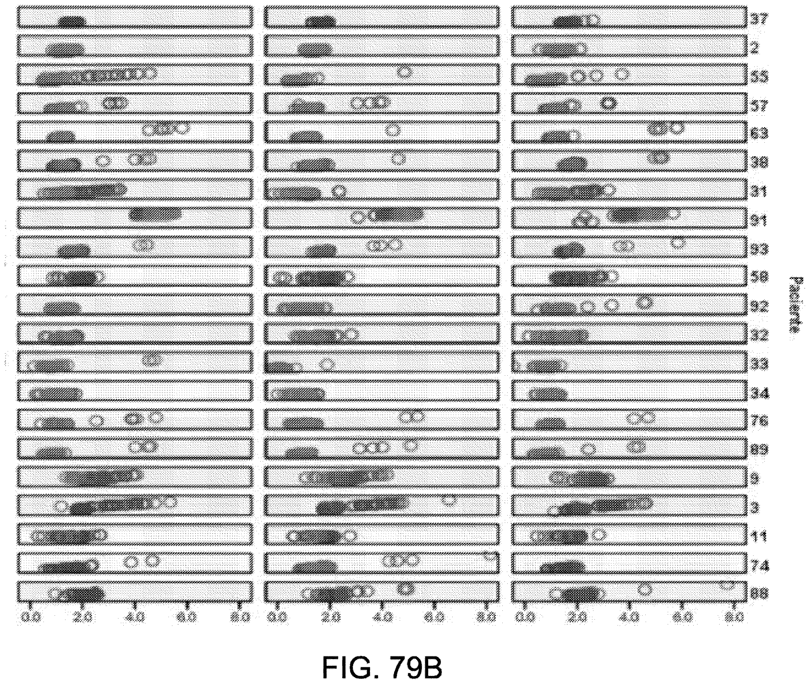

FIG. 79B illustrates a health condition-specific SFq from amplitude modulated from 100 Hz to 40,000 Hz are distributed in a determined manner in different health conditions. The artificial intelligence algorithms allow the identification of patterns in series of SFq used the diagnosis of patients. The example showed the distribution of disease-specific Sfq (red) and healthy-specific Sfq (Brue) during the exposure to three different groups of cancer-specific frequencies in 21 patients.

### EXAMPLE 6

In vitro studies suggest that low levels of amplitude-modulated electromagnetic fields may modify cell growth. Specific frequencies have been identified specific frequencies that may block cancer cell growth. A portable and programmable device capable of delivering low levels of amplitude-modulated electromagnetic fields has been developed. The device emits a 27.12 MHz radiofrequency signal, amplitude-modulated at cancer-specific frequencies ranging from 0.2 to 23,000 Hz with high precision. The device is connected to a spoon-like coupler, which is placed in the patient's mouth during treatment.

Methods:

A phase I study was conducted consisting of three daily 40 min treatments. From March 2004 to September 2006, 24 patients with advanced solid tumors were enrolled. The median age was 57.0 +/- 12.2 years. 16 patients were female. As of January 2007, 5 patients are still on therapy, 13 patients died of tumor progression, two patients are lost to follow-up and one patient withdrew consent. The most common tumor types were breast (7), ovary (5) and pancreas (3). 22 patients had received prior systemic therapy and 16 had documented tumor progression prior to study entry.

Results:

The median duration of therapy was 15.7 +/- 19.9 weeks (range: 0.4-72.0 weeks). There were no NCI grade 2, 3 or 4 toxicities. Three patients experienced grade 1 fatigue during and immediately after treatment. 12 patients reported severe pain prior to study entry. Two of them reported significant pain relief with the treatment. Objective response could be assessed in 13 patients, six of whom also had elevated tumor markers. Six additional patients could only be assessed by tumor markers. Among patients with progressive disease at study entry, one had a partial response for >14.4 weeks associated with >50% decrease in CEA, CA 125 and CA 15-3 (previously untreated metastatic breast cancer); one patient had stable disease for 34.6 weeks (add info); one patient had a 50% decrease in CA 19-9 for 12.4 weeks (recurrent pancreatic cancer). Among patients with stable disease at enrollment, four patients maintained stable disease for 17.0, >19.4, 30.4 and >63.4 weeks.

Conclusions:

The treatment is a safe and promising novel treatment modality for advanced cancer. A phase II study and molecular studies are ongoing to confirm those results.

**EXAMPLE 7**

A Phase II Study of Therapeutic Amplitude-Modulated Electromagnetic Fields in the Treatment of Advanced Hepatocellular Carcinoma (HCC) Federico P Costa, Andre Cosme de Oliveira, Roberto Meirelles Jr., Rodrigo Surjan, Tatiana Zanesco, Maria Cristina Chammas, Alexandre Barbault, Boris Pasche.

Background:

Phase I data suggest that low levels of electromagnetic fields amplitude-modulated at specific frequencies administered intrabuccally with the device of Example A are a safe and potentially effective treatment for advanced cancer. The device emits a 27.12 MHz RF signal, amplitude-modulated with cancer-specific frequencies ranging from 0.2 to 23,000 Hz with high precision. The device is connected to a spoon-like coupler placed in the patient's mouth during treatment. Patients with advanced hepatocellular carcinoma HCC and limited therapeutic options were offered treatment with a combination of HCC-specific frequencies.

Methods:

From October 2005 to July 2007, 43 patients with advanced HCC were recruited in a phase II study. Two patients were considered screening-failures. The patients received three daily 1 hour treatments until disease progression or death. The median age was 64.0 +/- 14.2 years. 17 patients were Child-Pugh status A5-6 and 24 patients were Child-Pugh B7-9. 75.6% of the patients had documented progression of disease (POD) prior to study entry.

Results:

The overall objective response rate as defined by partial response (PR) or stable disease (SD) in patients with documented POD at study entry; 4 PR (1 with near complete response for 58 months) and 16 SD. The median survival was 6.7 months (95% CI 3.0-10.2) and median progression-free survival of 4.4 months (95% 2.1 - 5.3). 14 patients have received therapy for more than six months. The estimated survival at 12, 24 and 36 months were 27.9%, 15.2% and 10.1% respectively. 12 patients reported pain at study entry: eight of them (66%) experienced decreased pain during treatment. There were no NCI grade 2/3/4 toxicities. One patient developed grade 1 mucositis and grade 1 fatigue.

Conclusions:

In patients with advanced HCC the treatment is a safe and effective novel therapeutic option, which has antitumor effect and provides pain relief in the majority of patients.
Thus, it seen that the electronic device of the present invention, comprising means for the accurate control over the frequencies and stability of amplitude modulations of a high frequency carrier signal, provides a safe and promising novel treatment modality for the treatment of patients suffering from various types of advanced forms of cancer.
Exemplary of above accurately controlled amplitude modulated frequencies controlling the frequency of amplitude modulations of a high frequency carrier signal are set forth below along with the type of cancer or tumor harbored by a subject to be treated.

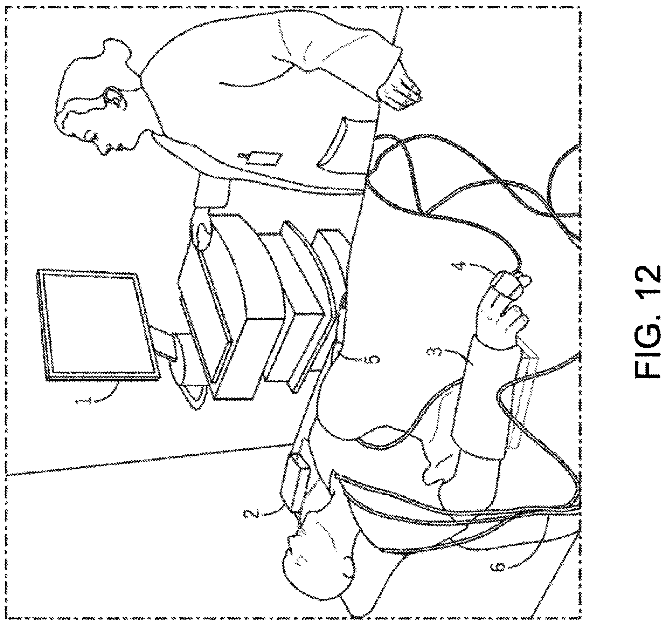

Referring again to the figures, FIG. 12 depicts an illustrative patient during experimental setup for continuous monitoring of hemodynamic parameters before and during AM RF EMF exposure. Non-invasive hemodynamic measurement was performed using a Task Force® Monitor (CNSystems Medizintechnik GmbH, version 2.2.12.0, Reininghausstraße 13, 8020 Graz, Austria). Numerical values of heart rate, blood pressure and blood flow are measured by digital photoplethysmography, pressure cuff and ECG. The hemodynamic parameters are transformed into absolute values for each consecutive heartbeat before and during AM RF EMF exposure. The spoon-shaped antenna for intrabuccal administration of AM RF EMF was placed in the patient's mouth during the entire experiment. In FIG. 12, the Task Force® monitor is labelled 1. The AM RF EMF emitting device is labelled 2. The AM RF EMF emitting device connected to a coaxial cable, which is connected to the spoon-shaped antenna 3. A right arm digital pressure cuff is labelled 4. A digital photoplethysmography is labelled 5. A left arm digital pressure cuff is labelled 6. Electrodes' cables for ECG and impedance cardiography are provided.

Numerical values of heart rate variability, blood pressure, baroreceptor sensitivity and blood pressure were measured by digital photoplethysmography and ECG acquired through three thoracic adhesive electrodes for high resolution for RR interval analysis. Digital pressure cuff was placed on the right arm and around the middle phalanx of the third and fourth right finger and another on the left arm between the shoulder and the elbow. Blood pressure measurements were transformed into absolute values for each consecutive heartbeat.

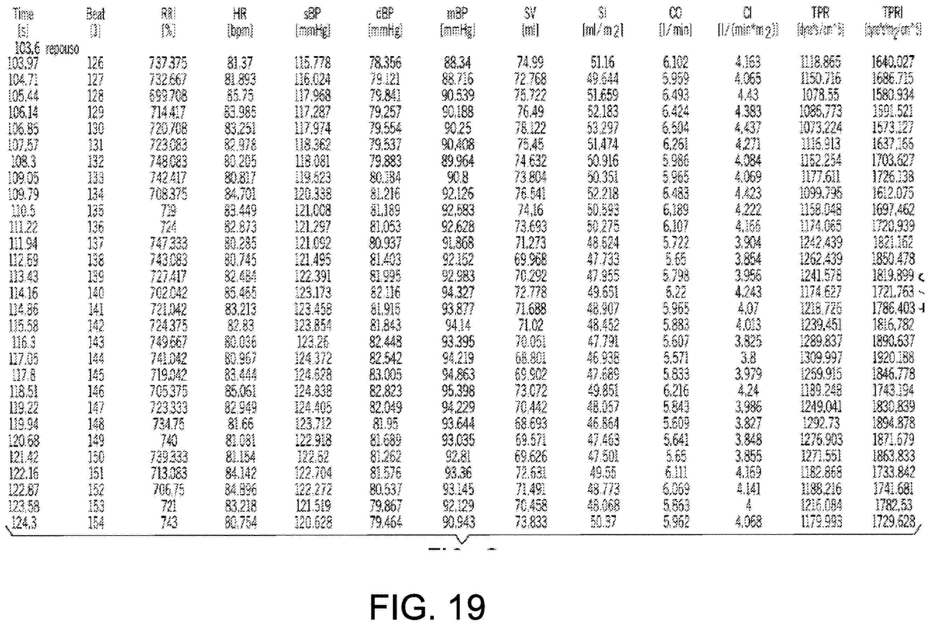

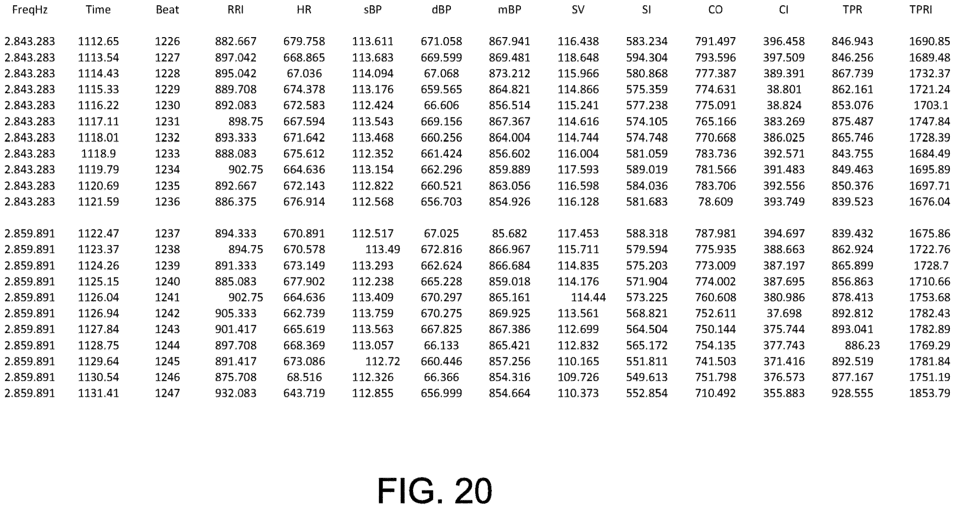

Referring to FIGS. 19-20, examples are seen of eleven hemodynamic parameters simultaneously measured during each heartbeat: heart rate (HR), systolic blood pressure (sBP), median blood pressure (mBP), diastolic blood pressure (dBP), total peripheral resistance (TPR), total peripheral resistance index (TPRI), cardiac output (CO), cardiac index (CI), RR interval (RRI), stroke volume (SV), and systolic index (SI). Hemodynamic recording was performed continuously in supine position before and during exposure to AM RF EMF. A total of three million hemodynamic parameters were analyzed in this study.

Participants held the spoon-shaped antenna in their mouth during the entire experiment. The three different devices each programmed with one of the treatment programs (HCC specific, breast cancer specific, and randomly chosen frequencies) were connected prior to initiation of each of the AM RF EMF exposure period. The protocol was conducted in a double-blind fashion.

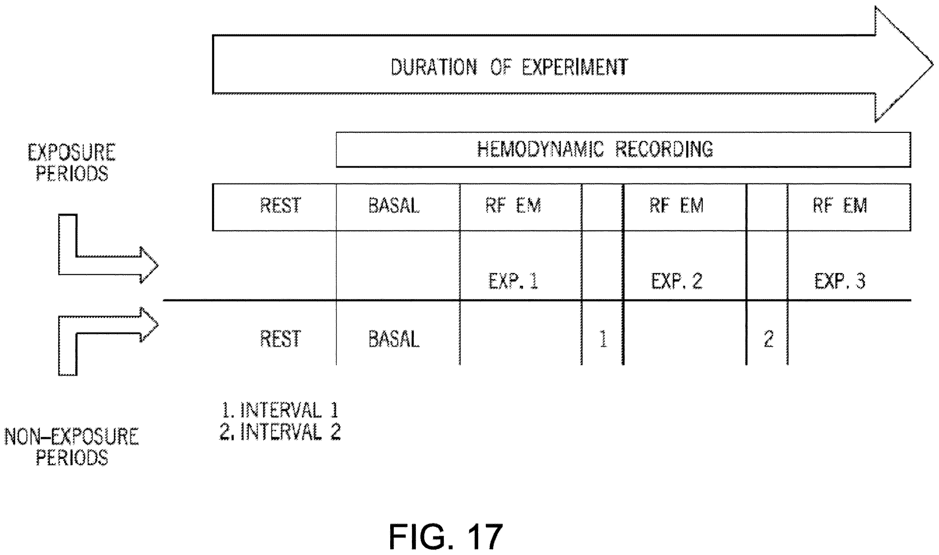

Referring to FIGS. 17-18, hemodynamic recording was performed continuously during non-exposure and exposure periods in a double-blind fashion. The non-exposure periods were the initial basal and resting intervals between RF EMF exposures of five minutes. During the exposure periods, patients received AM RF EMF (HCC-specific, breast cancer-specific, randomly chosen frequencies).

Hemodynamic parameters were analyzed according to three factors: diagnosis (HCC, breast cancer, healthy control), gender, and recording period (baseline and exposure to HCC-specific, breast cancer-specific, and randomly chosen modulation frequencies).

Analysis of the recorded hemodynamic data was only conducted after completion of patient accrual. Patients were selected to constitute a knowledge base for machine learning. The anticipated outcome of the knowledge base group analysis was the creation of computative specific for patients with hepatocellular carcinoma, patients with breast cancer, and healthy controls. Once the computations were constructed, the data from the validation group was analyzed in a blinded fashion in order to validate the computative.

Analysis of six patients diagnosed with potentially resectable HCC was included in the validation group analysis. These patients underwent the same non-invasive hemodynamic parameter measurements within 24 hours prior to HCC surgical resection and after complete recovery within four to six weeks post-surgery. Pre- vs post-surgical analysis was conducted.

Analysis of hemodynamic parameters during the basal non-exposure period was significantly different among healthy controls, patients with hepatocellular carcinoma, and patients with breast cancer in the "Discovery Group" ($p<0.0001$). There were significant differences in hemodynamic parameters between male and female participants as well.

Hemodynamic parameter analysis during the basal non-exposure and exposure periods were conducted separately. Differences of representative Hdp variation values are determined during the exposure to baseline and exposure to HCC-specific, breast cancer-specific, and randomly chosen modulation frequencies separated in different SFq. The application of mathematical algorithms and artificial intelligence processing identify HRV patterns for each different SFq for each individual being diagnosed by HCC, breast cancer, healthy control.

The identification of HRV patterns with the application of mathematical algorithms and artificial intelligence processing resulting from the exposure to modulated frequencies in healthy controls and patients with cancer (HCC or Breast cancer) demonstrated differences in the HRV patterns that could be used in the identification of the patient's diagnosis.

Using a previously selected knowledge base group constituted by 10 patients with biopsy proven HCC and 10 male healthy controls, the artificial intelligent processing analyzed the 10 min baseline non-exposure period in combination of 582 modulated frequencies in order to identify specific HRV patterns used in the diagnosis of a patient.

A validation group of 40 male individuals was tested. The diagnosis was correctly identified in 37 individuals. There were 2 healthy controls labeled as HCC patients and one HCC patient labeled as healthy individual. The artificial intelligent processing algorithm also indicated relevant modulated frequencies or SFq used in the correct discrimination between HCC and healthy controls.

Using a previously selected knowledge base group constituted by 10 patients with biopsy proven breast cancer and 10 female healthy controls, the artificial intelligent processing analyzed the 10 min baseline non-exposure period in combination of 582 modulated frequencies in order to identify specific HRV patterns used in the diagnosis of a patient.

A validation group of 27 female individuals was tested. The diagnosis was correctly identified in 17 of 18 breast cancer patients. The artificial intelligent processing algorithm also indicated relevant modulated frequencies or SFq used in the correct discrimination between HCC and healthy controls.

The identify of specific HRV patterns for modulated frequencies predominantly observed in HCC that significantly differ from healthy controls are selected to be used in the treatment programs of patients with HCC as SFq. The same rational applies for breast cancer patients and possibly other healthy conditions.

In accordance with another aspect of the present invention, the identification and characterization of new methods allowing for the diagnosis of hepatocellular carcinoma and breast cancer in a blinded fashion based solely on the identification of HRV patterns during exposure to 27.12 MHz RF EMF amplitude modulated at tumor-specific frequencies are provided. These findings may have broad clinical implications for the diagnosis of cancer.

U.S. Patent Application Ser. No. 12/450,450 listed those frequencies known as of the filing date of 25 September 2009 while U.S. Patent No. 8,977,365 added those frequencies known as of its filing date of 22 August 2012. Since those filings, additional AM frequencies have been determined by a bio-feedback procedure involving very substantial observations and measurements of physiological responses (at certain well defined AM frequencies) by subjects exposed to low energy electromagnetic emission excitation have been determined to be efficacious in the characterization, diagnosis, treatment, and frequency discovery of the type of cancer or tumor harbored by a subject to be treated. Based on such multiple amplitude modulation frequency values it has been surprisingly discovered that a relationship exists between sequential values or sequential groups of values within the range of frequency values.

Given that SFq are linearly correlated to a series of numbers which constitute a superset of the series of prime numbers, the construction of series of common denominators characterizing all SFq that have been previously patented and determined by a bio-feedback procedure involving very substantial observations and measurements of physiological responses (at certain well defined AM frequencies) by subjects exposed to low energy electromagnetic emission excitation in any health condition of a patient is now being determined by a new the mathematical model described below as an important aspect of the invention in order to perform precise diagnosis and treatment of warm-blooded mammalian subjects.

SFq can be organized in seven infinite families of congruent elements (mod 7), using $n = 7x + i$, where $x = 1, 2, ...$ is a natural number and $i = 0,1,2, ...,6$ is the family index or remainder. Allocating $n$ in blocks b of $i \times k = 42$ positions, where $k = 1,2, ...,6$ defines the position of $n$ in the family $i$ in the respective block resulting, by construction, $x = 6b + k$ where we obtain a convenient representation of natural numbers: $n = 42b + 7k + i = 42b + 0$.

It is not difficult to verify that all prime numbers ($Pn$) except for 2 and 3 belong to the following two $AP's$ ratio $r = 6$: $x = 0,1, ...$ : $AP_1 = 6x + 5$, $AP_2 = 6x + 7$. Evidently, these two $AP's$ also contain compose numbers ($Cn$). This superset of natural numbers represents candidates of primes ($Cp$). Considering the representation of natural numbers in terms of $b$, $k$ and $i$, we obtain Cp given by the superset of natural numbers such that $i + k = 5$ or 7 or 11, $n \geq 5$. Since each family of natural numbers is crossed by two $AP's$, such as $n = 42b + 7k + i = 42b + \theta$, under the restrictions of $i$ and $k$, we obtain two values of $k$ and $\theta$ per family ($i = 1,2, ... ,6$).

Cp can be organized in subdivisions. Restricting to natural numbers such as $i \neq 0$ and $i + k = 5$ or 7 or 11, we obtain 6 linear equations named *families of natural numbers* $n = 7x + i$, $AP's$ ratio $r = 7$, crossed by 2 $AP's$ ratio r = 6. As a result, there are 12 *groups of Cp* $n = 42b + 7k + i = 42b + \theta$, which is equivalent to 6 families by transformation $x = 6b + k$. Cp families are the basis for the construction of common denominators for SFq,

Constructed series of common denominators for SFq can be tested and validated in warm-blooded mammalian subjects and patients by the exposure of single, a series or combination of high specific frequency radio frequency carrier signals predetermined by the new mathematical model described above as an important aspect of the invention. Validation results named representative Hdp variation values and the correlated SFq obtained by the integrated solution of the invention provides a permanent refinement and adjustment of the linear models, based on artificial intelligence methods employed for pattern recognition described above. The methodology describe above provides the identification and generation of infinite series of SFq correlated with a heath condition of a warm-blooded mammalian subject.

## EXAMPLE 8

A total of 81 patients were prospectively evaluated in three separate groups of patients and healthy controls: 1) The discovery group consisted of 6 patients with advanced hepatocellular carcinoma, 6 patients with advanced breast cancer, and 6 healthy controls; 2) The validation group consisted of 25 patients with a diagnosis of cancer (14 female patients with advanced breast cancer and 11 male patients with advanced HCC) and 31 healthy controls (18 females and 13 males); 3) 6 patients (5 males and one female) with potentially resectable hepatocellular carcinoma. All individuals were exposed once to each of the 194 tumor-specific frequencies (HCC specific and breast cancer specific) and 194 randomly chosen frequencies previously reported, which were each emitted for 3 s.

Numerical values of heart rate variability, blood pressure, baroreceptor sensitivity and blood pressure were measured by digital photoplethysmography and ECG. Hemodynamic recording was performed with the patient in supine position before and during exposure to AM RF EMF.

Data analysis began with the discovery group for the creation of mathematical algorithms specific for patients with hepatocellular carcinoma, patients with breast cancer, and healthy controls. The data from the validation group was analyzed in a blinded fashion in order to validate the algorithms.

In the discovery group, we observed a similar hemodynamic response pattern to exposure of AM RF EMF in 6 (100.0%) of 6 patients with HCC, which only occurred during exposure to HCC-specific modulation frequencies. In patients with breast cancer, we identified a similar hemodynamic response pattern in 5 (83.3%) of 6 cases, during exposure to Breast cancer-specific modulation frequencies. We did not observe a tumor-specific hemodynamic response in healthy controls or during exposure to random chosen frequencies. In the validation group tumor-specific hemodynamic response pattern was identified in 22 (88.0%) of 25 patients.

Pre-planned post-Hoc analysis demonstrated that phenomenon could be explained by the oscillation in the interval between consecutive heart-beats as well as the oscillations between consecutive instantaneous heart rates known as heart rate variability (HRV), a described variation of both instantaneous heart rate and RR intervals (RRI). Variations in heart rate can be studied by the time-domain measures in a continuous consecutive RRI measurements. RRI fluctuations has a complex non-linear behavior. For the analysis of very short R-R intervals, Poincaré plots is often used and validated, but it requires precision in data collection (data synchronization).

## EXAMPLE 9

We conducted two prospective clinical studies exposing cancer patients as single treatment modality to AM RF EMF selected by HRV biological surrogate: a feasibility study with 28 patients with various tumor types and a phase I/II study in 41 patients with advanced hepatocellular carcinoma patients (HCC). Two of the seven patients with metastatic breast cancer and one patient with recurrent metastatic thyroid cancer had major durable responses in the feasibility study. Objective response was observed in four (9.8%) patients with HCC. One patient had a near-complete response lasting over 5 years and 7 patients had a disease control over 2 years of observation. No patient experienced any limiting toxicity even after over 7 years of treatment.

In vitro experiments using frequency modulation identified by HRV biological surrogate in patients with cancer blocked the growth of breast cancer cells and HCC cells at specific cancer frequency modulation. Confocal laser scanning microscopy revealed pronounced disruption of the mitotic spindle. Alterations in gene expression in key modulators of the IP3/DAG signaling arm of the PI3K pathway and calcium binding proteins were identified by RNA-seq and Micro-RNA analysis. Alterations in calcium flux through mictrotubles were identified by a calcium indicator analysis. Some anti-tumor effects may be calcium dependent.

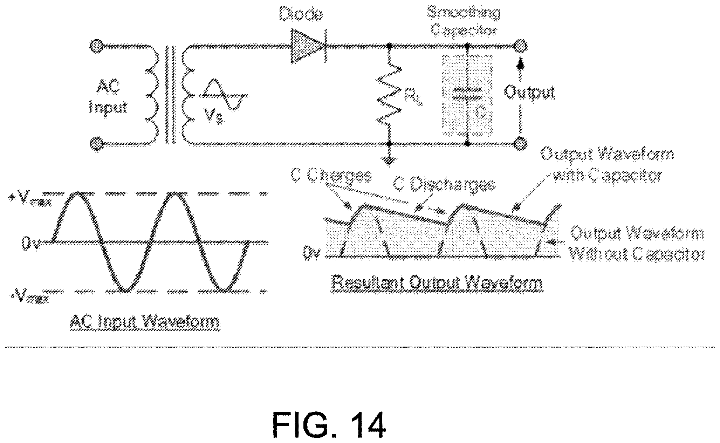

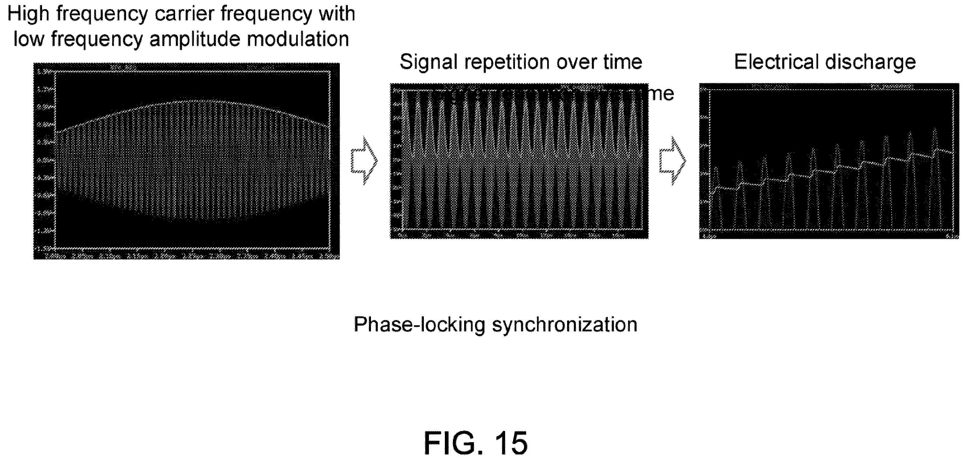

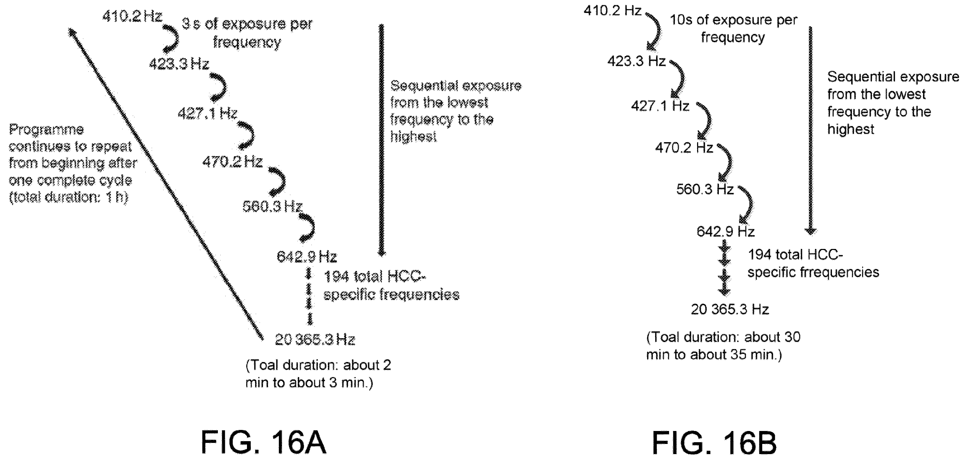

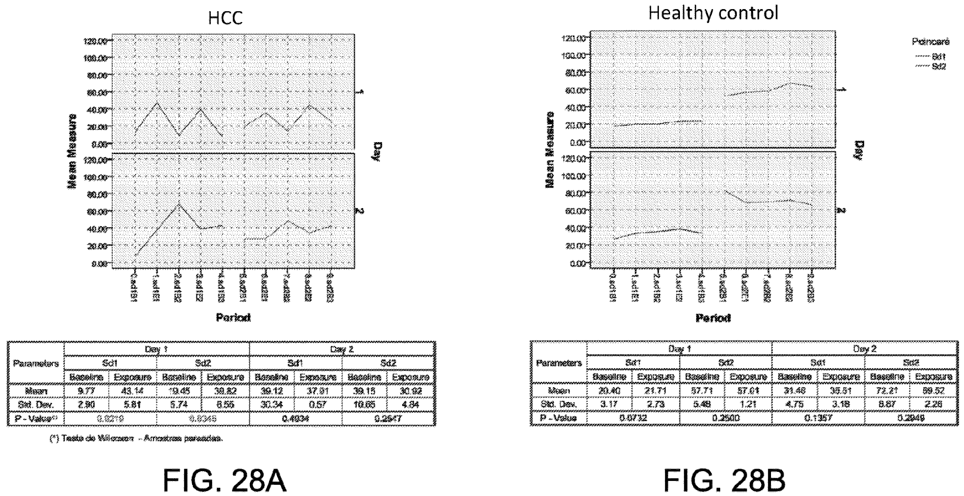

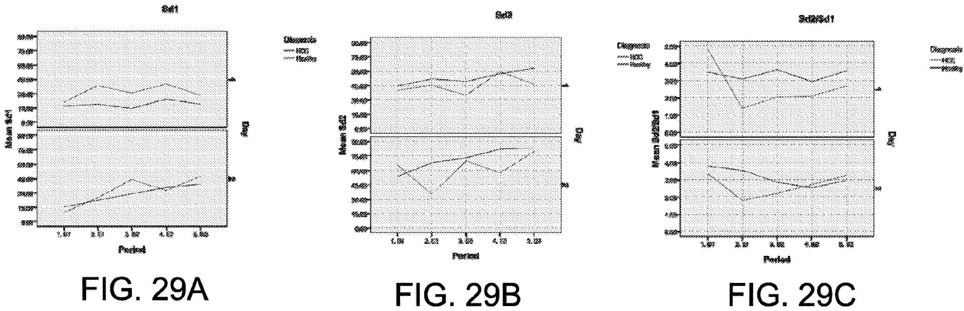

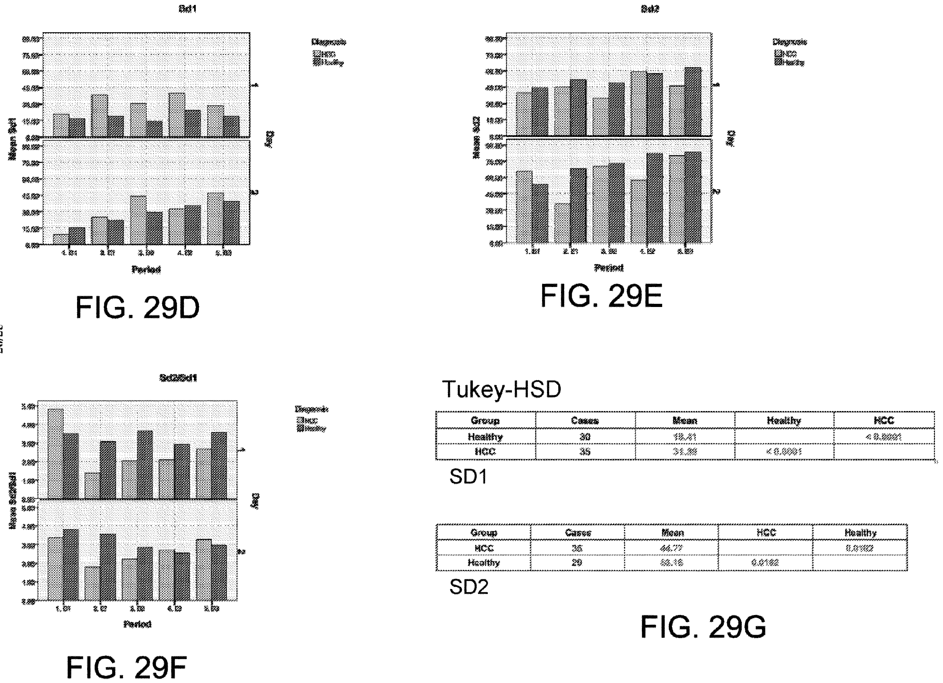

In order to demonstrate if the biological effects in humans are dependent on the carrier frequency or on the amplitude modulation signal (see FIG. 13), we conducted a prospective study in 14 individuals (7 healthy controls and 7 HCC) exposed to 27.12 MHz carrier frequency ± 194 amplitude modulated frequencies (10 second sequential exposure ranging from of 400 Hz to 20 kHz) in 5 alternating periods of exposure in two consecutive days. Hemodynamic parameters were recorded using a high precision and non-invasive hemodynamic monitor (Task Force monitor, CNSystem) synchronized with the frequency modulation radiofrequency generator (see FIGS 14-16). Real time HRV data analysis per period of exposure was performed using an Artificial Intelligence computing process. In addition, single frequency modulation responses per patient were identified and used for patient's diagnosis identification using Support Vector Machine algorithms. We were able to demonstrate that the human body produces a reproducible autonomic stress response to the exposure of HCC-specific frequency amplitude modulation of a 27.12 MHz carrier frequency significantly more pronounced in the HCC patients (Tukey-HSD test, $p < 0.05$) (FIG. 29). The response pattern identification was able to differentiate between healthy individuals and HCC patient groups (FIG. 28). The hemodynamic response to frequency amplitude modulation was immediate, supporting the idea of a frequency-specific intracellular electrical signal demodulation system resulting in tissue-specific biological alterations (e.g. antitumoral effect) and patient's diagnosis in a non-invasive and non-toxic fashion. We hypothesize that the cell-level mechanism of action of these fields involves ionic conduction along microtubules and possibly actin filaments consistently with earlier computational simulations.

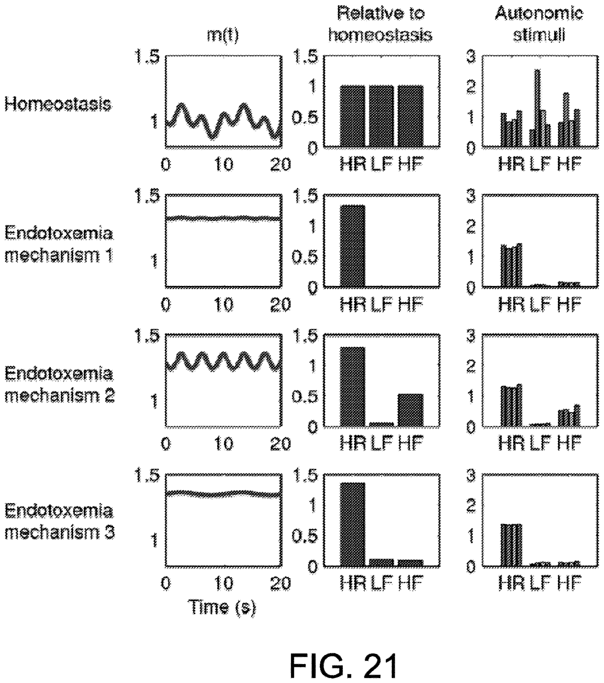

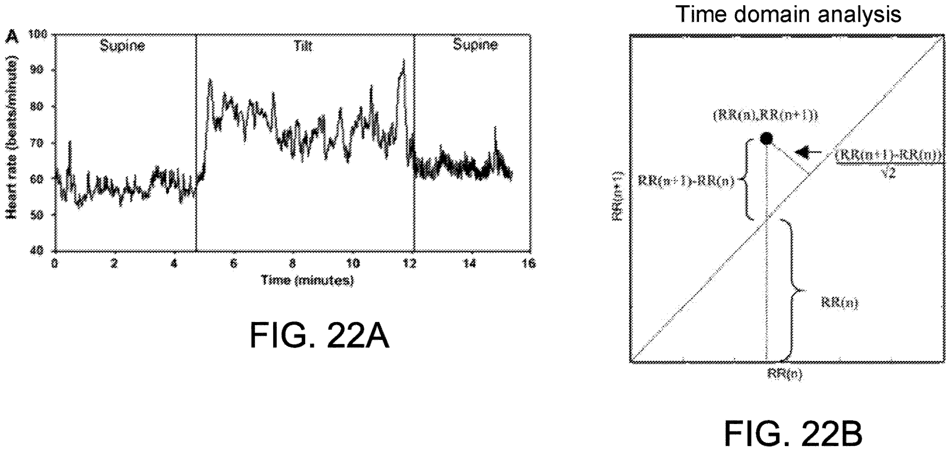

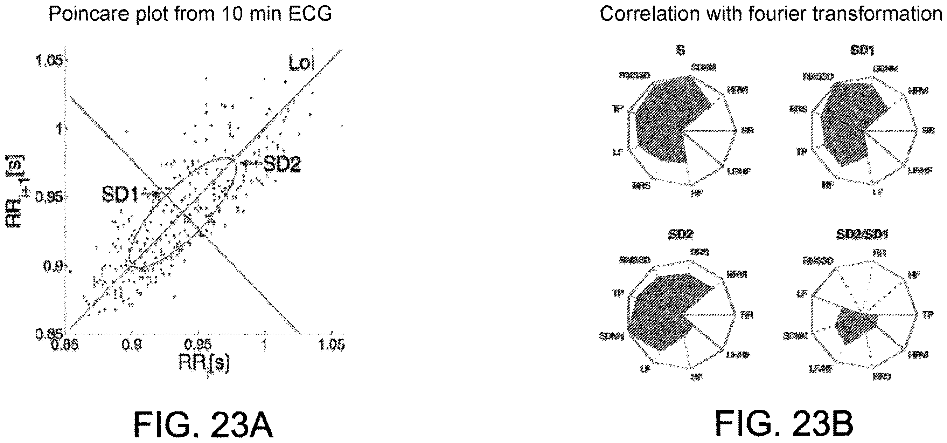

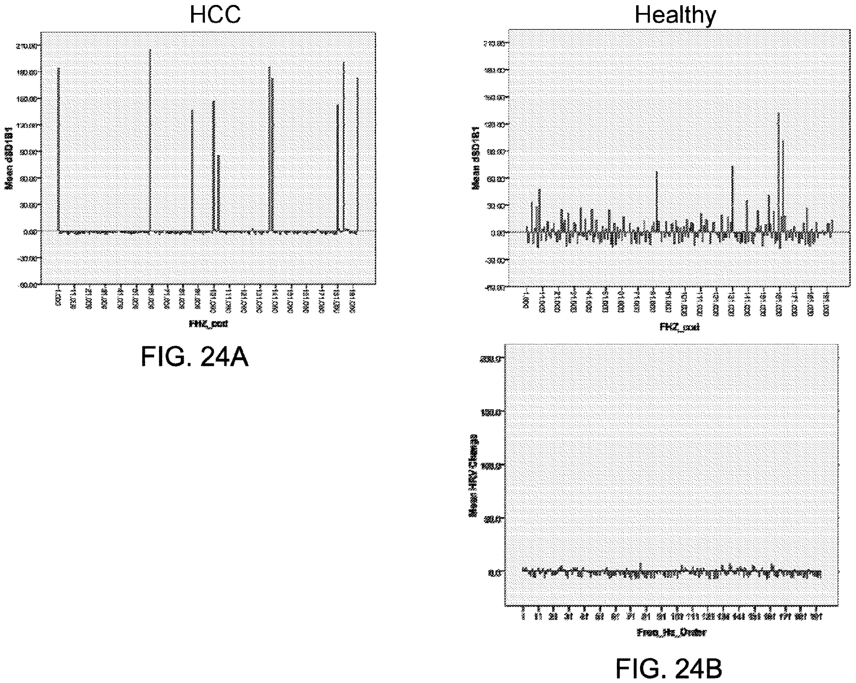

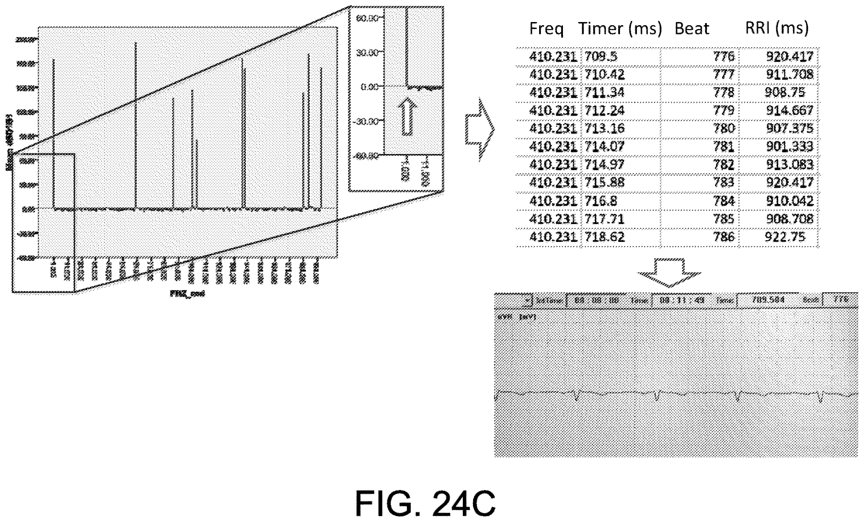

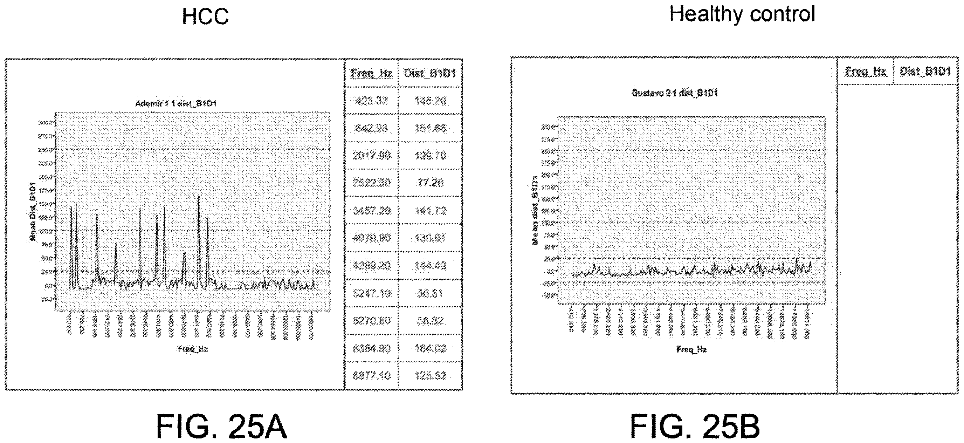

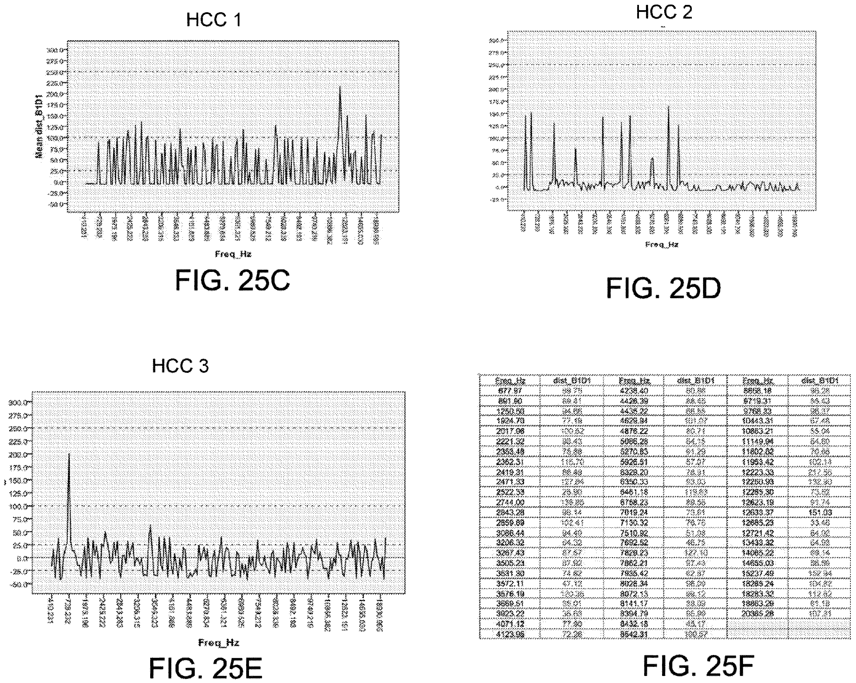

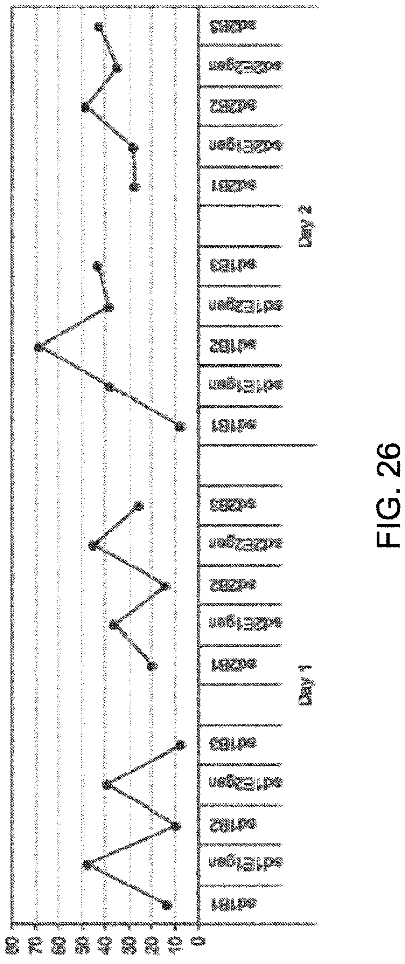

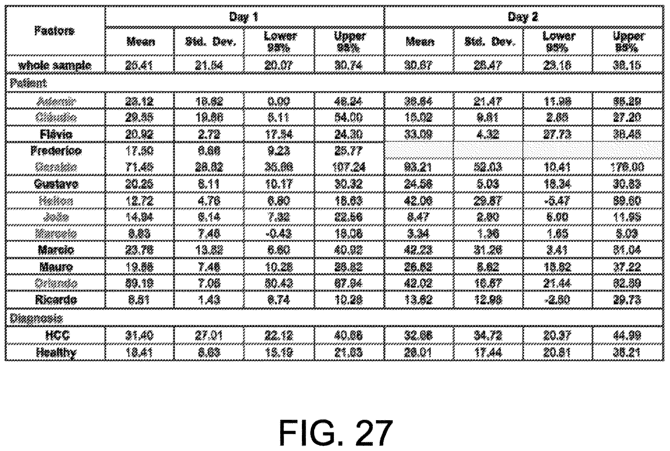

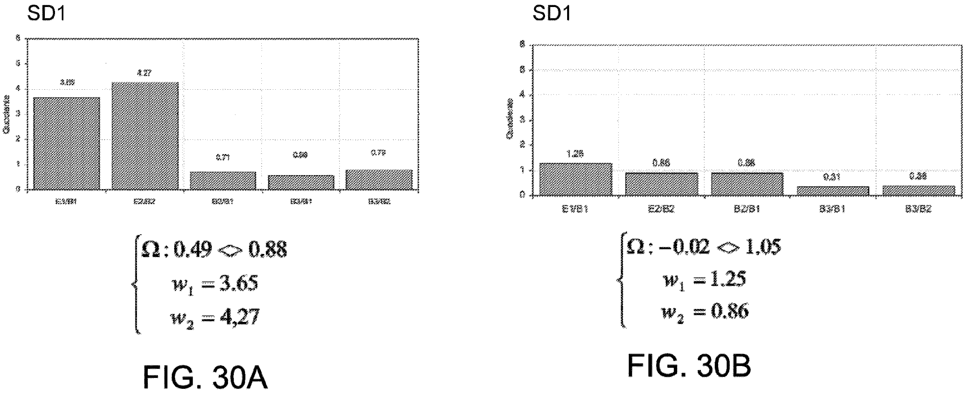

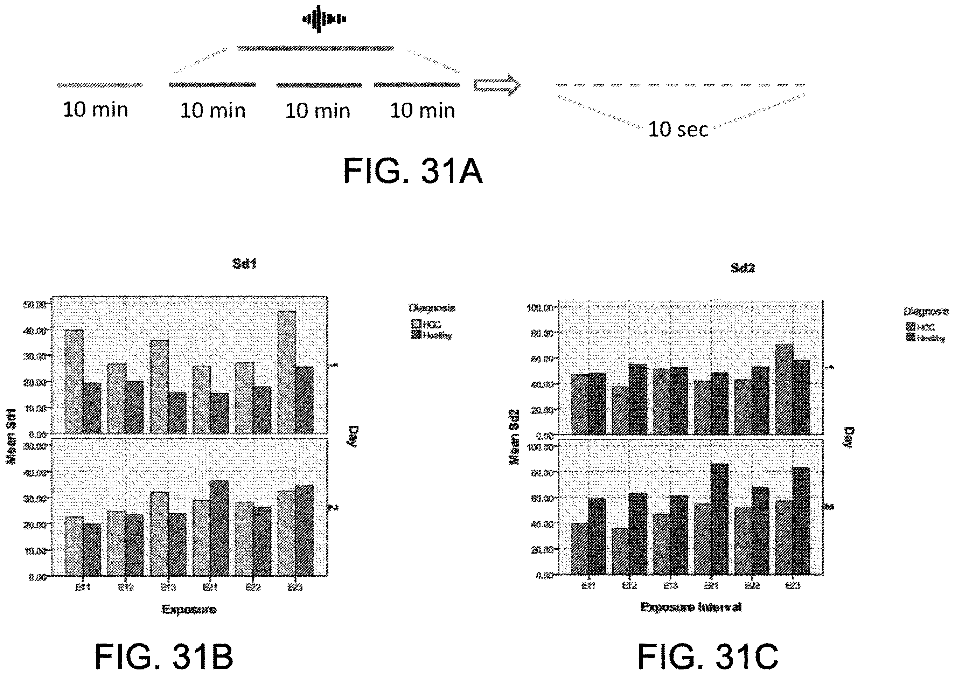

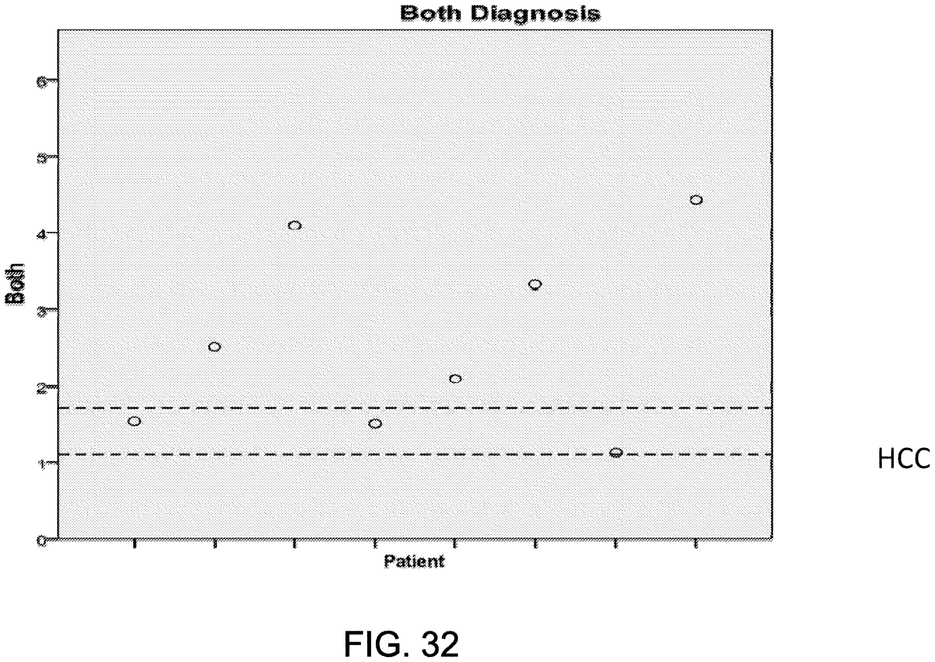

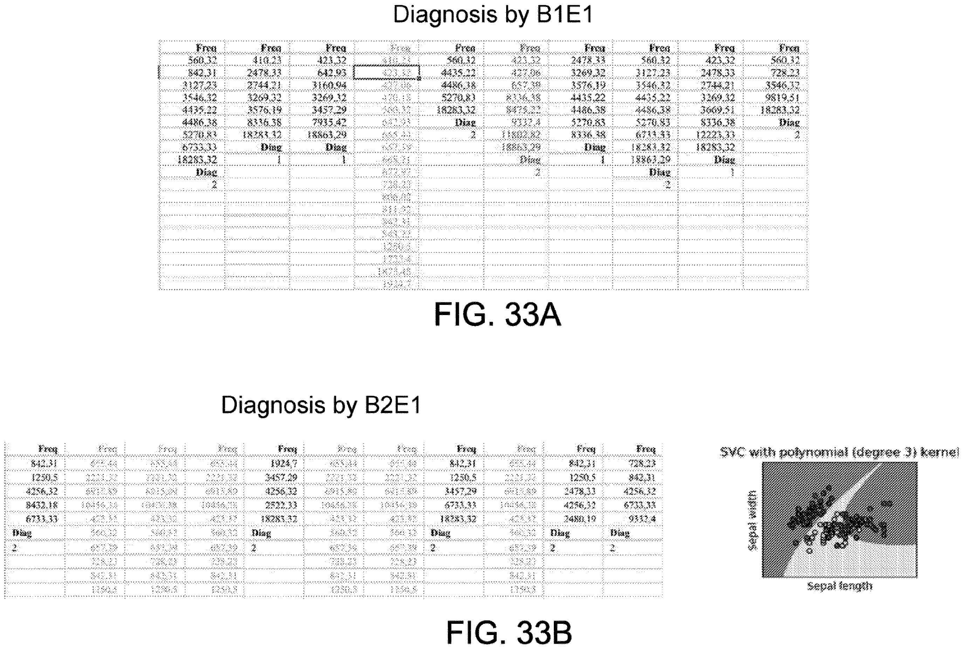

FIGS. 21-33 depict significant differences in Sd2/Sd1 (Poincaré plot hemi-axis) between the carrier frequency exposure periods (B1, B2 and B3) and carrier frequency modulated at HCC-specific frequency amplitude modulation exposure periods (E1 and E2) over the course of multiple days.

**EXAMPLE 10**

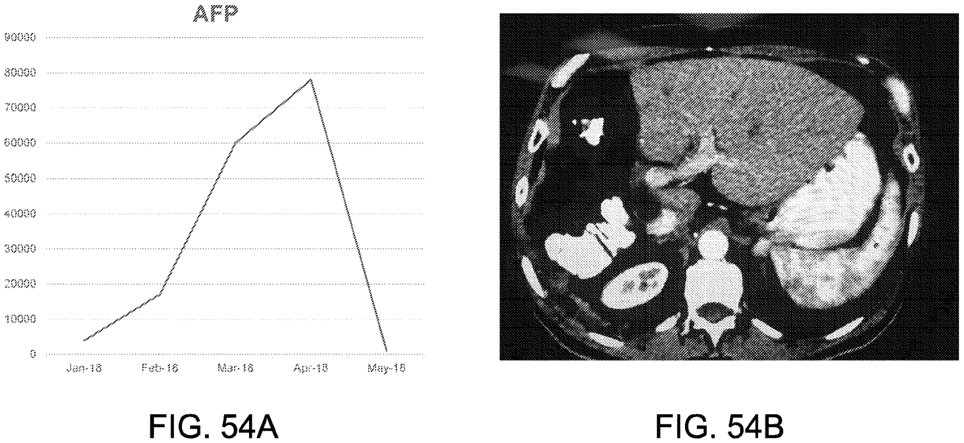

We conducted a study in a patient with advanced hepatocellular carcinoma (HCC) with hepatitis B. Progressive tumor mass was present in the right lobe of the liver with > 20cm in tumor mass size. Pathology confirmed poorly differentiated HCC with portal invasion. Alpha-fetoprotein (AFP) tumor marker was first measured at 3,883 (FIG. 54A). Patient started on sorafenib 800 mg/day, and subsequently reduced to 400 mg/day. Subsequent AFP measurement included values of 17,000 and 60,5000 (FIG. 54A). The patient was exposed to 27.12 MHz carrier frequency $\pm$ 194 amplitude modulated frequencies (10 second sequential exposure ranging from of 400 Hz to 20 kHz) in 2 alternating periods of exposure for two consecutive days (FIG. 18A). Hemodynamic parameters were recorded using a high precision and non-invasive hemodynamic monitor (Task Force monitor, CNSystem) synchronized with the frequency modulation radiofrequency generator (see FIGS 14-16). Real time HRV data analysis per period of exposure was performed using an Artificial Intelligence computing process. In addition, single frequency modulation responses per patient were identified and used for patient's diagnosis / treatment identification using Support Vector Machine algorithms. Following the exposure protocol, the patient's AFP level was reduced to 1,080 (FIG. 54A) and CT scan revealed a complete clinical response (FIG. 54B).

**EXAMPLE 11**

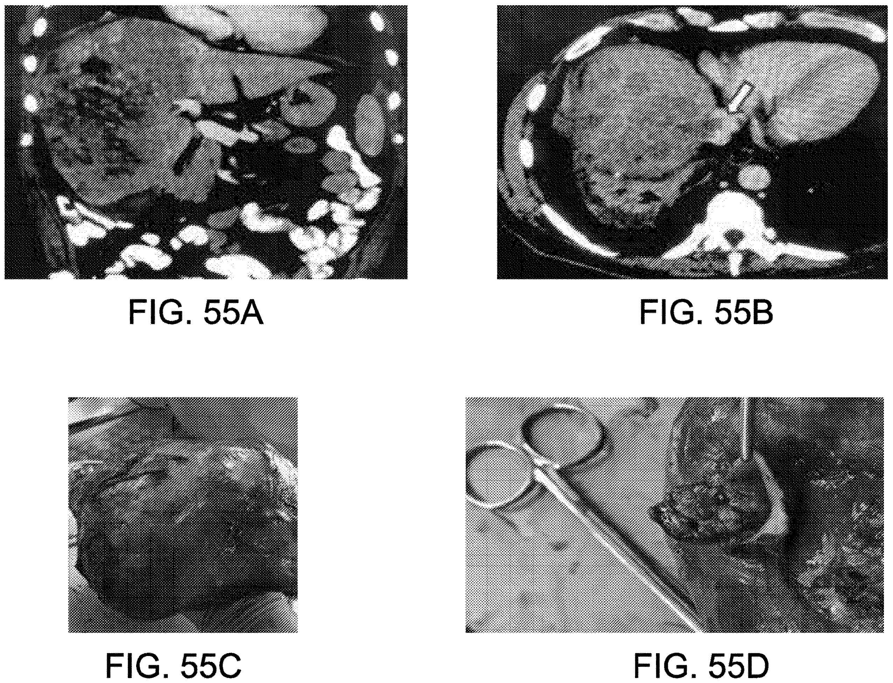

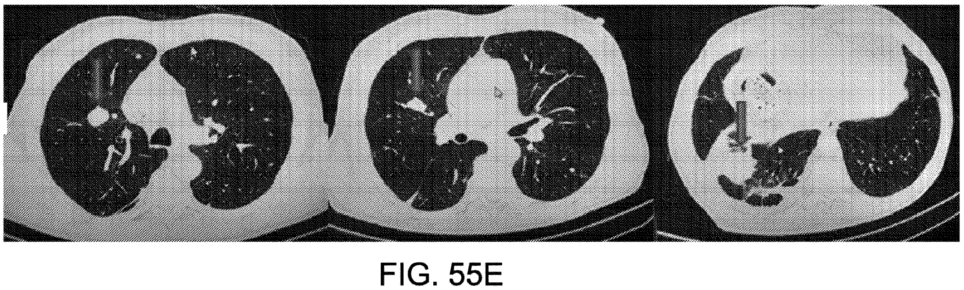

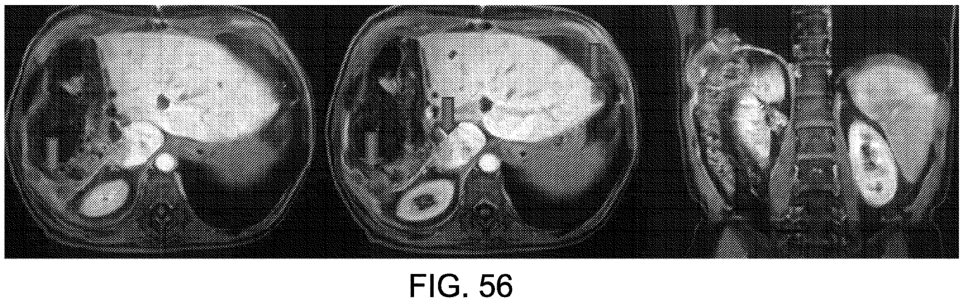

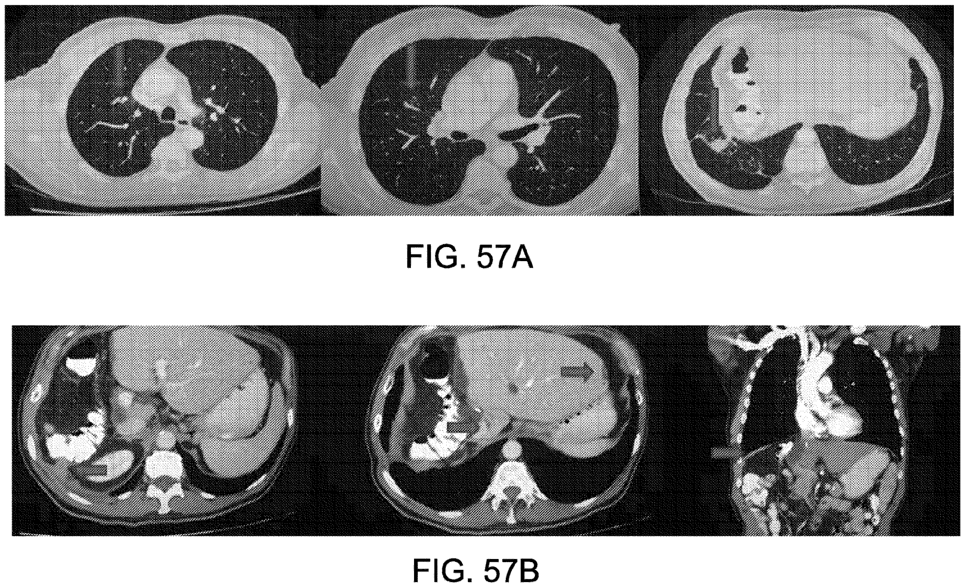

We conducted a study in a 71 year old caucasian male with pulmonary thromboembolism and chronic hepatitis B virus. Initial abdominal CT scan revealed a large tumor mass in the right hepatic lobe with dimensions of 18.7 cm $\times$ 14.2 cm $\times$ 14.5 cm with tumor invasion of the inferior vena cava (FIG. 55A & FIG. 55B). Blood results revealed total bilirubin of 1.12 mg/dL; total protein INR 1.31; and creatinine of 0.8 mg/dl. The patient was then submitted to palliative extended right hepatectomy with positive resection margins. Tumor invasion of the inferior vena cava and right portal vein and a poorly differentiated hepatocellular carcinoma was confirmed by pathology (FIGS. 55C-55D). Subsequent CT scan revealed bilateral metastatic pulmonary nodules (FIG. 55E), and an abdominal MRI showed a progressive tumor mass in Seg II/III with 5.7 cm $\times$ 4.5 cm $\times$ 6.8 cm and Seg IVA with 1.5 cm associated with multiple subphrenic and peritoneal metastatic lesions with an average of 3.0 cm in size (FIG. 56). The patient then started on the anti-cancer therapy of sorafenib 800 mg/day, and the dose was reduced to 400 mg/day due to hand and foot syndrome. The patient maintained an irregular use of sorafenib 400 mg/day. Blood work was again ordered and revealed total bilirubin of 0.98 mg/dL; total protein 1.27; and creatinine of 0.84. Initial AFP values revealed 38,834 (nl < 7.0) at the beginning of sorafenib therapy, and 60,500 following 2 months of sorafenib therapy. The patient was exposed to 27.12 MHz carrier frequency $\pm$ 194 amplitude modulated frequencies (10 second sequential exposure ranging from 400 Hz to 20 kHz, followed by 3 second sequential exposure ranging from 400 Hz to 20 kHz) for 1 period of exposure for one day (FIG. 18D). Hemodynamic parameters were recorded using a high precision and non-invasive hemodynamic monitor (Task Force monitor, CNSystem) synchronized with the frequency modulation radiofrequency generator (see FIGS 14-16). Real time HRV data analysis per period of exposure was performed using an Artificial Intelligence computing process. In addition, single frequency modulation responses per patient were identified and used for patient's diagnosis / treatment identification using Support Vector Machine algorithms. Following the exposure protocol, the patient's AFP level was reduced to 1,008 and CT scan revealed a significant clinical response as shown in FIG. 57A the cavitation of pulmonary metastasis and in FIG. 57 B the disappearance of hepatic tumor mass and residual peritoneal implant following the exposure protocol.

**EXAMPLE 12**

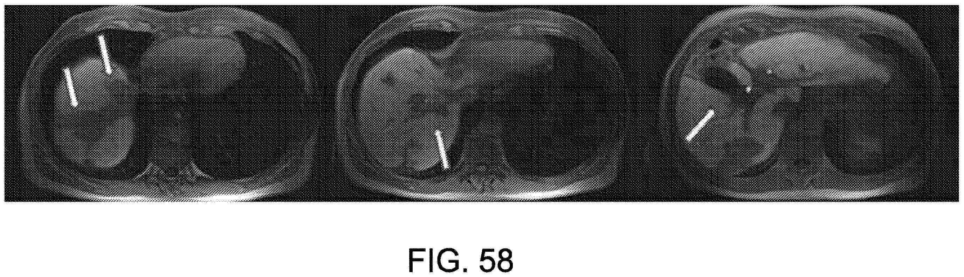

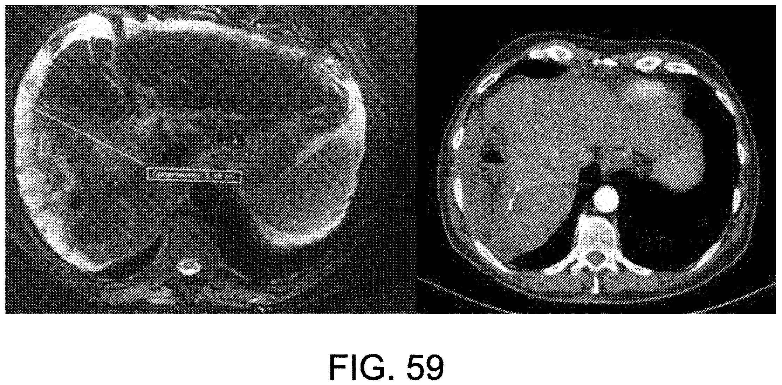

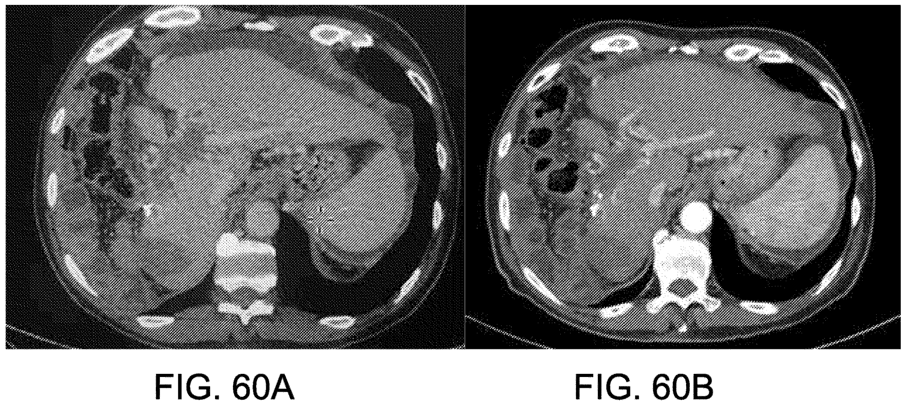

We conducted a study in a 60 year old caucasian male diagnosed with chronic hepatitis C virus who was subsequently treated for hepatitis C with interferon therapy and had a complete virologic response. Follow up abdominal ultrasound revealed liver cirrhosis and portal hypertension. Follow up abdominal CT scan revealed a single right hepatic nodule with dimensions of 4.5 cm $\times$ 4.22 cm $\times$ 4.35 cm. Follow up abdominal CT scan confirmed a 4.9 cm nodule in SEG VIII and a 0.7 cm nodule in Seg VIII and a 1.4 cm nodule in SEG VII. Hepatic MRI confirmed the same hepatic nodules consistent with hepatocellular carcinoma. The patient then received transarterial chemoembolization (TACE). Six months later, the patient received radioablation in Seg VIII. The patient subsequently received radioablation in Segs II, IV, VI, VII and VIII. At follow up, the hepatic MRI nodule in Seg VIII revealed progression and invasion of the portal vein (FIG. 58). Blood results revealed total bilirubin of 1.66 mg/dL; total protein INR 1.1; and creatinine of 1.1 mg/dl. The patient then underwent a tentative resection of the right hepatic lobe without success. Abdominal CT and MRI revealed progressive disease in Seg VIII when compared to patient's previous hepatic MRI scan. The patient then displayed tumoral thrombosis of the portal vein and new nodules in the left hepatic lobe (FIG. 59). The patient then started on the anti-cancer therapy Nexavar at 800 mg/day. Blood work was again ordered and revealed total bilirubin of 0.9 mg/dL; total protein INR 1.03; and creatinine of 0.95 mg/dL. The patient was exposed to 27.12 MHz carrier frequency $\pm$ 194 amplitude modulated frequencies (10 second sequential exposure ranging from of 400 Hz to 20 kHz) for 2 periods of exposure for two consecutive days. Hemodynamic parameters were recorded using a high precision and non-invasive hemodynamic monitor (Task Force monitor, CNSystem) synchronized with the frequency modulation radiofrequency generator (see FIGS 14-16). Real time HRV data analysis per period of exposure was performed using an Artificial Intelligence computing process. In addition, single frequency modulation responses per patient were identified and used for patient's diagnosis / treatment identification using Support Vector Machine algorithms. Following the exposure protocol, the patient's CT scan revealed a complete clinical response as shown in FIG. 60A the disappearance of tumor mass following two weeks after the exposure protocol and in FIG. 60B the disappearance of tumor mass following four weeks after the exposure protocol.

**EXAMPLE 13**

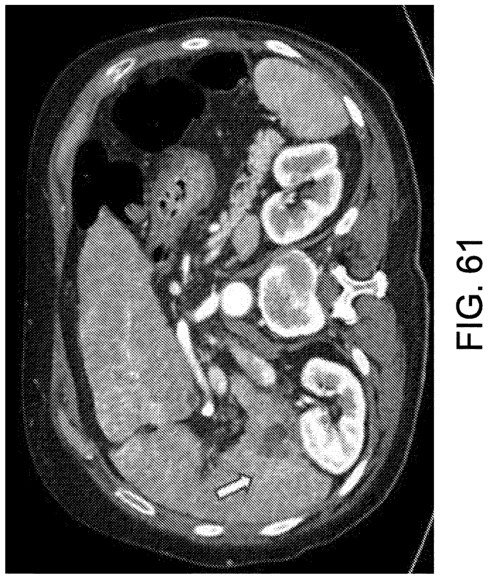

We conducted a study in a 75 year old caucasian male diagnosed with alcoholic cirrhosis. Abdominal ultrasound revealed new nodules in the right hepatic lobe. Abdominal MRI revealed multiple hepatic nodules in Seg VIII/V with 2.3 and 1.6 cm dimensions, along with dysplastic nodules. The patient then received transarterial chemoembolization (TACE) and began anti-cancer therapy of Nexavar at 200 mg/day with poor tolerability. Total column MRI revealed metastatic lesions in T%, T11, T9, T12, along with L3, L4 and L5. The patient was submitted to external beam radiation for the treatment of bone lesions. Blood results revealed total bilirubin of 1.75 mg/dL; total protein INR 1.2; and creatinine of 0.97 mg/dl and an AFP of 257.6. The patient was exposed to 27.12 MHz carrier frequency $\pm$ 194 amplitude modulated frequencies (10 second sequential exposure ranging from 400 Hz to 20 kHz) for 2 periods of exposure for two consecutive days. Hemodynamic parameters were recorded using a high precision and non-invasive hemodynamic monitor (Task Force monitor, CNSystem) synchronized with the frequency modulation radiofrequency generator (see FIGS 14-16). Real time HRV data analysis per period of exposure was performed using an Artificial Intelligence computing process. In addition, single frequency modulation responses per patient were identified and used for patient's diagnosis / treatment identification using Support Vector Machine algorithms. Twelve days following the exposure protocol, the patient's CT scan revealed near complete clinical response with tumor reduction as shown in FIG. 61.

**EXAMPLE 14**

We conducted a study in twenty patients patient diagnosed with hepatocellular carcinoma. The patients were exposed to 27.12 MHz carrier frequency $\pm$ 194 amplitude modulated frequencies (10 second or 3 second sequential exposure ranging from 400 Hz to 20 kHz) for 2 consecutive periods of exposure for one day. Following the first exposure period, hemodynamic parameters were recorded using a high precision and non-invasive hemodynamic monitor (Task Force monitor, CNSystem) synchronized with the frequency modulation radiofrequency generator (see FIGS 14-16). Real time HRV data analysis per period of exposure was performed using an Artificial Intelligence computing process. In addition, single frequency modulation responses per patient were identified and used for the patients' treatment identification using Support Vector Machine algorithms. Following the initial exposure protocol, the patients were exposed to a subsequent exposure protocol, wherein the subsequent exposure protocol comprised only the amplitude modulated frequencies from the initial exposure protocol that were determined to alter the heart rate variability, as illustrated in FIG. 18D. The patients' CT scan revealed a complete clinical response the disappearance of tumor mass. As such, by exposing the same range of amplitude modulated frequencies to various patients with the same diagnosis (e.g. hepatocellular carcinoma), we are able to identify the patients' "active" frequencies (i.e. those amplitude modulated frequencies that result in an alteration in heart rate variability) and thus able to construct an individualized treatment protocol tailored to the patient.

**EXAMPLE 15**

We conducted a study in a patient diagnosed with hepatocellular carcinoma. The patient was exposed to 27.12 MHz carrier frequency $\pm$ 194 amplitude modulated frequencies (10 second or 3 second sequential exposure ranging from 400 Hz to 20 kHz) for 2 consecutive periods of exposure for one day. Following the first exposure period, hemodynamic parameters were recorded using a high precision and non-invasive hemodynamic monitor (Task Force monitor, CNSystem) synchronized with the frequency modulation radiofrequency generator (see FIGS 14-16). Real time HRV data analysis per period of exposure was performed using an Artificial Intelligence computing process. In addition, single frequency modulation responses per patient were identified and used for patient's treatment identification using Support Vector Machine algorithms. Following the initial exposure protocol, the patient was exposed to a subsequent exposure protocol, wherein the subsequent exposure protocol comprised the 27.12 MHz carrier frequency $\pm$ 194 amplitude modulated frequencies (10 second or 3 second sequential exposure ranging from 400 Hz to 20 kHz), as illustrated in FIG. 18D. The patient's CT scan revealed a complete clinical response the disappearance of tumor mass. As such, by exposing the same range of amplitude modulated frequencies to various patients with the same diagnosis (e.g. hepatocellular carcinoma), we are able to construct an individualized treatment protocol tailored to the patient.

**EXAMPLE 16**

We conducted a study in a patient diagnosed with hepatocellular carcinoma. The patient was exposed to 27.12 MHz carrier frequency ± 194 amplitude modulated frequencies (10 second or 3 second sequential exposure ranging from 400 Hz to 20 kHz) for 2 consecutive periods of exposure for one day. Following the first exposure period, hemodynamic parameters were recorded using a high precision and non-invasive hemodynamic monitor (Task Force monitor, CNSystem) synchronized with the frequency modulation radiofrequency generator (see FIGS 14-16). Real time HRV data analysis per period of exposure was performed using an Artificial Intelligence computing process. In addition, single frequency modulation responses per patient were identified and used for patient's treatment identification using Support Vector Machine algorithms. Following the initial exposure protocol, the patient was exposed to a subsequent exposure protocol, wherein the subsequent exposure protocol comprised only the amplitude modulated frequencies from the initial exposure protocol that were determined to not alter the heart rate variability, as illustrated in FIG. 18D. The patient's CT scan revealed a complete clinical response the disappearance of tumor mass.

**EXAMPLE 17**

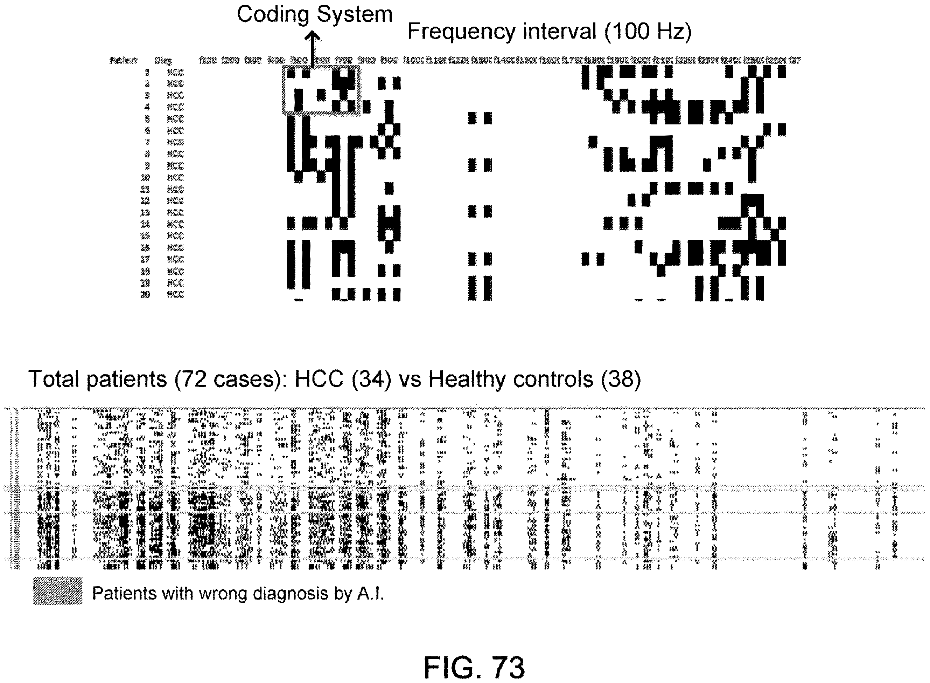

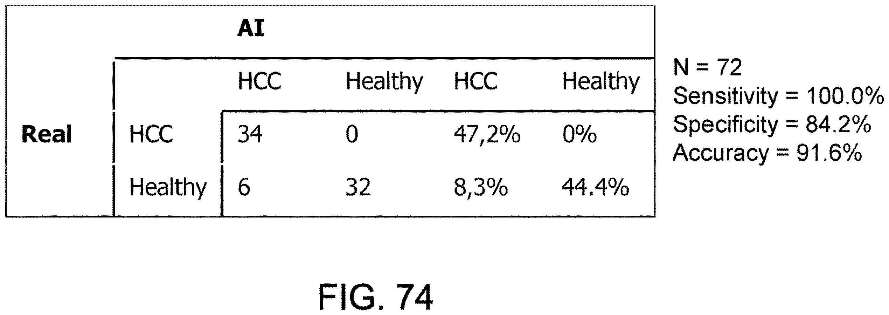

We conducted a study in 72 patients. The patients were exposed to a device emitting a 27.12 MHz RF signal, amplitude-modulated with cancer-specific frequencies ranging from 0.2 to 23,000 Hz with high precision. The device is connected to a spoon-like coupler placed in the patient's mouth during treatment. Patients with advanced hepatocellular carcinoma HCC and limited therapeutic options were offered treatment with a combination of HCC-specific frequencies.
34 patients with advanced HCC and 38 healthy controls received one or two (either concurrently or following a rest period of 10 minutes) exposure protocols of 35 or 10 minutes in accordance with embodiments disclosed in FIGS. 18A, 18C, 18D, and FIGS. 67A-B. Each patient was exposed to 27.12 MHz carrier frequency ± 194 amplitude modulated frequencies or a series of electromagnetic frequencies that occur every 3 Hz or 10 Hz in a range from 0.01 Hz to 20 KHz, in a range from 10 Hz to 1,000 Hz, or in a range from 10 Hz to 2,000 Hz (10 seconds per frequency or 3 seconds per frequency sequential exposure ranging from of 50 Hz to 20 kHz). The system may determine a set of frequencies that altered or did not alter a hemodynamic parameter during the first exposure period and provided a frequency attribute to the set of frequencies that altered or did not alter a hemodynamic parameter. As depicted in FIG. 73, the frequency attribute is a bar coding system as depicted in FIG. 71. Each frequency is given a frequency attribute of either code 0, code -1, code 1, code 2 according to whether a specific frequency did or did not produce a change in a hemodynamic parameter (e.g. heart rate variability). As illustrated in FIG. 74, the system has a sensitivity of 100%, a specificity of 84.2%, and an accuracy of 91.6% in the diagnosis of the 72 patients studied.

**EXAMPLE 18**

As depicted in FIGS. 63A-D, reflected energy data from 58 individuals (27 patients with hepatocellular carcinoma) demonstrated a different pattern between patients with hepatocellular carcinoma and healthy controls (Mann-Whitney 2-sided test, p = 1.9E-101). Reflected energy values were 1051.5 V (95% CI: 1050.1 - 1052.89) and 1008.53 V (95% CI: 1007.44 - 1009.61) respectively in healthy controls and hepatocellular carcinoma. This analysis may support the concept that patients with HCC absorb significant high energy during the exposure to electromagnetic fields modulated at HCC-specific frequencies compared to healthy controls.

**EXAMPLE 19**

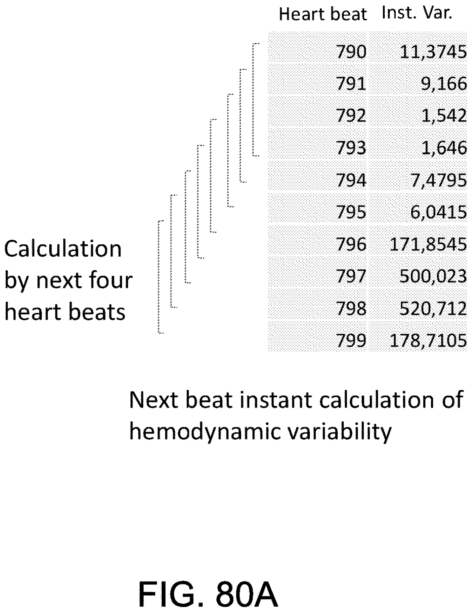

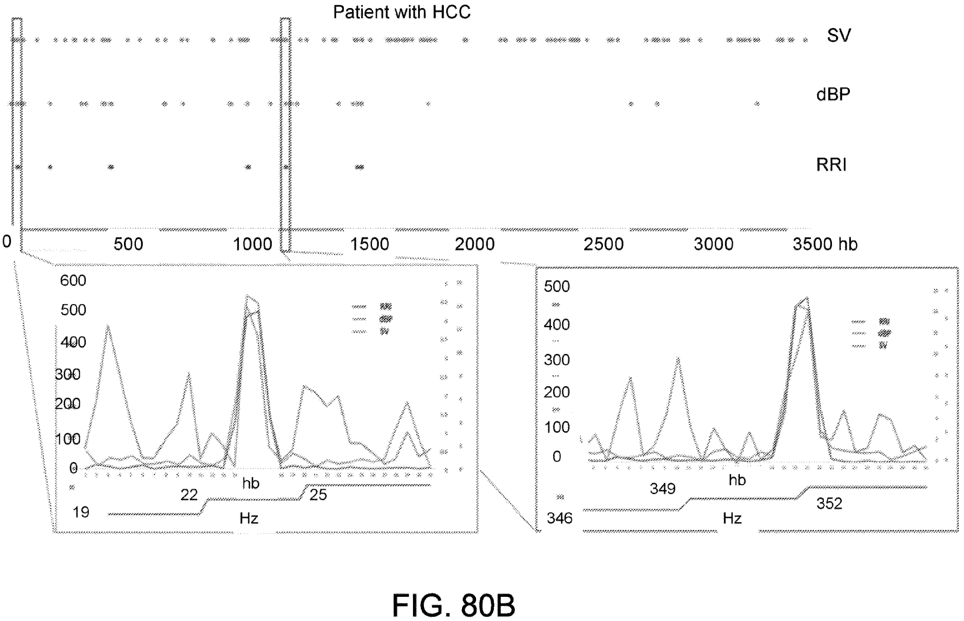

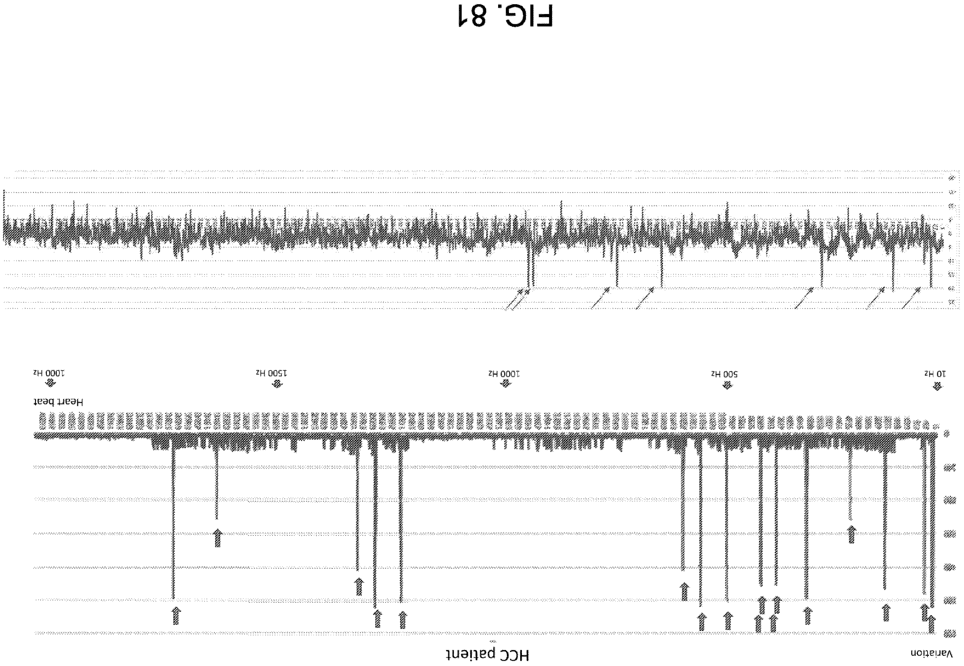

The temporal fluctuations in heart rate exhibit a marked synchrony and may reflect changes in cardiac autonomic regulation. In order to analyze hemodynamic patterns of heart rate variability, a series of algorithms for instant variability analysis based on the next heart rate were created and validated (FIG. 80A-B). The study of interaction of variables between RRI, dBP, and SV determined that RRI had the highest effect in the hemodynamic response induced by the frequency modulation (Partial Eta Squared = 0.7973; MANOVA test p = 2.54E-06) (FIG. 80B).
A cohort of 65 patients, 20 patients with hepatocellular carcinoma, were exposed to a series of electromagnetic frequencies that occurred every 3 Hz in a range from 10 Hz to 1,000 Hz, with each frequency exposure being for 3 seconds. As illustrated in FIG. 81, a number of frequencies elicited a change in heart rate variability, and these frequencies were correlated with tumor burden (FIG. 67B). Partial response / remission was observed in 2 patients (11.7%). Stable disease was observed in 9 patients (52.9%). Discharge was observed in 64.7% at follow up of + 3.5 months (range: 0 - 5.3 months). 41 cancer patient quality of life EORTC-C30 v3.0 questionnaires were analyzed with 64% showing improvement in global health status. No grade ≥ 2 toxicity was reported. The hemodynamic variability data can be processed, and variability results can be categorized as being consistent with a tumor-specific frequency in real time. Tumor-specific frequencies can further be identified for every patient in a dynamic and real time continuous procedure capable of determining instant variability values that may indicate a new tumor-specific frequency within any desired frequency interval. This procedure may identify any number of tumor-specific frequencies from different patients with the same disease in order to create and update tumor-specific frequencies to be used in the treatment of patients diagnosed with any type of cancer. Single patient's tumor-specific frequencies may further be identified in order to support a dynamic treatment of cancer in a single patient.

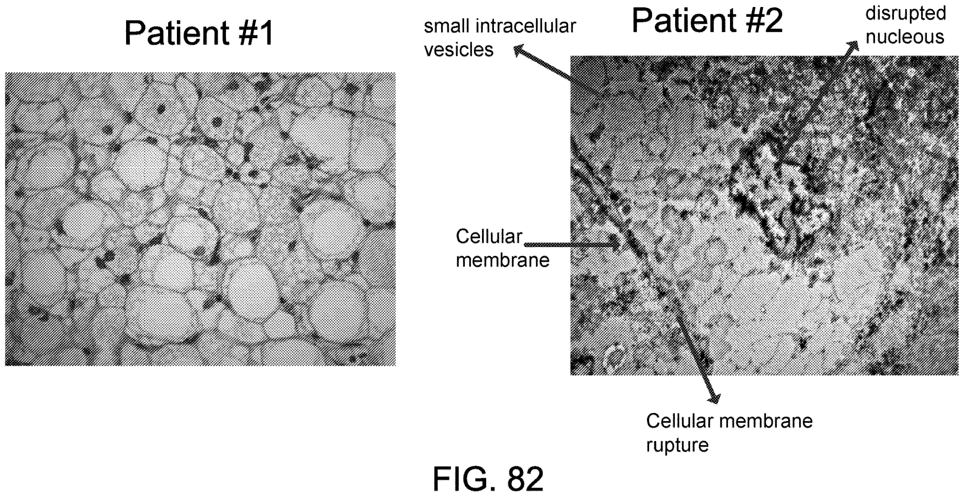

Following exposure of the series of electromagnetic frequencies detailed above, tumor resection occurred in two patients (FIG. 82). Histological examination of the tumors revealed hydropic degeneration identified by cellular swelling, small intracellular vesicles, cellular membrane rupture, and disrupted nucleous.

In the above detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be used, and other changes may be made, without departing from the spirit or scope of the subject matter presented herein. It will be readily understood that various features of the present disclosure, as generally described herein, and illustrated in the Figures, can be arranged, substituted, combined, separated, and designed in a wide variety of different configurations, all of which are explicitly contemplated herein.

The present disclosure is not to be limited in terms of the particular embodiments described in this application, which are intended as illustrations of various features. Many modifications and variations can be made without departing from its spirit and scope, as will be apparent to those skilled in the art. Functionally equivalent methods and apparatuses within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, reagents, compounds, compositions or biological systems, which can, of course, vary. It is also to be understood that the terminology used herein is for the purpose of describing particular embodiments only, and is not intended to be limiting.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (for example, bodies of the appended claims) are generally intended as "open" terms (for example, the term "including" should be interpreted as "including but not limited to," the term "having" should be interpreted as "having at least," the term "includes" should be interpreted as "includes but is not limited to," et cetera). While various compositions, methods, and devices are described in terms of "comprising" various components or steps (interpreted as meaning "including, but not limited to"), the compositions, methods, and devices can also "consist essentially of' or "consist of" the various components and steps, and such terminology should be interpreted as defining essentially closed-member groups. It will be further understood by those within the art that if a specific number of an introduced claim recitation is intended, such an intent will be explicitly recited in the claim, and in the absence of such recitation no such intent is present.

For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (for example, "a" and/or "an" should be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should be interpreted to mean at least the recited number (for example, the bare recitation of "two recitations," without other modifiers, means at least two recitations, or two or more recitations). Furthermore, in those instances where a convention analogous to "at least one of A, B, and C, et cetera" is used, in general such a construction is intended in the sense one having skill in the art would understand the convention (for example, "a system having at least one of A, B, and C" would include but not be limited to systems that have A alone, B alone, C alone, A and B together, A and C together, B and C together, and/or A, B, and C together, et cetera). In those instances where a convention analogous to "at least one of A, B, or C, et cetera" is used, in general such a construction is intended in the sense one having skill in the art would understand the convention (for example, "a system having at least one of A, B, or C" would include but not be limited to systems that have A alone, B alone, C alone, A and B together, A and C together, B and C together, and/or A, B, and C together, et cetera). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, et cetera. As a non-limiting example, each range discussed herein can be readily broken down into a lower third, middle third and upper third, et cetera. As will also be understood by one skilled in the art all language such as "up to," "at least," and the like include the number recited and refer to ranges that can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

The term "about," as used herein, refers to variations in a numerical quantity that can occur, for example, through measuring or handling procedures in the real world; through inadvertent error in these procedures; through differences in the manufacture, source, or purity of compositions or reagents; and the like. Typically, the term "about" as used herein means greater or lesser than the value or range of values stated by 1/10 of the stated values, e.g., $\pm$10%. The term "about" also refers to variations that would be recognized by one skilled in the art as being equivalent so long as such variations do not encompass known values practiced by the prior art. Each value or range of values preceded by the term "about" is also intended to encompass the embodiment of the stated absolute value or range of values. Whether or not modified by the term "about," quantitative values recited in the claims include equivalents to the recited values, e.g., variations in the numerical quantity of such values that can occur, but would be recognized to be equivalents by a person skilled in the art.

Various of the above-disclosed and other features and functions, or alternatives thereof, may be combined into many other different systems or applications. Various presently unforeseen or unanticipated alternatives, modifications, variations or improvements therein may be subsequently made by those skilled in the art, each of which is also intended to be encompassed by the disclosed embodiments.

The invention is further characterized by the following numbered clauses:

1. A system for diagnosing a health condition of a patient, comprising:

an electrocardiogram monitoring system configured to detect, measure and store a plurality of first values for R-R interval values exhibited by the patient during a basal or non-exposure period and a plurality of second values for R-R interval values exhibited by the patient during or after an exposure period in which the patient is exposed to low-energy electromagnetic carrier output signals;
an electrically powered generator adapted to be actuated to generate the low-energy electromagnetic carrier output signals for exposing or applying the low-energy electromagnetic carrier output signals to the patient during the exposure period; and
a processing system configured to synchronize the electrocardiogram monitoring system and the electrically powered generator.

2. The system of clause 1, wherein the processing system is internal to the electrocardiogram monitoring system.
3. The system of clause 1, wherein the processing system is external to the electrocardiogram monitoring system.
4. The system of clause 1, wherein the processing system is configured to sense and identify one or more of a specific electromagnetic field amplitude modulated frequency.
5. The system of clause 1, further comprising an interface controller in operable communication with the electrocardiogram monitoring system and the electrically powered generator.
6. The system of clause 1, wherein the electrocardiogram monitoring system is configured to measure one or more R-R interval values, calculate one or more heart rate variability values, record one or more heart rate variability values, identify one or more specific electromagnetic field amplitude modulated frequencies, or a combination thereof.
7. The system of clause 6, wherein the processing system integrates the one or more heart rate variability values with an intelligent learning library and the electrically powered generator.
8. The system of clause 1, wherein the low-energy electromagnetic carrier output signals comprises an amplitude modulation frequency in a range from about 0.01 Hz to about 150 kHz.
9. An electrocardiogram monitoring system configured to detect, measure and store a plurality of first values for R-R interval values exhibited by one or more surrogate patients during a basal or non-exposure period and a plurality of second values for R-R interval values exhibited by the one or more surrogate patients during or after an exposure period in which the one or more surrogate patients are exposed to low-energy electromagnetic output signals, the system comprising:

an electrically powered generator adapted to be actuated to generate the low-energy electromagnetic carrier output signals for exposing or applying the low-energy electromagnetic carrier output signals to the surrogate patients during said exposure period.

10. The system of clause 9, further comprising a processing system configured to synchronize the electrocardiogram monitoring system and the electrically powered generator.

11. The system of clause 10, wherein the processing system is internal to the electrocardiogram monitoring system.

12. The system of clause 10, wherein the processing system is external to the electrocardiogram monitoring system.

13. The system of clause 10, wherein the processing system is configured to sense and identify one or more of a specific electromagnetic field amplitude modulated frequency.

14. The system of clause 9, further comprising an interface controller in operable communication with the electrocardiogram monitoring system and the electrically powered generator.

15. The system of clause 9, wherein the electrocardiogram monitoring system is configured to measure one or more R-R interval values, calculate one or more heart rate variability values, record one or more heart rate variability values, identify one or more specific electromagnetic field amplitude modulated frequencies, or a combination thereof.

16. The system of clause 15, wherein the processing system integrates the one or more heart rate variability value with an intelligent learning library and the electrically powered generator.

17. The system of clause 9, wherein the low-energy electromagnetic carrier output signals comprises an amplitude modulation frequency in a range from about 0.01 HZ to about 150 kHz.

18. A system for diagnosing a health condition of a patient, comprising:

a hemodynamic parameter monitoring system configured to detect, measure, and record a plurality of first values for each of a plurality of hemodynamic parameters exhibited by a patient during an exposure period, wherein the exposure period comprises a time period in which the patient is exposed to a one or more of electromagnetic frequency signals;

an electrically-powered frequency generator adapted to generate the one or more electromagnetic frequency signals during the exposure period, wherein the one or more electromagnetic frequency signals is configured to influence a cellular function; and

a processing system configured to:

synchronize the hemodynamic parameter monitoring system and the frequency generator,
auto-tune a carrier signal to adjust forward energy delivered to the patient, and instruct the frequency generator to expose the patient to each of the one or more of electromagnetic frequency signals by modulating an amplitude of the carrier signal to produce an amplitude modulated electromagnetic frequency signal.

19. The system of clause 18, wherein the amplitude modulated electromagnetic signal is selected from within a range of 10 Hz to 2,000 Hz.

20. The system of clause 18, wherein the cellular function is microtubule conductivity.

21. The system of clause 18, wherein the plurality of hemodynamic parameters comprise one or more of RR interval, heart rate, systolic blood pressure, diastolic blood pressure, median blood pressure, pulse pressure, stroke volume, cardiac output, and total peripheral resistance.

22. The system of clause 18, wherein the processing system is further configured to actuate the frequency generator to generate one or more highly specific radio frequency carrier signals based on at least the plurality of first values for each of the plurality of hemodynamic parameters.

23. The system of clause 18, wherein the frequency generator comprises a programmable generator.

24. The system of clause 23, wherein the programmable generator comprises one or more controllable generator circuits, wherein each controllable generator circuit is configured to generate one or more highly specific radio frequency carrier signals.

25. The system of clause 24, wherein each controllable generator circuit comprises an amplitude modulation frequency control signal generator configured to control amplitude modulated variations of the one or more highly specific radio frequency carrier signals.

26. The system of clause 18, wherein the carrier signal comprises a 27.12 MHz signal.

27. The system of clause 18, further comprising a computing device operably connected to the processing system and configured to store at least one machine learning algorithm configured to output one or more variables for use in auto-tuning the carrier signal.

28. A system for treating cancer of a patient, comprising:

a hemodynamic parameter monitoring system configured to detect, measure, and record a plurality of first values for each of a plurality of hemodynamic parameters exhibited by a patient during an exposure period, wherein the exposure period comprises a time period in which the patient is exposed to a plurality of electromagnetic frequency signals;

an electrically-powered frequency generator adapted to generate the one or more electromagnetic signals during the exposure period, wherein the one or more electromagnetic signals is configured to influence microtubule conductivity; and

a processing system configured to:

synchronize the hemodynamic parameter monitoring system and the frequency generator, auto-tune a carrier signal to adjust forward energy delivered to the patient, and instruct the frequency generator to expose the patient to each of the plurality of electromagnetic frequency signals by modulating an amplitude of the carrier signal to produce a desired electromagnetic frequency signal, wherein the amplitude modulated electromagnetic signal is selected from within a range of 10 Hz to 2,000 Hz.

29. A method of diagnosing a health condition of a patient, the method comprising:

measuring, by a electrocardiogram monitoring system, a plurality of first values for R-R interval values;

calculating heart rate variability values exhibited by a patient during exposure of the patient to highly specific frequency radio frequency carrier signals;

processing heart rate variability values and exposure of the patient to the highly specific frequency radio frequency carrier signals;

recording representative heart rate variability values exhibited by one or more surrogate patients during exposure of the one or more surrogate patients to the highly specific frequency radio frequency carrier signals;

storing the plurality of first values and a plurality of second values of representative heart rate variability values; and

transferring the representative heart rate variability values from a pre-diagnosis or diagnosed patient to a library for further processing.

30. The method of clause 29, wherein the highly specific frequency radio frequency carrier signals comprise an amplitude modulation frequency in a range from about 0.01 HZ to about 150 kHz.

31. A method of diagnosing a health condition of a patient, the method comprising:

determining, by a processing system, a plurality of electromagnetic frequency signals to apply to the patient; auto-tuning, by the processing system, a carrier wave to balance forward energy delivered to the patient;

exposing, by a frequency generator operably connected to the processing system, the patient to each of the plurality of electromagnetic frequency signals by modulating an amplitude of the carrier signal to produce a desired electromagnetic frequency signal that is configured to influence a cellular function of the patient

measuring, by a hemodynamic parameter monitoring system, a plurality of first values for a plurality of hemodynamic parameters exhibited by a patient during exposure of the patient to the plurality of electromagnetic frequency signals; and

analyzing the plurality of first values to provide for a diagnosis of a health condition of the patient.

32. The method of clause 31, wherein the amplitude modulated signal is selected from within a range of 10 Hz to 2,000 Hz.

33. The method of clause 31, further comprising:

identifying a specific frequency response to a single frequency exposure of an electromagnetic amplitude modulation signal as non-reactive, reactive, or post-reactive.

34. The method of clause 31, wherein the plurality of hemodynamic parameters comprise one or more of RR interval, heart rate, systolic blood pressure, diastolic blood pressure, median blood pressure, pulse pressure, stroke volume, cardiac output, and total peripheral resistance.

35. The method of clause 31, further comprising:

determining highly specific radio frequency carrier signals that cause significant hemodynamic parameter value changes in the patient.

36. The method of clause 31, wherein the carrier signal comprises a 27.12 MHz signal.

37. The method of clause 31, wherein auto-tuning comprises adjusting the forward energy of the carrier signal based upon an output of a machine learning algorithm.

38. The method of clause 31, wherein the cellular function is microtubule conductivity.

39. A method of treating cancer, the method comprising:
administering one or more high frequency carrier signals and at least one amplitude modulation control signal for controlling amplitude modulated variations of the one or more high frequency carrier signals, wherein the amplitude modulation frequency is selected from within a range of 0.01 Hz to 150 kHz.

40. The method of clause 39, wherein the amplitude modulation frequency is selected from within a range of 10 Hz to 2,000 Hz.

41. The method of clause 39, wherein the at least one amplitude modulation control signal modulates microtubule ion conductivity.

42. A programmable generator activatable by electrical power and structured to influence cellular functions or malfunctions in a warm-blooded mammalian subject, the programmable generator comprising:
at least one controllable low-energy electromagnetic energy generator circuit for generating one or more high frequency carrier signals, wherein the at least one generator circuit includes:
at least one amplitude modulation control signal generator for controlling amplitude modulated variations of the one or more high frequency carrier signals, and

at least one programmable amplitude modulation frequency control signal generator for controlling frequencies at which amplitude modulations are generated, wherein each programmable amplitude modulation frequency control signal generator is adapted to accurately control the frequencies at which the amplitude modulations are generated to within an accuracy of at least 1000 parts per million relative to a reference amplitude modulation frequency selected from within a range of 0.01 Hz to 150 kHz;
at least one data processor constructed and arranged for communication with the at least one generator circuit and for receiving control information from a source of control information; and
a connection position configured to connect to an electrically conductive applicator configured to apply one or more amplitude-modulated low energy emissions at a program-controlled frequency to the warm-blooded mammalian subject, wherein the reference amplitude modulation frequencies are selected dependent on a health condition of the warm-blooded mammalian subject.

## Claims

1. A programmable generator activatable by electrical power and structured to influence cellular functions or malfunctions in a warm-blooded mammalian subject, the programmable generator comprising:

   a controllable low-energy electromagnetic energy generator circuit for generating high frequency carrier signals, wherein the controllable low-energy electromagnetic energy generator circuit comprises:

   an amplitude modulation control signal generator for controlling amplitude modulated variations of the high frequency carrier signals, and
   a programmable amplitude modulation frequency control signal generator for controlling frequencies at which amplitude modulations are generated, wherein the programmable amplitude modulation frequency control signal generator is configured to accurately control the frequencies at which the amplitude modulations are generated to within an accuracy of at least 1000 parts per million relative to a reference amplitude modulation frequency selected from within a range of 10 Hz to 2,000 Hz;

   a data processor constructed and arranged for communication with the controllable low-energy electromagnetic energy generator circuit and for receiving control information from a source of control information; and
   a connection position configured to connect to an electrically conductive applicator configured to apply amplitude-modulated low energy emissions at a program-controlled frequency to the warm-blooded mammalian subject, wherein the reference amplitude modulation frequency is selected dependent on a health condition of the warm-blooded mammalian subject, wherein the amplitude-modulated low energy emissions are configured to influence the cellular functions or malfunctions in the warm-blooded mammalian subject.

2. The programmable generator of claim 1, wherein the programmable generator is configured to be synchronized with a hemodynamic parameter monitoring system configured to detect a hemodynamic parameter.

3. The programmable generator of claim 2, wherein the hemodynamic parameter comprises one or more of RR interval, heart rate, systolic blood pressure, diastolic blood pressure, median blood pressure, pulse pressure, stroke volume, cardiac output, and total peripheral resistance.

**4.** The programmable generator of any one of claims 1-3, wherein the programmable generator adjusts the high frequency carrier signals based upon an output of a machine learning algorithm.

**5.** The programmable generator of any one of claims 1-4, wherein the cellular functions comprise microtubule conductivity.

100

110

106

102

104

108

FIG. 1

FIG. 2

**FIG. 3**

FIG. 4

Balanced class AB amplifier

500

502

Output from
the modulator

PIN Diode
Attenuator

Level control

$V_{Bias}$

$V_{Batt}$

504

Low Pass Filter

RF Output

# FIG. 5

RF forward and reflected power monitor

FIG. 6

EP 4 438 107 A2

FIG. 7

FIG. 8

## FIG. 9A

Patients

Database

Device

Processing

RF

## 900

908 — Database

910 — AI

906 — Orchestrator

902 — HD monitor

904 — RF generator

## FIG. 9B

EP 4 438 107 A2

FIG. 10

FIG. 11

FIG. 12

FIG. 13A

FIG. 13B

Sinusoid wave signal

FIG. 13C

A↔ C vectorial signal

FIG. 13D

| | | | |
|---|---|---|---|
| 410,231 | 3161,331 | 6361,321 | 9381,221 |
| 423,321 | 3206,315 | 6364,928 | 9719,314 |
| 427,062 | 3255,219 | 6383,321 | 9740,219 |
| 470,181 | 3267,433 | 6461,175 | 9768,331 |
| 560,32 | 3269,321 | 6733,331 | 9797,294 |
| 655,435 | 3457,291 | 6758,232 | 9819,511 |
| 657,394 | 3516,296 | 6779,482 | 10317,499 |
| 668,209 | 3530,188 | 6856,222 | 10438,495 |
| 677,972 | 3531,296 | 6877,183 | 10443,311 |
| 728,232 | 3546,323 | 6915,886 | 10456,383 |
| 806,021 | 3572,106 | 6980,525 | 10579,425 |
| 811,924 | 3576,189 | 7019,235 | 10863,209 |
| 842,311 | 3923,221 | 7043,209 | 10866,382 |
| 843,22 | 3927,331 | 7130,323 | 11067,418 |
| 891,9 | 4071,121 | 7144,142 | 11149,935 |
| 1250,504 | 4079,951 | 7210,223 | 11163,895 |
| 1755,402 | 4123,953 | 7291,21 | 11802,821 |
| 1873,477 | 4161,889 | 7482,245 | 11953,424 |
| 1924,702 | 4222,821 | 7510,92 | 12223,329 |
| 1975,196 | 4238,402 | 7529,233 | 12260,933 |
| 2017,962 | 4289,296 | 7549,212 | 12265,295 |
| 2053,396 | 4312,947 | 7650,028 | 12267,233 |
| 2083,419 | 4375,962 | 7680,518 | 12623,191 |
| 2190,731 | 4426,387 | 7692,522 | 12633,372 |
| 2324,393 | 4435,219 | 7829,231 | 12685,231 |
| 2353,478 | 4471,188 | 7862,209 | 12721,423 |
| 2362,309 | 4483,889 | 7932,482 | 12785,342 |
| 2419,309 | 4486,384 | 7935,423 | 13433,323 |
| 2425,222 | 4556,322 | 7947,392 | 14085,222 |
| 2430,219 | 4629,941 | 7979,308 | 14333,209 |
| 2431,094 | 4732,211 | 8028,339 | 14537,331 |
| 2471,328 | 4876,218 | 8055,942 | 14542,432 |
| 2478,331 | 5086,281 | 8072,134 | 14655,03 |
| 2480,191 | 5124,084 | 8141,174 | 14828,234 |
| 2522,328 | 5133,121 | 8336,383 | 15237,489 |
| 2743,995 | 5247,142 | 8394,793 | 15717,221 |
| 2744,211 | 5270,834 | 8432,181 | 16110,932 |
| 2831,951 | 5340,497 | 8452,119 | 16144,343 |
| 2843,283 | 5520,218 | 8475,221 | 18265,238 |
| 2859,891 | 5570,234 | 8492,193 | 18863,292 |
| 2873,542 | 5882,292 | 8818,104 | 18930,995 |
| 2886,232 | 6180,334 | 8852,329 | 19970,311 |
| 3086,443 | 6329,195 | 8853,444 | 20330,294 |
| 3127,232 | 6350,333 | 8858,179 | 20365,284 |
| 3160,942 | | 8939,212 | |
| | | | |

FIG. 13E

AC Input Waveform

Resultant Output Waveform

FIG. 14

EP 4 438 107 A2

High frequency carrier frequency with
low frequency amplitude modulation

Signal repetition over time

Electrical discharge

Phase-locking synchronization

FIG. 15

EP 4 438 107 A2

410.2 Hz  3 s of exposure per
frequency

423.3 Hz

427.1 Hz

470.2 Hz

560.3 Hz

642.9 Hz

194 total HCC-
specific frequencies

20 365.3 Hz

(Toal duration: about 2
min to about 3 min.)

Programme
continues to repeat
from beginning after
one complete cycle
(total duration: 1 h)

Sequential exposure
from the lowest
frequency to the
highest

FIG. 16A

410.2 Hz  10s of exposure per
frequency

423.3 Hz

427.1 Hz

470.2 Hz

560.3 Hz

642.9 Hz

194 total HCC-
specific frrequencies

20 365.3 Hz

(Toal duration: about 30
min to about 35 min.)

Sequential exposure
from the lowest
frequency to the
highest

FIG. 16B

FIG. 17

EP 4 438 107 A2

194 frequencies (HCC-specific)

| | normal |
| | HCC |

Supine position

CF     Carrier frequency

CF+ MF     Carrier frequency +
Modulation frequencies

Day 1

| 10 min Non-exposure | 10 min CF | 35 min CF+MF | 10 min CF | 35 min CF+MF | 10 min CF |

Day 2

| 10 min Non-exposure | 10 min CF | 35 min CF+MF | 10 min CF | 35 min CF+MF | 10 min CF |

FIG. 18A

EP 4 438 107 A2

FIG. 18B

FIG. 18C

FIG. 18D

Modulation frequencies every 3 Hz step from:
about 0.01 Hz to about 20 Khz,
or
about 10 Hz to about 1,000 Hz

Supine position

CF — Carrier frequency

CF+ MF — Carrier frequency + Modulation frequencies

normal

HCC

3s exposure / frequency

Day 1

10 min
Non-exposure

10 min
CF

120 min
CF + MF

FIG. 18E

| Time [s] | Beat [J] | RRI [s] | HR [bpm] | sBP [mmHg] | dBP [mmHg] | mBP [mmHg] | SV [ml] | SI [ml/m2] | CO [l/min] | CI [l/(min*m2)] | TPR [dyn*s/cm^5] | TPRI [dyn*s*m2/cm^5] |
|---|---|---|---|---|---|---|---|---|---|---|---|---|
| 103.6 reposo | | | | | | | | | | | | |
| 103.97 | 126 | 737.375 | 81.37 | 115.778 | 78.356 | 88.34 | 74.99 | 51.16 | 6.102 | 4.163 | 1118.865 | 1640.027 |
| 104.71 | 127 | 732.667 | 81.893 | 116.024 | 79.121 | 88.716 | 72.768 | 49.644 | 5.959 | 4.065 | 1150.716 | 1686.715 |
| 105.44 | 128 | 699.708 | 85.75 | 117.968 | 79.841 | 90.539 | 75.722 | 51.659 | 6.493 | 4.43 | 1078.55 | 1580.934 |
| 106.14 | 129 | 714.417 | 83.986 | 117.287 | 79.257 | 90.188 | 76.49 | 52.183 | 6.424 | 4.383 | 1086.773 | 1591.521 |
| 106.85 | 130 | 720.708 | 83.251 | 117.974 | 79.554 | 90.25 | 78.122 | 53.297 | 6.504 | 4.437 | 1073.224 | 1573.127 |
| 107.57 | 131 | 723.083 | 82.978 | 118.362 | 79.537 | 90.408 | 75.45 | 51.474 | 6.251 | 4.271 | 1116.913 | 1637.166 |
| 108.3 | 132 | 748.083 | 80.205 | 118.081 | 79.883 | 89.964 | 74.632 | 50.916 | 5.986 | 4.084 | 1152.234 | 1703.627 |
| 109.05 | 133 | 742.417 | 80.817 | 119.523 | 80.184 | 90.8 | 73.804 | 50.351 | 5.965 | 4.069 | 1177.631 | 1726.138 |
| 109.79 | 134 | 708.375 | 84.701 | 120.338 | 81.216 | 92.126 | 76.541 | 52.218 | 6.483 | 4.423 | 1099.795 | 1612.075 |
| 110.5 | 135 | 719 | 83.449 | 121.008 | 81.189 | 92.583 | 74.16 | 50.593 | 6.189 | 4.222 | 1158.048 | 1697.462 |
| 111.22 | 136 | 724 | 82.873 | 121.297 | 81.053 | 92.626 | 73.693 | 50.275 | 6.107 | 4.166 | 1174.065 | 1720.939 |
| 111.94 | 137 | 747.233 | 80.285 | 121.092 | 80.937 | 91.868 | 71.273 | 48.624 | 5.722 | 3.904 | 1242.439 | 1821.162 |
| 112.69 | 138 | 743.083 | 80.745 | 121.495 | 81.403 | 92.152 | 69.968 | 47.733 | 5.65 | 3.854 | 1262.439 | 1850.478 |
| 113.43 | 139 | 727.417 | 82.484 | 122.391 | 81.995 | 92.983 | 70.292 | 47.955 | 5.798 | 3.956 | 1241.578 | 1819.899 |
| 114.16 | 140 | 702.042 | 85.465 | 123.173 | 82.116 | 94.327 | 72.778 | 49.651 | 6.22 | 4.243 | 1174.627 | 1721.763 |
| 114.86 | 141 | 721.042 | 83.213 | 123.458 | 81.915 | 93.877 | 71.688 | 48.907 | 5.965 | 4.07 | 1218.725 | 1786.403 |
| 115.58 | 142 | 724.375 | 82.83 | 123.854 | 81.843 | 94.14 | 71.02 | 48.452 | 5.883 | 4.013 | 1239.451 | 1816.782 |
| 116.3 | 143 | 749.667 | 80.036 | 123.26 | 82.448 | 93.395 | 70.051 | 47.791 | 5.607 | 3.825 | 1289.837 | 1890.637 |
| 117.05 | 144 | 741.042 | 80.967 | 124.372 | 82.542 | 94.219 | 68.801 | 46.938 | 5.571 | 3.8 | 1309.997 | 1920.188 |
| 117.8 | 145 | 719.042 | 83.444 | 124.628 | 83.005 | 94.863 | 69.902 | 47.689 | 5.833 | 3.979 | 1259.915 | 1846.778 |
| 118.51 | 146 | 705.375 | 85.061 | 124.838 | 82.823 | 95.398 | 73.072 | 49.851 | 6.216 | 4.24 | 1189.248 | 1743.194 |
| 119.22 | 147 | 723.333 | 82.949 | 124.405 | 82.049 | 94.229 | 70.442 | 48.057 | 5.843 | 3.986 | 1249.041 | 1830.839 |
| 119.94 | 148 | 734.75 | 81.66 | 123.712 | 81.95 | 93.644 | 68.693 | 46.864 | 5.609 | 3.827 | 1292.73 | 1894.878 |
| 120.68 | 149 | 740 | 81.081 | 122.918 | 81.689 | 93.035 | 69.571 | 47.463 | 5.641 | 3.848 | 1276.903 | 1871.679 |
| 121.42 | 150 | 739.333 | 81.154 | 122.52 | 81.262 | 92.81 | 69.626 | 47.501 | 5.65 | 3.855 | 1271.551 | 1863.833 |
| 122.16 | 151 | 713.083 | 84.142 | 122.704 | 81.576 | 93.36 | 72.631 | 49.55 | 6.111 | 4.169 | 1182.868 | 1733.842 |
| 122.87 | 152 | 706.75 | 84.896 | 122.272 | 80.537 | 93.145 | 71.491 | 48.773 | 6.069 | 4.141 | 1188.216 | 1741.681 |
| 123.58 | 153 | 721 | 83.218 | 121.519 | 79.867 | 92.129 | 70.458 | 48.068 | 5.863 | 4 | 1216.084 | 1782.53 |
| 124.3 | 154 | 743 | 80.754 | 120.628 | 79.464 | 90.943 | 73.833 | 50.37 | 5.962 | 4.068 | 1179.993 | 1729.628 |

FIG. 19

EP 4 438 107 A2

| FreqHz | Time | Beat | RRI | HR | sBP | dBP | mBP | SV | SI | CO | CI | TPR | TPRI |
|---|---|---|---|---|---|---|---|---|---|---|---|---|---|
| 2.843.283 | 1112.65 | 1226 | 882.667 | 679.758 | 113.611 | 671.058 | 867.941 | 116.438 | 583.234 | 791.497 | 396.458 | 846.943 | 1690.85 |
| 2.843.283 | 1113.54 | 1227 | 897.042 | 668.865 | 113.683 | 669.599 | 869.481 | 118.648 | 594.304 | 793.596 | 397.509 | 846.256 | 1689.48 |
| 2.843.283 | 1114.43 | 1228 | 895.042 | 67.036 | 114.094 | 67.068 | 873.212 | 115.966 | 580.868 | 777.387 | 389.391 | 867.739 | 1732.37 |
| 2.843.283 | 1115.33 | 1229 | 889.708 | 674.378 | 113.176 | 659.565 | 864.821 | 114.866 | 575.359 | 774.631 | 38.801 | 862.161 | 1721.24 |
| 2.843.283 | 1116.22 | 1230 | 892.083 | 672.583 | 112.424 | 66.606 | 856.514 | 115.241 | 577.238 | 775.091 | 38.824 | 853.076 | 1703.1 |
| 2.843.283 | 1117.11 | 1231 | 898.75 | 667.594 | 113.543 | 669.156 | 867.367 | 114.616 | 574.105 | 765.166 | 383.269 | 875.487 | 1747.84 |
| 2.843.283 | 1118.01 | 1232 | 893.333 | 671.642 | 113.468 | 660.256 | 864.004 | 114.744 | 574.748 | 770.668 | 386.025 | 865.746 | 1728.39 |
| 2.843.283 | 1118.9 | 1233 | 888.083 | 675.612 | 112.352 | 661.424 | 856.602 | 116.004 | 581.059 | 783.736 | 392.571 | 843.755 | 1684.49 |
| 2.843.283 | 1119.79 | 1234 | 902.75 | 664.636 | 113.154 | 662.296 | 859.889 | 117.593 | 589.019 | 781.566 | 391.483 | 849.463 | 1695.89 |
| 2.843.283 | 1120.69 | 1235 | 892.667 | 672.143 | 112.822 | 660.521 | 863.056 | 116.598 | 584.036 | 783.706 | 392.556 | 850.376 | 1697.71 |
| 2.843.283 | 1121.59 | 1236 | 886.375 | 676.914 | 112.568 | 656.703 | 854.926 | 116.128 | 581.683 | 78.609 | 393.749 | 839.523 | 1676.04 |
| | | | | | | | | | | | | | |
| 2.859.891 | 1122.47 | 1237 | 894.333 | 670.891 | 112.517 | 67.025 | 85.682 | 117.453 | 588.318 | 787.981 | 394.697 | 839.432 | 1675.86 |
| 2.859.891 | 1123.37 | 1238 | 894.75 | 670.578 | 113.49 | 672.816 | 866.967 | 115.711 | 579.594 | 775.935 | 388.663 | 862.924 | 1722.76 |
| 2.859.891 | 1124.26 | 1239 | 891.333 | 673.149 | 113.293 | 662.624 | 866.684 | 114.835 | 575.203 | 773.009 | 387.197 | 865.899 | 1728.7 |
| 2.859.891 | 1125.15 | 1240 | 885.083 | 677.902 | 112.238 | 665.228 | 859.018 | 114.176 | 571.904 | 774.002 | 387.695 | 856.863 | 1710.66 |
| 2.859.891 | 1126.04 | 1241 | 902.75 | 664.636 | 113.409 | 670.297 | 865.161 | 114.44 | 573.225 | 760.608 | 380.986 | 878.413 | 1753.68 |
| 2.859.891 | 1126.94 | 1242 | 905.333 | 662.739 | 113.759 | 670.275 | 869.925 | 113.561 | 568.821 | 752.611 | 37.698 | 892.812 | 1782.43 |
| 2.859.891 | 1127.84 | 1243 | 901.417 | 665.619 | 113.563 | 667.825 | 867.386 | 112.699 | 564.504 | 750.144 | 375.744 | 893.041 | 1782.89 |
| 2.859.891 | 1128.75 | 1244 | 897.708 | 668.369 | 113.057 | 66.133 | 865.421 | 112.832 | 565.172 | 754.135 | 377.743 | 886.23 | 1769.29 |
| 2.859.891 | 1129.64 | 1245 | 891.417 | 673.086 | 112.72 | 660.446 | 857.256 | 110.165 | 551.811 | 741.503 | 371.416 | 892.519 | 1781.84 |
| 2.859.891 | 1130.54 | 1246 | 875.708 | 68.516 | 112.326 | 66.366 | 854.316 | 109.726 | 549.613 | 751.798 | 376.573 | 877.167 | 1751.19 |
| 2.859.891 | 1131.41 | 1247 | 932.083 | 643.719 | 112.855 | 656.999 | 854.664 | 110.373 | 552.854 | 710.492 | 355.883 | 928.555 | 1853.79 |

# FIG. 20

FIG. 21

Time domain analysis

FIG. 22B

FIG. 22A

Poincare plot from 10 min ECG

FIG. 23A

Correlation with fourier transformation

FIG. 23B

EP 4 438 107 A2

FIG. 24A

FIG. 24B

FIG. 24C

HCC

Healthy control

FIG. 25A

FIG. 25B

EP 4 438 107 A2

HCC 1

FIG. 25C

HCC 2

FIG. 25D

HCC 3

FIG. 25E

FIG. 25F

FIG. 26

EP 4 438 107 A2

| Factors | Day 1 | | | | Day 2 | | | |
|---|---|---|---|---|---|---|---|---|
| | Mean | Std. Dev. | Lower 95% | Upper 95% | Mean | Std. Dev. | Lower 95% | Upper 95% |
| whole sample | 25.41 | 21.54 | 20.07 | 30.74 | 30.67 | 25.47 | 23.18 | 38.15 |
| Patient | | | | | | | | |
| Ademir | 23.12 | 18.92 | 0.00 | 48.24 | 38.64 | 21.47 | 11.98 | 65.29 |
| Cláudio | 29.55 | 19.88 | 5.11 | 54.00 | 15.02 | 9.81 | 2.85 | 27.20 |
| Flávia | 20.92 | 2.72 | 17.54 | 24.30 | 33.09 | 4.32 | 27.73 | 38.45 |
| Frederico | 17.50 | 6.68 | 9.23 | 25.77 | | | | |
| Genário | 71.45 | 28.82 | 35.66 | 107.24 | 93.21 | 52.03 | 10.41 | 176.00 |
| Gustavo | 20.25 | 8.11 | 10.17 | 30.32 | 24.58 | 5.03 | 18.34 | 30.83 |
| Hélton | 12.72 | 4.76 | 6.80 | 18.63 | 42.06 | 29.87 | -5.47 | 89.60 |
| João | 14.94 | 6.14 | 7.32 | 22.56 | 8.47 | 2.80 | 5.00 | 11.95 |
| Marcela | 8.83 | 7.46 | -0.43 | 18.08 | 3.34 | 1.36 | 1.65 | 5.03 |
| Marcio | 23.76 | 13.82 | 6.60 | 40.92 | 42.23 | 31.28 | 3.41 | 81.04 |
| Mauro | 19.56 | 7.46 | 10.28 | 28.82 | 26.52 | 8.62 | 15.82 | 37.22 |
| Orlando | 59.19 | 7.05 | 50.43 | 67.94 | 42.02 | 16.57 | 21.44 | 62.60 |
| Ricardo | 8.51 | 1.43 | 6.74 | 10.28 | 13.62 | 12.98 | -2.50 | 29.73 |
| Diagnosis | | | | | | | | |
| HCC | 31.40 | 27.01 | 22.12 | 40.68 | 32.68 | 34.72 | 20.37 | 44.99 |
| Healthy | 18.41 | 8.63 | 15.19 | 21.63 | 28.01 | 17.44 | 20.81 | 35.21 |

# FIG. 27

## HCC

EP 4 438 107 A2

| Parameters | Day 1 | | | | Day 2 | | | |
|---|---|---|---|---|---|---|---|---|
| | Sd1 | | Sd2 | | Sd1 | | Sd2 | |
| | Baseline | Exposure | Baseline | Exposure | Baseline | Exposure | Baseline | Exposure |
| Mean | 9.77 | 43.14 | 19.45 | 38.82 | 39.12 | 37.91 | 39.15 | 30.92 |
| Std. Dev. | 2.90 | 5.81 | 6.74 | 6.55 | 30.34 | 0.57 | 10.65 | 4.84 |
| P – Value[*] | 0.8319 | | 0.8349 | | 0.4924 | | 0.2547 | |

(*) Teste de Wilcoxon – Amostras pareadas.

## FIG. 28A

## Healthy control

| Parameters | Day 1 | | | | Day 2 | | | |
|---|---|---|---|---|---|---|---|---|
| | Sd1 | | Sd2 | | Sd1 | | Sd2 | |
| | Baseline | Exposure | Baseline | Exposure | Baseline | Exposure | Baseline | Exposure |
| Mean | 20.40 | 21.71 | 57.71 | 57.91 | 31.48 | 35.51 | 72.21 | 69.52 |
| Std. Dev. | 3.17 | 2.73 | 5.48 | 1.21 | 4.75 | 3.18 | 8.87 | 2.28 |
| P – Value | 0.0732 | | 0.2500 | | 0.1357 | | 0.2949 | |

## FIG. 28B

FIG. 29A

FIG. 29B

FIG. 29C

FIG. 29E

FIG. 29G

FIG. 29D

FIG. 29F

SD1

$$\begin{cases} \Omega : 0.49 \diamond 0.88 \\ w_1 = 3.65 \\ w_2 = 4{,}27 \end{cases}$$

FIG. 30A

SD1

$$\begin{cases} \Omega : -0.02 \diamond 1.05 \\ w_1 = 1.25 \\ w_2 = 0.86 \end{cases}$$

FIG. 30B

FIG. 31A

FIG. 31B

FIG. 31C

**Both Diagnosis**

FIG. 32

Diagnosis by B1E1

FIG. 33A

Diagnosis by B2E1

FIG. 33B

## Platform for transmission of data signals to specific cell types

FIG. 34

FIG. 35A

FIG. 35B

FIG. 35C

FIG. 35D

FIG. 35E

FIG. 35F

FIG. 36A

FIG. 36B

FIG. 36C

FIG. 36E

FIG. 36D

4 mM ionic strength Buffer with Taxol
x1 MT in 4 mM ionic strength Buffer
x2 MT in 4 mM ionic strength Buffer
x5 MT in 4 mM ionic strength Buffer
4 mM ionic strenth Buffer with Colchicine
x1 Tubulin in 4 mM ionic strength Buffer
x1 Tubulin in 4 mM ionic strength Buffer

FIG. 37A

FIG. 37B

| Relative MT Concentration | Amplitude A (%) | Width (FWHM) B (kHz) | Central Frequency C (kHz) | Conductance Offset (%) | $R^2$ |
|---|---|---|---|---|---|
| 1x | 3±7 | 809±1448 | 116±98 | 2±2 | 0.9794 |
| 2x | 4±2 | 586±288 | 100±17 | 8±2 | 0.9928 |
| 5x | 11±2 | 503±118 | 111±11 | 12±2 | 0.9991 |

FIG. 37C

EP 4 438 107 A2

Red – negative charges
Blue – positive charges

FIG. 38B

Microtube polymer structure

FIG. 38A

FIG. 39

FIG. 40A

FIG. 40B

EP 4 438 107 A2

FIG. 41D

FIG. 41E

FIG. 41A

FIG. 41B

FIG. 41C

FIG. 42A

Low pH (acidic)

FIG. 42B

Neutral pH

FIG. 42C

EP 4 438 107 A2

FIG. 43

FIG. 44

FIG. 45

FIG. 46

FIG. 47B

FIG. 47A

FIG. 48

FIG. 49A

EP 4 438 107 A2

FIG. 49B

FIG. 50

FIG. 51

FIG. 52

FIG. 53

FIG. 54B

FIG. 54A

FIG. 55B

FIG. 55D

FIG. 55A

FIG. 55C

FIG. 55E

FIG. 56

FIG. 57A

FIG. 57B

FIG. 58

FIG. 59

FIG. 60B

FIG. 60A

FIG. 61

Reflection Coefficients

FIG. 62B

reflection

absorption

transmission

incoming radiation

FIG. 62A

EP 4 438 107 A2

| Asymptotic Estimates | | | | |
|---|---|---|---|---|
| Dependent Variable: Reflected | | | | |
| Diagnosis | Mean | Std. Error | 95% Confidence Interval | |
| | | | LI | LS |
| Control | 1051.50 | 0.71 | 1050.10 | 1052.89 |
| HCC | 1008.53 | 0.55 | 1007.44 | 1009.61 |

Test Statistic: $H_0 : \mu_{control} = \mu_{HCC}$

| Mann-Whitney U | 691204.000 |
|---|---|
| Z | -21.383 |
| Asymp. Sig. (2-tailed) | 1.00E-101 |

## FIG. 63A

Reflected energy – individual data

Forward energy

Reflection energy

Forward energy

Reflection energy

## FIG. 63B

## Mean values for entire cohort

FIG. 63C

$Y = 2.2894\,x^3 + 0.0066\,x^2 + 1032.1731$
$R^2 = 64.37\%$

FIG. 63D

EP 4 438 107 A2

```
┌─────────────────┐
│   Auto-Tuning   │
│ Forward Energy  │
│      6405       │
└─────────────────┘
         │
         ▼
┌─────────────────┐
│ 10 Minute Carrier│
│  Wave Exposure  │
│      6410       │
└─────────────────┘
         │
         ▼
┌─────────────────┐
│  35 or 10 Minute │
│    Modulated    │
│ Frequency Exposure│
│      6415       │
└─────────────────┘
         │
         ▼
┌─────────────────┐
│ 10 Minute Carrier│
│  Wave Exposure  │
│      6420       │
└─────────────────┘
         │
         ▼
      ◇ Additional ◇
  YES  Exposure?   NO   ( END )
        6425
```

FIG. 64

FIG. 65

FIG. 66

Rest       CF       CF + 194 MF
10 min    10 min    35 min

Rest       CF       CF + sel MF
10 min    10 min    x min

New frequencies          New frequencies

- 7 Hz  x 10          + 7 Hz  x 10

Filtering program          Updated program          194 MF + new frequencies
(removing uncommon
frequencies)

FIG. 67A

EP 4 438 107 A2

High tumor load

Very low tumor load

FIG. 67B

EP 4 438 107 A2

| | Delineadas | | | Padronizadas | |
|---|---|---|---|---|---|
| | Freq_Hz | DISD1 | | Z (Freq) | Z (DISD1) |
| | 572.93 | -3.16 | | -1.61 | -0.54 |
| | 579.93 | -3.84 | | -1.45 | -0.85 |
| | 586.93 | -3.23 | | -1.29 | -0.57 |
| | 593.93 | -2.88 | | -1.13 | -0.41 |
| | 600.93 | -3.01 | | -0.97 | -0.47 |
| | 607.93 | 1.21 | | -0.81 | 1.44 |
| | 614.93 | -1.00 | | -0.64 | 0.44 |
| | 621.93 | 2.66 | | -0.48 | 2.09 |
| | 628.93 | -2.08 | | -0.32 | -0.05 |
| | 635.93 | -4.84 | | -0.16 | -1.30 |
| | 642.93 | -3.75 | | 0.00 | -0.80 |
| | 649.93 | -2.11 | | 0.16 | -0.06 |
| | 656.93 | 2.01 | | 0.32 | 1.80 |
| | 663.93 | -3.83 | | 0.48 | -0.84 |
| | 670.93 | 0.73 | | 0.64 | 1.22 |
| | 677.93 | -3.67 | | 0.81 | -0.77 |
| | 684.93 | 0.02 | | 0.97 | 0.90 |
| | 691.93 | -1.06 | | 1.13 | 0.41 |
| | 698.93 | -1.91 | | 1.29 | 0.03 |
| | 705.93 | -5.30 | | 1.45 | -1.51 |
| | 712.93 | -2.33 | | 1.61 | -0.16 |
| Mean | 642.93 | -1.97 | | 0.00 | 0.00 |
| Std. Dev. | 43.43 | 2.21 | | 1.00 | 1.00 |

FIG. 68

EP 4 438 107 A2

| Delineadas | | Z (freq) |
|---|---|---|
| 642.93 | 2743.99 | |
| 572.93 | 2673.99 | -1.61 |
| 579.93 | 2680.99 | -1.45 |
| 586.93 | 2687.99 | -1.29 |
| 593.93 | 2694.99 | -1.13 |
| 600.93 | 2701.99 | -0.97 |
| 607.93 | 2708.99 | -0.81 |
| 614.93 | 2715.99 | -0.64 |
| 621.93 | 2722.99 | -0.48 |
| 628.93 | 2729.99 | -0.32 |
| 635.93 | 2736.99 | -0.16 |
| 642.93 | 2743.99 | 0.00 |
| 649.93 | 2750.99 | 0.16 |
| 656.93 | 2757.99 | 0.32 |
| 663.93 | 2764.99 | 0.48 |
| 670.93 | 2771.99 | 0.64 |
| 677.93 | 2778.99 | 0.81 |
| 684.93 | 2785.99 | 0.97 |
| 691.93 | 2792.99 | 1.13 |
| 698.93 | 2799.99 | 1.29 |
| 705.93 | 2806.99 | 1.45 |
| 712.93 | 2813.99 | 1.61 |
| Mean | 642.93 | 2743.99 | 0.00 |
| Std.dev | 43.43 | 43.43 | 1.00 |
| Var. | 1886.50 | 1886.50 | 1.00 |

Normal Probability Plot

% Probability

Variables

642.93, P = 0.7776    2743.99, P = 0.7776
Z (freq), P = 0.7761

FIG. 69

Normal Probability Plot

| Ordem | Freq_Hz | Z(DISD1) | Studentized Residuals | Cook's Distance |
|---|---|---|---|---|
| 1 | 2673.99 | -0.26 | -0.5739 | 0.0355 |
| 2 | 2680.99 | -0.15 | -0.4186 | 0.0158 |
| 3 | 2687.99 | -0.12 | -0.3532 | 0.0094 |
| 4 | 2694.99 | -0.25 | -0.4578 | 0.0131 |
| 5 | 2701.99 | -0.17 | -0.3427 | 0.0061 |
| 6 | 2708.99 | -0.25 | -0.3951 | 0.0068 |
| 7 | 2715.99 | 4.35 | 4.3482 | 0.6941 |
| 8 | 2722.99 | -0.20 | -0.2854 | 0.0026 |
| 9 | 2729.99 | -0.12 | -0.1761 | 0.0009 |
| 10 | 2736.99 | -0.26 | -0.2907 | 0.0022 |
| 11 | 2743.99 | -0.18 | -0.1824 | 0.0008 |
| 12 | 2750.99 | -0.34 | -0.3178 | 0.0026 |
| 13 | 2757.99 | -0.24 | -0.1898 | 0.0010 |
| 14 | 2764.99 | -0.13 | -0.0511 | 0.0001 |
| 15 | 2771.99 | -0.19 | -0.0856 | 0.0003 |
| 16 | 2778.99 | -0.34 | -0.2133 | 0.0020 |
| 17 | 2785.99 | -0.23 | -0.0730 | 0.0003 |
| 18 | 2792.99 | -0.22 | -0.0353 | 0.0001 |
| 19 | 2799.99 | -0.11 | 0.1092 | 0.0009 |
| 20 | 2806.99 | -0.24 | -0.0004 | 0.0000 |
| 21 | 2813.99 | -0.35 | -0.0913 | 0.0009 |

FIG. 70

FIG. 71

FIG. 72

Coding System

Frequency interval (100 Hz)

Total patients (72 cases): HCC (34) vs Healthy controls (38)

Patients with wrong diagnosis by A.I.

FIG. 73

EP 4 438 107 A2

| | | AI | | | |
|---|---|---|---|---|---|
| | | HCC | Healthy | HCC | Healthy |
| **Real** | HCC | 34 | 0 | 47,2% | 0% |
| | Healthy | 6 | 32 | 8,3% | 44.4% |

N = 72
Sensitivity = 100.0%
Specificity = 84.2%
Accuracy = 91.6%

## FIG. 74

| Time [s] | Beat [1] | RRI [%] | HR [bpm] | sBP [mmHg] | dBP [mmHg] | mBP [mmHg] | SV [ml] | SI [ml/m²] | CO [l/min] | CI [l/(min*m²)] | TPR [dyne*s/cm*(*)] | TPRI [dyne*s*m²/cm^5] |
|---|---|---|---|---|---|---|---|---|---|---|---|---|
| 86.23 | 88 | 865.667 | 69.311 | 111.109 | 81.39 | 93.019 | 61.348 | 31.88 | 4.252 | 2.208 | 1693.647 | 3261.151 |
| 87.09 | 89 | 873.375 | 68.699 | 111.892 | 82.068 | 94.581 | 59.202 | 30.746 | 4.067 | 2.112 | 1801.402 | 3468.635 |
| 87.97 | 90 | 864.667 | 69.391 | 112.089 | 81.795 | 94.622 | 55.133 | 28.633 | 3.826 | 1.987 | 1915.923 | 3689.146 |
| 88.83 | 91 | 859.042 | 69.845 | 110.589 | 82.004 | 94.27 | 55.858 | 29.009 | 3.901 | 2.026 | 1871.508 | 3603.625 |
| 89.69 | 92 | 842.708 | 71.199 | 110.359 | 79.861 | 93.377 | 53.523 | 27.797 | 3.811 | 1.979 | 1897.299 | 3653.285 |
| 90.53 | 93 | 857.417 | 69.978 | 109.808 | 81.868 | 92.172 | 55.005 | 28.567 | 3.849 | 1.999 | 1853.334 | 3568.831 |
| 91.39 | 94 | 876.375 | 68.464 | 110.711 | 81.306 | 93.933 | 58.008 | 30.126 | 3.971 | 2.063 | 1831.749 | 3527.089 |
| 92.27 | 95 | 864 | 69.444 | 111.357 | 81.383 | 94.184 | 54.844 | 28.483 | 3.809 | 1.978 | 1915.33 | 3688.006 |
| 93.13 | 96 | 858.333 | 69.903 | 109.975 | 80.965 | 93.399 | 55.252 | 28.695 | 3.862 | 2.006 | 1872.449 | 3605.438 |
| 93.99 | 97 | 851.75 | 70.443 | 108.478 | 79.125 | 92.46 | 53.177 | 27.617 | 3.746 | 1.945 | 1910.548 | 3678.798 |
| 94.84 | 98 | 847 | 70.838 | 107.625 | 79.9 | 90.667 | 54.014 | 28.051 | 3.826 | 1.987 | 1832.962 | 3529.405 |
| 95.69 | 99 | 870.333 | 68.939 | 108.472 | 81.437 | 92.267 | 59.124 | 30.706 | 4.076 | 2.117 | 1752.081 | 3373.666 |
| 96.56 | 100 | 866.75 | 69.224 | 110.854 | 80.662 | 93.625 | 56.905 | 29.553 | 3.939 | 2.046 | 1840.477 | 3543.875 |
| 97.42 | 101 | 857.667 | 69.957 | 109.651 | 80.548 | 93.082 | 59.392 | 30.845 | 4.155 | 2.158 | 1734.467 | 3339.75 |
| 98.28 | 102 | 852.042 | 70.419 | 108.832 | 79.499 | 92.771 | 56.965 | 29.584 | 4.011 | 2.083 | 1790.329 | 3447.314 |
| 99.13 | 103 | 844.042 | 71.087 | 108.254 | 79.411 | 91.44 | 55.587 | 28.868 | 3.951 | 2.052 | 1790.527 | 3447.694 |
| 99.98 | 104 | 862.375 | 69.575 | 105.461 | 80.022 | 89.712 | 58.751 | 30.512 | 4.088 | 2.123 | 1697.054 | 3267.711 |
| 100.84 | 105 | 866 | 69.284 | 107.714 | 79.491 | 91.673 | 57.207 | 29.71 | 3.964 | 2.058 | 1789.787 | 3446.271 |
| 101.71 | 106 | 858 | 69.93 | 107.364 | 79.047 | 91.513 | 54.291 | 28.195 | 3.797 | 1.972 | 1865.124 | 3591.332 |
| 102.58 | 107 | 853.333 | 70.313 | 106 | 79.964 | 91.438 | 51.111 | 26.544 | 3.594 | 1.866 | 1968.712 | 3790.793 |
| 103.42 | 108 | 847.75 | 70.776 | 107.409 | 78.102 | 91.28 | 54.451 | 28.279 | 3.854 | 2.001 | 1832.565 | 3528.64 |
| 104.28 | 109 | 847.667 | 70.783 | 106.429 | 78.788 | 89.288 | 55.211 | 28.673 | 3.908 | 2.03 | 1766.407 | 3401.251 |
| 105.11 | 110 | 871.375 | 68.857 | 106.965 | 79.41 | 90.751 | 59.018 | 30.651 | 4.064 | 2.11 | 1727.465 | 3326.207 |

FIG. 75A

| Beat | RRISD1 | RRISD2 | RRISD1xSD2 | RRICCM | RRITPVa | Frequency | DI |
|---|---|---|---|---|---|---|---|
| 1 | 13,8789264 | 53,7068018 | 745,392747 | -6907,8288 | 57,3037533 | 4 | 8,55E+07 |
| 2 | 51,6567542 | 84,9515646 | 4388,32209 | -6935,7519 | 89,7056474 | 14 | 8,58E+07 |
| 3 | 27,8759158 | 40,6380658 | 1132,8233 | 11809,1202 | 43,0042157 | 24 | 1,46E+08 |
| 4 | 40,5454393 | 34,3807524 | 1393,98271 | -26582,556 | 36,3831032 | 34 | 3,29E+08 |
| 5 | 35,0480871 | 40,5817451 | 1422,31253 | -3496,0755 | 42,8842484 | 44 | 4,32E+07 |
| 6 | 42,5039809 | 29,4086701 | 1249,98555 | 990,223308 | 30,8444667 | 54 | 1,24E+07 |
| 7 | 45,1901092 | 36,0755196 | 1630,25667 | 3137,92983 | 38,4382246 | 64 | 3,90E+07 |
| 8 | 46,765476 | 33,6755254 | 1574,85198 | -22828,727 | 35,9011603 | 74 | 2,83E+08 |
| 9 | 39,8256199 | 32,1894788 | 1281,96595 | 47,5721464 | 34,3384147 | 84 | 1,78E+06 |
| 10 | 30,2713377 | 47,7661583 | 1445,94551 | 4519,26636 | 50,6761039 | 94 | 5,61E+07 |
| 11 | 35,186602 | 23,8825096 | 840,344361 | 2685,20061 | 25,1495254 | 104 | 3,33E+07 |
| 12 | 37,479101 | 52,3296708 | 1961,26902 | -18712,683 | 55,6259921 | 114 | 2,32E+08 |
| 13 | 53,6928877 | 42,3805183 | 2275,53241 | 4844,24643 | 44,7894832 | 124 | 6,02E+07 |
| 14 | 34,0572425 | 44,5420995 | 1516,98109 | -11106,596 | 47,2445818 | 134 | 1,37E+08 |
| 15 | 46,6213262 | 24,6540229 | 1149,40324 | 12824,1034 | 26,2531618 | 144 | 1,59E+08 |
| 16 | 32,445878 | 39,6020208 | 1284,92233 | -30406,893 | 41,9408539 | 154 | 3,77E+08 |
| 17 | 74,9106161 | 98,5793958 | 7384,64327 | -13693,11 | 104,217338 | 164 | 1,69E+08 |
| 18 | 44,7166188 | 40,2697042 | 1800,72501 | -11245,794 | 42,4305224 | 174 | 1,39E+08 |
| 19 | 49,3476794 | 49,535279 | 2444,45107 | -1585,6643 | 52,3216238 | 184 | 1,97E+07 |
| 20 | 34,0536542 | 34,4127831 | 1171,88101 | 4490,32455 | 36,3052018 | 194 | 5,57E+07 |
| 21 | 35,2361001 | 23,5102587 | 828,409828 | -11087,768 | 24,8419718 | 204 | 1,37E+08 |
| 22 | 37,1406638 | 54,5350674 | 2025,46861 | 9562,95019 | 57,4914902 | 214 | 1,19E+08 |
| 23 | 32,5973769 | 42,9152428 | 1398,92435 | -11403,819 | 45,5420335 | 224 | 1,41E+08 |

FIG. 75B

EP 4 438 107 A2

FIG. 76

FIG. 77

| Patient code | frequency code | SD1 | SD2 | CMSD1 | CMSD2 |
|---|---|---|---|---|---|
| 4 | 4 | | | | |
| 4 | 14 | | | | |
| 4 | 24 | | | | |
| 4 | 34 | | | | |
| 4 | 44 | | | | |
| 4 | 54 | | | | |
| 4 | 64 | | | | |
| 4 | 74 | | | | |
| 4 | 84 | | | | |
| 4 | 94 | | | | |
| 4 | 104 | | | | |
| 4 | 114 | | | | |
| 4 | 124 | | | | |
| 4 | 134 | | | | |
| 4 | 144 | | | | |
| 4 | 154 | | | | |
| 4 | 164 | | | | |
| 4 | 174 | | | | |
| 4 | 184 | | | | |
| 4 | 194 | | | | |

FIG. 78

FIG. 79A

FIG. 79B

|  | Heart beat | Inst. Var. |
| --- | --- | --- |
|  | 790 | 11,3745 |
|  | 791 | 9,166 |
|  | 792 | 1,542 |
|  | 793 | 1,646 |
|  | 794 | 7,4795 |
|  | 795 | 6,0415 |
| Calculation by next four heart beats | 796 | 171,8545 |
|  | 797 | 500,023 |
|  | 798 | 520,712 |
|  | 799 | 178,7105 |

Next beat instant calculation of hemodynamic variability

# FIG. 80A

EP 4 438 107 A2

Patient with HCC

SV

dBP

RRI

0    500    1000    1500    2000    2500    3000    3500 hb

600
500
400
300
200
100
0

19    22    hb    25
      Hz

500
400
300
200
100
0

346    349    hb    352
       Hz

FIG. 80B

FIG. 81

Patient #1

Patient #2

small intracellular vesicles

disrupted nucleous

Cellular membrane

Cellular membrane rupture

FIG. 82

## REFERENCES CITED IN THE DESCRIPTION

### Patent documents cited in the description

- US 62690709
- US 62688296
- US 62688282
- US 62572170
- US 20150005647
- US 4649935 A
- US 4765322 A
- US 84421417
- US 45045009
- US 8977365 B

### Non-patent literature cited in the description

- *European Heart Journal,* 1996, vol. 17, 354-381
- **ZIMMERMAN, J. W. et al.** Cancer cell proliferation is inhibited by specific modulation frequencies. *British Journal of Cancer,* 2012, vol. 106, 307-313
- **COSTA, F. P. et al.** Treatment of advanced hepatocellular carcinoma with very low levels of amplitude-modulated electromagnetic fields. *British Journal of Cancer,* 2011, vol. 105, 640-648
- **COSTA, F. P. et al.** *British journal of cancer,* 2011, vol. 105, 640-648
- **BARBAULT, A. et al.** *J. Exp. Clin. Cancer Res.,* 2009, vol. 28, 51
- **ZIMMERMAN, J. W. et al.** *British Journal of Cancer,* 2012, vol. 106, 307-313
- **COSTA, F. P. et al.** *British Journal of Cancer,* 2011, vol. 105, 640-648
- **ZIMMERMAN, J. W. et al.** *British journal of cancer,* 2012, vol. 106, 307-313